(19) **Europäisches Patentamt / European Patent Office / Office européen des brevets**

(11) **EP 4 008 005 B1**

(12) # EUROPEAN PATENT SPECIFICATION

(45) Date of publication and mention
of the grant of the patent:
**11.02.2026 Bulletin 2026/07**

(21) Application number: **20847239.9**

(22) Date of filing: **31.07.2020**

(51) International Patent Classification (IPC):
**G16B 20/00** (2019.01)     **G16B 30/00** (2019.01)
**G16B 40/20** (2019.01)     **G16H 50/30** (2018.01)

(52) Cooperative Patent Classification (CPC):
**G16H 50/30; G16B 20/00; G16B 30/00;
G16B 40/20**

(86) International application number:
**PCT/US2020/044637**

(87) International publication number:
**WO 2021/022225 (04.02.2021 Gazette 2021/05)**

(54) **METHODS AND SYSTEMS FOR DETECTING MICROSATELLITE INSTABILITY OF A CANCER IN A LIQUID BIOPSY ASSAY**

VERFAHREN UND SYSTEME ZUM NACHWEIS VON MIKROSATELLITENINSTABILITÄT VON KREBS IN EINEM FLÜSSIGEN BIOPSIETEST

PROCÉDÉS ET SYSTÈMES DE DÉTECTION D'INSTABILITÉ DE MICROSATELLITES D'UN CANCER DANS UN DOSAGE DE BIOPSIE LIQUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.08.2019 US 201962881845 P
06.11.2019 US 201962931600 P
18.02.2020 US 202062978130 P**

(43) Date of publication of application:
**08.06.2022 Bulletin 2022/23**

(73) Proprietor: **Tempus AI, Inc.
Chicago, IL 60654 (US)**

(72) Inventors:
- **LOZAC'HMEUR, Ariane
Chicago, IL 60610 (US)**
- **KHAN, Aly A.
Chicago, IL 60605 (US)**
- **PERERA, Jason
Chicago, IL 60622 (US)**
- **LAU, Denise
Santa Monica, CA 90404 (US)**
- **HAFEZ, Ashraf
Woodinville, WA 98077 (US)**

(74) Representative: **Grund, Martin et al
Grund Intellectual Property Group
Patentanwälte und Solicitor PartG mbB
Steinsdorfstraße 2
80538 München (DE)**

(56) References cited:
**WO-A2-2019/079624     US-A1- 2019 169 685**

- **ARTYOMENKO A ET AL: "Microsatellite instability detection with cell-free DNA nextgeneration sequencing", 24 October 2018 (2018-10-24), XP093074387, Retrieved from the Internet <URL:https://www.annalsofoncology.org/action/showPdf?pii=S0923-7534(19)49647-6> [retrieved on 20230816]**
- **ATKINS ALEXANDER ET AL: "Detection of Circulating Tumor DNA with a Single-Molecule Sequencing Analysis Validated for Targeted and Immunotherapy Selection", MOLECULAR DIAGNOSIS AND THERAPY, vol. 23, no. 4, 17 June 2019 (2019-06-17), NZ, pages 521 - 535, XP055780866, ISSN: 1177-1062, DOI: 10.1007/s40291-019-00406-0**

EP 4 008 005 B1

- DATABASE EMBASE [online] ELSEVIER SCIENCE PUBLISHERS, AMSTERDAM, NL; 6 November 2019 (2019-11-06), LOZAC'HMEUR A ET AL: "Microsatellite instability detection with cell-free DNA nextgeneration sequencing", XP002809978, Database accession no. EMB-629890407
- JOURNAL FOR IMMUNOTHERAPY OF CANCER 2019 BIOMED CENTRAL LTD. NLD, vol. 7, no. Supplement 1, 2019, ISSN: 2051-1426
- JASON WILLIS ET AL: "Validation of Microsatellite Instability Detection Using a Comprehensive Plasma-Based Genotyping Panel", CLINICAL CANCER RESEARCH : AN OFFICIAL JOURNAL OF THE AMERICAN ASSOCIATION FOR CANCER RESEARCH, 4 August 2019 (2019-08-04), United States, pages 7035 - 7045, XP055662308, Retrieved from the Internet <URL:https://clincancerres. aacrjournals.org/content/25/23/7035.full-text. pdf> DOI: 10.1158/1078-0432.CCR-19-1324

**Description**

**FIELD OF THE INVENTION**

The present disclosure relates generally to the use of cell-free DNA sequencing data to provide clinical support for personalized treatment of cancer. In particular, the present invention relates to computer-implemented methods for detecting a microsatellite instability status in a liquid biopsy sample from a test subject.

**BACKGROUND**

Precision oncology is the practice of tailoring cancer therapy to the unique genomic, epigenetic, and/or transcriptomic profile of an individual's cancer. Personalized cancer treatment builds upon conventional therapeutic regimens used to treat cancer based only on the gross classification of the cancer, e.g., treating all breast cancer patients with a first therapy and all lung cancer patients with a second therapy. This field was borne out of many observations that different patients diagnosed with the same type of cancer, e.g., breast cancer, responded very differently to common treatment regimens. Over time, researchers have identified genomic, epigenetic, and transcriptomic markers that improve predictions as to how an individual cancer will respond to a particular treatment modality.

There is growing evidence that cancer patients who receive therapy guided by their genetics have better outcomes. For example, studies have shown that targeted therapies result in significantly improved progression-free cancer survival. See, e.g., Radovich M. et al., Oncotarget, 7(35):56491-500 (2016). Similarly, reports from the IMPACT trial-a large (n = 1307) retrospective analysis of consecutive, prospectively molecularly profiled patients with advanced cancer who participated in a large, personalized medicine trial-indicate that patients receiving targeted therapies matched to their tumor biology had a response rate of 16.2%, as opposed to a response rate of 5.2% for patients receiving non-matched therapy. Tsimberidou AM et al., ASCO 2018, Abstract LBA2553 (2018).

In fact, therapy targeted to specific genomic alterations is already the standard of care in several tumor types, e.g., as suggested in the National Comprehensive Cancer Network (NCCN) guidelines for melanoma, colorectal cancer, and non-small cell lung cancer. In practice, implementation of these targeted therapies requires determining the status of the diagnostic marker in each eligible cancer patient. While this can be accomplished for the few, well known mutations associated with treatment recommendations in the NCCN guidelines using individual assays or small next generation sequencing (NGS) panels, the growing number of actionable genomic alterations and increasing complexity of diagnostic classifiers necessitates a more comprehensive evaluation of each patient's cancer genome, epigenome, and/or transcriptome.

For instance, some evidence suggests that use of combination therapies where each component is matched to an actionable genomic alteration holds the greatest potential for treating individual cancers. To this point, a retroactive study of cancer patients treated with one or more therapeutic regimens revealed that patients who received therapies matched to a higher percentage of their genomic alterations experienced a greater frequency of stable disease (e.g., a longer time to recurrence), longer time to treatment failure, and greater overall survival. Wheeler JJ et al., Cancer Res., 76:3690-701 (2016). Thus, comprehensive evaluation of each cancer patient's genome, epigenome, and/or transcriptome should maximize the benefits provided by precision oncology, by facilitating more fine-tuned combination therapies, use of novel off-label drug indications, and/or tissue agnostic immunotherapy. See, for example, Schwaederle M. et al., J Clin Oncol., 33(32):3817-25 (2015); Schwaederle M. et al., JAMA Oncol., 2(11):1452-59 (2016); and Wheler JJ et al., Cancer Res., 76(13):3690-701 (2016). Further, the use of comprehensive next generation sequencing analysis of cancer genomes facilitates better access and a larger patient pool for clinical trial enrollment. Coyne GO et al., Curr. Probl. Cancer, 41(3):182-93 (2017); and Markman M., Oncology, 31(3):158, 168.

One example of a genomic trait that has been linked to the efficacy of particular therapies is microsatellite instability. Microsatellite instability is a clinically actionable genomic indication for cancer immunotherapy. For instance, despite the generally poor performance of checkpoint inhibitor therapies across the general cancer patient population, some patients with microsatellite-instability-high (MSI-H) metastatic colorectal cancer respond favorably to checkpoint inhibitors, such as PD-1 and PD-L1 inhibitors. See, Oliveira AF et al., Front Oncol., 9:396 (2019). In microsatellite instability-high (MSI-H) tumors, defects in DNA mismatch repair (MMR) can cause a hypermutated phenotype where alterations accumulate in the repetitive microsatellite regions of DNA.

MSI detection is typically performed by subjecting tumor tissue ("solid biopsy") to clinical next-generation sequencing or specific assays, such as MMR IHC or MSI PCR See, Lozac'hmeur et al. Journal for Immunotherapy of Cancer, 7:Supplement 1 (2019). However, collection of solid tumor biopsies is an invasive procedure. Moreover, the sample material recovered from the biopsy is only representative of the portion of the individual tumor sampled. This is particularly problematic in cancer patients with multiple tumors and patients with cancers having high tumor mutational burdens, because different tumors-and even different sections of the same tumor-can have different genomic, epigenetic, and transcriptomic profiles. Circulating cell-free tumor DNA (cfDNA) testing ("liquid biopsy") is rapidly emerging as a less invasive method for cancer detection and monitoring disease progression. *See, e.g.,* Artyomenko et al., Annales of Oncology, 29(8): Supplement 8 (2018). However, liquid biopsy methodologies are hampered by the fact that DNA fragments from both cancerous and non-cancerous cells are present in blood plasma. In addition, the ratio of DNA from cancerous and non-cancerous cells varies from patient to patient, based on the tumor burden of the patient.

The use of large NGS genomic analysis is growing in order to address the need for more comprehensive characterization of an individual's cancer genome. See, for example, Fernandes GS et al., Clinics, 72(10):588-94. Recent studies indicate that of the patients for which large NGS genomic analysis is performed, 30-40% then receive clinical care based on the assay results, which is limited by at least the identification of actionable genomic alterations, the availability of medication for treatment of identified actionable genomic alterations, and the clinical condition of the subject. See, Ross JS et al., JAMA Oncol., 1(1):40-49 (2015); Ross JS et al., Arch. Pathol. Lab Med., 139:642-49 (2015); Hirshfield KM et al., Oncologist, 21(11):1315-25 (2016); and Groisberg R. et al., Oncotarget, 8:39254-67 (2017).

However, these large NGS genomic analyses are conventionally performed on solid tumor samples. For instance, each of the studies referenced in the paragraph above performed NGS analysis of FFPE tumor blocks from patients. Solid tissue biopsies remain the gold standard for diagnosis and identification of predictive biomarkers because they represent well-known and validated methodologies that provide a high degree of accuracy. Nevertheless, there are significant limitations to the use of solid tissue material for large NGS genomic analyses of cancers. For example, tumor biopsies are subject to sampling bias caused by spatial and/or temporal genetic heterogeneity, e.g., between two regions of a single tumor and/or between different cancerous tissues (such as between primary and metastatic tumor sites or between two different primary tumor sites). Such intertumor or intratumor heterogeneity can cause subclonal or emerging mutations to be overlooked when using localized tissue biopsies, with the potential for sampling bias to be exacerbated over time as subclonal populations further evolve and/or shift in predominance.

Additionally, the acquisition of solid tissue biopsies often requires invasive surgical procedures, *e.g.*, when the primary tumor site is located at an internal organ. These procedures can be expensive, time consuming, and carry a significant risk to the patient, *e.g.*, when the patient's health is poor and may not be able to tolerate invasive medical procedures and/or the tumor is located in a particularly sensitive or inoperable location, such as in the brain or heart. Further, the amount of tissue, if any, that can be procured depends on multiple factors, including the location of the tumor, the size of the tumor, the fragility of the patient, and the risk of comorbidities related to biopsies, such as bleeding and infections. For instance, recent studies report that tissue samples in a majority of advanced non-small cell lung cancer patients are limited to small biopsies, and cannot be obtained at all in up to 31% of patients. Ilie and Hofman, Transl. Lung Cancer Res., 5(4):420-23 (2016). Even when a tissue biopsy is obtained, the sample may be too scant for comprehensive testing.

Further, the method of tissue collection, preservation (e.g., formalin fixation), and/or storage of tissue biopsies can result in sample degradation and variable quality DNA. This, in turn, leads to inaccuracies in downstream assays and analysis, including next-generation sequencing (NGS) for the identification of biomarkers. Ilie and Hofman, Transl Lung Cancer Res., 5(4):420-23 (2016).

In addition, the invasive nature of the biopsy procedure, the time and cost associated with obtaining the sample, and the compromised state of cancer patients receiving therapy render repeat testing of cancerous tissues impracticable, if not impossible. As a result, solid tissue biopsy analysis is not amenable to many monitoring schemes that would benefit cancer patients, such as disease progression analysis, treatment efficacy evaluation, disease recurrence monitoring, and other techniques that require data from several time points. Comprehensive genetic cancer profiling using circulating tumor DNA has enabled the detection of NCCN guideline-recommended somatic alterations from a single, non-invasive blood draw. Based thereon, a single-molecule sequencing (SMSEQ) platform has been developed to address the challenges of reliably detecting somatic variants at low variant level. See, Atkins et al., Molecular Diagnosis and Therapy, 23(4):521-535 (2019).

Cell-free DNA (cfDNA) has been identified in various bodily fluids, *e.g.,* blood serum, plasma, urine, *etc.* Chan et al., Ann. Clin. Biochem., 40(Pt 2):122-30 (2003). This cfDNA originates from necrotic or apoptotic cells of all types, including germline cells, hematopoietic cells, and diseased (e.g., cancerous) cells. Advantageously, genomic alterations in cancerous tissues can be identified from cfDNA isolated from cancer patients. See, e.g., Stroun et al., Oncology, 46(5):318-22 (1989); Goessl et al., Cancer Res., 60(21):5941-45 (2000); and Frenel et al., Clin. Cancer Res. 21(20):4586-96 (2015). Thus, one approach to overcoming the problems presented by the use of solid tissue biopsies described above is to analyze cell-free nucleic acids (e.g., cfDNA) and/or nucleic acids in circulating tumor cells present in biological fluids, *e.g.,* via a liquid biopsy.

Specifically, liquid biopsies offer several advantages over conventional solid tissue biopsy analysis. For instance, because bodily fluids can be collected in a minimally-invasive or non-invasive fashion, sample collection is simpler, faster, safer, and less expensive than solid tumor biopsies. Such methods require only small amounts of sample (e.g., 10 mL or less of whole blood per biopsy) and reduce the discomfort and risk of complications experienced by patients during conventional tissue biopsies. In fact, liquid biological samples can be collected with limited or no assistance from medical professionals, and can be performed at almost any location. Further, liquid biological samples can be collected from any patient, regardless of the location of their cancer, their overall health, and any previous biopsy collection. This allows for analysis of the cancer genome of patients from which a solid tumor sample cannot be easily and/or safely obtained. In addition, because cell-free DNA in the bodily fluids arise from many different types of tissues in the patient, the genomic alterations present in the pool of cell-free DNA are representative of various different clonal sub-populations of the cancerous tissue of the subject, facilitating a more comprehensive analysis of the cancerous genome of the subject than is possible from one or more sections of a single solid tumor sample.

Liquid biopsies also enable serial genetic testing prior to cancer detection, during the early stages of cancer progression, throughout the course of treatment, and during remission, *e.g.,* to monitor for disease recurrence. The ability to conduct serial testing via non-invasive liquid biopsies throughout the course of disease could prove beneficial for many patients, e.g., through monitoring patient response to therapies, the emergence of new actionable genomic alterations, and/or drug-resistance alterations. These types of information allow medical professionals to more quickly tailor and update therapeutic regimens, e.g., facilitating more timely intervention in the case of disease progression. See, *e.g.,* Ilie and Hofman, Transl. Lung Cancer Res., 5(4):420-23 (2016).

Nevertheless, while liquid biopsies are promising tools for improving outcomes using precision oncology, there are significant challenges specific to the use of cell-free DNA for evaluation of a subject's cancer genome. For instance, there is a highly variable signal-to-noise ratio from one liquid biological sample to the next. This occurs because cfDNA originates from a variety of different cells in a subject, both healthy and diseased. Depending on the stage and type of cancer in any particular subject, the fraction of cfDNA fragments originating from cancerous cells (the "tumor fraction" or "ctDNA fraction" of the sample/subject) can range from almost 0% to well over 50%. Other factors, including tumor type and mutation profile, can also impact the amount of DNA released from cancerous tissues. For instance, cfDNA clearance through the liver and kidneys is affected by a variety of factors, including renal dysfunction or other tissue damaging factors (e.g., chemotherapy, surgery, and/or radiotherapy).

This, in turn, leads to problems detecting and/or validating cancer-specific genomic alterations in a liquid sample. This is particularly true during early stages of the disease-when cancer therapies have much higher success rates-because the tumor fraction in the patient is lowest at this point. Thus, early stage cancer patients can have ctDNA fractions below the limit of detection (LOD) for one or more informative genomic alterations, limiting clinical utility because of the risk of false negatives and/or providing an incomplete picture of the cancer genome of the patient. Further, because cancers, and even individual tumors, can be clonally diverse, actionable genomic alterations that arise in only a subset of clonal populations are diluted below the overall tumor fraction of the sample, further frustrating attempts to tailor combination therapies to the various actionable mutations in the patient's cancer genome. Consequently, most studies using liquid biopsy samples to date have focused on late stage patients for assay validation and research.

Another challenge associated with liquid biopsies is the accurate determination of tumor fraction in a sample. This difficulty arises from at least the heterogeneity of cancers and the increased frequency of large chromosomal duplications and deletions found in cancers. As a result, the frequency of genomic alterations from cancerous tissues varies from locus to locus based on at least (i) their prevalence in different subclonal populations of the subject's cancer, and (ii) their location within the genome, relative to large chromosomal copy number variations. The difficulty in accurately determining the tumor fraction of liquid biological samples affects accurate measurement of various cancer features shown to have diagnostic value for the analysis of solid tumor biopsies. These include allelic ratios, copy number variations, overall mutational burden, frequency of abnormal methylation patterns, *etc.,* all of which are correlated with the percentage of DNA fragments that arise from cancerous tissue, as opposed to healthy tissue.

Altogether, these factors result in highly variable concentrations of ctDNA-from patient to patient and possibly from locus to locus-that confound accurate measurement of disease indicators and actionable genomic alterations. Further, the quantity and quality of cfDNA obtained from liquid biopsy samples are highly dependent on the particular methodology for collecting the samples, storing the samples, sequencing the samples, and standardizing the sequencing data.

While validation studies of existing liquid biopsy assays have shown high sensitivity and specificity, few studies have corroborated results with orthogonal methods, or between particular testing platforms, e.g., different NGS technologies and/or targeted panel sequencing versus whole genome/exome sequence. Reports of liquid biopsy-based studies are limited by comparison to non-comprehensive tissue testing algorithms including Sanger sequencing, small NGS hotspot panels, polymerase chain reaction (PCR), and fluorescent in situ hybridization (FISH), which may not contain all NCCN guideline genes in their reportable range, thus suffering in comparison to a more comprehensive liquid biopsy assay.

The information disclosed in this Background section is only for enhancement of understanding of the general background of the invention and should not be taken as an acknowledgement or any form of suggestion that this information forms the prior art already known to a person skilled in the art.

# EP 4 008 005 B1

## SUMMARY

Given the above background, there is a need in the art for improved methods and systems for supporting clinical decisions in precision oncology using liquid biopsy assays. In particular, there is a need in the art for improved methods and systems for classifying the microsatellite stability of a cancer, in order to better inform treatment of the patient. The present disclosure solves these and other needs in the art by providing improvements in classifying the microsatellite stability of a cancer using DNA sequencing results of a liquid biopsy. Advantageously, the methods disclosed herein are compatible with other cancer genotyping assays, allowing a spectrum of different information to be captured from a single liquid biopsy sequencing. In some embodiments, the methods disclosed herein are more effective than other liquid biopsy-based microsatellite instability (MSI) assays because of the improved statistical and classification methodologies described herein.

Microsatellite instability is a clinically actionable genomic indication for cancer immunotherapy. In microsatellite instability-high (MSI-H) tumors, defects in DNA mismatch repair (MMR) can cause a hypermutated phenotype where alterations accumulate in the repetitive microsatellite regions of DNA. MSI detection is conventionally performed by subjecting tumor tissue ("solid biopsy") to clinical next-generation sequencing or specific assays, such as MMR IHC or MSI PCR. Circulating cell-free tumor DNA (cfDNA) testing ("liquid biopsy") is rapidly emerging as a less invasive method for cancer detection and monitoring disease progression. Disclosed herein are methods and systems for detecting MSI using a cfDNA MSI detection assay with high specificity.

The invention is defined in the appended claims.

The invention relates to a computer-implemented method of determining a microsatellite instability (MSI) status in a liquid biopsy sample obtained from a test subject, the method comprising:

at a computer system having one or more processors, and memory storing one or more programs for execution by the one or more processors:

(A) obtaining, for each respective DNA fragment in a plurality of DNA fragments from the liquid biopsy sample, a corresponding nucleotide sequence that has been aligned to a reference genome for the species of the test subject, thereby obtaining a plurality of aligned nucleotide sequences;

(B) using the plurality of aligned nucleotide sequences to determine, for each respective microsatellite locus in a plurality of predetermined microsatellite loci, wherein each respective microsatellite locus in the plurality of microsatellite loci has a nucleic acid sequence that includes a variable number of repeat units, a first corresponding metric value for a first metric, a second corresponding metric value for a second metric, and a third corresponding metric value for a third metric, wherein the first corresponding metric value, the second corresponding metric value and the third corresponding metric value is determined, at least in part, by a distribution of the number of repeat units in each aligned nucleotide sequence, in the plurality of nucleotide sequences, encompassing the respective microsatellite locus, wherein

the first metric is a percentage score and the corresponding first metric value for the percentage score is a percentage of aligned nucleotide sequences, in the plurality of aligned nucleotides, encompassing the respective microsatellite locus that have a number of repeat units that is less than a first corresponding reference number of repeat units representative of a corresponding stable construct at the respective microsatellite locus,

the second metric is a lower mean score and the corresponding metric value for the lower mean score is determined by the corresponding mean number of repeat units in aligned nucleotide sequences in the plurality of aligned nucleotides that both (i) encompass the respective microsatellite locus and (ii) have a number of repeat units that is less than a second corresponding reference number of repeat units representative of a corresponding stable construct at the respective microsatellite locus, and

the third metric is a likelihood score and the corresponding metric value for the likelihood score is a mean of a distribution of probabilities, wherein each respective probability in the distribution of probabilities is a probability that a corresponding aligned nucleotide sequence in a respective subset of the set of aligned nucleotide sequences encompassing the respective microsatellite locus is a member of a distribution of aligned nucleotide sequences encompassing a corresponding stable construct at the respective microsatellite locus, wherein the respective subset of aligned nucleotide sequences consists of the aligned nucleotide sequences that (i) encompass the respective microsatellite locus and (ii) have a number of repeat units within a threshold percentile of the smallest number of repeat units observed in the respective subset of the set of aligned nucleotide sequences; and

(C) classifying the MSI status of the test subject by inputting, for each respective microsatellite locus in the plurality of predetermined microsatellite loci, at least the first corresponding metric value, the second corresponding metric value and the third corresponding metric value into a classifier that distinguishes between stable and unstable microsatellite loci, thereby determining the MSI status of the test subject.

## BRIEF DESCRIPTION OF THE DRAWINGS

Figures 1A, 1B, 1C, and 1D collectively illustrate a block diagram of an example computing device for determining the microsatellite stability status of a cancer.

Figure 2A illustrates an example workflow for generating a clinical report based on information generated from analysis of one or more patient specimens.

Figure 2B illustrates an example of a distributed diagnostic environment for collecting and evaluating patient data for the purpose of precision oncology.

Figure 3 provides an example flow chart of processes and features for liquid biopsy sample collection and analysis for use in precision oncology.

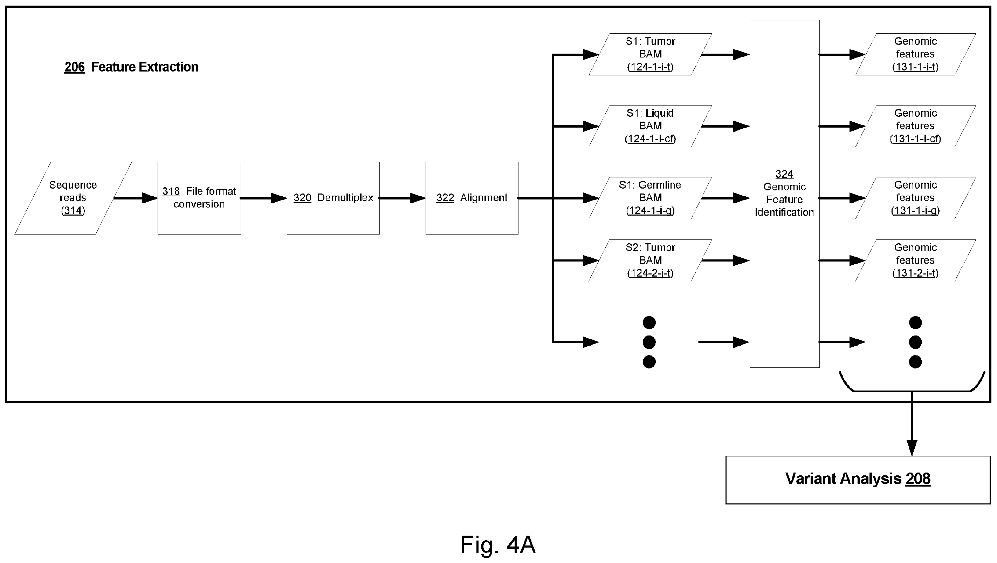

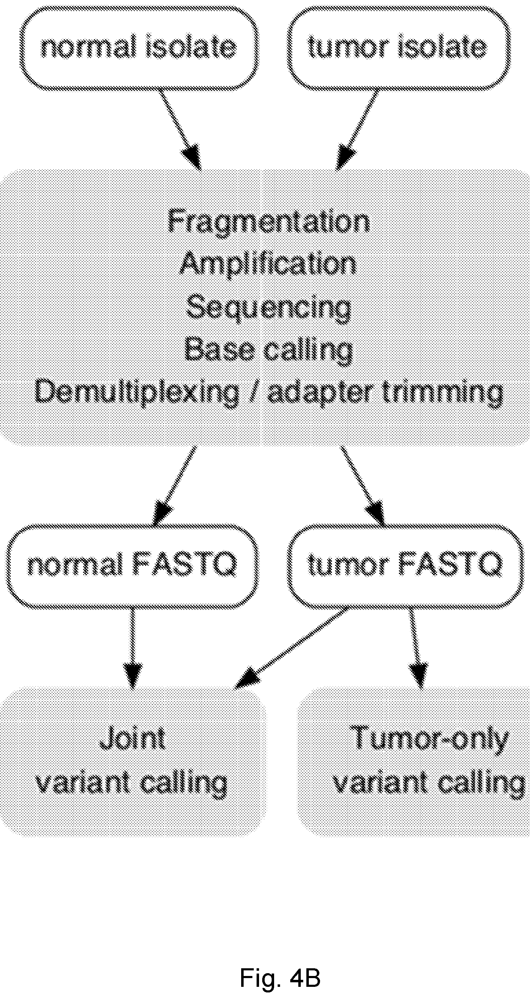

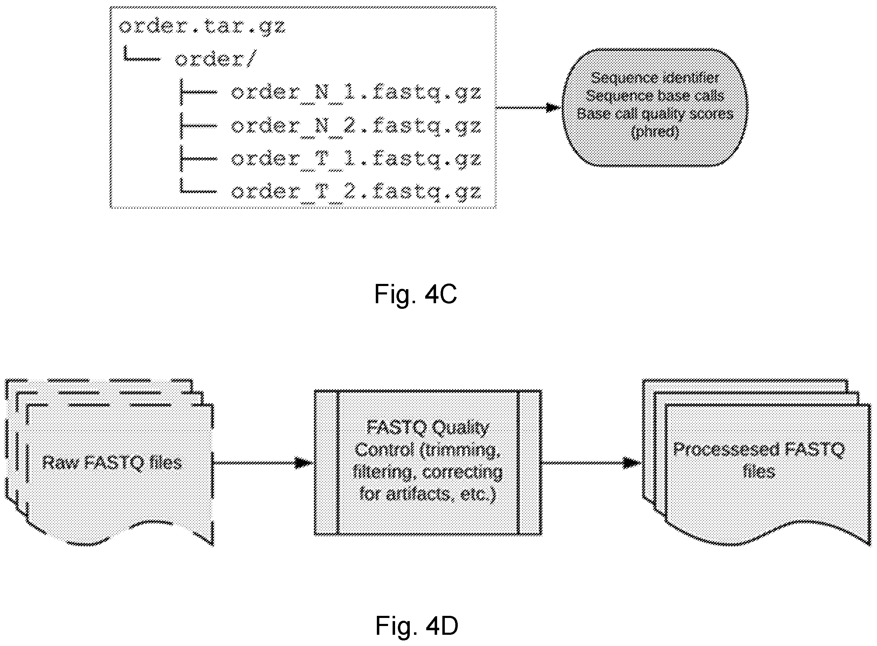

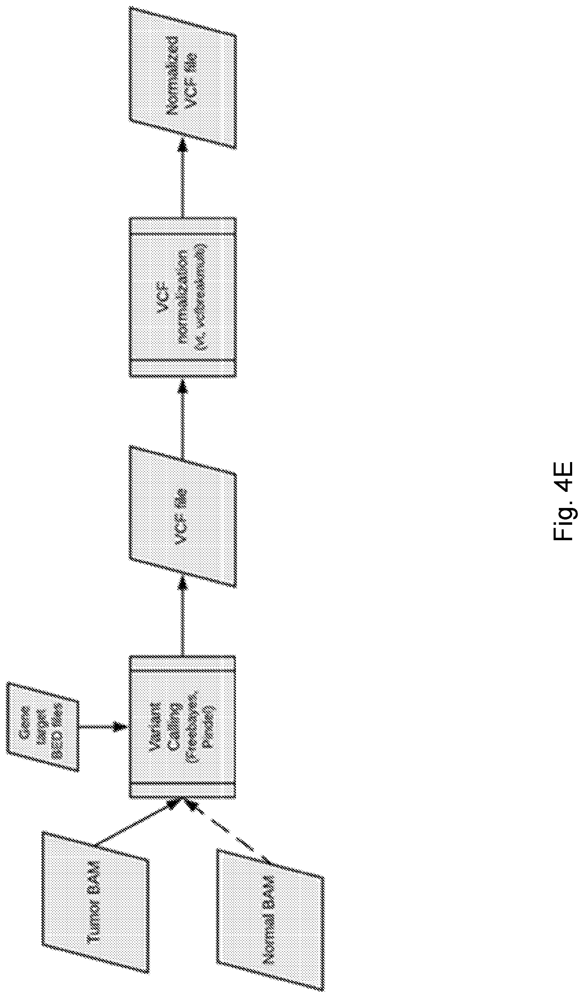

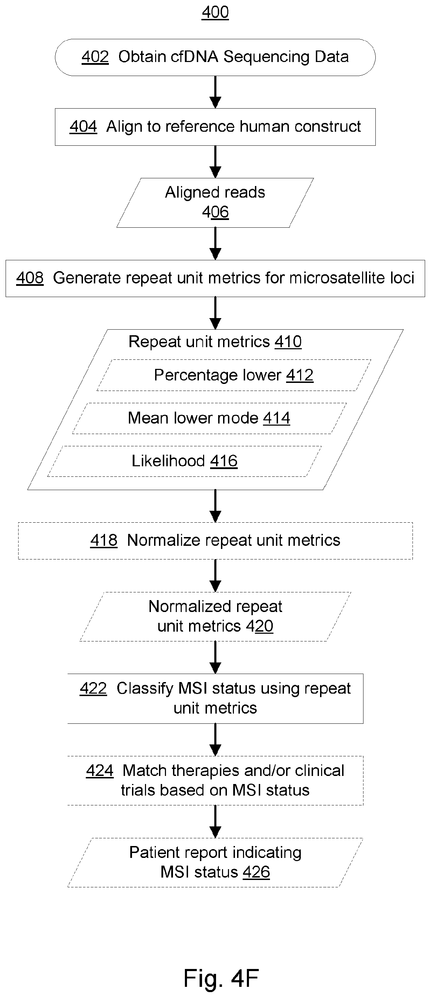

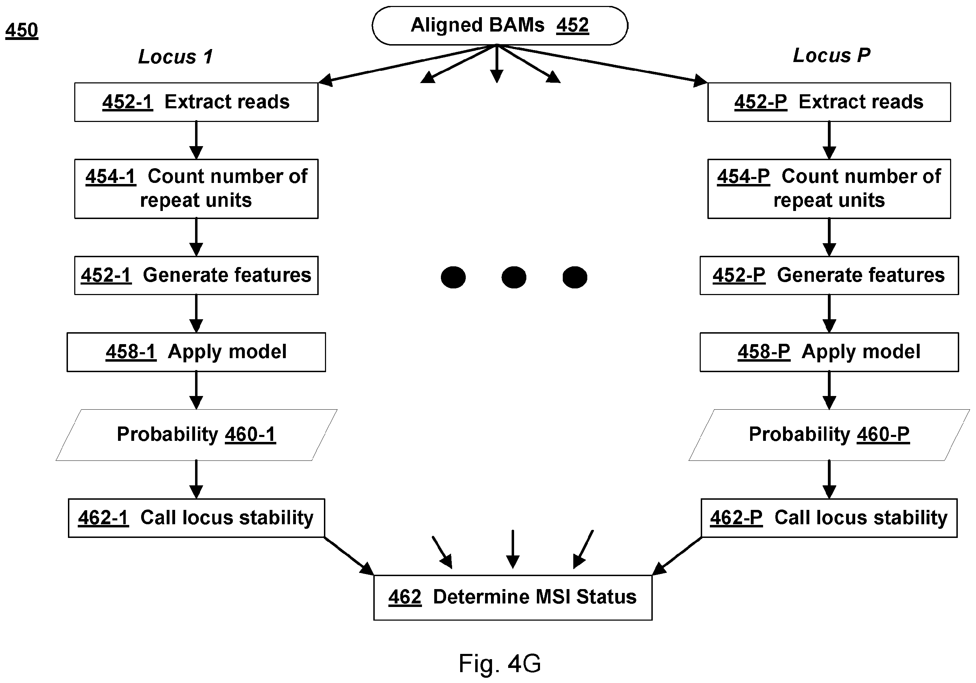

Figures 4A, 4B, 4C, 4D, 4E, 4F, and 4G collectively illustrate an example bioinformatics pipeline for precision oncology. Figure 4A provides an overview flow chart of processes and features in a bioinformatics pipeline. Figure 4B provides an overview of a bioinformatics pipeline executed with either a liquid biopsy sample alone or a liquid biopsy sample and a matched normal sample. Figure 4C illustrates that paired-end reads from tumor and normal isolates are zipped and stored separately under the same order identifier. Figure 4D illustrates quality correction for FASTQ files. Figure 4E illustrates processes for obtaining tumor and normal BAM alignment files. Figure 4F provides a flow chart of a method 400 for determining the microsatellite stability status of a cancer, in which dashed boxes represent optional portions of the method. Figure 4G provides a flow chart of a method 450 for determining the microsatellite stability status of a cancer, in which dashed boxes represent optional portions of the method.

Figures 5A, 5B, and 5C collectively illustrate an example distribution of repeat units in sequence reads from an MSI-H sample (5A; red) and an MSS sample (5A; blue), and an illustration of how the percent lower metric is calculated for a stable microsatellite locus (5B) and an unstable microsatellite locus (5V).

Figures 6A and 6B illustrate density estimates for reference distributions from a microsatellite locus with a single stable number of repeat units (6A) and a microsatellite locus with multiple stable numbers of repeat units (6B).

Figures 7A, 7B, and 7C collectively illustrate performance metrics for a locus classifier trained against a percent lower metric, a mean lower mode metric, and a likelihood metric. Figure 7A illustrates a receiver operating characteristic (ROC) for the locus classifier evaluated with a 10-fold cross validation. Figure 7B illustrates a prediction distribution for the microsatellite loci. Figure 7C illustrates a prediction scatter plot for the microsatellite loci.

Figures 8A, 8B, and 8C collectively illustrate performance metrics for a locus classifier trained against a percent lower metric, a mean lower mode metric, and a likelihood metric, showing the percentage of unstable loci.

Figures 9A and 9B collectively illustrate performance metrics for a k-nearest neighbor algorithm trained to distinguish MSI-H cancers from MSS cancers using individual locus statuses generated by a locus classifier trained against a percent lower metric, a mean lower mode metric, and a likelihood metric.

Figure 10 is a table showing the number of samples in the training and validation data sets, divided according to the MSI status and cancer type associated with each sample.

Figure 11 illustrates a block diagram of an example of a computing device for determining the microsatellite stability status of a cancer.

Figure 12 illustrates an example distribution of repeat units in sequence reads from an MSI-H locus (red bars) and an MSS locus (blue bars).

Figure 13 illustrates the probability density for an unstable microsatellite locus and how the percent lower metric is calculated.

Figure 14 illustrates a k Nearest Neighbors model displayed as a principal component analysis of three metrics (percent lower metric, mean lower mode metric, and likelihood metric) associated with each locus.

Figures 15A and 15B collectively illustrate performance metrics for a locus classifier trained against a percent lower metric, a mean lower mode metric, and a likelihood metric. Figure 15A illustrates a prediction distribution for the microsatellite loci. Figure 15B illustrates the number of MSI-H (red) and MSS (blue) samples having a given percentage of loci predicted to be unstable.

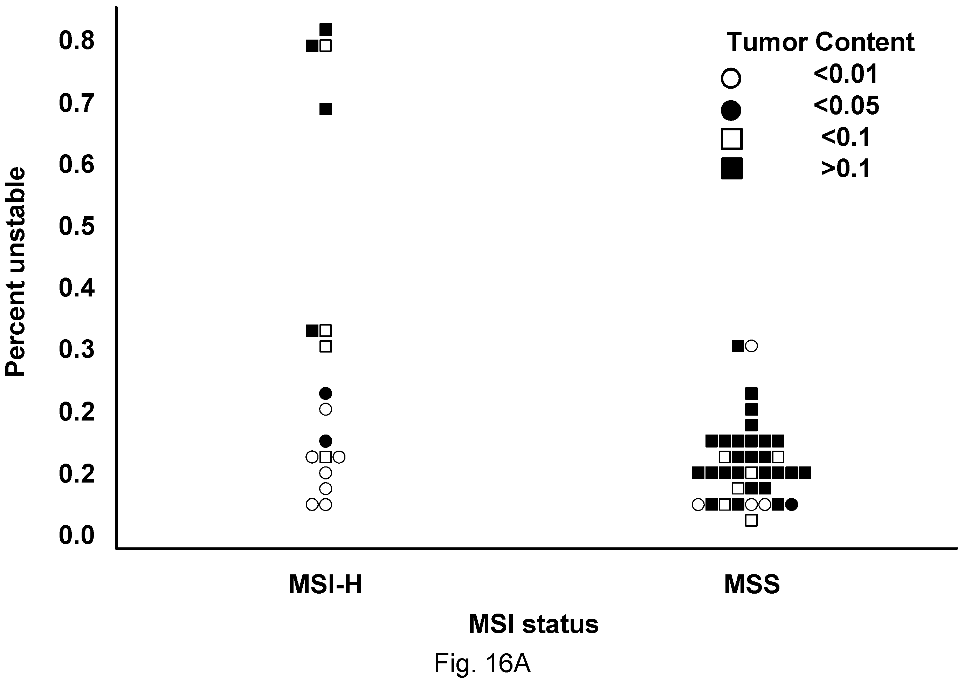

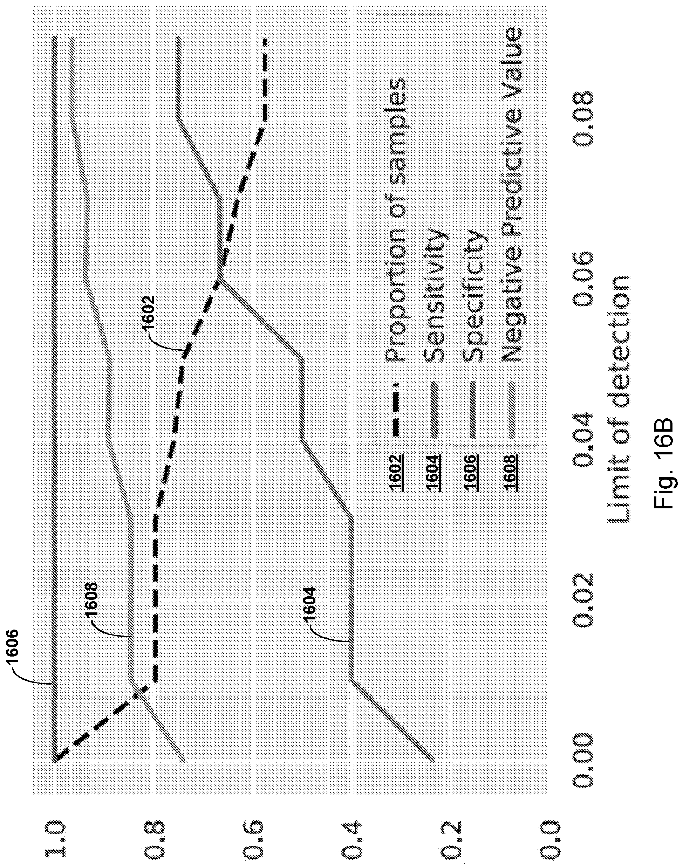

Figures 16A and 16B collectively illustrate the tumor content limit of detection achieved by a locus classifier. Figure 16A illustrates the relationship among tumor content, the orthogonal MSI status of a sample, and the predicted MSI status provided by the locus classifier. Figure 16B illustrates the relationships between the tumor content limit of detection and each of the following performance metrics of a locus classifier: sensitivity, specificity, and negative predictive value, as well as the proportion of samples in an example cohort having a tumor content higher than a given limit of detection.

Figure 17 compares MSI status predictions provided by a locus classifier to orthogonal MSI status results provided by a solid tumor test for several loci in three independent samples.

Figure 18 illustrates aligned sequence reads from a microsatellite locus, containing varying numbers of repeated sequence units, as shown in underlined font.

Figures 19A and 19B collectively provide a flow chart of processes and features for determining the microsatellite stability status of a cancer from a liquid biopsy sample, in which dashed boxes represent optional portions of the method.

Figures 20A and 20B collectively provide a flow chart of processes and features for determining the microsatellite stability status of a cancer from a liquid biopsy sample, in which dashed boxes represent optional portions of the method.

Figures 21A, 21B, and 21C collectively provide a flow chart of processes and features for determining the microsatellite stability status of a cancer from a liquid biopsy sample, in which dashed boxes represent optional portions of the method.

Figures 22A, 22B, and 22C collectively provide a flow chart of processes and features for determining the microsatellite stability status of a cancer from a liquid biopsy sample, in which dashed boxes represent optional portions of the method.

Figures 23A and 23B collectively provide a flow chart of processes and features for determining the microsatellite stability status of a cancer from a liquid biopsy sample, in which dashed boxes represent optional portions of the method.

Figures 24A and 24B collectively provide a flow chart of processes and features for determining the microsatellite stability status of a cancer from a liquid biopsy sample, in which dashed boxes represent optional portions of the method.

Figures 25A, 25B, and 25C collectively provide a flow chart of processes and features for determining the microsatellite stability status of a cancer from a liquid biopsy sample, in which dashed boxes represent optional portions of the method.

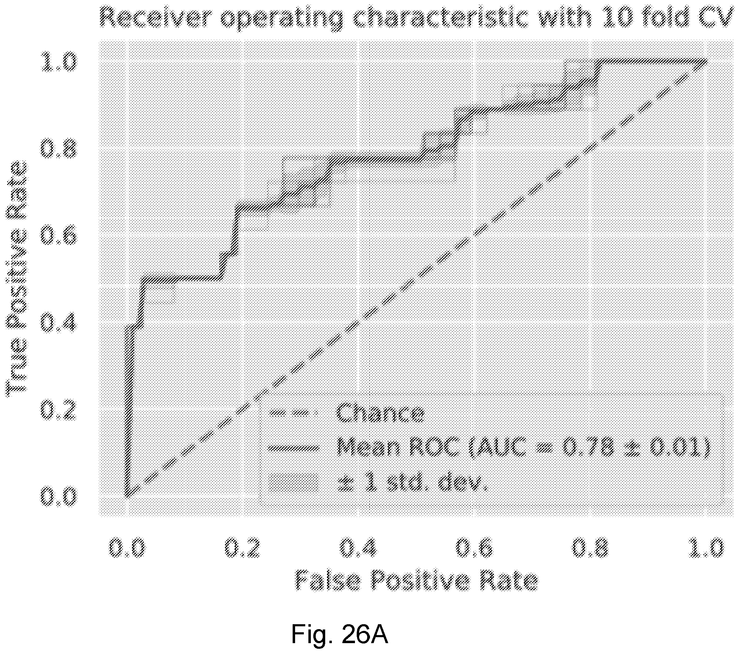

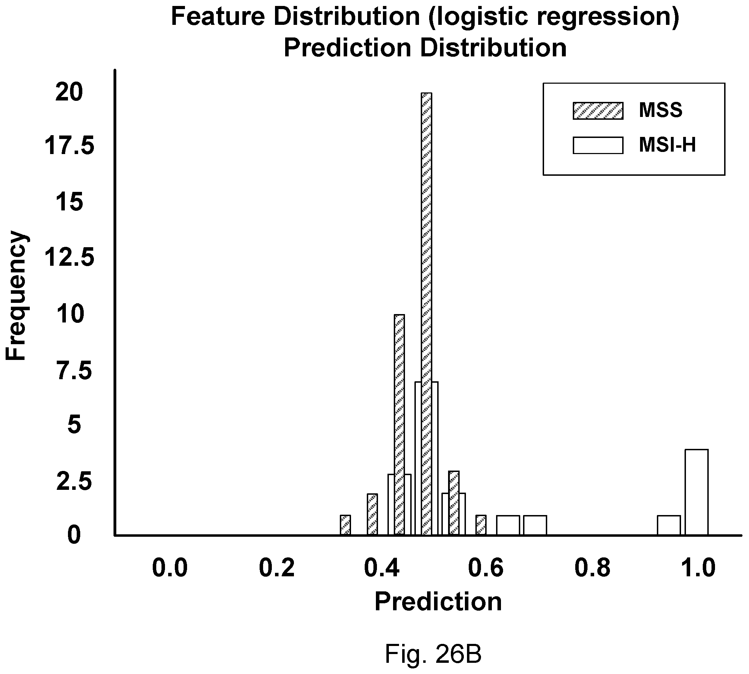

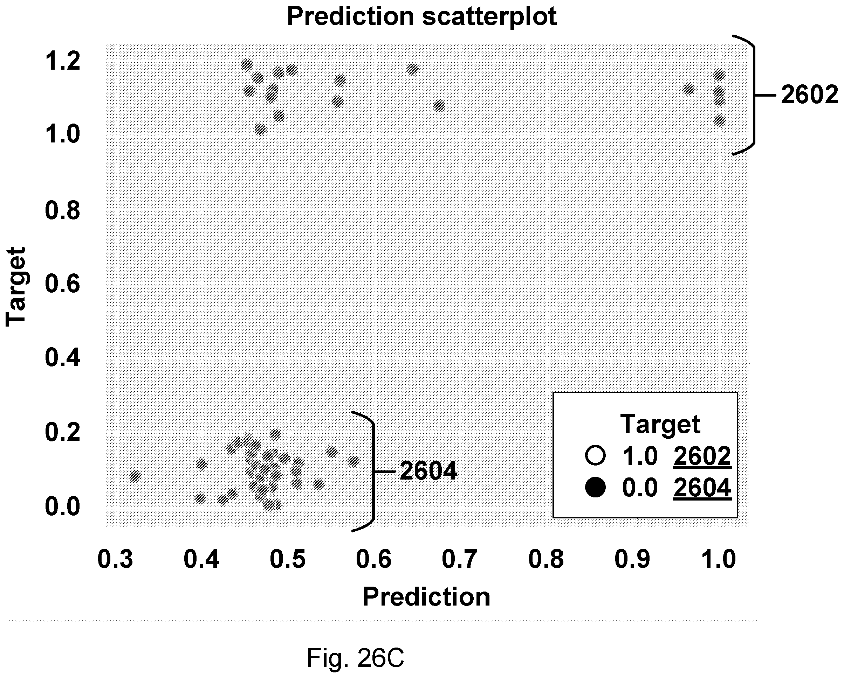

Figures 26A, 26B, and 26C collectively illustrate performance metrics for a logistic regression model trained to distinguish MSI-H cancers from MSS cancers using the 15 highest metric values, from 39 microsatellite loci, for each of a percent lower metric, a mean lower mode metric, and a likelihood metric.

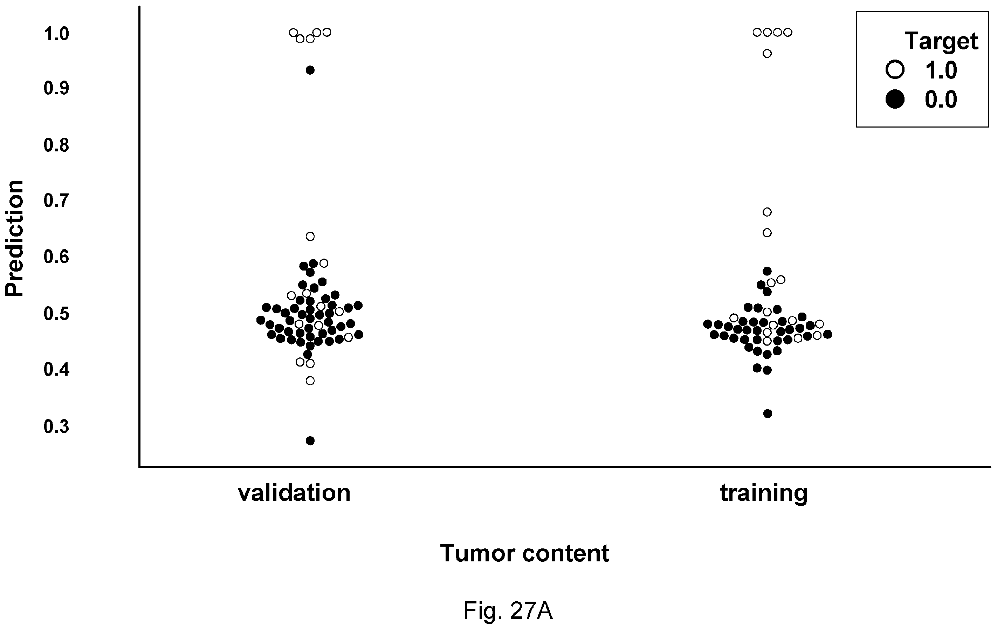

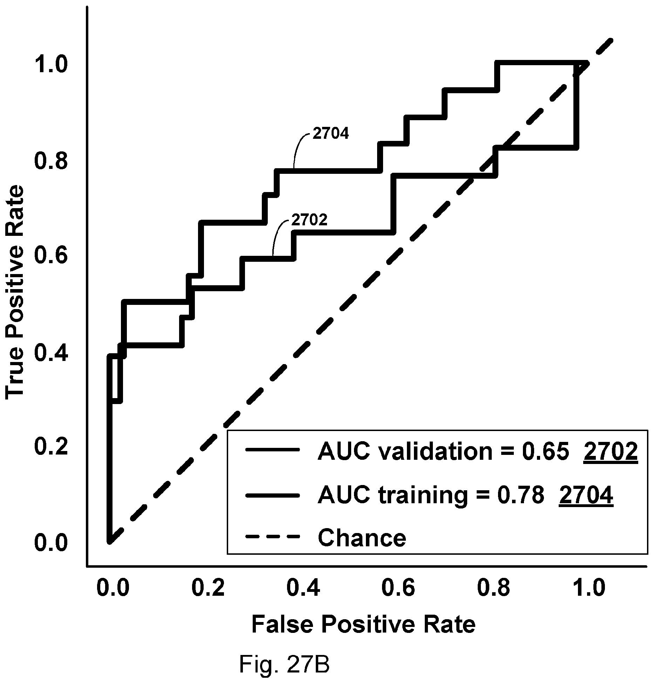

Figures 27A and 27B collectively illustrate performance metrics for a logistic regression model trained to distinguish MSI-H cancers from MSS cancers using the 15 highest metric values, from 39 microsatellite loci, for each of a percent lower metric, a mean lower mode metric, and a likelihood metric.

Figure 28A illustrates a method for determining a mode-specific, e.g., mode = 16, 17, or 18, repeat unit density estimate for a microsatellite locus using a set of reference samples of microsatellite stable tissue.

Figure 28B shows an example distribution of repeat unit lengths at a microsatellite locus and the mode of the distribution.

Figure 28C illustrates mode-specific kernel density estimates for the distribution of repeat unit lengths from a set of reference samples of microsatellite stable tissues, determined as outlined in Figure 28A.

Figures 29A, 29B, and 29C collectively provide a flow chart of a method 2900 for determining the microsatellite stability status of a cancer, in which dashed boxes represent optional portions of the method.

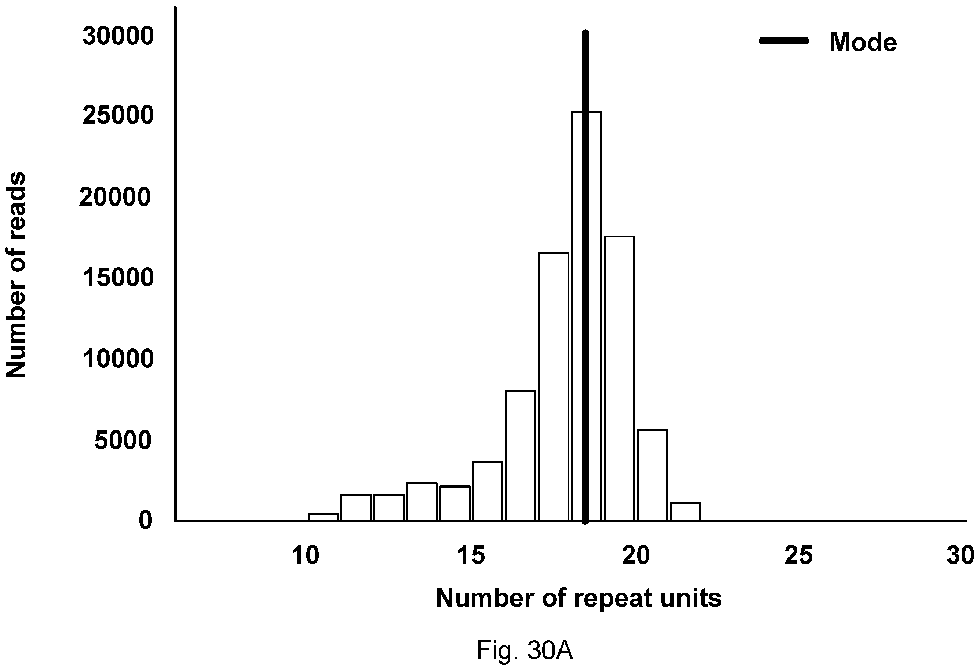

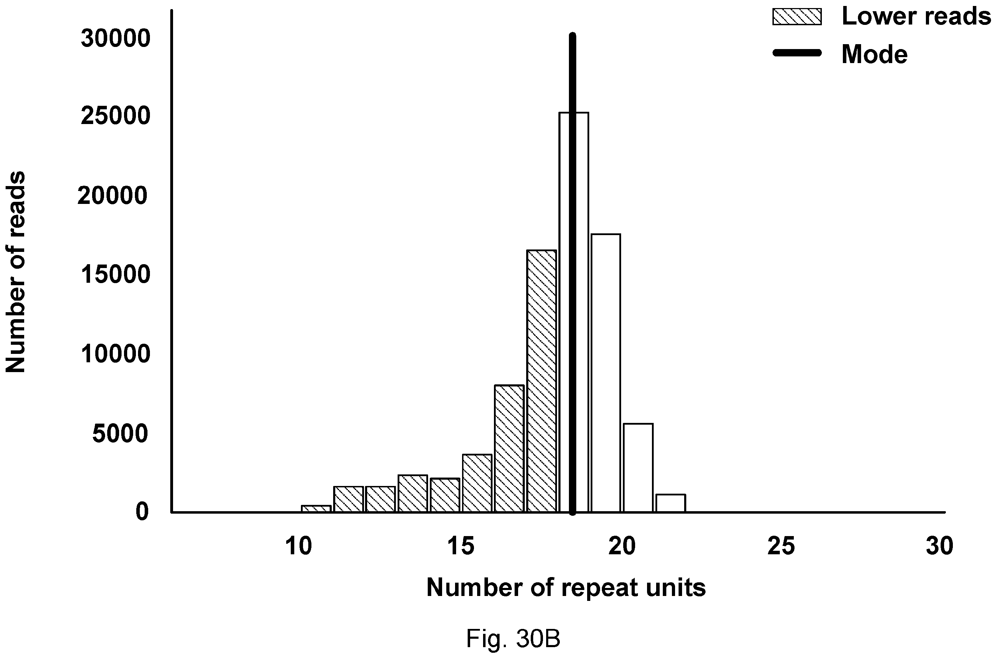

Figures 30A and 30B collectively show an example distribution of repeat unit lengths at a microsatellite locus, the mode of the distribution, and those members of the distribution used to determine a lower mean metric.

Like reference numerals refer to corresponding parts throughout the several views of the drawings.

## DETAILED DESCRIPTION

### Introduction

Conventional liquid biopsy assays do not provide accurate determination of the microsatellite stability status of a cancer. Advantageously, the present disclosure provides methods and systems that do provide accurate determination of the microsatellite stability status of a cancer from a liquid biopsy sample.

For example, the methods and systems described herein use of one or more novel metrics of microsatellite stability, e.g., a percentage score, a lower mean score, a likelihood score, and/or a standard deviation, to make a determination of the stability of each of a plurality of microsatellite loci in a cancer. When considered together, these intermediate determinations allow for accurate prediction of the overall microsatellite stability of a cancer. The methods and systems described herein improve the accuracy of a liquid biopsy-based classifier for the microsatellite stability of a cancer by training the classifier against training cohorts that include solid tumor biopsy tissue from microsatellite-stable (MSS) cancers and/or normal tissue samples. The methods and systems described herein improve the accuracy of a classifier for the microsatellite stability of a cancer by using a reference value, representative of a stable microsatellite locus, that is derived from the test patient, allowing for more accurate identification of unstable microsatellite loci. For example, as described in Examples 2 and 4, accurate k-nearest neighbor (Example 2) and logistic regression (Example 4) classifiers have been developed using the microsatellite stability metrics and training methods described herein.

The identification of actionable genomic alterations in a patient's cancer genome is a difficult and computationally demanding problem. For instance, the determination of various prognostic metrics useful for precision oncology, such as variant allelic ratio, copy number variation, tumor mutational burden, microsatellite instability status, etc., requires analysis of hundreds of millions to billions, of sequenced nucleic acid bases. An example of a typical bioinformatics pipeline established for this purpose includes at least five stages of analysis: assessment of the quality of raw next generation sequencing data, generation of collapsed nucleic acid fragment sequences and alignment of such sequences to a reference genome, detection of structural variants in the aligned sequence data, annotation of identified variants, and visualization of the data. See, Wadapurkar and Vyas, Informatics in Medicine Unlocked, 11:75-82 (2018). Each one of these procedures is computationally taxing in its own right.

For instance, the overall temporal and spatial computation complexity of simple global and local pairwise sequence alignment algorithms are quadratic in nature (e.g., second order problems), that increase rapidly as a function of the size of the nucleic acid sequences (n and m) being compared. Specifically, the temporal and spatial complexities of these sequence alignment algorithms can be estimated as $O(mn)$, where $O$ is the upper bound on the asymptotic growth rate of the algorithm, $n$ is the number of bases in the first nucleic acid sequence, and $m$ is the number of bases in the second nucleic acid sequence. See, Baichoo and Ouzounis, BioSystems, 156-157:72-85 (2017). Given that the human genome contains more than 3 billion bases, these alignment algorithms are extremely computationally taxing, especially when used to analyze next generation sequencing (NGS) data, which can generate more than 3 billion sequence reads per reaction.

This is particularly true when performed in the context of a liquid biopsy assay, because liquid biological samples contain a complex mixture of short DNA fragments originating from many different germline (e.g., healthy) and diseased (e.g., cancerous) tissues. Thus, the cellular origins of the sequence reads are unknown, and the sequence signals originating from cancerous cells, which may constitute multiple sub-clonal populations, must be computationally deconvoluted from signals originating from germline and hematopoietic origins, in order to provide relevant information about the subject's cancer. Thus, in addition to the computationally taxing processes required to align sequence reads to a human genome, there is a computation problem of determining whether a particular abnormal signal, *e.g.,* one or more sequence reads corresponding to a genomic alteration, (i) is not an artifact, and (ii) originated from a cancerous source in the subject. This is increasingly difficult during the early stages of cancer-when treatment is presumably most effective-when only small amounts of ctDNA are diluted by germline and hematopoietic DNA.

Advantageously, the present disclosure provides various systems and methods that improve the computational elucidation of actionable genomic alterations from a liquid biopsy sample of a cancer patient. Advantageously, the methods and systems described herein improve upon the accuracy of microsatellite stability determination from a liquid biopsy assay, which is inherently a computational problem. The methods and systems described herein also improve precision oncology methodologies for assigning and/or administering personalized treatment because of the improved accuracy of microsatellite stability classification the present disclosure facilitates.

**Definitions**

As used herein, the term "subject" refers to any living or non-living organism including, but not limited to, a human (*e.g.*, a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine (*e.g.,* cattle), equine (*e.g.,* horse), caprine and ovine (*e.g.,* sheep, goat), swine (*e.g.,* pig), camelid (*e.g.,* camel, llama, alpaca), monkey, ape (*e.g.,* gorilla, chimpanzee), ursid (*e.g.,* bear), poultry, dog, cat, mouse, rat, fish, dolphin, whale and shark. In some embodiments, a subject is a male or female of any age (*e.g.,* a man, a woman, or a child).

As used herein, the terms "control," "control sample," "reference," "reference sample," "normal," and "normal sample" describe a sample from a non-diseased tissue. In some embodiments, such a sample is from a subject that does not have a particular condition (e.g., cancer). In other embodiments, such a sample is an internal control from a subject, e.g., who may or may not have the particular disease (e.g., cancer), but is from a healthy tissue of the subject. For example, where a liquid or solid tumor sample is obtained from a subject with cancer, an internal control sample may be obtained from a healthy tissue of the subject, e.g., a white blood cell sample from a subject without a blood cancer or a solid germline tissue sample from the subject. Accordingly, a reference sample can be obtained from the subject or from a database, *e.g.,* from a second subject who does not have the particular disease (*e.g.,* cancer).

As used herein the term "cancer," "cancerous tissue," or "tumor" refers to an abnormal mass of tissue in which the growth of the mass surpasses, and is not coordinated with, the growth of normal tissue, including both solid masses (e.g., as in a solid tumor) or fluid masses (e.g., as in a hematological cancer). A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be a poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein.

Non-limiting examples of cancer types include ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural cancer, neuroblastoma, basal cell carcinoma, brain cancer, breast cancer, non-clear cell renal cell carcinoma, glioblastoma, glioma, kidney cancer, gastrointestinal stromal tumor, medulloblastoma, bladder cancer, gastric cancer, bone cancer, non-small cell lung cancer, thymoma, prostate cancer, clear cell renal cell carcinoma, skin cancer, thyroid cancer, sarcoma, testicular cancer, head and neck cancer (e.g., head and neck squamous cell carcinoma), meningioma, peritoneal cancer, endometrial cancer, pancreatic cancer, mesothelioma, esophageal cancer, small cell lung cancer, Her2 negative breast cancer, ovarian serous carcinoma, HR+ breast cancer, uterine serous carcinoma, uterine corpus endometrial carcinoma, gastroesophageal junction adenocarcinoma, gallbladder cancer, chordoma, and papillary renal cell carcinoma.

As used herein, the terms "cancer state" or "cancer condition" refer to a characteristic of a cancer patient's condition, *e.g.,* a diagnostic status, a type of cancer, a location of cancer, a primary origin of a cancer, a cancer stage, a cancer prognosis, and/or one or more additional characteristics of a cancer (e.g., tumor characteristics such as morphology, heterogeneity, size, *etc.*). In some embodiments, one or more additional personal characteristics of the subject are used further describe the cancer state or cancer condition of the subject, e.g., age, gender, weight, race, personal habits (*e.g.,* smoking, drinking, diet), other pertinent medical conditions (*e.g.,* high blood pressure, dry skin, other diseases), current medications, allergies, pertinent medical history, current side effects of cancer treatments and other medications, *etc.*

As used herein, the term "liquid biopsy" sample refers to any sample taken from a subject, which can reflect a biological state associated with the subject, and that includes cell-free DNA. Examples of liquid biopsy samples include, but are not limited to, blood, whole blood, plasma, serum, urine, cerebrospinal fluid, fecal, saliva, sweat, tears, pleural fluid, pericardial fluid, or peritoneal fluid of the subject. A liquid biopsy sample can include any tissue or material derived from a living or dead subject. A liquid biopsy sample can be a cell-free sample. A liquid biopsy sample can comprise a nucleic acid *(e.g.,* DNA or RNA) or a fragment thereof. The term "nucleic acid" can refer to deoxyribonucleic acid (DNA), ribonucleic acid (RNA) or any hybrid or fragment thereof.

As used herein, the term "cell-free DNA" and "cfDNA" interchangeably refer to DNA fragments that circulate in a subject's body (e.g., bloodstream) and originate from one or more healthy cells and/or from one or more cancer cells. These DNA molecules are found outside cells, in bodily fluids such as blood, whole blood, plasma, serum, urine, cerebrospinal fluid, fecal, saliva, sweat, sweat, tears, pleural fluid, pericardial fluid, or peritoneal fluid of a subject, and are believed to be fragments of genomic DNA expelled from healthy and/or cancerous cells, *e.g.,* upon apoptosis and lysis of the cellular envelope.

As used herein, the term "locus" refers to a position *(e.g.,* a site) within a genome, *e.g.,* on a particular chromosome. In some embodiments, a locus refers to a single nucleotide position, on a particular chromosome, within a genome. In some embodiments, a locus refers to a group of nucleotide positions within a genome. In some instances, a locus is defined by a mutation (e.g., substitution, insertion, deletion, inversion, or translocation) of consecutive nucleotides within a cancer genome. In some instances, a locus is defined by a gene, a sub-genic structure (e.g., a regulatory element, exon, intron, or combination thereof), or a predefined span of a chromosome. Because normal mammalian cells have diploid genomes, a normal mammalian genome (e.g., a human genome) will generally have two copies of every locus in the genome, or at least two copies of every locus located on the autosomal chromosomes, e.g., one copy on the maternal autosomal chromosome and one copy on the paternal autosomal chromosome.

As used herein, the term "allele" refers to a particular sequence of one or more nucleotides at a chromosomal locus. In a haploid organism, the subject has one allele at every chromosomal locus. In a diploid organism, the subject has two alleles at every chromosomal locus.

As used herein, the term "base pair" or "bp" refers to a unit consisting of two nucleobases bound to each other by hydrogen bonds. Generally, the size of an organism's genome is measured in base pairs because DNA is typically double stranded. However, some viruses have single-stranded DNA or RNA genomes.

As used herein, the terms "genomic alteration," "mutation," and "variant" refer to a detectable change in the genetic material of one or more cells. A genomic alteration, mutation, or variant can refer to various type of changes in the genetic material of a cell, including changes in the primary genome sequence at single or multiple nucleotide positions, e.g., a single nucleotide variant (SNV), a multi-nucleotide variant (MNV), an indel *(e.g.,* an insertion or deletion of nucleotides), a DNA rearrangement (e.g., an inversion or translocation of a portion of a chromosome or chromosomes), a variation in the copy number of a locus *(e.g.,* an exon, gene, or a large span of a chromosome) (CNV), a partial or complete change in the ploidy of the cell, as well as in changes in the epigenetic information of a genome, such as altered DNA methylation patterns. In some embodiments, a mutation is a change in the genetic information of the cell relative to a particular reference genome, or one or more 'normal' alleles found in the population of the species of the subject. For instance, mutations can be found in both germline cells *(e.g.,* non-cancerous, 'normal' cells) of a subject and in abnormal cells *(e.g.,* pre-cancerous or cancerous cells) of the subject. As such, a mutation in a germline of the subject (e.g., which is found in substantially all 'normal cells' in the subject) is identified relative to a reference genome for the species of the subject. However, many loci of a reference genome of a species are associated with several variant alleles that are significantly represented in the population of the subject and are not associated with a diseased state, e.g., such that they would not be considered 'mutations.' By contrast, in some embodiments, a mutation in a cancerous cell of a subject can be identified relative to either a reference genome of the subject or to the subject's own germline genome. In certain instances, identification of both types of variants can be informative. For instance, in some instances, a mutation that is present in both the cancer genome of the subject and the germline of the subject is informative for precision oncology when the mutation is a so-called 'driver mutation," which contributes to the initiation and/or development of a cancer. However, in other instances, a mutation that is present in both the cancer genome of the subject and the germline of the subject is not informative for precision oncology, e.g., when the mutation is a so-called "passenger mutation," which does not contribute to the initiation and/or development of the cancer. Likewise, in some instances, a mutation that is present in the cancer genome of the subject but not the germline of the subject is informative for precision oncology, e.g., where the mutation is a driver mutation and/or the mutation facilitates a therapeutic approach, e.g., by differentiating cancer cells from normal cells in a therapeutically actionable way. However, in some instances, a mutation that is present in the cancer genome but not the germline of a subject is not informative for precision oncology, e.g., where the mutation is a passenger mutation and/or where the mutation fails to differentiate the cancer cell from a germline cell in a therapeutically actionable way.

As used herein, the term "reference allele" refers to the sequence of one or more nucleotides at a chromosomal locus that is either the predominant allele represented at that chromosomal locus within the population of the species (e.g., the "wild-type" sequence), or an allele that is predefined within a reference genome for the species.

As used herein, the term "variant allele" refers to a sequence of one or more nucleotides at a chromosomal locus that is either not the predominant allele represented at that chromosomal locus within the population of the species (e.g., not the "wild-type" sequence), or not an allele that is predefined within a reference genome for the species.

As used herein, the term "variant allele fraction," "VAF," "allelic fraction," or "AF" refers to the number of times a variant or mutant allele was observed (e.g., a number of reads supporting a candidate variant allele) divided by the total number of times the position was sequenced (e.g., a total number of reads covering a candidate locus).

As used herein, the term "germline variants" refers to genetic variants inherited from maternal and paternal DNA. Germline variants may be determined through a matched tumor-normal calling pipeline.

As used herein, the term "somatic variants" refers to variants arising as a result of dysregulated cellular processes associated with neoplastic cells, *e.g.,* a mutation. Somatic variants may be detected via subtraction from a matched normal sample.

As used herein, the term "single nucleotide variant" or "SNV" refers to a substitution of one nucleotide to a different nucleotide at a position *(e.g.,* site) of a nucleotide sequence, *e.g.,* a sequence read from an individual. A substitution from a first nucleobase X to a second nucleobase Y may be denoted as "X>Y." For example, a cytosine to thymine SNV may be denoted as "C>T."

As used herein, the term "insertions and deletions" or "indels" refers to a variant resulting from the gain or loss of DNA base pairs within an analyzed region.

As used herein, the term "copy number variation" or "CNV" refers to the process by which large structural changes in a genome associated with tumor aneuploidy and other dysregulated repair systems are detected. These processes are used to detect large scale insertions or deletions of entire genomic regions. CNV is defined as structural insertions or deletions greater than a certain base pair ("bp") in size, such as 500 bp.

As used herein, the term "gene fusion" refers to the product of large scale chromosomal aberrations resulting in the creation of a chimeric protein. These expressed products can be non-functional, or they can be highly over or underactive. This can cause deleterious effects in cancer such as hyper-proliferative or anti-apoptotic phenotypes.

As used herein, the term "loss of heterozygosity" refers to the loss of one copy of a segment *(e.g.,* including part or all of one or more genes) of the genome of a diploid subject *(e.g.,* a human) or loss of one copy of a sequence encoding a functional gene product in the genome of the diploid subject, in a tissue, e.g., a cancerous tissue, of the subject. As used herein, when referring to a metric representing loss of heterozygosity across the entire genome of the subject, loss of heterozygosity is caused by the loss of one copy of various segments in the genome of the subject. Loss of heterozygosity across the entire genome may be estimated without sequencing the entire genome of a subject, and such methods for such estimations based on gene panel targeting-based sequencing methodologies are described in the art. Accordingly, in some embodiments, a metric representing loss of heterozygosity across the entire genome of a tissue of a subject is represented as a single value, *e.g.,* a percentage or fraction of the genome. In some cases a tumor is composed of various sub-clonal populations, each of which may have a different degree of loss of heterozygosity across their respective genomes. Accordingly, in some embodiments, loss of heterozygosity across the entire genome of a cancerous tissue refers to an average loss of heterozygosity across a heterogeneous tumor population. As used herein, when referring to a metric for loss of heterozygosity in a particular gene, *e.g.,* a DNA repair protein such as a protein involved in the homologous DNA recombination pathway (e.g., BRCA1 or BRCA2), loss of heterozygosity refers to complete or partial loss of one copy of the gene encoding the protein in the genome of the tissue and/or a mutation in one copy of the gene that prevents translation of a full-length gene product, *e.g.,* a frameshift or truncating (creating a premature stop codon in the gene) mutation in the gene of interest. In some cases a tumor is composed of various sub-clonal populations, each of which may have a different mutational status in a gene of interest. Accordingly, in some embodiments, loss of heterozygosity for a particular gene of interest is represented by an average value for loss of heterozygosity for the gene across all sequenced sub-clonal populations of the cancerous tissue. In other embodiments, loss of heterozygosity for a particular gene of interest is represented by a count of the number of unique incidences of loss of heterozygosity in the gene of interest across all sequenced sub-clonal populations of the cancerous tissue (e.g., the number of unique frame-shift and/or truncating mutations in the gene identified in the sequencing data).

As used herein, the term "microsatellites" refers to short, repeated sequences of DNA. The smallest nucleotide repeated unit of a microsatellite is referred to as the "repeated unit" or "repeat unit." In some embodiments, the stability of a microsatellite locus is evaluated by comparing some metric of the distribution of the number of repeated units at a microsatellite locus to a reference number or distribution.

As used herein, the term "microsatellite instability" or "MSI" refers to a genetic hypermutability condition associated with various cancers that results from impaired DNA mismatch repair (MMR) in a subject. Among other phenotypes, MSI causes changes in the size of microsatellite loci, i.e., a change in the number of repeated units at microsatellite loci, during DNA replication. Accordingly, the size of microsatellite repeats is varied in MSI cancers as compared to the size of the corresponding microsatellite repeats in the germline of a cancer subject. The term "Microsatellite Instability-High" or "MSI-H" refers to a state of a cancer *(e.g.,* a tumor) that has a significant MMR defect, resulting in microsatellite loci with significantly different lengths than the corresponding microsatellite loci in normal cells of the same individual. The term "Microsatellite Stable" or "MSS" refers to a state of a cancer *(e.g.,* a tumor) without significant MMR defects, such that there is no significant difference between the lengths of the microsatellite loci in cancerous cells and the lengths of the corresponding microsatellite loci in normal (e.g., non-cancerous) cells in the same individual. The term "Microsatellite Equivocal" or "MSE" refers to a state of a cancer *(e.g.,* a tumor) having an intermediate microsatellite length phenotype, that cannot be clearly classified as MSI-H or MSS based on statistical cutoffs used to define those two categories.

As used herein, the term "gene product" refers to an RNA (e.g., mRNA or miRNA) or protein molecule transcribed or translated from a particular genomic locus, *e.g.,* a particular gene. The genomic locus can be identified using a gene name, a chromosomal location, or any other genetic mapping metric.

As used herein, the terms "expression level," "abundance level," or simply "abundance" refers to an amount of a gene product, (an RNA species, e.g., mRNA or miRNA, or protein molecule) transcribed or translated by a cell, or an average amount of a gene product transcribed or translated across multiple cells. When referring to mRNA or protein expression, the term generally refers to the amount of any RNA or protein species corresponding to a particular genomic locus, *e.g.,* a particular gene. However, in some embodiments, an expression level can refer to the amount of a particular isoform of an mRNA or protein corresponding to a particular gene that gives rise to multiple mRNA or protein isoforms. The genomic locus can be identified using a gene name, a chromosomal location, or any other genetic mapping metric.

As used herein, the term "ratio" refers to any comparison of a first metric X, or a first mathematical transformation thereof X' (e.g., measurement of a number of units of a genomic sequence in a first one or more biological samples or a first mathematical transformation thereof) to another metric Y or a second mathematical transformation thereof Y' *(e.g.,* the number of units of a respective genomic sequence in a second one or more biological samples or a second mathematical transformation thereof) expressed as $X/Y$, $Y/X$, $\log_N(X/Y)$, $\log_N(Y/X)$, $X'/Y$, $Y/X'$, $\log_N(X'/Y)$, or $\log_N(Y/X')$, $X/Y'$, $Y'/X$, $\log_N(X/Y')$, $\log_N(Y'/X)$, $X'/Y'$, $Y'/X'$, $\log_N(X'/Y')$, or $\log_N(Y'/X')$, where N is any real number greater than 1 and where example mathematical transformations of X and Y include, but are not limited to. raising X or Y to a power Z, multiplying X or Y by a constant Q, where Z and Q are any real numbers, and/or taking an M based logarithm of X and/or Y, where M is a real number greater than 1. In one non-limiting example, X is transformed to X' prior to ratio calculation by raising X by the power of two ($X^2$) and Y is transformed to Y' prior to ratio calculation by raising Y by the power of 3.2 ($Y^{3.2}$) and the ratio of X and Y is computed as $\log_2(X'/Y')$.

As used herein, the term "relative abundance" refers to a ratio of a first amount of a compound measured in a sample, *e.g.,* a gene product (an RNA species, *e.g.,* mRNA or miRNA, or protein molecule) or nucleic acid fragments having a particular characteristic (e.g., aligning to a particular locus or encompassing a particular allele), to a second amount of a compound measured in a second sample. In some embodiments, relative abundance refers to a ratio of an amount of species of a compound to a total amount of the compound in the same sample. For instance, a ratio of the amount of mRNA transcripts encoding a particular gene in a sample (e.g., aligning to a particular region of the exome) to the total amount of mRNA transcripts in the sample. In other embodiments, relative abundance refers to a ratio of an amount of a compound or species of a compound in a first sample to an amount of the compound of the species of the compound in a second sample. For instance, a ratio of a normalized amount of mRNA transcripts encoding a particular gene in a first sample to a normalized amount of mRNA transcripts encoding the particular gene in a second and/or reference sample.

As used herein, the terms "sequencing," "sequence determination," and the like refer to any biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

As used herein, the term "genetic sequence" refers to a recordation of a series of nucleotides present in a subject's RNA or DNA as determined by sequencing of nucleic acids from the subject.

As used herein, the term "sequence reads" or "reads" refers to nucleotide sequences produced by any nucleic acid sequencing process described herein or known in the art. Reads can be generated from one end of nucleic acid fragments ("single-end reads") or from both ends of nucleic acid fragments (e.g., paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long *(e.g.,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp, 2000 bp, 5000 bp, 10,000 bp, or 50,000 bp or more. Nanopore® sequencing, for example, can provide sequence reads that can vary in size from tens to hundreds to thousands of base pairs. Illumina® parallel sequencing, for example, can provide sequence reads that do not vary as much, for example, most of the sequence reads can be smaller than 200 bp. A sequence read (or sequencing read) can refer to sequence information corresponding to a nucleic acid molecule *(e.g.,* a string of nucleotides). For example, a sequence read can correspond to a string of nucleotides (e.g., about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, *e.g.,* using sequencing techniques or using probes, *e.g.,* in hybridization arrays or capture probes, or amplification techniques, such as the polymerase chain reaction (PCR) or linear amplification using a single primer or isothermal amplification.

As used herein, the term "unique sequence read" or "DNA fragment sequence" refers to the nucleotide sequence of a unique DNA fragment produced by any nucleic acid sequencing process described herein or known in the art. Unique sequence reads are generated by collapsing non-unique, raw sequence reads generated from the same unique DNA fragment into a consensus sequence for the unique DNA fragment. In this fashion, a sequencing reaction will generate many non-unique, raw sequence reads for each DNA fragment in the reaction, but only a single DNA fragment sequence for the unique DNA fragment will be output from analysis of the raw sequence data.

As used herein, the term "read segment" refers to any form of nucleotide sequence read including the raw sequence reads obtained directly from a nucleic acid sequencing technique or from a sequence derived therefrom, e.g., an aligned sequence read, a collapsed sequence read, or a stitched sequence read.

As used herein, the term "read count" refers to the total number of nucleic acid reads generated, which may or may not be equivalent to the number of nucleic acid molecules generated, during a nucleic acid sequencing reaction.

As used herein, the term "read-depth," "sequencing depth," or "depth" can refer to a total number of unique nucleic acid fragments encompassing a particular locus or region of the genome of a subject that are sequenced in a particular sequencing reaction. Sequencing depth can be expressed as "Yx", *e.g.,* 50x, 100x, *etc.,* where "Y" refers to the number of unique nucleic acid fragments encompassing a particular locus that are sequenced in a sequencing reaction. In such a case, Y is necessarily an integer, because it represents the actual sequencing depth for a particular locus. Alternatively, read-depth, sequencing depth, or depth can refer to a measure of central tendency (e.g., a mean or mode) of the number of unique nucleic acid fragments that encompass one of a plurality of loci or regions of the genome of a subject that are sequenced in a particular sequencing reaction. For example, in some embodiments, sequencing depth refers to the average depth of every locus across an arm of a chromosome, a targeted sequencing panel, an exome, or an entire genome. In such case, Y may be expressed as a fraction or a decimal, because it refers to an average coverage across a plurality of loci. When a mean depth is recited, the actual depth for any particular locus may be different than the overall recited depth. Metrics can be determined that provide a range of sequencing depths in which a defined percentage of the total number of loci fall. For instance, a range of sequencing depths within which 90% or 95%, or 99% of the loci fall. As understood by the skilled artisan, different sequencing technologies provide different sequencing depths. For instance, low-pass whole genome sequencing can refer to technologies that provide a sequencing depth of less than 5x, less than 4x, less than 3x, or less than 2x, *e.g.,* from about 0.5x to about 3x.

As used herein, the term "sequencing breadth" refers to what fraction of a particular reference exome *(e.g.,* human reference exome), a particular reference genome *(e.g.,* human reference genome), or part of the exome or genome has been analyzed. Sequencing breadth can be expressed as a fraction, a decimal, or a percentage, and is generally calculated as (the number of loci analyzed / the total number of loci in a reference exome or reference genome). The denominator of the fraction can be a repeat-masked genome, and thus 100% can correspond to all of the reference genome minus the masked parts. A repeat-masked exome or genome can refer to an exome or genome in which sequence repeats are masked (e.g., sequence reads align to unmasked portions of the exome or genome). In some embodiments, any part of an exome or genome can be masked and, thus, sequencing breadth can be evaluated for any desired portion of a reference exome or genome. In some embodiments, "broad sequencing" refers to sequencing/analysis of at least 0.1% of an exome or genome.

As used herein, the term "sequencing probe" refers to a molecule that binds to a nucleic acid with affinity that is based on the expected nucleotide sequence of the RNA or DNA present at that locus.

As used herein, the term "targeted panel" or "targeted gene panel" refers to a combination of probes for sequencing (e.g., by next-generation sequencing) nucleic acids present in a biological sample from a subject (e.g., a tumor sample, liquid biopsy sample, germline tissue sample, white blood cell sample, or tumor or tissue organoid sample), selected to map to one or more loci of interest on one or more chromosomes. An example set of loci/genes useful for precision oncology, e.g., via solid or liquid biopsy assay, that can be analyzed using a targeted panel is described in Table 1. In some embodiments, in addition to loci that are informative for precision oncology, a targeted panel includes one or more probes for sequencing one or more of a loci associated with a different medical condition, a loci used for internal control purposes, or a loci from a pathogenic organism (e.g., an oncogenic pathogen).

As used herein, the term, "reference exome" refers to any sequenced or otherwise characterized exome, whether partial or complete, of any tissue from any organism or pathogen that may be used to reference identified sequences from a subject. Typically, a reference exome will be derived from a subject of the same species as the subject whose sequences are being evaluated. Example reference exomes used for human subjects as well as many other organisms are provided in the on-line genome browser hosted by the National Center for Biotechnology Information ("NCBI"). An "exome" refers to the complete transcriptional profile of an organism or pathogen, expressed in nucleic acid sequences. As used herein, a reference sequence or reference exome often is an assembled or partially assembled exomic sequence from an individual or multiple individuals. In some embodiments, a reference exome is an assembled or partially assembled exomic sequence from one or more human individuals. The reference exome can be viewed as a representative example of a species' set of expressed genes. In some embodiments, a reference exome comprises sequences assigned to chromosomes.

As used herein, the term "reference genome" refers to any sequenced or otherwise characterized genome, whether partial or complete, of any organism or pathogen that may be used to reference identified sequences from a subject. Typically, a reference genome will be derived from a subject of the same species as the subject whose sequences are being evaluated. Exemplary reference genomes used for human subjects as well as many other organisms are provided in the on-line genome browser hosted by the National Center for Biotechnology Information ("NCBI") or the University of California, Santa Cruz (UCSC). A "genome" refers to the complete genetic information of an organism or pathogen, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38). For a haploid genome, there can be only one nucleotide at each locus. For a diploid genome, heterozygous loci can be identified; each heterozygous locus can have two alleles, where either allele can allow a match for alignment to the locus.

As used herein, the term "bioinformatics pipeline" refers to a series of processing stages used to determine characteristics of a subject's genome or exome based on sequencing data of the subject's genome or exome. A bioinformatics pipeline may be used to determine characteristics of a germline genome or exome of a subject and/or a cancer genome or exome of a subject. In some embodiments, the pipeline extracts information related to genomic alterations in the cancer genome of a subject, which is useful for guiding clinical decisions for precision oncology, from sequencing results of a biological sample, *e.g.,* a tumor sample, liquid biopsy sample, reference normal sample, *etc.,* from the subject. Certain processing stages in a bioinformatics may be 'connected,' meaning that the results of a first respective processing stage is informative and/or essential for execution of a second, downstream processing stage. For instance, in some embodiments, a bioinformatics pipeline includes a first respective processing stage for identifying genomic alterations that are unique to the cancer genome of a subject and a second respective processing stage that uses the quantity and/or identity of the identified genomic alterations to determine a metric that is informative for precision oncology, *e.g.,* a tumor mutational burden. In some embodiments, the bioinformatics pipeline includes a reporting stage that generates a report of relevant and/or actionable information identified by upstream stages of the pipeline, which may or may not further include recommendations for aiding clinical therapy decisions.

As used herein, a "percentage score" for a microsatellite locus refers to a measure of the percentage of DNA fragment sequences encompassing the microsatellite locus having a number of repeat units falling below a reference number of repeat units.

As used herein, a "lower mean score" for a microsatellite locus refers to a measure of central tendency, e.g., a mean, of the number of repeat units in a subset of unique sequence reads that (i) encompass the microsatellite locus, and (ii) have a repeat unit length falling below a reference number of repeat units.

As used herein, a "likelihood score" for a microsatellite locus refers to a statistical measure of a distribution of probabilities that each unique sequence read in all or a subset of the set of unique sequence reads encompassing the respective microsatellite locus is a member of a distribution of unique sequence reads encompassing a stable construct at the respective microsatellite locus. In some embodiments, a subset of unique sequence reads including only those unique sequence reads that fall within a threshold percentile of the smallest number of repeat units, e.g., 1%, 2%, 3%, 4%, 5%, 10%, 15%, 20%, 25%, 30%, or more, are used to generate the likelihood score. In some embodiments, the statistical measure is a measure of central tendency, e.g., a mean, of the distribution of probabilities.

Generally, a "reference number of repeat units" is selected to represent a stable construct at the microsatellite locus. However, in some embodiments, a reference value may be selected to represent a number of repeat units falling above or below a stability threshold, e.g., higher or lower than a maximum number of repeat units that would be considered to be stable at the microsatellite locus. In some embodiments, the reference number is selected based on the repeat unit lengths of one or more reference samples, e.g., healthy tissue samples from the patient or from another individual. In some embodiments, the reference number is selected based on a metric of the distribution of repeat unit lengths in the cancerous sample from the test subject, e.g., a median value, representative of the germline state of the microsatellite locus in the test subject.

As used herein, the term "limit of detection" or "LOD" refers to the minimal quantity of a feature that can be identified with a particular level of confidence. Accordingly, level of detection can be used to describe an amount of a substance that must be present in order for a particular assay to reliably detect the substance. A level of detection can also be used to describe a level of support needed for an algorithm to reliably identify a genomic alteration based on sequencing data. For example, a minimal number of unique sequence reads to support identification of a sequence variant such as a SNV.

As used herein, the term "BAM File" or "Binary file containing Alignment Maps" refers to a file storing sequencing data aligned to a reference sequence (e.g., a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "Positive Predictive Value" or "PPV" means the likelihood that a variant is properly called given that a variant has been called by an assay. PPV can be expressed as (number of true positives)/ (number of false positives + number of true positives).

As used herein, the term "assay" refers to a technique for determining a property of a substance, *e.g.,* a nucleic acid, a protein, a cell, a tissue, or an organ. An assay *(e.g.,* a first assay or a second assay) can comprise a technique for determining the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art can be used to detect any of the properties of nucleic acids mentioned herein. Properties of a nucleic acids can include a sequence, genomic identity, copy number, methylation state at one or more nucleotide positions, size of the nucleic acid, presence or absence of a mutation in the nucleic acid at one or more nucleotide positions, and pattern of fragmentation of a nucleic acid *(e.g.,* the nucleotide position(s) at which a nucleic acid fragments). An assay or method can have a particular sensitivity and/or specificity, and their relative usefulness as a diagnostic tool can be measured using ROC-AUC statistics.

As used herein, the term "classification" can refer to any number(s) or other characters(s) that are associated with a particular property of a sample. For example, in some embodiments, the term "classification" can refer to a type of cancer in a subject, a stage of cancer in a subject, a prognosis for a cancer in a subject, a tumor load, a presence of tumor metastasis in a subject, and the like. The classification can be binary (e.g., positive or negative) or have more levels of classification (e.g., a scale from 1 to 10 or 0 to 1). The terms "cutoff" and "threshold" can refer to predetermined numbers used in an operation. For example, a cutoff size can refer to a size above which fragments are excluded. A threshold value can be a value above or below which a particular classification applies. Either of these terms can be used in either of these contexts.

As used herein, the term "untrained classifier" refers to a classifier that has not been trained on a training dataset.

As used herein, the term "sensitivity" or "true positive rate" (TPR) refers to the number of true positives divided by the sum of the number of true positives and false negatives. Sensitivity can characterize the ability of an assay or method to correctly identify a proportion of the population that truly has a condition. For example, sensitivity can characterize the ability of a method to correctly identify the number of subjects within a population having cancer. In another example, sensitivity can characterize the ability of a method to correctly identify the one or more markers indicative of cancer.

As used herein, the term "specificity" or "true negative rate" (TNR) refers to the number of true negatives divided by the sum of the number of true negatives and false positives. Specificity can characterize the ability of an assay or method to correctly identify a proportion of the population that truly does not have a condition. For example, specificity can characterize the ability of a method to correctly identify the number of subjects within a population not having cancer. In another example, specificity characterizes the ability of a method to correctly identify one or more markers indicative of cancer.

As used herein, an "actionable genomic alteration" or "actionable variant" refers to a genomic alteration (e.g., a SNV, MNV, indel, rearrangement, copy number variation, or ploidy variation), or value of another cancer metric derived from nucleic acid sequencing data *(e.g.,* a tumor mutational burden, MSI status, or tumor fraction), that is known or believed to be associated with a therapeutic course of action that is more likely to produce a positive effect in a cancer patient that has the actionable variant than in a similarly situated cancer patient that does not have the actionable variant. For instance, administration of EGFR inhibitors (e.g., afatinib, erlotinib, gefitinib) is more effective for treating non-small cell lung cancer in patients with an EGFR mutation in exons 19/21 than for treating non-small cell lung cancer in patients that do not have an EGFR mutations in exons 19/21. Accordingly, an EGFR mutation in exon 19/21 is an actionable variant. In some instances, an actionable variant is only associated with an improved treatment outcome in one or a group of specific cancer types. In other instances, an actionable variant is associated with an improved treatment outcome in substantially all cancer types.

As used herein, a "variant of uncertain significance" or "VUS" refers to a genomic alteration *(e.g.,* a SNV, MNV, indel, rearrangement, copy number variation, or ploidy variation), or value of another cancer metric derived from nucleic acid sequencing data *(e.g.,* a tumor mutational burden, MSI status, or tumor fraction), whose impact on disease development/progression is unknown.

As used herein, a "benign variant" or "likely benign variant" refers to a genomic alteration *(e.g.,* a SNV, MNV, indel, rearrangement, copy number variation, or ploidy variation), or value of another cancer metric derived from nucleic acid sequencing data *(e.g.,* a tumor mutational burden, MSI status, or tumor fraction), that is known or believed to not contribute to disease development/progression.

As used herein, a "pathogenic variant" or "likely pathogenic variant" refers to a genomic alteration *(e.g.,* a SNV, MNV, indel, rearrangement, copy number variation, or ploidy variation), or value of another cancer metric derived from nucleic acid sequencing data *(e.g.,* a tumor mutational burden, MSI status, or tumor fraction), that is known or believed to contribute to disease development/progression.

As used herein, an "effective amount" or "therapeutically effective amount" is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the therapeutic agent being administered.

As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

In the following detailed description, numerous specific details are set forth in order to provide a thorough understanding of the present disclosure, including example systems, methods, techniques, instruction sequences, and computing machine program products that embody illustrative implementations. The features described herein are not limited by the illustrated ordering of acts or events, as some acts can occur in different orders and/or concurrently with other acts or events.

**Example System Embodiments - Not part of the invention**

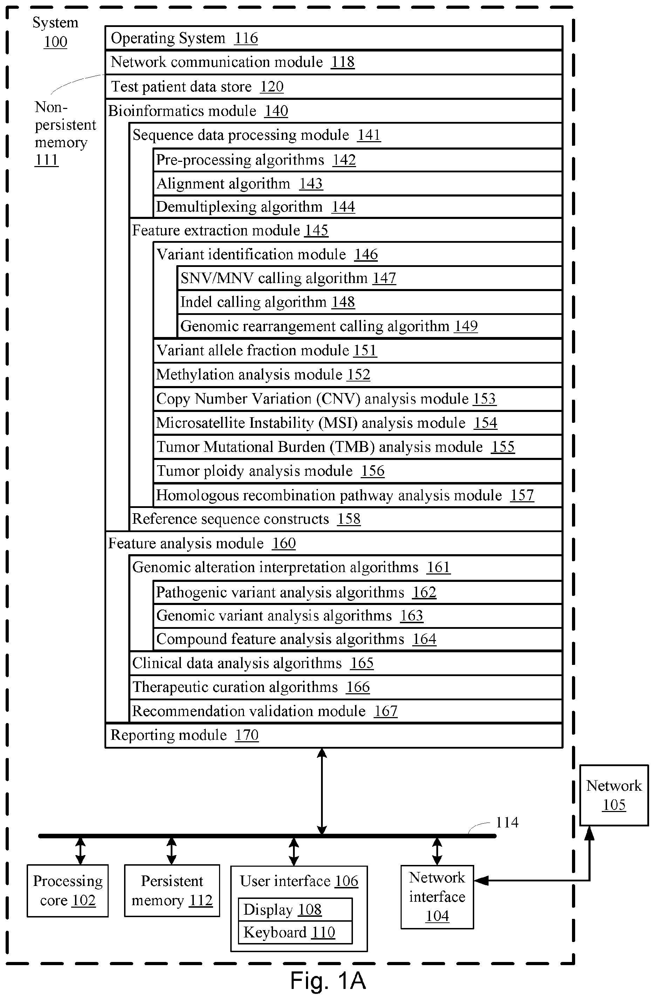

Now that some definitions used in the present disclosure have been provided, details of an exemplary system for providing clinical support for personalized cancer therapy using a liquid biopsy assay are now described in conjunction with Figures 1A-1D. Figures 1A-1D collectively illustrate the topology of an example system for providing clinical support for personalized cancer therapy using a liquid biopsy assay, in accordance with some embodiments of the present disclosure. Advantageously, the example system illustrated in Figures 1A-1D improves upon conventional methods for providing clinical support for personalized cancer therapy by providing improved classification of MSI status for the subject's cancer.

Figure 1A is a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 *(e.g.,* a mouse, touchpad, keyboard, *etc*.), a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:

- an operating system 116, which includes procedures for handling various basic system services and for performing hardware dependent tasks;
- a network communication module (or instructions) 118 for connecting the system 100 with other devices and/or a communication network 105;
- a test patient data store 120 for storing one or more collections of features from patients (e.g., subjects);
- a bioinformatics module 140 for processing sequencing data and extracting features from sequencing data, e.g., from liquid biopsy sequencing assays;
- a feature analysis module 160 for evaluating patient features, e.g., genomic alterations, compound genomic features, and clinical features; and
- a reporting module 180 for generating and transmitting reports that provide clinical support for personalized cancer therapy.

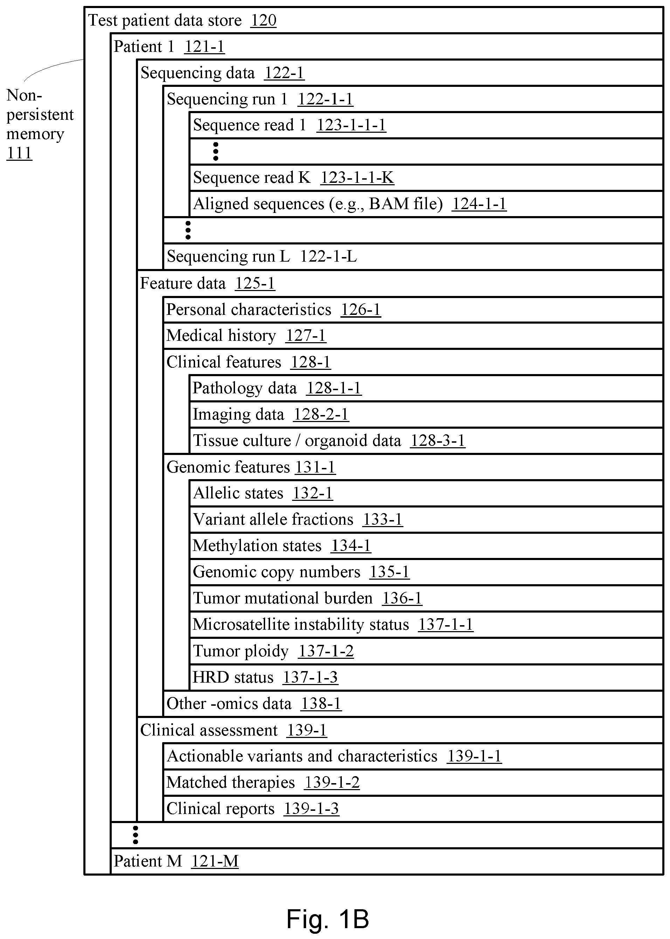

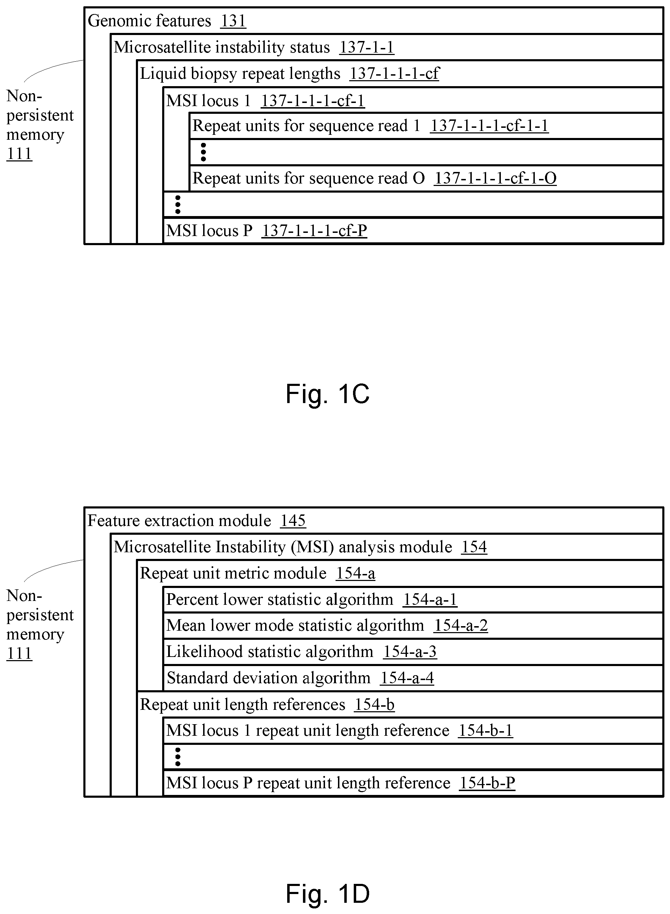

Although Figures 1A-1D depict a "system 100," the figures are intended more as a functional description of the various features that may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112. For example, in various implementations, one or more of the above identified elements are stored in one or more of the previously mentioned memory devices, and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 may retrieve all or a portion of such data when needed.

For purposes of illustration in Figure 1A, system 100 is represented as a single computer that includes all of the functionality for providing clinical support for personalized cancer therapy. However, while a single machine is illustrated, the term "system" shall also be taken to include any collection of machines that individually or jointly execute a set (or multiple sets) of instructions to perform any one or more of the methodologies discussed herein.

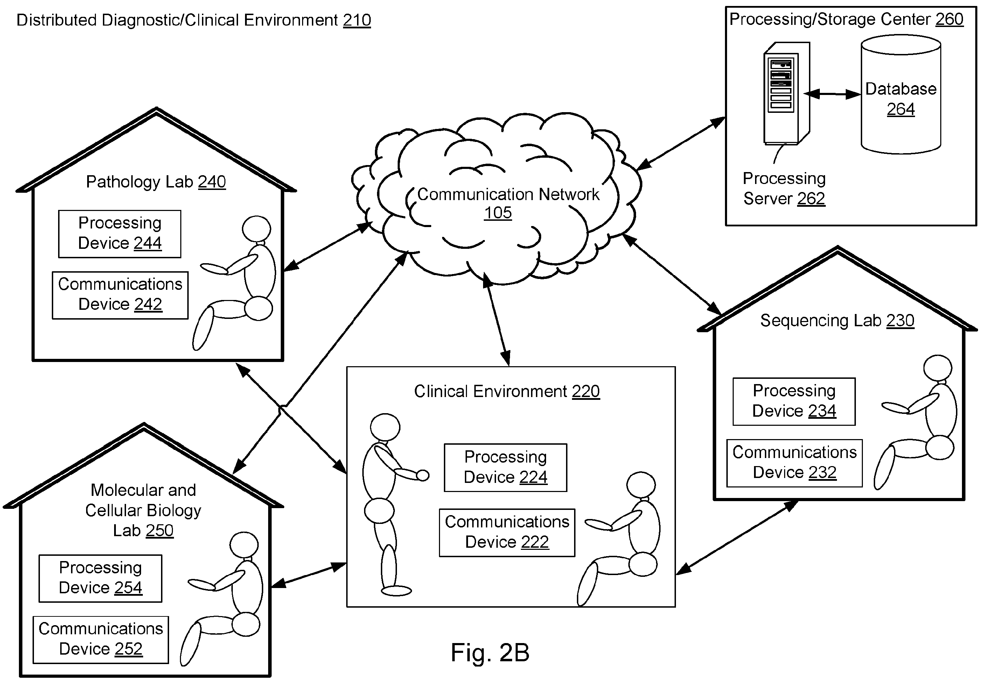

For example, in some embodiments, system 100 includes one or more computers. In some embodiments, the functionality for providing clinical support for personalized cancer therapy is spread across any number of networked computers and/or resides on each of several networked computers and/or is hosted on one or more virtual machines at a remote location accessible across the communications network 105. For example, different portions of the various modules and data stores illustrated in Figures 1A-1D can be stored and/or executed on the various instances of a processing device and/or processing server/database in the distributed diagnostic environment 210 illustrated in Figure 2B (e.g., processing devices 224, 234, 244, and 254, processing server 262, and database 264).

The system may operate in the capacity of a server or a client machine in client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

One of skill in the art will appreciate that any of a wide array of different computer topologies are used for the application and all such topologies are within the scope of the present disclosure.

Test Patient Data Store (120)

Referring to Figure 1B, in some embodiments, the system (e.g., system 100) includes a patient data store 120 that stores data for patients 121-1 to 121-M *(e.g.,* cancer patients or patients being tested for cancer) including one or more sequencing data 122, feature data 125, and clinical assessments 139. These data are used and/or generated by the various processes stored in the bioinformatics module 140 and feature analysis module 160 of system 100, to ultimately generate a report providing clinical support for personalized cancer therapy of a patient. While the feature scope of patient data 121 across all patients may be informationally dense, an individual patient's feature set may be sparsely populated across the entirety of the collective feature scope of all features across all patients. That is to say, the data stored for one patient may include a different set of features that the data stored for another patient. Further, while illustrated as a single data construct in Figure 1B, different sets of patient data may be stored in different databases or modules spread across one or more system memories.

In some embodiments, sequencing data 122 from one or more sequencing reactions 122-i, including a plurality of sequence reads 123-i-1 to 123-i-K, is stored in the test patient data store 120. The data store may include different sets of sequencing data from a single subject, corresponding to different samples from the patient, *e.g.,* a tumor sample, liquid biopsy sample, tumor organoid derived from a patient tumor, and/or a normal sample, and/or to samples acquired at different times, e.g., while monitoring the progression, regression, remission, and/or recurrence of a cancer in a subject. The sequence reads may be in any suitable file format, *e.g.,* BCL, FASTA, FASTQ, *etc.* In some embodiments, sequencing data 122 is accessed by a sequencing data processing module 141, which performs various pre-processing, genome alignment, and demultiplexing operations, as described in detail below with reference to bioinformatics module 140. In some embodiments, sequence data that has been aligned to a reference construct, e.g., BAM file 124, is stored in test patient data store 120.

In some embodiments, the test patient data store 120 includes feature data 125, *e.g.,* that is useful for identifying clinical support for personalized cancer therapy. In some embodiments, the feature data 125 includes personal characteristics 126 of the patient, such as patient name, date of birth, gender, ethnicity, physical address, smoking status, alcohol consumption characteristic, anthropomorphic data, *etc.*

In some embodiments, the feature data 125 includes medical history data 127 for the patient, such as cancer diagnosis information (e.g., date of initial diagnosis, date of metastatic diagnosis, cancer staging, tumor characterization, tissue of origin, previous treatments and outcomes, adverse effects of therapy, therapy group history, clinical trial history, previous and current medications, surgical history, *etc.*), previous or current symptoms, previous or current therapies, previous treatment outcomes, previous disease diagnoses, diabetes status, diagnoses of depression, diagnoses of other physical or mental maladies, and family medical history. In some embodiments, the feature data 125 includes clinical features 128, such as pathology data 128-1, medical imaging data 128-2, and tissue culture and/or tissue organoid culture data 128-3.

In some embodiments, yet other clinical features, such as previous laboratory testing results, are stored in the test patient data store 120. Medical history data 127 and clinical features may be collected from various sources, including at intake directly from the patient, from an electronic medical record (EMR) or electronic health record (EHR) for the patient, or curated from other sources, such as fields from various testing records (e.g., genetic sequencing reports).

In some embodiments, the feature data 125 includes genomic features 131 for the patient. Non-limiting examples of genomic features include allelic states 132 *(e.g.,* the identity of alleles at one or more loci, support for wild type or variant alleles at one or more loci, support for SNVs/MNVs at one or more loci, support for indels at one or more loci, and/or support for gene rearrangements at one or more loci), allelic fractions 133 *(e.g.,* ratios of variant to reference alleles (or vice versa), methylation states 132 *(e.g.,* a distribution of methylation patterns at one or more loci and/or support for aberrant methylation patterns at one or more loci), genomic copy numbers 135 *(e.g.,* a copy number value at one or more loci and/or support for an aberrant (increased or decreased) copy number at one or more loci), tumor mutational burden 136 *(e.g.,* a measure of the number of mutations in the cancer genome of the subject), and microsatellite instability status 137 *(e.g.,* a measure of the repeated unit length at one or more microsatellite loci and/or a classification of the MSI status for the patient's cancer). In some embodiments, one or more of the genomic features 131 are determined by a nucleic acid bioinformatics pipeline, *e.g.,* as described in detail below with reference to Figures 4A-4G. In particular, in some embodiments, the feature data 125 include liquid biopsy repeat lengths 137-1-1-1, as determined using the improved methods for classifying the microsatellite stability of a cancer, as described in further detail below with reference to Figures 1C, 1D, and 4C. In some embodiments, one or more of the genomic features 131 are obtained from an external testing source, e.g., not connected to the bioinformatics pipeline as described below.

In some embodiments, the feature data 125 further includes data 138 from other -omics fields of study. Non-limiting examples of -omics fields of study that may yield feature data useful for providing clinical support for personalized cancer therapy include transcriptomics, epigenomics, proteomics, metabolomics, metabonomics, microbiomics, lipidomics, glycomics, cellomics, and organoidomics.

In some embodiments, yet other features may include features derived from machine learning approaches, e.g., based at least in part on evaluation of any relevant molecular or clinical features, considered alone or in combination, not limited to those listed above. For instance, in some embodiments, one or more latent features learned from evaluation of cancer patient training datasets improve the diagnostic and prognostic power of the various analysis algorithms in the feature analysis module 160.

The skilled artisan will know of other types of features useful for providing clinical support for personalized cancer therapy. The listing of features above is merely representative and should not be construed to be limiting.

In some embodiments, a test patient data store 120 includes clinical assessment data 139 for patients, e.g., based on the feature data 125 collected for the subject. In some embodiments, the clinical assessment data 139 includes a catalogue of actionable variants and characteristics 139-1 (e.g., genomic alterations and compound metrics based on genomic features known or believed to be targetable by one or more specific cancer therapies), matched therapies 139-2 (e.g., the therapies known or believed to be particularly beneficial for treatment of subjects having actionable variants), and/or clinical reports 139-3 generated for the subject, e.g., based on identified actionable variants and characteristics 139-1 and/or matched therapies 139-2.

In some embodiments, clinical assessment data 139 is generated by analysis of feature data 125 using the various algorithms of feature analysis module 160, as described in further detail below. In some embodiments, clinical assessment data 139 is generated, modified, and/or validated by evaluation of feature data 125 by a clinician, e.g., an oncologist. For instance, in some embodiments, a clinician (e.g., at clinical environment 220) uses feature analysis module 160, or accesses test patient data store 120 directly, to evaluate feature data 125 to make recommendations for personalized cancer treatment of a patient. Similarly, in some embodiments, a clinician (e.g., at clinical environment 220) reviews recommendations determined using feature analysis module 160 and approves, rejects, or modifies the recommendations, e.g., prior to the recommendations being sent to a medical professional treating the cancer patient.

Bioinformatics Module (140)

Referring again to Figure 1A, the system (e.g., system 100) includes a bioinformatics module 140 that includes a feature extraction module 145 and optional ancillary data processing constructs, such as a sequence data processing module 141 and/or one or more reference sequence constructs 158 (e.g., a reference genome, exome, or targeted-panel construct that includes reference sequences for a plurality of loci targeted by a sequencing panel).

In some embodiments, bioinformatics module 140 includes a sequence data processing module 141 that includes instructions for processing sequence reads, e.g., raw sequence reads 123 from one or more sequencing reactions 122, prior to analysis by the various feature extraction algorithms, as described in detail below. In some embodiments, sequence data processing module 141 includes one or more pre-processing algorithms 142 that prepare the data for analysis. In some embodiments, the pre-processing algorithms 142 include instructions for converting the file format of the sequence reads from the output of the sequencer (e.g., a BCL file format) into a file format compatible with downstream analysis of the sequences (e.g., a FASTQ or FASTA file format). In some embodiments, the pre-processing algorithms 142 include instructions for evaluating the quality of the sequence reads (e.g., by interrogating quality metrics like Phred score, base-calling error probabilities, Quality (Q) scores, and the like) and/or removing sequence reads that do not satisfy a threshold quality (e.g., an inferred base call accuracy of at least 80%, at least 90%, at least 95%, at least 99%, at least 99.5%, at least 99.9%, or higher). In some embodiments, the pre-processing algorithms 142 include instructions for filtering the sequence reads for one or more properties, e.g., removing sequences failing to satisfy a lower or upper size threshold or removing duplicate sequence reads.

In some embodiments, sequence data processing module 141 includes one or more alignment algorithms 143, for aligning pre-processed sequence reads 123 to a reference sequence construct 158, e.g., a reference genome, exome, or targeted-panel construct. Many algorithms for aligning sequencing data to a reference construct are known in the art, for example, BWA, Blat, SHRiMP, LastZ, and MAQ. One example of a sequence read alignment package is the Burrows-Wheeler Alignment tool (BWA), which uses a Burrows-Wheeler Transform (BWT) to align short sequence reads against a large reference construct, allowing for mismatches and gaps. Li and Durbin, Bioinformatics, 25(14):1754-60 (2009). Sequence read alignment packages import raw or pre-processed sequence reads 122, e.g., in BCL, FASTA, or FASTQ file formats, and output aligned sequence reads 124, e.g., in SAM or BAM file formats.

In some embodiments, sequence data processing module 141 includes one or more demultiplexing algorithms 144, for dividing sequence read or sequence alignment files generated from sequencing reactions of pooled nucleic acids into separate sequence read or sequence alignment files, each of which corresponds to a different source of nucleic acids in the nucleic acid sequencing pool. For instance, because of the cost of sequencing reactions, it is common practice to pool nucleic acids from a plurality of samples into a single sequencing reaction. The nucleic acids from each sample are tagged with a sample-specific and/or molecule-specific sequence tag *(e.g.,* a UMI), which is sequenced along with the molecule. In some embodiments, demultiplexing algorithms 144 sort these sequence tags in the sequence read or sequence alignment files to demultiplex the sequencing data into separate files for each of the samples included in the sequencing reaction.

Bioinformatics module 140 includes a feature extraction module 145, which includes instructions for identifying diagnostic features, e.g., genomic features 131, from sequencing data 122 of biological samples from a subject, e.g., one or more of a solid tumor sample, a liquid biopsy sample, or a normal tissue (e.g., control) sample. For instance, in some embodiments, a feature extraction algorithm compares the identity of one or more nucleotides at a locus from the sequencing data 122 to the identity of the nucleotides at that locus in a reference sequence construct *(e.g.,* a reference genome, exome, or targeted-panel construct) to determine whether the subject has a variant at that locus. In some embodiments, a feature extraction algorithm evaluates data other than the raw sequence, to identify a genomic alteration in the subject, e.g., an allelic ratio, a relative copy number, a repeat unit distribution, *etc.*

For instance, in some embodiments, feature extraction module 145 includes one or more variant identification modules that include instructions for various variant calling processes. In some embodiments, variants in the germline of the subject are identified, *e.g.,* using a germline variant identification module 146. In some embodiments, variants in the cancer genome, *e.g.,* somatic variants, are identified, *e.g.,* using a somatic variant identification module 150. While separate germline and somatic variant identification modules are illustrated in Figure 1A, in some embodiments they are integrated into a single module. In some embodiments, the variant identification module includes instructions for identifying one or more of nucleotide variants *(e.g.,* single nucleotide variants (SNV) and multi-nucleotide variants (MNV)) using one or more SNV/MNV calling algorithms *(e.g.,* algorithms 147 and/or 151), indels *(e.g.,* insertions or deletions of nucleotides) using one or more indel calling algorithms *(e.g.,* algorithms 148 and/or 152), and genomic rearrangements *(e.g.,* inversions, translocation, and fusions of nucleotide sequences) using one or more genomic rearrangement calling algorithms (e.g., algorithms 149 and/or 153).

A SNV/MNV algorithm 147 may identify a substitution of a single nucleotide that occurs at a specific position in the genome. For example, at a specific base position, or locus, in the human genome, the C nucleotide may appear in most individuals, but in a minority of individuals, the position is occupied by an A. This means that there is a SNP at this specific position and the two possible nucleotide variations, C or A, are said to be alleles for this position. SNPs underlie differences in human susceptibility to a wide range of diseases (*e.g.* - sickle-cell anemia, β-thalassemia and cystic fibrosis result from SNPs). The severity of illness and the way the body responds to treatments are also manifestations of genetic variations. For example, a single-base mutation in the APOE (apolipoprotein E) gene is associated with a lower risk for Alzheimer's disease. A single-nucleotide variant (SNV) is a variation in a single nucleotide without any limitations of frequency and may arise in somatic cells. A somatic single-nucleotide variation (*e.g.,* caused by cancer) may also be called a single-nucleotide alteration. An MNP (Multiple-nucleotide polymorphisms) module may identify the substitution of consecutive nucleotides at a specific position in the genome.

An indel calling algorithm 148 may identify an insertion or deletion of bases in the genome of an organism classified among small genetic variations. While indels usually measure from 1 to 10 000 base pairs in length, a microindel is defined as an indel that results in a net change of 1 to 50 nucleotides. Indels can be contrasted with a SNP or point mutation. An indel inserts and/or deletes nucleotides from a sequence, while a point mutation is a form of substitution that replaces one of the nucleotides without changing the overall number in the DNA. Indels, being insertions and/or deletions, can be used as genetic markers in natural populations, especially in phylogenetic studies. Indel frequency tends to be markedly lower than that of single nucleotide polymorphisms (SNP), except near highly repetitive regions, including homopolymers and microsatellites.

A genomic rearrangement algorithm 149 may identify hybrid genes formed from two previously separate genes. It can occur as a result of: translocation, interstitial deletion, or chromosomal inversion. Gene fusion can play an important role in tumorigenesis. Fusion genes can contribute to tumor formation because fusion genes can produce much more active abnormal protein than non-fusion genes. Often, fusion genes are oncogenes that cause cancer; these include BCR-ABL, TEL-AML1 (ALL with t(12 ; 21)), AML1-ETO (M2 AML with t(8 ; 21)), and TMPRSS2-ERG with an interstitial deletion on chromosome 21, often occurring in prostate cancer. In the case of TMPRSS2-ERG, by disrupting androgen receptor (AR) signaling and inhibiting AR expression by oncogenic ETS transcription factor, the fusion product regulates prostate cancer. Most fusion genes are found from hematological cancers, sarcomas, and prostate cancer. BCAM-AKT2 is a fusion gene that is specific and unique to high-grade serous ovarian cancer. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. The latter is common in lymphomas, where oncogenes are juxtaposed to the promoters of the immunoglobulin genes. Oncogenic fusion transcripts may also be caused by trans-splicing or read-through events. Since chromosomal translocations play such a significant role in neoplasia, a specialized database of chromosomal aberrations and gene fusions in cancer has been created. This database is called Mitelman Database of Chromosome Aberrations and Gene Fusions in Cancer.

In some embodiments, feature extraction module 145 includes instructions for identifying one or more complex genomic alterations (e.g., features that incorporate more than a change in the primary sequence of the genome) in the cancer genome of the subject. For instance, in some embodiments, feature extraction module 145 includes modules for identifying one or more of copy number variation (e.g., copy number variation analysis module 153), microsatellite instability status (e.g., microsatellite instability analysis module 154), tumor mutational burden *(e.g.,* tumor mutational burden analysis module 155), tumor ploidy *(e.g.,* tumor ploidy analysis module 156), and homologous recombination pathway deficiencies (e.g., homologous recombination pathway analysis module 157).

Feature Analysis Module (160)

Referring again to Figure 1A, the system (e.g., system 100) includes a feature analysis module 160 that includes one or more genomic alteration interpretation algorithms 161, one or more optional clinical data analysis algorithms 165, an optional therapeutic curation algorithm 165, and an optional recommendation validation module 167. In some embodiments, feature analysis module 160 identifies actionable variants and characteristics 139-1 and corresponding matched therapies 139-2 and/or clinical trials using one or more analysis algorithms (e.g., algorithms 162, 163, 164, and 165) to evaluate feature data 125. The identified actionable variants and characteristics 139-1 and corresponding matched therapies 139-2, which are optionally stored in test patient data store 120, are then curated by feature analysis module 160 to generate a clinical report 139-3, which is optionally validated by a user, *e.g.,* a clinician, before being transmitted to a medical professional, e.g., an oncologist, treating the patient.

In some embodiments, the genomic alteration interpretation algorithms 161 include instructions for evaluating the effect that one or more genomic features 131 of the subject, e.g., as identified by feature extraction module 145, have on the characteristics of the patient's cancer and/or whether one or more targeted cancer therapies may improve the clinical outcome for the patient. For example, in some embodiments, one or more genomic variant analysis algorithms 163 evaluate various genomic features 131 by querying a database, e.g., a look-up-table ("LUT") of actionable genomic alterations, targeted therapies associated with the actionable genomic alterations, and any other conditions that should be met before administering the targeted therapy to a subject having the actionable genomic alteration. For instance, evidence suggests that depatuxizumab mafodotin (an anti-EGFR mAb conjugated to monomethyl auristatin F) has improved efficacy for the treatment of recurrent glioblastomas having EGFR focal amplifications. van den Bent M. *et al.,* Cancer Chemother Pharmacol., 80(6):1209-17 (2017). Accordingly, the actionable genomic alteration LUT would have an entry for the focal amplification of the EGFR gene indicating that depatuxizumab mafodotin is a targeted therapy for glioblastomas (e.g., recurrent glioblastomas) having a focal gene amplification. In some instances, the LUT may also include counter indications for the associated targeted therapy, e.g., adverse drug interactions or personal characteristics that are counter-indicated for administration of the particular targeted therapy.

In some embodiments, a genomic alteration interpretation algorithm 161 determines whether a particular genomic feature 131 should be reported to a medical professional treating the cancer patient. In some embodiments, genomic features 131 *(e.g.,* genomic alterations and compound features) are reported when there is clinical evidence that the feature significantly impacts the biology of the cancer, impacts the prognosis for the cancer, and/or impacts pharmacogenomics, e.g., by indicating or counter-indicating particular therapeutic approaches. For instance, a genomic alteration interpretation algorithm 161 may classify a particular CNV feature 135 as "Reportable," e.g., meaning that the CNV has been identified as influencing the character of the cancer, the overall disease state, and/or pharmacogenomics, as "Not Reportable," *e.g*., meaning that the CNV has not been identified as influencing the character of the cancer, the overall disease state, and/or pharmacogenomics, as "No Evidence," *e.g.,* meaning that no evidence exists supporting that the CNV is "Reportable" or "Not Reportable,"or as "Conflicting Evidence," *e.g.,* meaning that evidence exists supporting both that the CNV is "Reportable" and that the CNV is "Not Reportable."

In some embodiments, the genomic alteration interpretation algorithms 161 include one or more pathogenic variant analysis algorithms 162, which evaluate various genomic features to identify the presence of an oncogenic pathogen associated with the patient's cancer and/or targeted therapies associated with an oncogenic pathogen infection in the cancer. For instance, RNA expression patterns of some cancers are associated with the presence of an oncogenic pathogen that is helping to drive the cancer. See, for example, U.S. Patent Application Serial No. 16/802,126, filed February 26, 2020. In some instances, the recommended therapy for the cancer is different when the cancer is associated with the oncogenic pathogen infection than when it is not. Accordingly, in some embodiments, *e.g.,* where feature data 125 includes RNA abundance data for the cancer of the patient, one or more pathogenic variant analysis algorithms 162 evaluate the RNA abundance data for the patient's cancer to determine whether a signature exists in the data that indicates the presence of the oncogenic pathogen in the cancer. Similarly, in some embodiments, bioinformatics module 140 includes an algorithm that searches for the presence of pathogenic nucleic acid sequences in sequencing data 122. See, for example, U.S. Provisional Patent Application Serial No. 62/978,067, filed February 18, 2020. Accordingly, in some embodiments, one or more pathogenic variant analysis algorithms 162 evaluates whether the presence of an oncogenic pathogen in a subject is associated with an actionable therapy for the infection. In some embodiments, system 100 queries a database, *e.g.,* a look-up-table ("LUT"), of actionable oncogenic pathogen infections, targeted therapies associated with the actionable infections, and any other conditions that should be met before administering the targeted therapy to a subject that is infected with the oncogenic pathogen. In some instances, the LUT may also include counter indications for the associated targeted therapy, *e.g.,* adverse drug interactions or personal characteristics that are counter-indicated for administration of the particular targeted therapy.

In some embodiments, the genomic alteration interpretation algorithms 161 include one or more multi-feature analysis algorithms 164 that evaluate a plurality of features to classify a cancer with respect to the effects of one or more targeted therapies. For instance, in some embodiments, feature analysis module 160 includes one or more classifiers trained against feature data, one or more clinical therapies, and their associated clinical outcomes for a plurality of training subjects to classify cancers based on their predicted clinical outcomes following one or more therapies.

In some embodiments, the classifier is implemented as an artificial intelligence engine and may include gradient boosting models, random forest models, neural networks (NN), regression models, Naive Bayes models, and/or machine learning algorithms (MLA). A MLA or a NN may be trained from a training data set that includes one or more features 125, including personal characteristics 126, medical history 127, clinical features 128, genomic features 131, and/or other -omic features 138. MLAs include supervised algorithms (such as algorithms where the features/classifications in the data set are annotated) using linear regression, logistic regression, decision trees, classification and regression trees, naive Bayes, nearest neighbor clustering; unsupervised algorithms (such as algorithms where no features/classification in the data set are annotated) using Apriori, means clustering, principal component analysis, random forest, adaptive boosting; and semi-supervised algorithms (such as algorithms where an incomplete number of features/classifications in the data set are annotated) using generative approach (such as a mixture of Gaussian distributions, mixture of multinomial distributions, hidden Markov models), low density separation, graph-based approaches (such as mincut, harmonic function, manifold regularization), heuristic approaches, or support vector machines.

NNs include conditional random fields, convolutional neural networks, attention based neural networks, deep learning, long short term memory networks, or other neural models where the training data set includes a plurality of tumor samples, RNA expression data for each sample, and pathology reports covering imaging data for each sample.

While MLA and neural networks identify distinct approaches to machine learning, the terms may be used interchangeably herein. Thus, a mention of MLA may include a corresponding NN or a mention of NN may include a corresponding MLA unless explicitly stated otherwise. Training may include providing optimized datasets, labeling these traits as they occur in patient records, and training the MLA to predict or classify based on new inputs. Artificial NNs are efficient computing models which have shown their strengths in solving hard problems in artificial intelligence. They have also been shown to be universal approximators, that is, they can represent a wide variety of functions when given appropriate parameters.

In some embodiments, system 100 includes a classifier training module that includes instructions for training one or more untrained or partially trained classifiers based on feature data from a training dataset. In some embodiments, system 100 also includes a database of training data for use in training the one or more classifiers. In other embodiments, the classifier training module accesses a remote storage device hosting training data. In some embodiments, the training data includes a set of training features, including but not limited to, various types of the feature data 125 illustrated in Figure 1B. In some embodiments, the classifier training module uses patient data 121, *e.g.,* when test patient data store 120 also stores a record of treatments administered to the patient and patient outcomes following therapy.

In some embodiments, feature analysis module 160 includes one or more clinical data analysis algorithms 165, which evaluate clinical features 128 of a cancer to identify targeted therapies which may benefit the subject. For example, in some embodiments, e.g., where feature data 125 includes pathology data 128-1, one or more clinical data analysis algorithms 165 evaluate the data to determine whether an actionable therapy is indicated based on the histopathology of a tumor biopsy from the subject, e.g., which is indicative of a particular cancer type and/or stage of cancer. In some embodiments, system 100 queries a database, e.g., a look-up-table ("LUT"), of actionable clinical features (e.g., pathology features), targeted therapies associated with the actionable features, and any other conditions that should be met before administering the targeted therapy to a subject associated with the actionable clinical features 128 (*e.g.,* pathology features 128-1). In some embodiments, system 100 evaluates the clinical features 128 (*e.g.*, pathology features 128-1) directly to determine whether the patient's cancer is sensitive to a particular therapeutic agent. Further details on example methods, systems, and algorithms for classifying cancer and identifying targeted therapies based on clinical data, such as pathology data 128-1, imaging data 138-2, and/or tissue culture/organoid data 128-3 are discussed, for example, in U.S. Patent Application No. 16/830,186, filed on March 25, 2020, U.S. Patent Application No. 16/789,363, filed on Feb. 12, 2020, and U.S. Provisional Application No. 63/007,874, filed on April. 9, 2020.

In some embodiments, feature analysis module 160 includes a clinical trials module that evaluates test patient data 121 to determine whether the patient is eligible for inclusion in a clinical trial for a cancer therapy, *e.g.,* a clinical trial that is currently recruiting patients, a clinical trial that has not yet begun recruiting patients, and/or an ongoing clinical trial that may recruit additional patients in the future. In some embodiments, a clinical trial module evaluates test patient data 121 to determine whether the results of a clinical trial are relevant for the patient, *e.g.,* the results of an ongoing clinical trial and/or the results of a completed clinical trial. For instance, in some embodiments, system 100 queries a database, e.g., a look-up-table ("LUT") of clinical trials, *e.g.,* active and/or completed clinical trials, and compares patient data 121 with inclusion criteria for the clinical trials, stored in the database, to identify clinical trials with inclusion criteria that closely match and/or exactly match the patient's data 121. In some embodiments, a record of matching clinical trials, *e.g.,* those clinical trials that the patient may be eligible for and/or that may inform personalized treatment decisions for the patient, are stored in clinical assessment database 139.

In some embodiments, feature analysis module 160 includes a therapeutic curation algorithm 166 that assembles actionable variants and characteristics 139-1, matched therapies 139-2, and/or relevant clinical trials identified for the patient, as described above. In some embodiments, a therapeutic curation algorithm 166 evaluates certain criteria related to which actionable variants and characteristics 139-1, matched therapies 139-2, and/or relevant clinical trials should be reported and/or whether certain matched therapies, considered alone or in combination, may be counter-indicated for the patient, e.g., based on personal characteristics 126 of the patient and/or known drug-drug interactions. In some embodiments, the therapeutic curation algorithm then generates one or more clinical reports 139-3 for the patient. In some embodiments, the therapeutic curation algorithm generates a first clinical report 139-3-1 that is to be reported to a medical professional treating the patient and a second clinical report 139-3-2 that will not be communicated to the medical professional, but may be used to improve various algorithms within the system.

In some embodiments, feature analysis module 160 includes a recommendation validation module 167, that includes an interface allowing a clinician to review, modify, and approve a clinical report 139-3 prior to the report being sent to a medical professional, e.g., an oncologist, treating the patient.

In some embodiments, each of the one or more feature collections, sequencing modules, bioinformatics modules (including, e.g., alteration module(s), structural variant calling and data processing modules), classification modules and outcome modules are communicatively coupled to a data bus to transfer data between each module for processing and/or storage. In some alternative embodiments, each of the feature collection, alteration module(s), structural variant and feature store are communicatively coupled to each other for independent communication without sharing the data bus.

Further details on systems and exemplary embodiments of modules and feature collections are discussed in PCT Application PCT/US19/69149, titled "A METHOD AND PROCESS FOR PREDICTING AND ANALYZING PATIENT COHORT RESPONSE, PROGRESSION, AND SURVIVAL," filed December 31, 2019.

Classifiers and Machine Learning:

In some embodiments, the methods and systems described herein train and/or employ classifiers and/or machine learning strategies to improve characterization of a patient's cancer and/or improve clinical outcomes by improving clinical support for personalized cancer therapies.

In some embodiments, one or more results obtained from a bioinformatics analysis pipeline for a respective biological sample *(e.g.,* a liquid biopsy sample) are used as features to train a classifier, *e.g.,* to improve methods of determining the microsatellite stability of a cancer, to classify a cancer condition of a patient, to identify personalized therapeutic strategies for a cancer patient, and/or to identify clinical trials relevant to a cancer patient. In some embodiments, a classifier is trained against data from a plurality of patients, where each respective patient in the plurality of patients has the same cancer condition *(e.g.,* a presence or absence of cancer, a type of cancer, a stage of cancer, and/or a tissue-of-origin). In some alternative embodiments, a first one or more patients (or a first subset of patients) in the plurality of patients has a cancer condition that is different from a second one or more patients (or a second subset of patients) in the plurality of training patients. In some embodiments, to improve the training of the classifier, the classifier is trained against data from a plurality of patients, where one or more patients in the plurality of patients have two or more cancer conditions *(e.g.,* 2, 3, 4, 5, 6, 7, 8, 9, 10, or more than 10 cancer conditions).

In some embodiments, features used to train a classifier include any of the features described above with respect to patient data store 120. For example, in some embodiments, a classifier is trained against distributions of the length of repeat units at microsatellite loci in the cancer of each training subject. In some such embodiments, the classifier is trained against the number of repeat units of nucleic acids, *e.g.,* cfDNA from a liquid biopsy sample *(e.g.,* a blood sample). In some embodiments, the features used to train the classifier include the repeat unit length at microsatellite loci of one or more genes listed in Table 2. In some embodiments, the classifier is trained against features of at least five of the genes listed in Table 2. In some embodiments, the classifier is trained against the number of repeat units at microsatellite loci of at least 10, at least 15, at least 20, at least 25, at least 30, at least 35, or all of the genes listed in Table 2. In some embodiments, the classifier is trained against the number of repeat units of one or more microsatellite loci in genes not listed in Table 2. In some alternative embodiments, the classifier is trained against the number of repeat units of one or more microsatellite loci in at least 5, at least 10, at least 15, at least 20, at least 30, at least 40, at least 50, at least 60, at least 80, or at least 100 genes not listed in Table 2.

However, the skilled artisan will appreciate that, in some instances, the use of different training data sets may yield different results. These differences may arise, for example, when different criteria are used to select the training population, *e.g.,* different inclusion and/or exclusion criteria such as cancer type, personal characteristics (*e.g.,* age, gender, ethnicity, family history, smoking status, *etc.),* or simply by using a smaller or larger data set.

The skilled artisan will also appreciate that some features will be more informative than other features in a particular classifier. One measure of the predictive power of respective features in a classifier based on multiple features is the regression coefficient calculated for the features during training of the model. Regression coefficients describe the relationship between each feature and the response of the model. The coefficient value represents the mean change in the response given a one-unit increase in the feature value. As such, at least for variables of the same type, the magnitude, *e.g.,* absolute value, of a regression coefficient is correlated with the importance of the feature in the model. That is, the higher the magnitude of the regression coefficient, the more important the variable is to the model. As such, in some embodiments, a feature set is selected based, at least in part, upon the importance of the respective features in one or more classification models. For instance, in some embodiments, one or more genes with lower predictive power in a classification model may be left out during classifier training.

In some embodiments, the size of the feature set may be affected by which features are included and/or excluded. For instance, in some embodiments, if particular features having high predictive power are included in a classification model, fewer total features may be included in the model. Similarly, in some embodiments, if features having high predictive power are excluded from the classification model, more of the other features may be included in the model. In some embodiments, other metrics are also available for evaluating the importance of a feature in a model, such as standardized regression coefficients and change in R-squared when the feature is added to the model last.

When selecting a feature set, the skilled artisan will also consider the degree to which features are correlated to each other. Correlation is a statistical measure of how linearly dependent two variables are upon each other. As such, two correlated features provide duplicative information to a predictive model, which can be detrimental to a classifier. As such, there are several reasons why a correlated feature may be excluded from a model. For instance, removing a correlated feature will make the algorithm faster, as the larger the number of features in a classifier the more computations that need to be made. Removing a correlated feature may also remove harmful bias, arising from the correlation, from a model. Finally, removing a correlated feature may make the model more interpretable. In some embodiments, the selection to remove one or the other feature of a correlated feature set is informed by predictive powers of the two features, *e.g.,* their respective regression coefficients.

Some MLA may identify features of importance and identify a coefficient, or weight, to them. The coefficient may be multiplied with the occurrence frequency of the feature to generate a score, and once the scores of one or more features exceed a threshold, certain classifications may be predicted by the MLA. A coefficient schema may be combined with a rule-based schema to generate more complicated predictions, such as predictions based upon multiple features. For example, ten key features may be identified across different classifications. A list of coefficients may exist for the key features, and a rule set may exist for the classification. A rule set may be based upon the number of occurrences of the feature, the scaled weights of the features, or other qualitative and quantitative assessments of features encoded in logic known to those of ordinary skill in the art.

In other MLA, features may be organized in a binary tree structure. For example, key features which distinguish between the most classifications may exist as the root of the binary tree and each subsequent branch in the tree until a classification may be awarded based upon reaching a terminal node of the tree. For example, a binary tree may have a root node which tests for a first feature. The occurrence or non-occurrence of this feature must exist (the binary decision), and the logic may traverse the branch which is true for the item being classified.

Additional rules may be based upon thresholds, ranges, or other qualitative and quantitative tests. While supervised methods are useful when the training dataset has many known values or annotations, the nature of EMR/EHR documents is that there may not be many annotations provided. When exploring large amounts of unlabeled data, unsupervised methods are useful for binning/bucketing instances in the data set. A single instance of the above models, or two or more such instances in combination, may constitute a model for the purposes of models, artificial intelligence, neural networks, or machine learning algorithms, herein.

In some embodiments, a classifier used in the methods described herein is a logistic regression algorithm, a neural network algorithm, a convolutional neural network algorithm, a support vector machine (SVM) algorithm, a Naive Bayes algorithm, a nearest neighbor algorithm, a boosted trees algorithm, a random forest algorithm, a decision tree algorithm, a clustering algorithm, or a combination thereof.

Logistic regression algorithms suitable for use as classifiers are disclosed, for example, in Agresti, An Introduction to Categorical Data Analysis, 1996, Chapter 5, pp. 103-144, John Wiley & Son, New York.

Neural network algorithms, including convolutional neural network algorithms, suitable for use as classifiers are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology. A neural network has a layered structure that includes a layer of input units (and the bias) connected by a layer of weights to a layer of output units. For regression, the layer of output units typically includes just one output unit. However, neural networks can handle multiple quantitative responses in a seamless fashion. In multilayer neural networks, there are input units (input layer), hidden units (hidden layer), and output units (output layer). There is, furthermore, a single bias unit that is con-nected to each unit other than the input units.

Additional example neural networks suitable for use as classifiers are disclosed in Duda et al., 2001, Pattern Classification, Second Edition, John Wiley & Sons, Inc., New York; and Hastie et al., 2001, The Elements of Statistical Learning, Springer-Verlag, New York. Additional example neural networks suitable for use as classifiers are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York.

SVM algorithms suitable for use as classifiers are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914. When used for classification, SVMs separate a given set of binary labeled data training set *(e.g.,* a first and second cancer condition of each respective subject in a plurality of subjects) with a hyperplane that is maximally distant from the labeled data. For cases in which no linear separation is possible, SVMs can work in combination with the technique of 'kernels, which automatically realize a non-linear mapping to a feature space. The hyperplane found by the SVM in feature space corresponds to a non-linear decision boundary in the input space.

Naive Bayes classifiers suitable for use as classifiers are disclosed, for example, in Ng et al., 2002, "On discriminative vs. generative classifiers: A comparison of logistic regression and naive Bayes," Advances in Neural Information Processing Systems, 14.

Decision trees algorithms suitable for use as classifiers are described in, for example, Duda, 2001, Pattern Classification, John Wiley & Sons, Inc., New York, pp. 395-396. Treebased methods partition the feature space into a set of rectangles, and then fit a model (like a constant) in each one. In some embodiments, the decision tree is random forest regression. One specific algorithm that can be used as a classifier is a classification and regression tree (CART). Other examples of specific decision tree algorithms that can be used as classifiers include, but are not limited to, ID3, C4.5, MART, and Random Forests. CART, ID3, and C4.5 are described in Duda, 2001, Pattern Classification, John Wiley & Sons, Inc., New York. pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random Forests--Random Features," Technical Report 567, Statistics Department, U.C. Berkeley, September 1999.

Clustering algorithms suitable for use as classifiers are described, for example, at pages 211-256 of Duda and Hart, Pattern Classification and Scene Analysis, 1973, John Wiley & Sons, Inc., New York, (hereinafter "Duda 1973"). As set forth in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in a dataset. If distance is a good measure of similarity, then the distance between samples in the same cluster will be significantly less than the distance between samples in different clusters. However, as stated on page 215 of Duda 1973, clustering does not require the use of a distance metric. For example, a nonmetric similarity function $s(x, x')$ can be used to compare two vectors x and x'. Conventionally, $s(x, x')$ is a symmetric function whose value is large when x and x' are somehow "similar." An example of a nonmetric similarity function $s(x, x')$ is provided on page 216 of Duda 1973.

Once a method for measuring "similarity" or "dissimilarity" between points in a dataset has been selected, clustering makes use of a criterion function that measures the clustering quality of any partition of the data. Partitions of the dataset that extremize the criterion function are used to cluster the data. See page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques suitable for use as classifiers are disclosed in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, N.J. Particular exemplary clustering techniques that can be used as classifiers include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering.

In some embodiments, a classifier is a nearest neighbor algorithm. For nearest neighbors, given a query point $x_0$ (a test subject), the k training points $x_{(r)}$, r, ... , k (here the training subjects) closest in distance to $x_0$ are identified and then the point $x_0$ is classified using the k nearest neighbors. Here, the distance to these neighbors is a function of the abundance values of the discriminating gene set. In some embodiments, Euclidean distance in feature space is used to determine distance as $d_{(i)} = ||x_{(i)} - x_{(o)}||$. Typically, when the nearest neighbor algorithm is used, the abundance data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York.

Cohorts and Clinical Trials:

In some embodiments, a plurality of biological subjects is a clinical cohort, *e.g.,* a group of participants in a clinical trial or study. In some embodiments, the plurality of subjects in the cohort comprises at least 10, at least 20, at least 30, at least 40, at least 50, at least 100, at least 200, at least 500, at least 1000, at least 2000, or at least 5000 subjects.

In some embodiments, each of the subjects in the plurality of biological subjects (*e.g.,* cohort) has a particular cancer with the same origin (*e.g.,* stomach cancer). In some embodiments, each of the subjects in the plurality of biological subjects has a cancer that is associated with a set of cancers.

In some embodiments, the plurality of biological subjects comprises one or more subsets of subjects with a respective cancer condition, where the respective cancer condition is not represented in any other subset of subjects in the plurality of subjects. For example, in some embodiments, a first subject (and/or a subset of subjects) in a plurality of subjects has a first cancer condition, and a second subject (and/or a subset of subjects) in a plurality of subjects has a second cancer condition.

In some embodiments, the first subset of subjects and the second subset of subjects each comprise the same number of subjects. For example, in some such embodiments, the plurality of subjects comprises one hundred subjects, the first subset of subjects (*e.g.,* with the first cancer condition) comprises twenty subjects, and the second subset of subjects (*e.g.,* with the second cancer condition) comprises twenty subjects. In other embodiments, the plurality of subjects comprises one thousand subjects, the first subset of subjects comprises one hundred subjects, and the second subset of subjects comprises one hundred subjects.

In some alternative embodiments, the first subset of subjects and the second subset of subjects comprise different numbers of subjects (*e.g.,* a greater number of subjects in the plurality of subjects have the first cancer condition than the second cancer condition). For instance, in some embodiments, more than ten percent, more than twenty percent, more than thirty percent, more than forty percent, more than fifty percent, more than sixty percent, more than seventy percent, more than eighty percent, or more than ninety percent of the subjects in the plurality of subjects have the first cancer condition while the remainder have the second cancer condition.

Training and Test Subjects:

In some embodiments, a plurality of subjects (*e.g.,* a cohort) is of sufficient size to develop a classifier that has suitable performance for screening subjects to ascertain whether they have a first or second cancer condition (see Cohorts, above).

In some embodiments, a plurality of subjects comprises training subjects (*e.g.,* subjects for which the cancer condition is known). In some embodiments, training subjects can be used to train a classifier to detect or distinguish a cancer condition.

In some embodiments, a plurality of subjects comprises test subjects (*e.g.,* subjects for which the cancer condition is unknown). For example, in typical instances, a test subject is a subject for which it has not been confirmed whether the subject has a first or second cancer condition.

In some such embodiments, a trained classifier is used to classify a test subject (*e.g.,* by detecting or distinguishing the cancer condition of the test subject). In some such embodiments, a test subject is a subject that was not used to train the classifier.

**Example Methods - part of the invention**

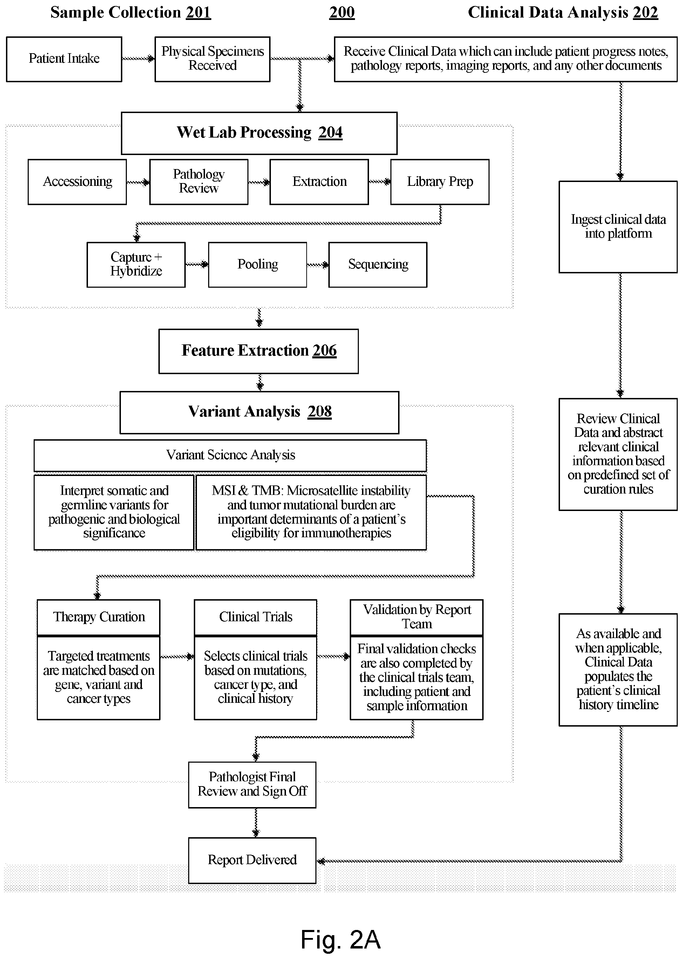

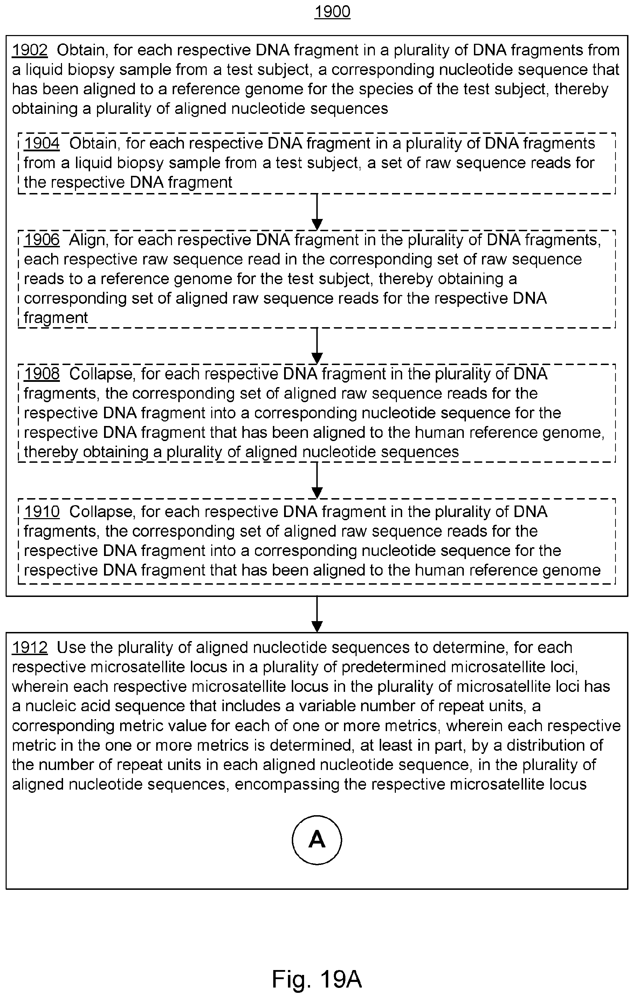

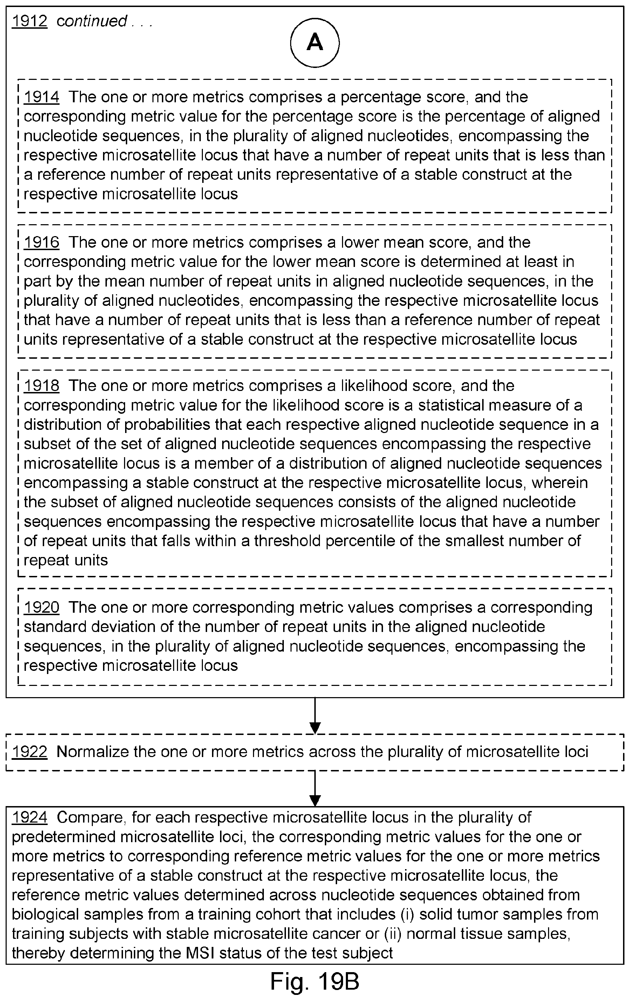

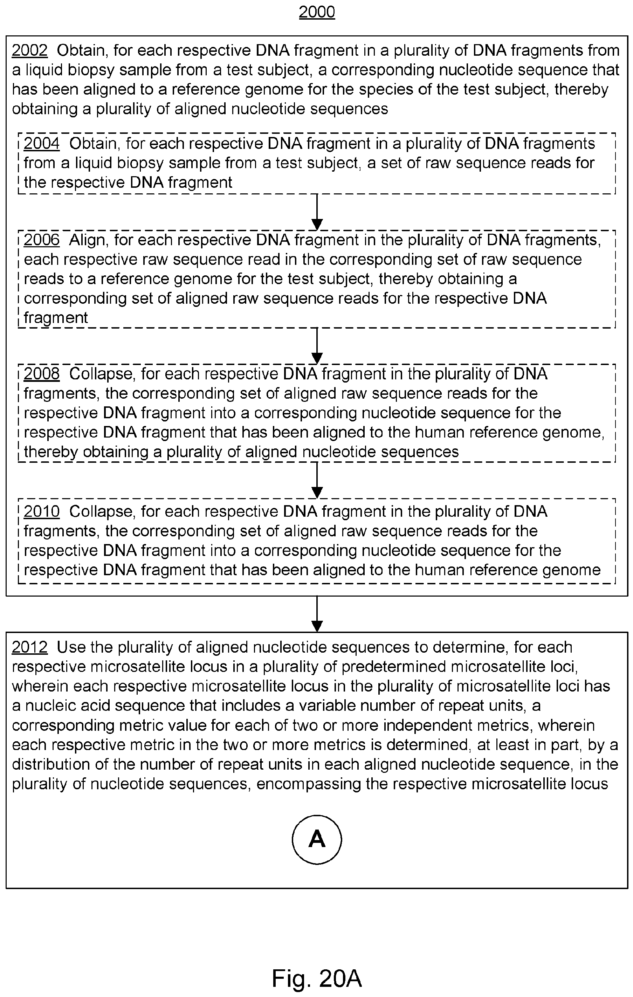

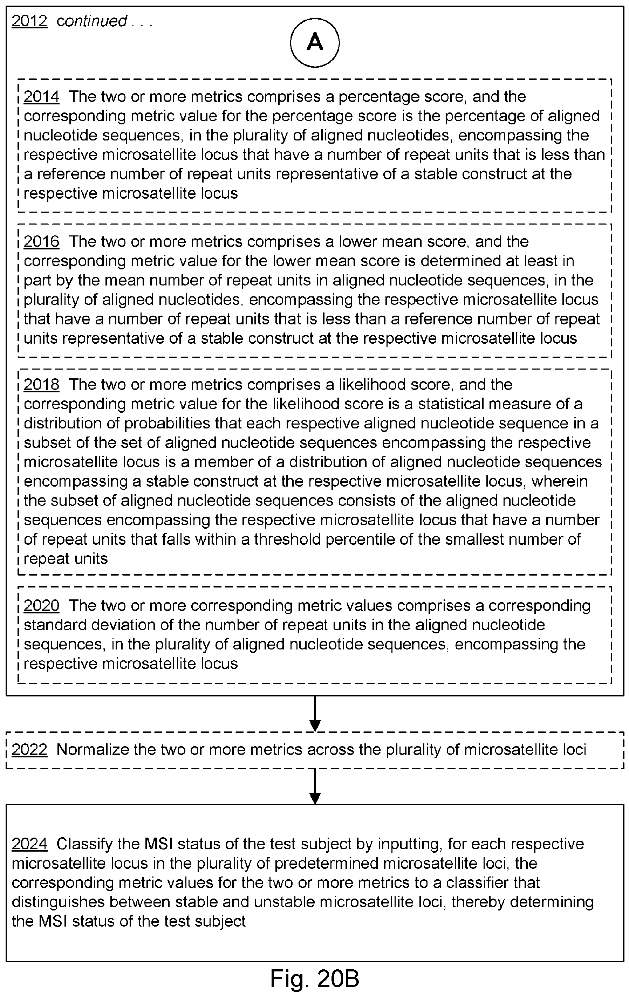

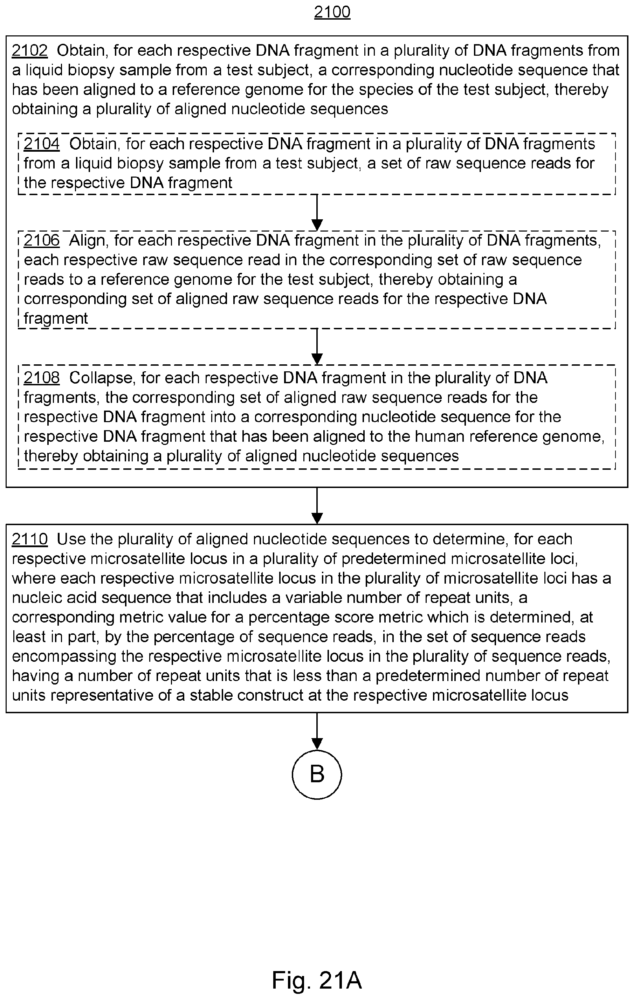

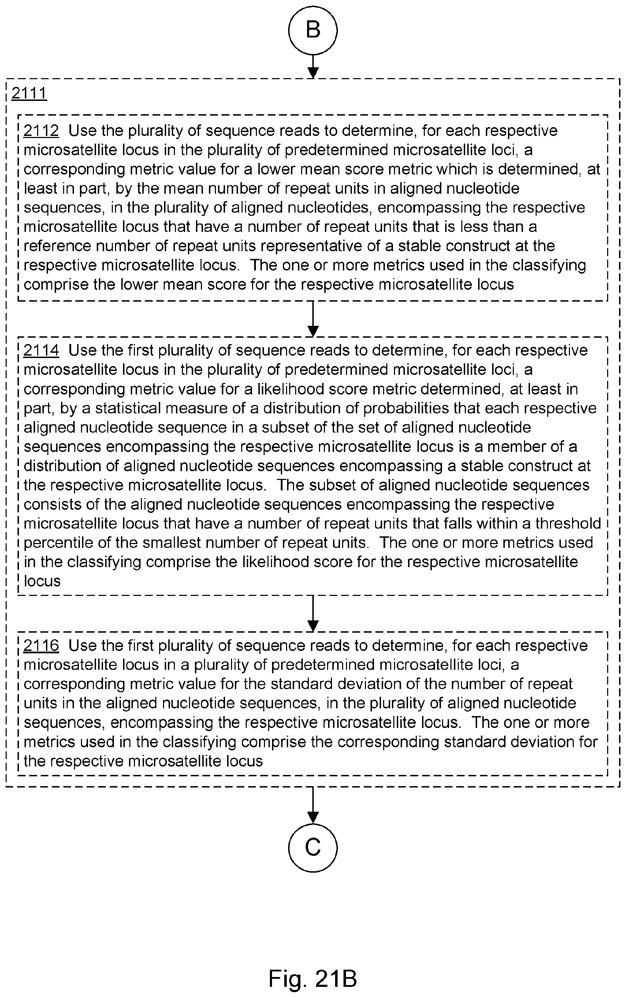

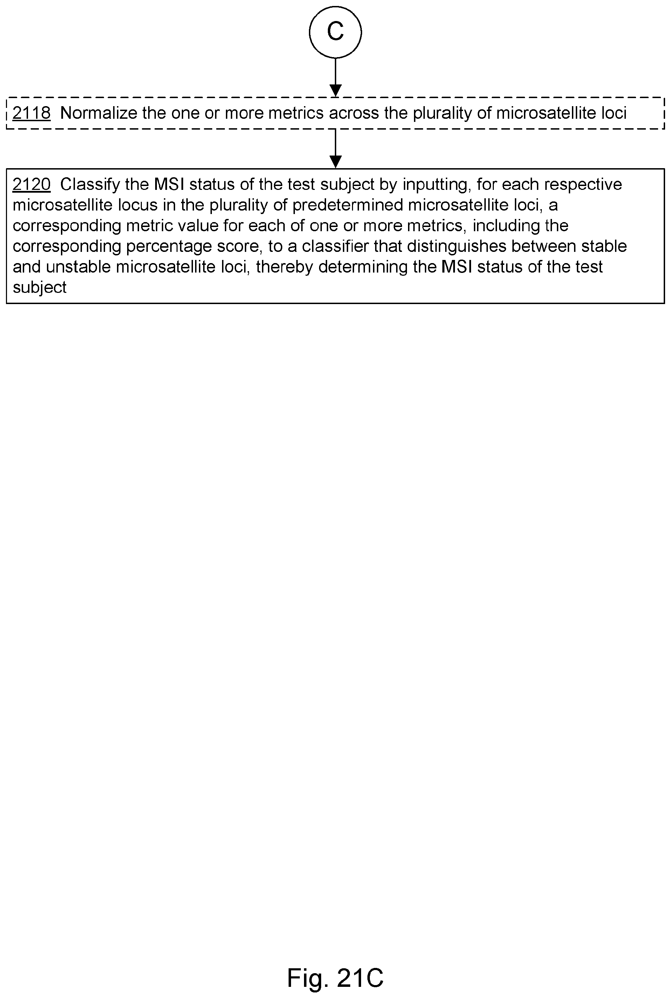

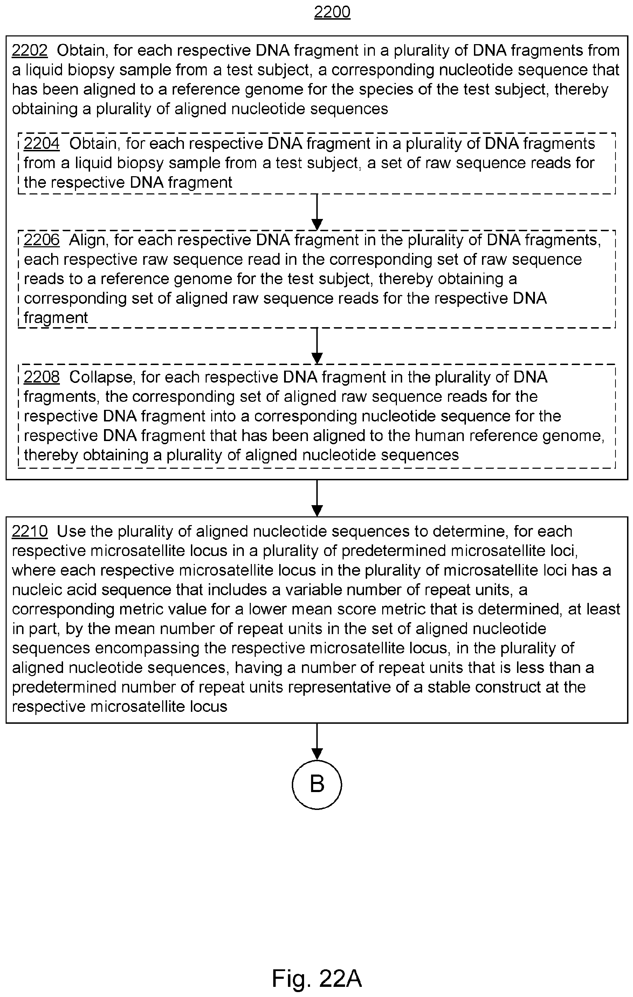

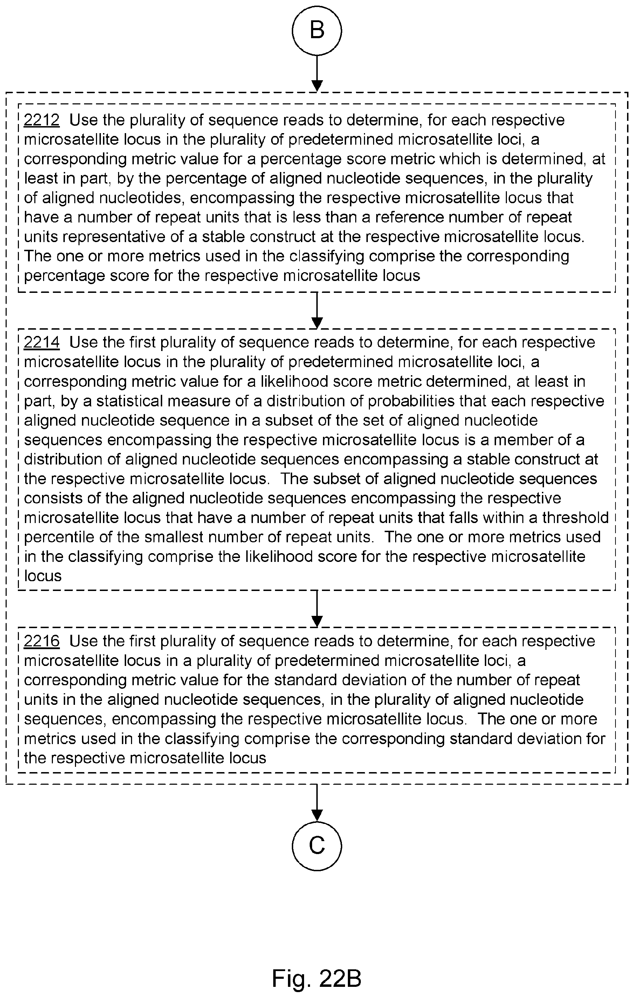

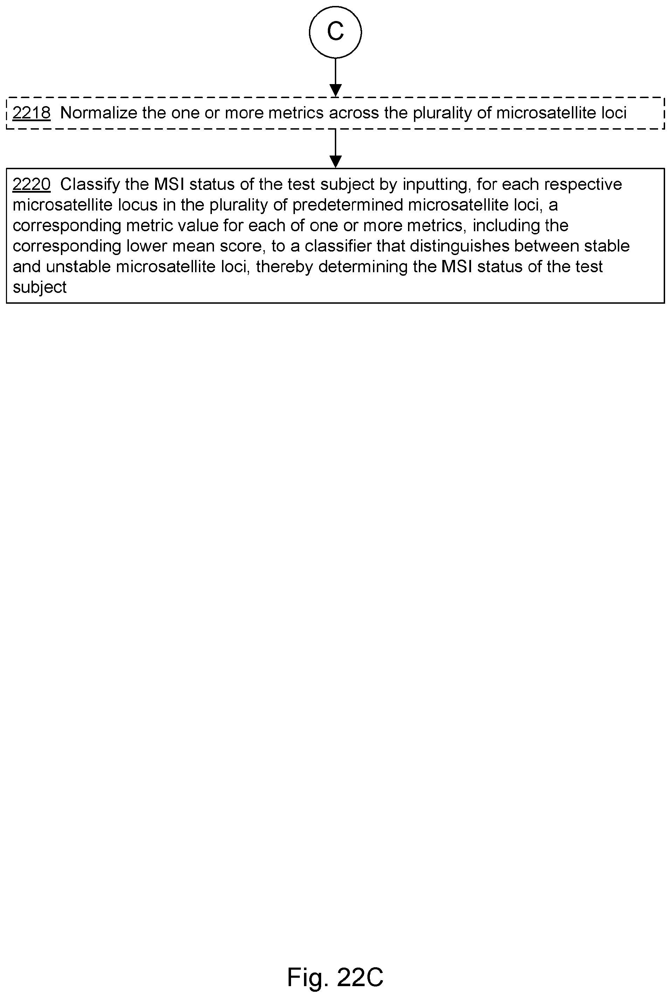

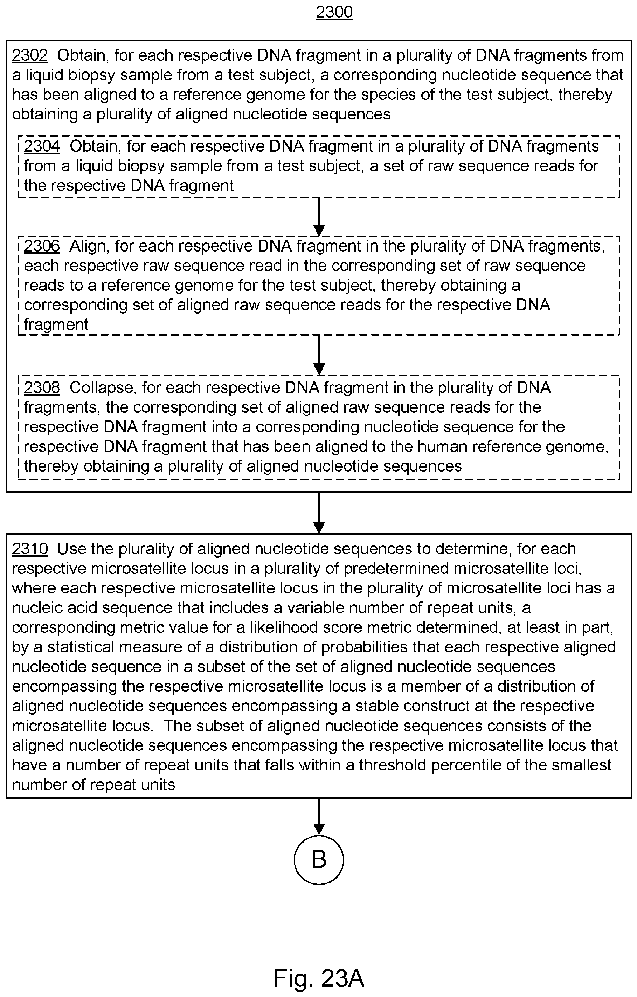

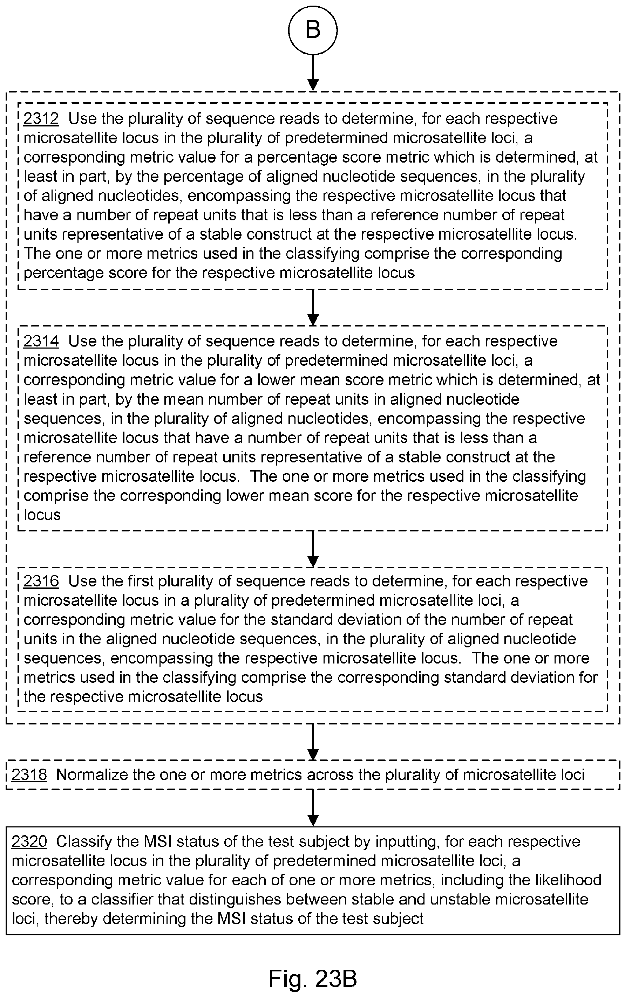

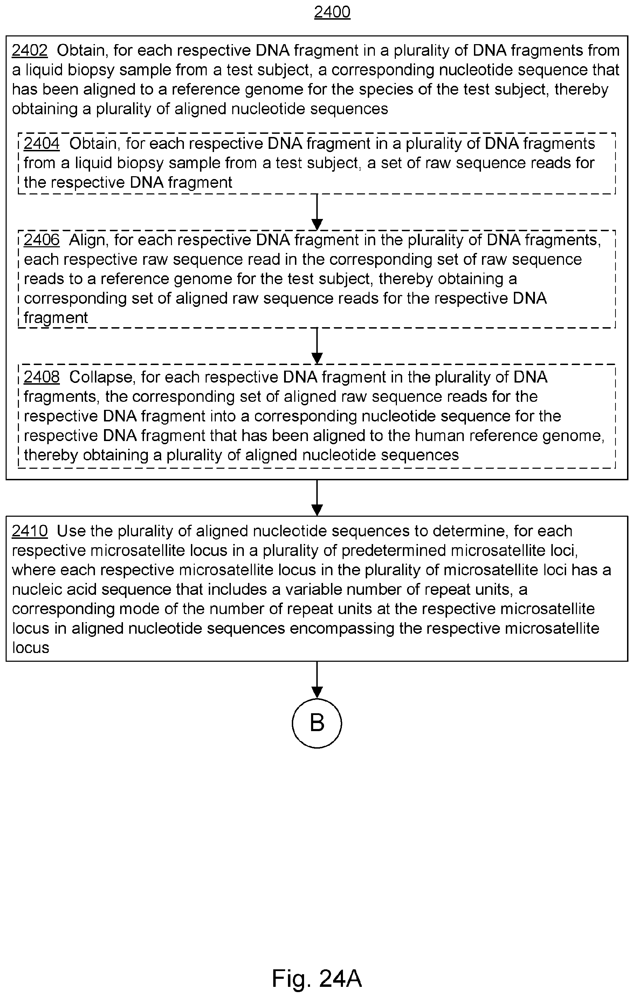

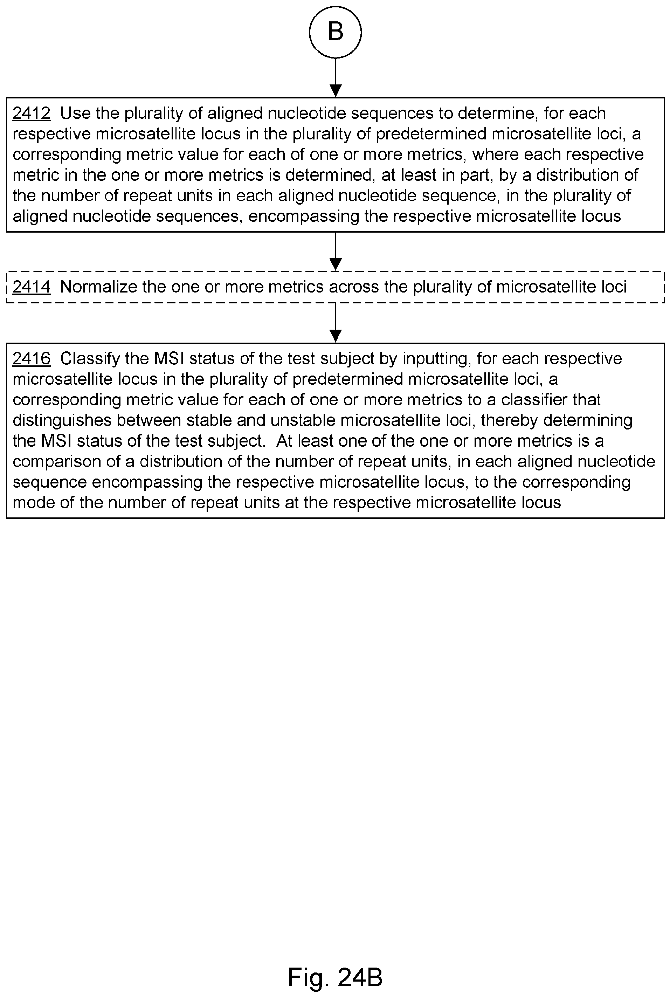

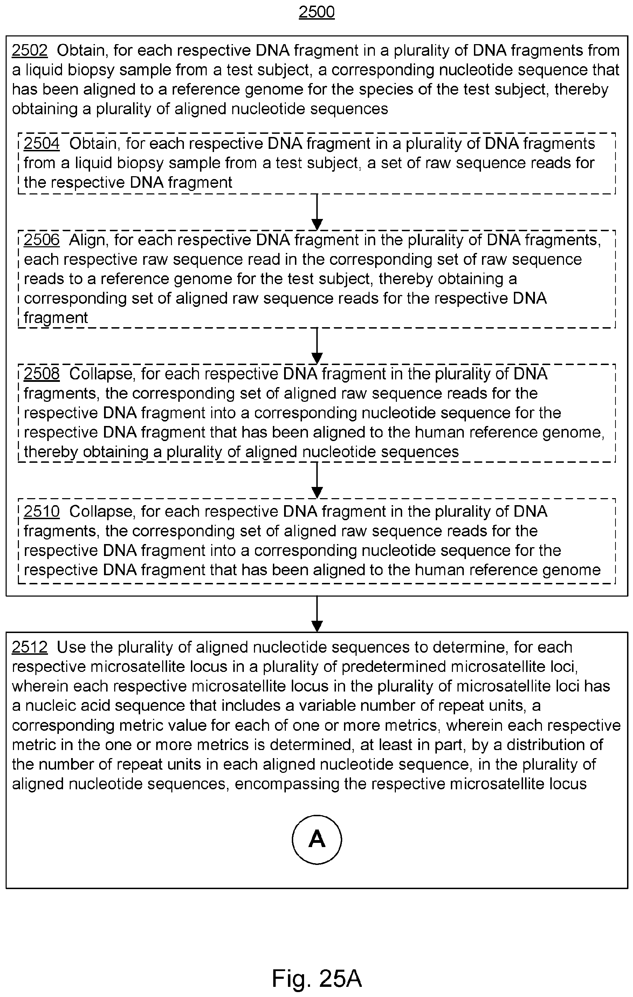

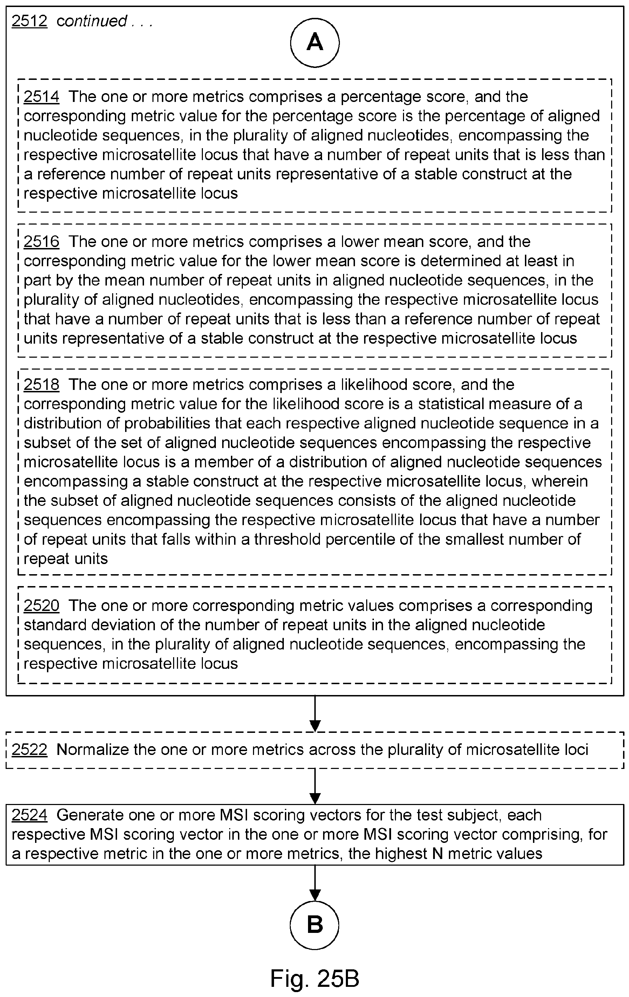

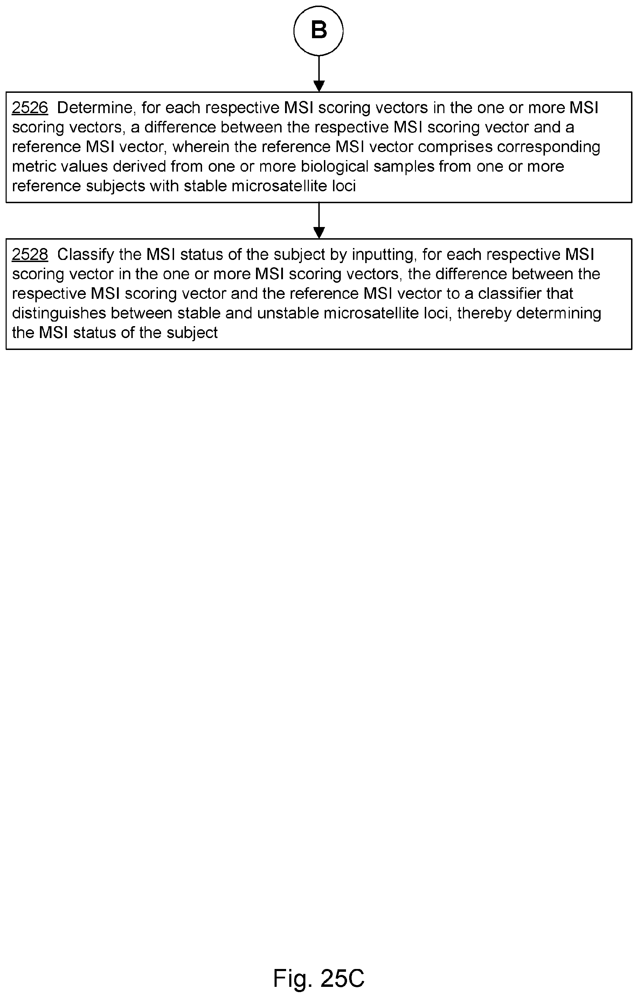

Now that details of a system 100 for providing clinical support for personalized cancer therapy, *e.g.,* with improved classification of microsatellite stability status, have been disclosed, details regarding processes and features of the system, in accordance with various embodiments of the present disclosure, are disclosed below. Specifically, example processes are described below with reference to Figures 2A, 3, 4A-4C, 19A-19B, 20A-20B, 21A-21C, 22A-22C, 23A-23B, 24A-24B, and 25A-25C. In some embodiments, such processes and features of the system are carried out by modules 118, 120, 140, 160, and/or 170, as illustrated in Figure 1A. Referring to these methods, the systems described herein (*e.g.,* system 100) include instructions for determining microsatellite stability status that are improved compared to conventional methods for determining microsatellite stability status.

Figure 2B: Distributed Diagnostic and Clinical Environment

The methods described herein for providing clinical support for personalized cancer therapy may be performed across a distributed diagnostic/clinical environment, *e.g.,* as illustrated in Figure 2B. However, the improved methods described herein for determining microsatellite stability status, may be performed at a single location, *e.g.,* at a single computing system or environment, although ancillary procedures supporting the methods described herein, and/or procedures that make further use of the results of the methods described herein, may be performed across a distributed diagnostic/clinical environment.

Figure 2B illustrates an example of a distributed diagnostic/clinical environment 210. The distributed diagnostic/clinical environment may be connected via communication network 105. One or more biological samples, *e.g.,* one or more liquid biopsy samples, solid tumor biopsy, normal tissue samples, and/or control samples, may be collected from a subject in clinical environment 220, *e.g.,* a doctor's office, hospital, or medical clinic, or at a home health care environment (not depicted). Advantageously, while solid tumor samples should be collected within a clinical setting, liquid biopsy samples can be acquired in a less invasive fashion and are more easily collected outside of a traditional clinical setting. One or more biological samples, or portions thereof, may be processed within the clinical environment 220 where collection occurred, using a processing device 224, *e.g.,* a nucleic acid sequencer for obtaining sequencing data, a microscope for obtaining pathology data, a mass spectrometer for obtaining proteomic data, *etc.* One or more biological samples, or portions thereof may be sent to one or more external environments, *e.g.,* sequencing lab 230, pathology lab 240, and/or molecular biology lab 250, each of which includes a processing device 234, 244, and 254, respectively, to generate biological data 121 for the subject. Each environment includes a communications device 222, 232, 242, and 252, respectively, for communicating biological data 121 about the subject to a processing server 262 and/or database 264, which may be located in yet another environment, *e.g.,* processing/storage center 260. Thus, different portions of the systems and methods described herein may be fulfilled by different processing devices located in different physical environments.

Accordingly, a method for providing clinical support for personalized cancer therapy, *e.g.,* with improved microsatellite stability status classification, may be performed across one or more environments, as illustrated in Figure 2B. For instance, a liquid biopsy sample is collected at clinical environment 220 or in a home healthcare environment. The sample, or a portion thereof, is sent to sequencing lab 230 where raw sequence reads 123 of nucleic acids in the sample are generated by sequencer 234. The raw sequencing data 123 is communicated, *e.g.,* from communications device 232, to database 264 at processing/storage center 260, where processing server 262 extracts features from the sequence reads by executing one or more of the processes in bioinformatics module 140, thereby generating genomic features 131 for the sample. Processing server 262 may then analyze the identified features by executing one or more of the processes in feature analysis module 160, thereby generating clinical assessment 139, including a clinical report 139-3. A clinician may access clinical report 139-3, *e.g.,* at processing/storage center 260 or through communications network 105, via recommendation validation module 167. After final approval, clinical report 139-3 is transmitted to a medical professional, *e.g.,* an oncologist, at clinical environment 220, who uses the report to support clinical decision making for personalized treatment of the patient's cancer.

Figure 2A: Example Workflow for Precision Oncology

Figure 2A is a flowchart of an example workflow 200 for collecting and analyzing data in order to generate a clinical report 139 to support clinical decision making in precision oncology. Advantageously, the methods described herein improve this process, for example, by improving various stages within feature extraction 206, including microsatellite stability status classification.

Briefly, the workflow begins with patient intake and sample collection 201, where one or more liquid biopsy samples, one or more tumor biopsy, and one or more normal and/or control tissue samples are collected from the patient *(e.g.,* at a clinical environment 220 or home healthcare environment, as illustrated in Figure 2B). In some embodiments, personal data 126 corresponding to the patient and a record of the one or more biological samples obtained (*e.g.,* patient identifiers, patient clinical data, sample type, sample identifiers, cancer conditions, *etc.)* are entered into a data analysis platform, *e.g.,* test patient data store 120. Accordingly, the methods disclosed herein include obtaining one or more biological samples from one or more subjects, *e.g.,* cancer patients. Obtaining the biological sample is not part of the invention but described for illustrative purposes only. In some embodiments, the subject is a human, *e.g.,* a human cancer patient.

In some embodiments, one or more of the biological samples obtained from the patient are a biological liquid sample, also referred to as a liquid biopsy sample. In some embodiments, one or more of the biological samples obtained from the patient are selected from blood, plasma, serum, urine, vaginal fluid, fluid from a hydrocele *(e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body *(e.g.,* thyroid, breast), *etc.* In some embodiments, the liquid biopsy sample includes blood and/or saliva. In some embodiments, the liquid biopsy sample is peripheral blood. In some embodiments, blood samples are collected from patients in commercial blood collection containers, *e.g.,* using a PAXgene® Blood DNA Tubes. In some embodiments, saliva samples are collected from patients in commercial saliva collection containers, *e.g.,* using an Oragene® DNA Saliva Kit.

In some embodiments, the liquid biopsy sample has a volume of from about 1 mL to about 50 mL. For example, in some embodiments, the liquid biopsy sample has a volume of about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 16 mL, about 17 mL, about 18 mL, about 19 mL, about 20 mL, or greater.

Liquid biopsy samples include cell free nucleic acids, including cell-free DNA (cfDNA). As described above, cfDNA isolated from cancer patients includes DNA originating from cancerous cells, also referred to as circulating tumor DNA (ctDNA), cfDNA originating from germline *(e.g.,* healthy or non-cancerous) cells, and cfDNA originating from hematopoietic cells *(e.g.,* white blood cells). The relative proportions of cancerous and non-cancerous cfDNA present in a liquid biopsy sample varies depending on the characteristics *(e.g.,* the type, stage, lineage, genomic profile, *etc.)* of the patient's cancer. As used herein, the 'tumor burden' of the subject refers to the percentage cfDNA that originated from cancerous cells.

As described herein, cfDNA is a particularly useful source of biological data for various implementations of the methods and systems described herein, because it is readily obtained from various body fluids. Advantageously, use of bodily fluids facilitates serial monitoring because of the ease of collection, as these fluids are collectable by non-invasive or minimally-invasive methodologies. This is in contrast to methods that rely upon solid tissue samples, such as biopsies, which often times require invasive surgical procedures. Further, because bodily fluids, such as blood, circulate throughout the body, the cfDNA population represents a sampling of many different tissue types from many different locations.

In some embodiments, a liquid biopsy sample is separated into two different samples. For example in some embodiments, a blood sample is separated into a blood plasma sample, containing cfDNA, and a buffy coat preparation, containing white blood cells.

A plurality of liquid biopsy samples may be obtained from a respective subject at intervals over a period of time *(e.g.,* using serial testing). For example, the time between obtaining liquid biopsy samples from a respective subject is at least 1 day, at least 2 days, at least 1 week, at least 2 weeks, at least 1 month, at least 2 months, at least 3 months, at least 4 months, at least 6 months, or at least 1 year.

One or more biological samples collected from the patient is a solid tissue sample, *e.g.,* a solid tumor sample or a solid normal tissue sample. Methods for obtaining solid tissue samples, *e.g.,* of cancerous and/or normal tissue are known in the art, and are dependent upon the type of tissue being sampled and are not part of the invention but described for illustrative purposes only. For example, bone marrow biopsies and isolation of circulating tumor cells can be used to obtain samples of blood cancers, endoscopic biopsies can be used to obtain samples of cancers of the digestive tract, bladder, and lungs, needle biopsies *(e.g.,* fine-needle aspiration, core needle aspiration, vacuum-assisted biopsy, and image-guided biopsy, can be used to obtain samples of subdermal tumors, skin biopsies, *e.g.,* shave biopsy, punch biopsy, incisional biopsy, and excisional biopsy, can be used to obtain samples of dermal cancers, and surgical biopsies can be used to obtain samples of cancers affecting internal organs of a patient. A solid tissue sample is a formalin-fixed tissue (FFT). A solid tissue sample is a macro-dissected formalin fixed paraffin embedded (FFPE) tissue. A solid tissue sample is a fresh frozen tissue sample.

In some embodiments, a dedicated normal sample is collected from the patient, for co-processing with a liquid biopsy sample. Generally, the normal sample is of a non-cancerous tissue, and can be collected using any tissue collection means described above. In some embodiments, buccal cells collected from the inside of a patient's cheeks are used as a normal sample. Buccal cells can be collected by placing an absorbent material, *e.g.,* a swab, in the subjects mouth and rubbing it against their cheek, *e.g.,* for at least 15 second or for at least 30 seconds. The swab is then removed from the patient's mouth and inserted into a tube, such that the tip of the tube is submerged into a liquid that serves to extract the buccal cells off of the absorbent material. An example of buccal cell recovery and collection devices is provided in U.S. Patent No. 9,138,205. In some embodiments, the buccal swab DNA is used as a source of normal DNA in circulating heme malignancies.

The biological samples collected from the patient are, optionally, sent to various analytical environments (e.g., sequencing lab 230, pathology lab 240, and/or molecular biology lab 250) for processing *(e.g.,* data collection) and/or analysis *(e.g.,* feature extraction). Wet lab processing 204 may include cataloguing samples *(e.g.,* accessioning), examining clinical features of one or more samples *(e.g.,* pathology review), and nucleic acid sequence analysis *(e.g.,* extraction, library prep, capture + hybridize, pooling, and sequencing). In some embodiments, the workflow includes clinical analysis of one or more biological samples collected from the subject, *e.g.*, at a pathology lab 240 and/or a molecular and cellular biology lab 250, to generate clinical features such as pathology features 128-3, imaging data 128-3, and/or tissue culture / organoid data 128-3.

The pathology data 128-1 collected during clinical evaluation includes visual features identified by a pathologist's inspection of a specimen (*e.g.*, a solid tumor biopsy), *e.g.*, of stained H&E or IHC slides. The sample may be a solid tissue biopsy sample. The tissue biopsy sample may be a formalin-fixed tissue (FFT), *e.g.*, a formalin-fixed paraffin-embedded (FFPE) tissue. The tissue biopsy sample may be an FFPE or FFT block. The tissue biopsy sample may be a fresh-frozen tissue biopsy. The tissue biopsy sample can be prepared in thin sections (*e.g.*, by cutting and/or affixing to a slide), to facilitate pathology review (*e.g.*, by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

In some embodiments, a liquid sample (*e.g.*, blood) collected from the patient (*e.g.*, in EDTA-containing collection tubes) is prepared on a slide (*e.g.*, by smearing) for pathology review. In some embodiments, macrodissected FFPE tissue sections, which may be mounted on a histopathology slide, from solid tissue samples (*e.g.*, tumor or normal tissue) are analyzed by pathologists. In some embodiments, tumor samples are evaluated to determine, *e.g.*, the tumor purity of the sample, the percent tumor cellularity as a ratio of tumor to normal nuclei, *etc.*. For each section, background tissue may be excluded or removed such that the section meets a tumor purity threshold, *e.g.*, where at least 20% of the nuclei in the section are tumor nuclei, or where at least 25%, 30%, 40%, 50%, 60%, 70%, 80%, 90%, or more of the nuclei in the section are tumor nuclei.

In some embodiments, pathology data 128-1 is extracted, in addition to or instead of visual inspection, using computational approaches to digital pathology, *e.g.*, providing morphometric features extracted from digital images of stained tissue samples. A review of digital pathology methods is provided in Bera, K. et al., Nat. Rev. Clin. Oncol., 16:703-15 (2019). In some embodiments, pathology data 128-1 includes features determined using machine learning algorithms to evaluate pathology data collected as described above.

Further details on methods, systems, and algorithms for using pathology data to classify cancer and identify targeted therapies are discussed, for example, in are discussed, for example, in U.S. Patent Application No. 16/830,186, filed on March 25, 2020, and U.S. Provisional Application No. 63/007,874, filed on April. 9, 2020.

In some embodiments, imaging data 128-2 collected during clinical evaluation includes features identified by review of in-vitro and/or in-vivo imaging results (*e.g.*, of a tumor site), for example a size of a tumor, tumor size differentials over time (such as during treatment or during other periods of change). In some embodiments, imaging data 128-2 includes features determined using machine learning algorithms to evaluate imaging data collected as described above.

Further details on methods, systems, and algorithms for using medical imaging to classify cancer and identify targeted therapies are discussed, for example, in are discussed, for example, in U.S. Patent Application No. 16/830,186, filed on March 25, 2020, and U.S. Provisional Application No. 63/007,874, filed on April. 9, 2020.

In some embodiments, tissue culture / organoid data 128-3 collected during clinical evaluation includes features identified by evaluation of cultured tissue from the subject. For instance, in some embodiments, tissue samples obtained from the patients (*e.g.*, tumor tissue, normal tissue, or both) are cultured (*e.g.*, in liquid culture, solid-phase culture, and/or organoid culture) and various features, such as cell morphology, growth characteristics, genomic alterations, and/or drug sensitivity, are evaluated. In some embodiments, tissue culture / organoid data 128-3 includes features determined using machine learning algorithms to evaluate tissue culture / organoid data collected as described above. Examples of tissue organoid (*e.g.*, personal tumor organoid) culturing and feature extractions thereof are described in U.S. Provisional Application Serial No. 62/924,621, filed on October 22, 2019, and U.S. Patent Application Serial No. 16/693,117, filed on November 22, 2019.

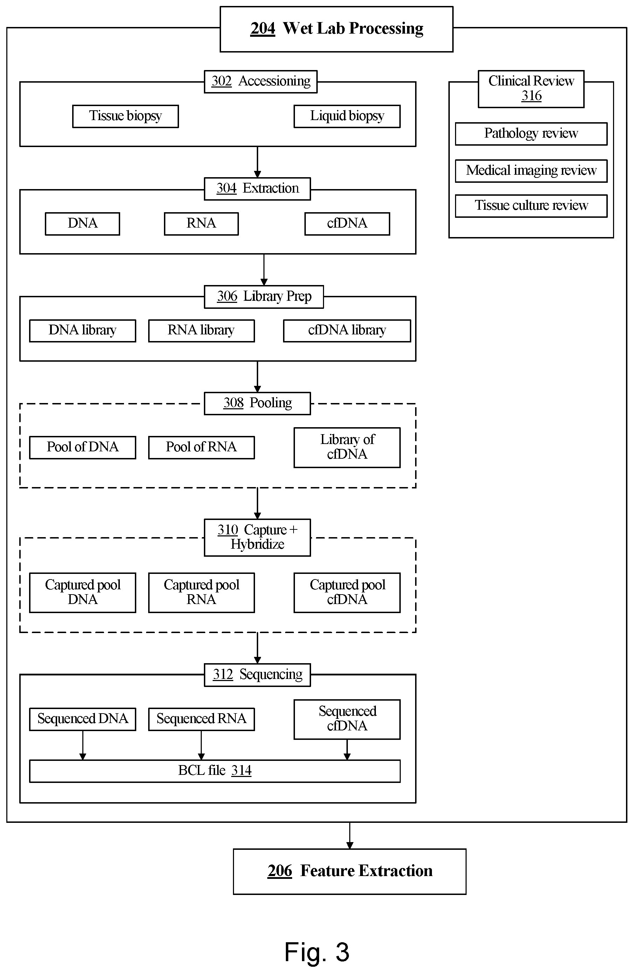

Nucleic acid sequencing of one or more samples collected from the subject is performed, *e.g.*, at sequencing lab 230, during wet lab processing 204. An example workflow for nucleic acid sequencing is illustrated in Figure 3. In some embodiments, the one or more biological samples obtained at the sequencing lab 230 are accessioned (302), to track the sample and data through the sequencing process.

Next, nucleic acids, *e.g.*, RNA and/or DNA are extracted (304) from the one or more biological samples. Methods for isolating nucleic acids from biological samples are known in the art, and are dependent upon the type of nucleic acid being isolated (*e.g.*, cfDNA, DNA, and/or RNA) and the type of sample from which the nucleic acids are being isolated (*e.g.*, liquid biopsy samples, white blood cell buffy coat preparations, formalin-fixed paraffin-embedded (FFPE) solid tissue samples, and fresh frozen solid tissue samples). The selection of any particular nucleic acid isolation technique for use in conjunction with the embodiments described herein is well within the skill of the person having ordinary skill in the art, who will consider the sample type, the state of the sample, the type of nucleic acid being sequenced and the sequencing technology being used.

For instance, many techniques for DNA isolation, *e.g.*, genomic DNA isolation, from a tissue sample are known in the art, such as organic extraction, silica adsorption, and anion exchange chromatography. Likewise, many techniques for RNA isolation, *e.g.*, mRNA isolation, from a tissue sample are known in the art. For example, acid guanidinium thiocyanate-phenolchloroform extraction (see, for example, Chomczynski and Sacchi, 2006, Nat Protoc, 1(2):581-85), and silica bead/glass fiber adsorption (see, for example, Poeckh, T. et al., 2008, Anal Biochem., 373(2):253-62). The selection of any particular DNA or RNA isolation technique for use in conjunction with the embodiments described herein is well within the skill of the person having ordinary skill in the art, who will consider the tissue type, the state of the tissue, *e.g.*, fresh, frozen, formalin-fixed, paraffin-embedded (FFPE), and the type of nucleic acid analysis that is to be performed.

According to the invention, the biological sample is a liquid biopsy sample, *e.g.*, a blood or blood plasma sample, cfDNA is isolated from blood samples using commercially available reagents, including proteinase K, to generate a liquid solution of cfDNA.

In some embodiments, isolated DNA molecules are mechanically sheared to an average length using an ultrasonicator (for example, a Covaris ultrasonicator). In some embodiments, isolated nucleic acid molecules are analyzed to determine their fragment size, *e.g.*, through gel electrophoresis techniques and/or the use of a device such as a LabChip GX Touch. The skilled artisan will know of an appropriate range of fragment sizes, based on the sequencing technique being employed, as different sequencing techniques have differing fragment size requirements for robust sequencing. In some embodiments, quality control testing is performed on the extracted nucleic acids (*e.g.*, DNA and/or RNA), *e.g.*, to assess the nucleic acid concentration and/or fragment size. For example, sizing of DNA fragments provides valuable information used for downstream processing, such as determining whether DNA fragments require additional shearing prior to sequencing.

Wet lab processing 204 then includes preparing a nucleic acid library from the isolated nucleic acids (*e.g.*, cfDNA, DNA, and/or RNA). For example, in some embodiments, DNA libraries (*e.g.*, gDNA and/or cfDNA libraries) are prepared from isolated DNA from the one or more biological samples. In some embodiments, the DNA libraries are prepared using a commercial library preparation kit, *e.g.*, the KAPA Hyper Prep Kit, a New England Biolabs (NEB) kit, or a similar kit.

In some embodiments, during library preparation, adapters (*e.g.*, UDI adapters, such as Roche SeqCap dual end adapters, or UMI adapters such as full length or stubby Y adapters) are ligated onto the nucleic acid molecules. In some embodiments, the adapters include unique molecular identifiers (UMIs), which are short nucleic acid sequences (*e.g.*, 3-10 base pairs) that are added to ends of DNA fragments during adapter ligation. In some embodiments, UMIs are degenerate base pairs that serve as a unique tag that can be used to identify sequence reads originating from a specific DNA fragment. In some embodiments, *e.g.*, when multiplex sequencing will be used to sequence DNA from a plurality of samples (*e.g.*, from the same or different subjects) in a single sequencing reaction, a patient-specific index is also added to the nucleic acid molecules. In some embodiments, the patient specific index is a short nucleic acid sequence (*e.g.*, 3-20 nucleotides) that are added to ends of DNA fragments during library construction, that serve as a unique tag that can be used to identify sequence reads originating from a specific patient sample. Examples of identifier sequences are described, for example, in Kivioja et al., Nat. Methods 9(1):72-74 (2011) and Islam et al., Nat. Methods 11(2):163-66 (2014).

In some embodiments, an adapter includes a PCR primer landing site, designed for efficient binding of a PCR or second-strand synthesis primer used during the sequencing reaction. In some embodiments, an adapter includes an anchor binding site, to facilitate binding of the DNA molecule to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached DNA fragment. This provides a way to identify sequence reads that came from the same original fragment in downstream analysis.

In some embodiments, DNA libraries are amplified and purified using commercial reagents, (e.g., Axygen MAG PCR clean up beads). In some such embodiments, the concentration and/or quantity of the DNA molecules are then quantified using a fluorescent dye and a fluorescence microplate reader, standard spectrofluorometer, or filter fluorometer. In some embodiments, library amplification is performed on a device (*e.g.*, an Illumina C-Bot2) and the resulting flow cell containing amplified target-captured DNA libraries is sequenced on a next generation sequencer (*e.g.*, an Illumina HiSeq 4000 or an Illumina NovaSeq 6000) to a unique on-target depth selected by the user. In some embodiments, DNA library preparation is performed with an automated system, using a liquid handling robot (*e.g.*, a SciClone NGSx).

In some embodiments, where feature data 125 includes methylation states 132 for one or more genomic locations, nucleic acids isolated from the biological sample (*e.g.*, cfDNA) are treated to convert unmethylated cytosines to uracils, *e.g.*, prior to generating the sequencing library. Accordingly, when the nucleic acids are sequenced, all cytosines called in the sequencing reaction were necessarily methylated, since the unmethylated cytosines were converted to uracils and accordingly would have been called as thymidines, rather than cytosines, in the sequencing reaction. Commercial kits are available for bisulfite-mediated conversion of methylated cytosines to uracils, for instance, the EZ DNA Methylation™-Gold, EZ DNA Methylation™-Direct, and EZ DNA Methylation™-Lightning kit (available from Zymo Research Corp (Irvine, CA)). Commercial kits are also available for enzymatic conversion of methylated cytosines to uracils, for example, the APOBEC-Seq kit (available from NEBiolabs, Ipswich, MA).

In some embodiments, wet lab processing 204 includes pooling (308) DNA molecules from a plurality of libraries, corresponding to different samples from the same and/or different patients, to forming a sequencing pool of DNA libraries. When the pool of DNA libraries is sequenced, the resulting sequence reads correspond to nucleic acids isolated from multiple samples. The sequence reads can be separated into different sequence read files, corresponding to the various samples represented in the sequencing read based on the unique identifiers present in the added nucleic acid fragments. In this fashion, a single sequencing reaction can generate sequence reads from multiple samples. Advantageously, this allows for the processing of more samples per sequencing reaction.

In some embodiments, wet lab processing 204 includes enriching (310) a sequencing library, or pool of sequencing libraries, for target nucleic acids, *e.g.,* nucleic acids encompassing loci that are informative for precision oncology and/or used as internal controls for the sequencing or bioinformatics processes. In some embodiments, enrichment is achieved by hybridizing target nucleic acids in the sequencing library to probes that hybridize to the target sequences, and then isolating the captured nucleic acids away from off-target nucleic acids that are not bound by the capture probes.

Advantageously, enriching for target sequences prior to sequencing nucleic acids significantly reduces the costs and time associated with sequencing, facilitates multiplex sequencing by allowing multiple samples to be mixed together for a single sequencing reaction, and significantly reduces the computation burden of aligning the resulting sequence reads, as a result of significantly reducing the total amount of nucleic acids analyzed from each sample.

In some embodiments, the enrichment is performed prior to pooling multiple nucleic acid sequencing libraries. However, in other embodiments, the enrichment is performed after pooling nucleic acid sequencing libraries, which has the advantage of reducing the number of enrichment assays that have to be performed.

In some embodiments, the enrichment is performed prior to generating a nucleic acid sequencing library. This has the advantage that fewer reagents are needed to perform both the enrichment (because there are fewer target sequences at this point, prior to library amplification) and the library production (because there are fewer nucleic acid molecules to tag and amplify after the enrichment). However, this raises the possibility of pull-down bias and/or that small variations in the enrichment protocol will result in less consistent results.

In some embodiments, nucleic acid libraries are pooled (two or more DNA libraries may be mixed to create a pool) and treated with reagents to reduce off-target capture, for example Human COT-1 and/or IDT xGen Universal Blockers. Pools may be dried in a vacufuge and resuspended. DNA libraries or pools may be hybridized to a probe set (for example, a probe set specific to a panel that includes loci from at least 100, 600, 1,000, 10,000, *etc.* of the 19,000 known human genes) and amplified with commercially available reagents (for example, the KAPA HiFi HotStart ReadyMix). For example, in some embodiments, a pool is incubated in an incubator, PCR machine, water bath, or other temperature-modulating device to allow probes to hybridize. Pools may then be mixed with Streptavidin-coated beads or another means for capturing hybridized DNA-probe molecules, such as DNA molecules representing exons of the human genome and/or genes selected for a genetic panel.

Pools may be amplified and purified more than once using commercially available reagents, for example, the KAPA HiFi Library Amplification kit and Axygen MAG PCR clean up beads, respectively. The pools or DNA libraries may be analyzed to determine the concentration or quantity of DNA molecules, for example by using a fluorescent dye (for example, PicoGreen pool quantification) and a fluorescence microplate reader, standard spectrofluorometer, or filter fluorometer. In one example, the DNA library preparation and/or capture is performed with an automated system, using a liquid handling robot (for example, a SciClone NGSx).

In some embodiments, *e.g.,* where a whole genome sequencing method will be used, nucleic acid sequencing libraries are not target-enriched prior to sequencing, in order to obtain sequencing data on substantially all of the competent nucleic acids in the sequencing library. Similarly, in some embodiments, *e.g.,* where a whole genome sequencing method will be used, nucleic acid sequencing libraries are not mixed, because of bandwidth limitations related to obtaining significant sequencing depth across an entire genome. However, in other embodiments, *e.g.,* where a low pass whole genome sequencing (LPWGS) methodology will be used, nucleic acid sequencing libraries can still be pooled, because very low average sequencing coverage is achieved across a respective genome, *e.g.,* between about 0.5x and about 5x.

In some embodiments, a plurality of nucleic acid probes *(e.g.,* a probe set) is used to enrich one or more target sequences in a nucleic acid sample *(e.g.,* an isolated nucleic acid sample or a nucleic acid sequencing library), *e.g.,* where one or more target sequences is informative for precision oncology. For instance, in some embodiments, one or more of the target sequences encompasses a locus that is associated with an actionable allele. That is, variations of the target sequence are associated with targeted therapeutic approaches. In some embodiments, one or more of the target sequences and/or a property of one or more of the target sequences is used in a classifier trained to distinguish two or more cancer states.

In some embodiments, the probe set includes probes targeting one or more gene loci, *e.g.,* exon or intron loci. In some embodiments, the probe set includes probes targeting one or more loci not encoding a protein, *e.g.,* regulatory loci, miRNA loci, and other non-coding loci, *e.g.,* that have been found to be associated with cancer. In some embodiments, the plurality of loci include at least 25, 50, 100, 150, 200, 250, 300, 350, 400, 500, 750, 1000, 2500, 5000, or more human genomic loci.

In some embodiments, the probe set includes probes targeting one or more of the genes listed in Table 1. In some embodiments, the probe set includes probes targeting at least 5 of the genes listed in Table 1. In some embodiments, the probe set includes probes targeting at least 10 of the genes listed in Table 1. In some embodiments, the probe set includes probes targeting at least 25 of the genes listed in Table 1. In some embodiments, the probe set includes probes targeting at least 50 of the genes listed in Table 1. In some embodiments, the probe set includes probes targeting at least 75 of the genes listed in Table 1. In some embodiments, the probe set includes probes targeting at least 100 of the genes listed in Table 1. In some embodiments, the probe set includes probes targeting all of the genes listed in Table 1.

**Table 1.** An example panel of 105 genes that are informative for precision oncology.

| Table 1: Liquid Biopsy Gene Panel | | | | | | |
|---|---|---|---|---|---|---|
| ALK | B2M | ERRFI1 | IDH2 | MSH6 | PIK3R1 | SPOP |
| FGFR2 | BAP1 | ESR1 | JAK1 | MTOR | PMS2 | STK11 |
| FGFR3 | BRCA1 | EZH2 | JAK2 | MYCN | PTCH1 | TERT |
| NTRK1 | BRCA2 | FBXW7 | JAK3 | NF1 | PTEN | TP53 |
| RET | BTK | FGFR1 | KDR | NF2 | PTPN11 | TSC1 |
| ROS1 | CCND1 | FGFR4 | KEAP1 | NFE2L2 | RAD51C | TSC2 |
| BRAF | CCND2 | FLT3 | KIT | NOTCH1 | RAF1 | UGT1A1 |
| AKT1 | CCND3 | FOXL2 | KRAS | NPM1 | RB1 | VHL |
| AKT2 | CDH1 | GATA3 | MAP2K1 | NRAS | RHEB | CCNE1 |
| APC | CDK4 | GNA11 | MAP2K2 | PALB2 | RHOA | CD274 |
| AR | CDK6 | GNAQ | MAPK1 | PBRM1 | RIT1 | EGFR |
| ARAF | CDKN2A | GNAS | MLH1 | PDCD1LG2 | RNF43 | ERBB2 |
| ARID1A | CTNNB1 | HNF1A | MPL | PDGFRA | SDHA | MET |
| ATM | DDR2 | HRAS | MSH2 | PDGFRB | SMAD4 | MYC |
| ATR | DPYD | IDH1 | MSH3 | PIK3CA | SMO | KMT2A |

While microsatellite loci may fall within a gene, there is no requirement that each microsatellite loci be associated with a gene. In some embodiments, one or more microsatellite loci falls within a non-coding region of the genome.

In some embodiments, the probe set includes probes targeting one or more of the microsatellite loci (regions) listed in Table 2. In some embodiments, the probe set includes probes targeting at least 5 of the microsatellite loci listed in Table 2. In some embodiments, the probe set includes probes targeting at least 10 of the microsatellite loci listed in Table 2. In some embodiments, the probe set includes probes targeting at least 15 of the microsatellite loci listed in Table 2. In some embodiments, the probe set includes probes targeting at least 20 of the microsatellite loci listed in Table 2. In some embodiments, the probe set includes probes targeting at least 25 of the microsatellite loci listed in Table 2. In some embodiments, the probe set includes probes targeting at least 30 of the microsatellite loci listed in Table 2. In some embodiments, the probe set includes probes targeting at least 35 of the microsatellite loci listed in Table 2. In some embodiments, the probe set includes probes targeting all of the microsatellite loci listed in Table 2.

**Table 2.** Example regions with informative microsatellite loci, for precision oncology.

| region name | repeat motif | chr | start | end | front_flank | rear_flank |
|---|---|---|---|---|---|---|
| chr18:649874-649899 | T | 18 | 649874 | 649899 | CTGCC | AAGGT |
| chr14:73959698-73959724 | T | 14 | 73959698 | 73959724 | CTACC | CCTTA |
| chr8:23712061-23712083 | T | 8 | 23712061 | 23712083 | TTGGG | CCTGC |
| chr3:71008336-71008359 | T | 3 | 71008336 | 71008359 | ACGTG | CCTTT |
| chr4:39501717-39501745 | A | 4 | 39501717 | 39501745 | GTACC | TCACA |
| chr3:113377476-113377497 | T | 3 | 113377476 | 113377497 | GAGGG | AGCAA |

(continued)

| region name | repeat motif | chr | start | end | front_flank | rear_flank |
|---|---|---|---|---|---|---|
| chr19:14104683-14104707 | T | 19 | 14104683 | 14104707 | AAGGC | AATTC |
| chr11:63149665-63149686 | A | 11 | 63149665 | 63149686 | AGCAC | CCTTC |
| chr6:11714634-11714658 | A | 6 | 11714634 | 11714658 | ACCAG | GATGA |
| chr1:47325294-47325317 | A | 1 | 47325294 | 47325317 | TTGTT | GTTGT |
| chr3:130733041-130733062 | T | 3 | 130733041 | 130733062 | CTGCC | GAATT |
| chr3:30691866-30691886 | A | 3 | 30691866 | 30691886 | GAAGG | GCCTG |
| chr11:115047027-115047051 | T | 11 | 115047027 | 115047051 | GAACA | ACACA |
| chr1:149900980-149901006 | A | 1 | 149900980 | 149901006 | GATAC | GATTA |
| chrX:101409249-101409275 | T | X | 101409249 | 101409275 | TCCTG | CTTCC |
| chr8:79629733-79629757 | A | 8 | 79629733 | 79629757 | ATCTC | GCCAA |
| chr11:125763605-125763628 | T | 11 | 125763605 | 125763628 | GTAAA | CCTTT |
| chr5:67584507-67584529 | T | 5 | 67584507 | 67584529 | GGGGA | CATTG |
| chr3:164905643-164905664 | A | 3 | 164905643 | 164905664 | TTTTG | GCACT |
| chr5:58270452-58270474 | A | 5 | 58270452 | 58270474 | TACTT | GGCAT |
| chr2:211179760-211179781 | T | 2 | 211179760 | 211179781 | TGCCA | AAAAG |
| chr1:237060940-237060965 | T | 1 | 237060940 | 237060965 | CTAAC | GCCTT |
| chr3:123332870-123332896 | T | 3 | 123332870 | 123332896 | TTGAG | GAGTT |
| chr18:319939-319960 | T | 18 | 319939 | 319960 | AGTGC | CAATC |
| chr2:64069272-64069297 | A | 2 | 64069272 | 64069297 | TCTAG | GCCGG |
| chr12:13364421-13364441 | A | 12 | 13364421 | 13364441 | TTACC | GAGCC |
| chr4:83785669-83785697 | TGC | 4 | 83785669 | 83785697 | CATGG | TCAGC |
| chr5:88018364-88018386 | CA | 5 | 88018364 | 88018386 | TATAG | CTGCA |
| chr5:88018383-88018406 | A | 5 | 88018383 | 88018406 | GCAAG | CTAGT |
| chr21:30339200-30339221 | T | 21 | 30339200 | 30339221 | TACCA | ACTTG |
| chr17:1326749-1326773 | A | 17 | 1326749 | 1326773 | GTTGG | GATTG |
| chr5:161494985-161495007 | A | 5 | 161494985 | 161495007 | GAAGG | GCGAT |
| chr15:37391747-37391769 | T | 15 | 37391747 | 37391769 | ATTCC | CCAAA |
| chr6:43021971-43021993 | G | 6 | 43021971 | 43021993 | AAAAC | CATTT |
| chr7:94989250-94989277 | T | 7 | 94989250 | 94989277 | TAGAC | ACTAT |
| chr2:99614590-99614613 | A | 2 | 99614590 | 99614613 | GTAGC | TCAGT |
| chr2:152236040-152236064 | A | 2 | 152236040 | 152236064 | CTTAT | GGATG |
| chr11:65268474-65268496 | T | 11 | 65268474 | 65268496 | GTGGG | ACACG |
| chr2:118854089-118854112 | T | 2 | 118854089 | 118854112 | TTCCA | AACGG |
| chr18:649874-649899 | T | 18 | 649874 | 649899 | CTGCC | AAGGT |

Generally, probes for enrichment of nucleic acids (e.g., cfDNA obtained from a liquid biopsy sample) include DNA, RNA, or a modified nucleic acid structure with a base sequence that is complementary to a loci of interest. For instance, a probe designed to hybridize to a loci in a cfDNA molecule can contain a sequence that is complementary to either strand, because the cfDNA molecules are double stranded. In some embodiments, each probe in the plurality of probes includes a nucleic acid sequence that is identical or complementary to at least 10, at least 11, at least 12, at least 13, at least 14, or at least 15 consecutive bases of a loci of interest. In some embodiments, each probe in the plurality of probes includes a nucleic acid sequence that is identical or complementary to at least 20, 25, 30, 40, 50, 75, 100, 150, 200, or more consecutive bases of a locus of interest.

Targeted-panels provide several benefits for nucleic acid sequencing. For example, in some embodiments, algorithms for discriminating between, *e.g.,* a first and second cancer condition can be trained on smaller, more informative data sets (*e.g.,* fewer genes), which leads to more computationally efficient training of classifiers that discriminate between the first and second cancer states. Such improvements in computational efficiency, owing to the reduced size of the discriminating gene set, can advantageously either be used to speed up classifier training or be used to improve the performance of such classifiers (*e.g.,* through more extensive training of the classifier).

In some embodiments, the gene panel is a whole-exome panel that analyzes the exomes of a biological sample. In some embodiments, the gene panel is a whole-genome panel that analyzes the genome of a specimen. In some preferred embodiments, the gene panel is optimized for use with liquid biopsy samples (*e.g.,* to provide clinical decision support for solid tumors). See, for example, Table 1 above.

In some embodiments, the probes include additional nucleic acid sequences that do not share any homology to the loci of interest. For example, in some embodiments, the probes also include nucleic acid sequences containing an identifier sequence, *e.g.,* a unique molecular identifier (UMI), *e.g.,* that is unique to a particular sample or subject. Examples of identifier sequences are described, for example, in Kivioja et al., 2011, Nat. Methods 9(1), pp. 72-74 and Islam et al., 2014, Nat. Methods 11(2), pp. 163-66. Similarly, in some embodiments, the probes also include primer nucleic acid sequences useful for amplifying the nucleic acid molecule of interest, *e.g.,* using PCR. In some embodiments, the probes also include a capture sequence designed to hybridize to an anti-capture sequence for recovering the nucleic acid molecule of interest.

Likewise, in some embodiments, the probes each include a non-nucleic acid affinity moiety covalently attached to nucleic acid molecule that is complementary to the loci of interest, for recovering the nucleic acid molecule of interest. Non-limited examples of non-nucleic acid affinity moieties include biotin, digoxigenin, and dinitrophenol. In some embodiments, the probe is attached to a solid-state surface or particle, *e.g.,* a dip-stick or magnetic bead, for recovering the nucleic acid of interest. In some embodiments, the methods described herein include amplifying the nucleic acids that bound to the probe set prior to further analysis, *e.g.,* sequencing. Methods for amplifying nucleic acids, *e.g.,* by PCR, are well known in the art.

Sequence reads are then generated (312) from the sequencing library or pool of sequencing libraries. Sequencing data may be acquired by any methodology known in the art. For example, next generation sequencing (NGS) techniques such as sequencing-by-synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators. In some embodiments, sequencing is performed using next generation sequencing technologies, such as short-read technologies. In other embodiments, long-read sequencing or another sequencing method known in the art is used.

Next-generation sequencing produces millions of short reads (*e.g.,* sequence reads) for each biological sample. Accordingly, in some embodiments, the plurality of sequence reads obtained by next-generation sequencing of cfDNA molecules are DNA sequence reads. In some embodiments, the sequence reads have an average length of at least fifty nucleotides. In other embodiments, the sequence reads have an average length of at least 50, 60, 70, 80, 90, 100, 150, 200, 250, 300, or more nucleotides.

In some embodiments, sequencing is performed after enriching for nucleic acids (*e.g.,* cfDNA, gDNA, and/or RNA) encompassing a plurality of predetermined target sequences, *e.g.,* human genes and/or non-coding sequences associated with cancer. Advantageously, sequencing a nucleic acid sample that has been enriched for target nucleic acids, rather than all nucleic acids isolated from a biological sample, significantly reduces the average time and cost of the sequencing reaction. Accordingly, in some preferred embodiments, the methods described herein include obtaining a plurality of sequence reads of nucleic acids that have been hybridized to a probe set for hybrid-capture enrichment (*e.g.,* of one or more genes listed in Table 1).

In some embodiments, panel-targeting sequencing is performed to an average on-target depth of at least 500x, at least 750x, at least 1000x, at least 2500x, at least 500x, at least 10,000x, or greater depth. In some embodiments, samples are further assessed for uniformity above a sequencing depth threshold (*e.g.,* 95% of all targeted base pairs at 300x sequencing depth). In some embodiments, the sequencing depth threshold is a minimum depth selected by a user or practitioner.

In some embodiments, the sequence reads are obtained by a whole genome or whole exome sequencing methodology. In some such embodiments, whole exome capture is performed with an automated system, using a liquid handling robot (for example, a SciClone NGSx). Whole genome sequencing, and to some extent whole exome sequencing, is typically performed at lower sequencing depth than smaller target-panel sequencing reactions, because many more loci are being sequenced. For example, in some embodiments, whole genome or whole exome sequencing is performed to an average sequencing depth of at least 3x, at least 5x, at least 10x, at least 15x, at least 20x, or greater. In some embodiments, low-pass whole genome sequencing (LPWGS) techniques are used for whole genome or whole exome sequencing. LPWGS is typically performed to an average sequencing depth of about 0.25x to about 5x, more typically to an average sequencing depth of about 0.5x to about 3x.

Because of the differences in the sequencing methodologies, data obtained from targeted-panel sequencing is better suited for certain analyses than data obtained from whole genome/whole exome sequencing, and vice versa. For instance, because of the higher sequencing depth achieved by targeted-panel sequencing, the resulting sequence data is better suited for the identification of variant alleles present at low allelic fractions in the sample, *e.g.,* less than 20%. By contrast, data generated from whole genome/whole exome sequencing is better suited for the estimation of genome-wide metrics, such as tumor mutational burden, because the entire genome is better represented in the sequencing data. Accordingly, in some embodiments, a nucleic acid sample, *e.g.,* a cfDNA, gDNA, or mRNA sample, is evaluated using both targeted-panel sequencing and whole genome/whole exome sequencing (*e.g.,* LPWGS).

In some embodiments, the raw sequence reads resulting from the sequencing reaction are output from the sequencer in a native file format, *e.g.,* a BCL file. In some embodiments, the native file is passed directly to a bioinformatics pipeline (*e.g.,* variant analysis 206), components of which are described in detail below. In other embodiments, pre-processing is performed prior to passing the sequences to the bioinformatics platform. For instance, in some embodiments, the format of the sequence read file is converted from the native file format (*e.g.,* BCL) to a file format compatible with one or more algorithms used in the bioinformatics pipeline (*e.g.,* FASTQ or FASTA). In some embodiments, the raw sequence reads are filtered to remove sequences that do not meet one or more quality thresholds. In some embodiments, raw sequence reads generated from the same unique nucleic acid molecule in the sequencing read are collapsed into a single sequence read representing the molecule, *e.g.,* using UMIs as described above. In some embodiments, one or more of these pre-processing activities is performed within the bioinformatics pipeline itself.

In one example, a sequencer may generate a BCL file. A BCL file may include raw image data of a plurality of patient specimens which are sequenced. BCL image data is an image of the flow cell across each cycle during sequencing. A cycle may be implemented by illuminating a patient specimen with a specific wavelength of electromagnetic radiation, generating a plurality of images which may be processed into base calls via BCL to FASTQ processing algorithms which identify which base pairs are present at each cycle. The resulting FASTQ file includes the entirety of reads for each patient specimen paired with a quality metric, *e.g.,* in a range from 0 to 64 where a 64 is the best quality and a 0 is the worst quality. In embodiments where both a liquid biopsy sample and a normal tissue sample are sequenced, sequence reads in the corresponding FASTQ files may be matched, such that a liquid biopsy-normal analysis may be performed.

FASTQ format is a text-based format for storing both a biological sequence, such as a nucleotide sequence, and its corresponding quality scores. These FASTQ files are analyzed to determine what genetic variants or copy number changes are present in the sample. Each FASTQ file contains reads that may be paired-end or single reads, and may be short-reads or long-reads, where each read represents one detected sequence of nucleotides in a nucleic acid molecule that was isolated from the patient sample or a copy of the nucleic acid molecule, detected by the sequencer. Each read in the FASTQ file is also associated with a quality rating. The quality rating may reflect the likelihood that an error occurred during the sequencing procedure that affected the associated read. In some embodiments, the results of paired-end sequencing of each isolated nucleic acid sample are contained in a split pair of FASTQ files, for efficiency. Thus, in some embodiments, forward (Read 1) and reverse (Read 2) sequences of each isolated nucleic acid sample are stored separately but in the same order and under the same identifier.

In various embodiments, the bioinformatics pipeline may filter FASTQ data from the corresponding sequence data file for each respective biological sample. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

While workflow 200 illustrates obtaining a biological sample, extracting nucleic acids from the biological sample, and sequencing the isolated nucleic acids, in some embodiments, sequencing data used in the improved systems and methods described herein (*e.g.,* which include improved methods for determining microsatellite stability status) is obtained by receiving previously generated sequence reads, in electronic form.

Referring again to Figure 2A, nucleic acid sequencing data 122 generated from the one or more patient samples is then evaluated (*e.g.,* via variant analysis 206) in a bioinformatics pipeline, *e.g.,* using bioinformatics module 140 of system 100, to identify genomic alterations and other metrics in the cancer genome of the patient. An example overview for a bioinformatics pipeline is described below with respect to Figures 4A-4G. Advantageously, in some embodiments, the present disclosure improves bioinformatics pipelines, like pipeline 206, by improving microsatellite stability status classification.

Figure 4A illustrates an example bioinformatics pipeline 206 (*e.g.,* as used for feature extraction in the workflows illustrated in Figures 2A and 3) for providing clinical support for precision oncology. As shown in Figure 4A, sequencing data 122 obtained from the wet lab processing 204 (*e.g.,* sequence reads 314) is input into the pipeline.

In various embodiments, the bioinformatics pipeline includes a circulating tumor DNA (ctDNA) pipeline for analyzing liquid biopsy samples. The pipeline may detect SNVs, INDELs, copy number amplifications/deletions and genomic rearrangements (for example, fusions). The pipeline may employ unique molecular index (UMI)-based consensus base calling as a method of error suppression as well as a Bayesian tri-nucleotide context-based position level error suppression. In various embodiments, it is able to detect variants having a 0.1%, 0.15%, 0.2%, 0.25%, 0.3%, 0.4%, or 0.5% variant allele fraction.

In some embodiments, the sequencing data is processed (*e.g.,* using sequence data processing module 141) to prepare it for genomic feature identification 385. For instance, in some embodiments as described above, the sequencing data is present in a native file format provided by the sequencer. Accordingly, in some embodiments, the system (*e.g., system 100*) applies a pre-processing algorithm 142 to convert the file format (318) to one that is recognized by one or more upstream processing algorithms. For example, BCL file outputs from a sequencer can be converted to a FASTQ file format using the bcl2fastq or bcl2fastq2 conversion software (Illumina®). FASTQ format is a text-based format for storing both a biological sequence, such as nucleotide sequence, and its corresponding quality scores. These FASTQ files are analyzed to determine what genetic variants, copy number changes, *etc.,* are present in the sample.

In some embodiments, other preprocessing functions are performed, *e.g.,* filtering sequence reads 122 based on a desired quality, *e.g.,* size and/or quality of the base calling. In some embodiments, quality control checks are performed to ensure the data is sufficient for variant calling. For instance, entire reads, individual nucleotides, or multiple nucleotides that are likely to have errors may be discarded based on the quality rating associated with the read in the FASTQ file, the known error rate of the sequencer, and/or a comparison between each nucleotide in the read and one or more nucleotides in other reads that has been aligned to the same location in the reference genome. Filtering may be done in part or in its entirety by various software tools, for example, a software tool such as Skewer. See, Jiang, H. et al., BMC Bioinformatics 15(182):1-12 (2014). FASTQ files may be analyzed for rapid assessment of quality control and reads, for example, by a sequencing data QC software such as AfterQC, Kraken, RNA-SeQC, FastQC, or another similar software program. For paired-end reads, reads may be merged.

In some embodiments, when both a liquid biopsy sample and a normal tissue sample from the patient are sequenced, two FASTQ output files are generated, one for the liquid biopsy sample and one for the normal tissue sample. A 'matched' (*e.g*., panel-specific) workflow is run to jointly analyze the liquid biopsy-normal matched FASTQ files. When a matched normal sample is not available from the patient, FASTQ files from the liquid biopsy sample are analyzed in the 'tumor-only' mode. See, for example, Figure 4B. If two or more patient samples are processed simultaneously on the same sequencer flow cell, *e.g.,* a liquid biopsy sample and a normal tissue sample, a difference in the sequence of the adapters used for each patient sample barcodes nucleic acids extracted from both samples, to associate each read with the correct patient sample and facilitate assignment to the correct FASTQ file.

For efficiency, in some embodiments, the results of paired-end sequencing of each isolate are contained in a split pair of FASTQ files. Forward (Read 1) and reverse (Read 2) sequences of each tumor and normal isolate are stored separately but in the same order and under the same identifier. See, for example, Figure 4C. In various embodiments, the bioinformatics pipeline may filter FASTQ data from each isolate. Such filtering may include correcting or masking sequencer errors and removing (trimming) low quality sequences or bases, adapter sequences, contaminations, chimeric reads, overrepresented sequences, biases caused by library preparation, amplification, or capture, and other errors. See, for example, Figure 4D.

Similarly, in some embodiments, sequencing (312) is performed on a pool of nucleic acid sequencing libraries prepared from different biological samples, *e.g.,* from the same or different patients. Accordingly, in some embodiments, the system demultiplexes (320) the data *(e.g.,* using demultiplexing algorithm 144) to separate sequence reads into separate files for each sequencing library included in the sequencing pool, *e.g.,* based on UMI or patient identifier sequences added to the nucleic acid fragments during sequencing library preparation, as described above. In some embodiments, the demultiplexing algorithm is part of the same software package as one or more pre-processing algorithms 142. For instance, the bcl2fastq or bcl2fastq2 conversion software (Illumina®) include instructions for both converting the native file format output from the sequencer and demultiplexing sequence reads 122 output from the reaction.

The sequence reads are then aligned (322), *e.g.,* using an alignment algorithm 143, to a reference sequence construct 158, *e.g.,* a reference genome, reference exome, or other reference construct prepared for a particular targeted-panel sequencing reaction. For example, in some embodiments, individual sequence reads 123, in electronic form *(e.g.,* in FASTQ files), are aligned against a reference sequence construct for the species of the subject (*e.g.,* a reference human genome) by identifying a sequence in a region of the reference sequence construct that best matches the sequence of nucleotides in the sequence read. In some embodiments, the sequence reads are aligned to a reference exome or reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. Any of a variety of alignment tools can be used for this task.

For instance, local sequence alignment algorithms compare subsequences of different lengths in the query sequence *(e.g.,* sequence read) to subsequences in the subject sequence *(e.g.,* reference construct) to create the best alignment for each portion of the query sequence. In contrast, global sequence alignment algorithms align the entirety of the sequences, *e.g.,* end to end. Examples of local sequence alignment algorithms include the Smith-Waterman algorithm (see, for example, Smith and Waterman, J Mol. Biol., 147(1):195-97 (1981)), Lalign (see, for example, Huang and Miller, Adv. Appl. Math, 12:337-57 (1991)), and PatternHunter (see, for example, Ma B. et al., Bioinformatics, 18(3):440-45 (2002)).

In some embodiments, the read mapping process starts by building an index of either the reference genome or the reads, which is then used to retrieve the set of positions in the reference sequence where the reads are more likely to align. Once this subset of possible mapping locations has been identified, alignment is performed in these candidate regions with slower and more sensitive algorithms. See, for example, Hatem et al., 2013, "Benchmarking short sequence mapping tools," BMC Bioinformatics 14: p. 184; and Flicek and Birney, 2009, "Sense from sequence reads: methods for alignment and assembly," Nat Methods 6(Suppl. 11), S6-S12. In some embodiments, the mapping tools methodology makes use of a hash table or a Burrows-Wheeler transform (BWT). See, for example, Li and Homer, 2010, "A survey of sequence alignment algorithms for next-generation sequencing," Brief Bioinformatics 11, pp. 473-483.

Other software programs designed to align reads include, for example, Novoalign (Novocraft, Inc.), Bowtie, Burrows Wheeler Aligner (BWA), and/or programs that use a Smith-Waterman algorithm. Candidate reference genomes include, for example, hg19, GRCh38, hg38, GRCh37, and/or other reference genomes developed by the Genome Reference Consortium. In some embodiments, the alignment generates a SAM file, which stores the locations of the start and end of each read according to coordinates in the reference genome and the coverage (number of reads) for each nucleotide in the reference genome.

For example, in some embodiments, each read of a FASTQ file is aligned to a location in the human genome having a sequence that best matches the sequence of nucleotides in the read. There are many software programs designed to align reads, for example, Novoalign (Novocraft, Inc.), Bowtie, Burrows Wheeler Aligner (BWA), programs that use a Smith-Waterman algorithm, *etc.* Alignment may be directed using a reference genome (for example, hg19, GRCh38, hg38, GRCh37, other reference genomes developed by the Genome Reference Consortium, *etc.)* by comparing the nucleotide sequences in each read with portions of the nucleotide sequence in the reference genome to determine the portion of the reference genome sequence that is most likely to correspond to the sequence in the read. In some embodiments, one or more SAM files are generated for the alignment, which store the locations of the start and end of each read according to coordinates in the reference genome and the coverage (number of reads) for each nucleotide in the reference genome. The SAM files may be converted to BAM files. In some embodiments, the BAM files are sorted and duplicate reads are marked for deletion, resulting in de-duplicated BAM files.

In some embodiments, adapter-trimmed FASTQ files are aligned to the 19th edition of the human reference genome build (HG19) using Burrows-Wheeler Aligner (BWA, Li and Durbin, Bioinformatics, 25(14):1754-60 (2009)). Following alignment, reads are grouped by alignment position and UMI family and collapsed into consensus sequences, for example, using fgbio tools (e.g., available on the Internet at fulcrumgenomics.github.io/fgbio/). Bases with insufficient quality or significant disagreement among family members (for example, when it is uncertain whether the base is an adenine, cytosine, guanine, *etc.)* may be replaced by N's to represent a wildcard nucleotide type. PHRED scores are then scaled based on initial base calling estimates combined across all family members. Following single-strand consensus generation, duplex consensus sequences are generated by comparing the forward and reverse oriented PCR products with mirrored UMI sequences. In various embodiments, a consensus can be generated across read pairs. Otherwise, single-strand consensus calls will be used. Following consensus calling, filtering is performed to remove low-quality consensus fragments. The consensus fragments are then re-aligned to the human reference genome using BWA. A BAM output file is generated after the re-alignment, then sorted by alignment position, and indexed.

In some embodiments, where both a liquid biopsy sample and a normal tissue sample are analyzed, this process produces a liquid biopsy BAM file *(e.g.,* Liquid BAM 124-1-i-cf) and a normal BAM file *(e.g.,* Germline BAM 124-1-i-g), as illustrated in Figure 4A. In various embodiments, BAM files may be analyzed to detect genetic variants and other genetic features, including single nucleotide variants (SNVs), copy number variants (CNVs), gene rearrangements, *etc.*

In some embodiments, the sequencing data is normalized, e.g., to account for pull-down, amplification, and/or sequencing bias *(e.g.,* mappability, GC bias *etc.).* See, for example, Schwartz et al., PLoS ONE 6(1):e16685 (2011) and Benjamini and Speed, Nucleic Acids Research 40(10):e72 (2012).

In some embodiments, SAM files generated after alignment are converted to BAM files 124. Thus, after preprocessing sequencing data generated for a pooled sequencing reaction, BAM files are generated for each of the sequencing libraries present in the master sequencing pools. For example, as illustrated in Figure 4A, separate BAM files are generated for each of three samples acquired from subject 1 at time i (*e.g.,* tumor BAM 124-1-i-t corresponding to alignments of sequence reads of nucleic acids isolated from a solid tumor sample from subject 1, Liquid BAM 124-1-i-cf corresponding to alignments of sequence reads of nucleic acids isolated from a liquid biopsy sample from subject 1, and Germline BAM 124-1-i-g corresponding to alignments of sequence reads of nucleic acids isolated from a normal tissue sample from subject 1), and one or more samples acquired from one or more additional subjects at time j (*e.g.*, Tumor BAM 124-2-j-t corresponding to alignments of sequence reads of nucleic acids isolated from a solid tumor sample from subject 2). In some embodiments, BAM files are sorted, and duplicate reads are marked for deletion, resulting in de-duplicated BAM files. For example, tools like SamBAMBA mark and filter duplicate alignments in the sorted BAM files.

Many of the embodiments described below, in conjunction with Figure 4, relate to analyses performed using sequencing data from cfDNA of a cancer patient, *e.g.,* obtained from a liquid biopsy sample of the patient. Generally, these embodiments are independent and, thus, not reliant upon any particular sequencing data generation methods, *e.g.,* sample preparation, sequencing, and/or data pre-processing methodologies. However, in some embodiments, the methods described below include one or more features 204 of generating sequencing data, as illustrated in Figures 2A and 3.

Alignment files prepared as described above (*e.g.,* BAM files 124) are then passed to a feature extraction module 145, where the sequences are analyzed (324) to identify genomic alterations (*e.g.,* SNVs/MNVs, indels, genomic rearrangements, copy number variations, *etc.)* and/or determine various characteristics of the patient's cancer (*e.g.,* MSI status, TMB, tumor ploidy, HRD status, tumor fraction, tumor purity, methylation patterns, *etc.).* Many software packages for identifying genomic alterations are known in the art, for example, freebayes, PolyBayse, samtools, GATK, pindel, SAMtools, Breakdancer, Cortex, Crest, Delly, Gridss, Hydra, Lumpy, Manta, and Socrates. For a review of many of these variant calling packages see, for example, Cameron, D.L. et al., Nat. Commun., 10(3240):1-11 (2019). Generally, these software packages identify variants in sorted SAM or BAM files 124, relative to one or more reference sequence constructs 158. The software packages then output a file *e.g.,* a raw VCF (variant call format), listing the variants (*e.g.,* genomic features 131) called and identifying their location relevant to the reference sequence construct (*e.g.,* where the sequence of the sample nucleic acids differ from the corresponding sequence in the reference construct). In some embodiments, system 100 digests the contents of the native output file to populate feature data 125 in test patient data store 120. In other embodiments, the native output file serves as the record of these genomic features 131 in test patient data store 120.

Generally, the systems described herein can employ any combination of available variant calling software packages and internally developed variant identification algorithms. In some embodiments, the output of a particular algorithm of a variant calling software is further evaluated, *e.g.,* to improve variant identification. Accordingly, in some embodiments, system 100 employs an available variant calling software package to perform some of all of the functionality of one or more of the algorithms shown in feature extraction module 145.

In some embodiments, as illustrated in Figure 1A, separate algorithms (or the same algorithm implemented using different parameters) are applied to identify variants unique to the cancer genome of the patient and variants existing in the germline of the subject. In other embodiments, variants are identified indiscriminately and later classified as either germline or somatic, *e.g.,* based on sequencing data, population data, or a combination thereof. In some embodiments, variants are classified as germline variants, and/or non-actionable variants, when they are represented in the population above a threshold level, *e.g.,* as determined using a population database such as ExAC or gnomAD. For instance, in some embodiments, variants that are represented in at least 1% of the alleles in a population are annotated as germline and/or non-actionable. In other embodiments, variants that are represented in at least 2%, at least 3%, at least 4%, at least 5%, at least 7.5%, at least 10%, or more of the alleles in a population are annotated as germline and/or non-actionable. In some embodiments, sequencing data from a matched sample from the patient, *e.g.,* a normal tissue sample, is used to annotate variants identified in a cancerous sample from the subject. That is, variants that are present in both the cancerous sample and the normal sample represent those variants that were in the germline prior to the patient developing cancer, and can be annotated as germline variants.

In various aspects, the detected genetic variants and genetic features are analyzed as a form of quality control. For example, a pattern of detected genetic variants or features may indicate an issue related to the sample, sequencing procedure, and/or bioinformatics pipeline (*e.g.,* example, contamination of the sample, mislabeling of the sample, a change in reagents, a change in the sequencing procedure and/or bioinformatics pipeline, *etc.).*

Figure 4E illustrates an example workflow for genomic feature identification (324). This particular workflow is only an example of one possible collection and arrangement of algorithms for feature extraction from sequencing data 124. Generally, any combination of the modules and algorithms of feature extraction module 145, *e.g.,* illustrated in Figure 1A, can be used for a bioinformatics pipeline, and particularly for a bioinformatics pipeline for analyzing liquid biopsy samples. For instance, in some embodiments, an architecture useful for the methods and systems described herein includes at least one of the modules or variant calling algorithms shown in feature extraction module 145. In some embodiments, an architecture includes at least 2, 3, 4, 5, 6, 7, 8, 9, 10, or more of the modules or variant calling algorithms shown in feature extraction module 145. Further, in some embodiments, feature extraction modules and/or algorithms not illustrated in Figure 1A find use in the methods and systems described herein.

Variant Identification

In some embodiments, variant analysis of aligned sequence reads, *e.g.,* in SAM or BAM format, includes identification of single nucleotide variants (SNVs), multiple nucleotide variants (MNVs), indels (*e.g.,* nucleotide additions and deletions), and/or genomic rearrangements (*e.g.*, inversions, translocations, and gene fusions) using variant identification module 146, *e.g.,* which includes a SNV/MNV calling algorithm (*e.g.,* SNV/MNV calling algorithm 147), an indel calling algorithm (*e.g.*, indel calling algorithm 148), and/or one or more genomic rearrangement calling algorithms (*e.g.*, genomic rearrangement calling algorithm 149). An overview of an example method for variant identification is shown in Figure 4E. Essentially, the module first identifies a difference between the sequence of an aligned sequence read 124 and the reference sequence to which the sequence read is aligned (*e.g.,* an SNV/MNV, an indel, or a genomic rearrangement) and makes a record of the variant, *e.g.,* in a variant call format (VCF) file. For instance, software packages such as freebayes and pindel are used to call variants using sorted BAM files and reference BED files as the input. For a review of variant calling packages see, for example, Cameron, D.L. et al., Nat. Commun., 10(3240):1-11 (2019). A raw VCF file (variant call format) file is output, showing the locations where the nucleotide base in the sample is not the same as the nucleotide base in that position in the reference sequence construct.

In some embodiments, as illustrated in Figure 4E, raw VCF data is then normalized, *e.g.,* by parsimony and left alignment. For example, software packages such as vcfbreakmulti and vt are used to normalize multi-nucleotide polymorphic variants in the raw VCF file and a variant normalized VCF file is output. See, for example, E. Garrison, "Vcflib: A C++ library for parsing and manipulating VCF files, GitHub, available on the Internet at github.com/ekg/vcflib (2012). In some embodiments, a normalization algorithm is included within the architecture of a broader variant identification software package.

An algorithm is then used to annotate the variants in the (*e.g.*, normalized) VCF file, *e.g.,* determines the source of the variation, *e.g.,* whether the variant is from the germline of the subject (*e.g.,* a germline variant), a cancerous tissue (*e.g.,* a somatic variant), a sequencing error, or of an undeterminable source. In some embodiments, an annotation algorithm is included within the architecture of a broader variant identification software package. However, in some embodiments, an external annotation algorithm is applied to (*e.g.,* normalized) VCF data obtained from a conventional variant identification software package. The choice to use a particular annotation algorithm is well within the purview of the skilled artisan, and in some embodiments is based upon the data being annotated.

For example, in some embodiments, where both a liquid biopsy sample and a normal tissue sample of the patient are analyzed, variants identified in the normal tissue sample inform annotation of the variants in the liquid biopsy sample. In some embodiments, where a particular variant is identified in the normal tissue sample, that variant is annotated as a germline variant in the liquid biopsy sample. Similarly, in some embodiments, where a particular variant identified in the liquid biopsy sample is not identified in the normal tissue sample, the variant is annotated as a somatic variant when the variant otherwise satisfies any additional criteria placed on somatic variant calling, *e.g.,* a threshold variant allele fraction (VAF) in the sample.

By contrast, in some embodiments, where only a liquid biopsy sample is being analyzed, the annotation algorithm relies on other characteristics of the variant in order to annotate the origin of the variant. For instance, in some embodiments, the annotation algorithm evaluates the VAF of the variant in the sample, *e.g.,* alone or in combination with additional characteristics of the sample, *e.g.,* tumor fraction. Accordingly, in some embodiments, where the VAF is within a first range encompassing a value that corresponds to a 1:1 distribution of variant and reference alleles in the sample, the algorithm annotates the variant as a germline variant, because it is presumably represented in cfDNA originating from both normal and cancer tissues. Similarly, in some embodiments, where the VAF is below a baseline variant threshold, the algorithm annotates the variant as undeterminable, because there is not sufficient evidence to distinguish between the possibility that the variant arose as a result of an amplification or sequencing error and the possibility that the variant originated from a cancerous tissue. Similarly, in some embodiments, where the VAF falls between the first range and the baseline variant threshold, the algorithm annotates the variant as a somatic variant.

In some embodiments, the baseline variant threshold is a value from 0.01% VAF to 0.5% VAF. In some embodiments, the baseline variant threshold is a value from 0.05% VAF to 0.35% VAF. In some embodiments, the baseline variant threshold is a value from 0.1% VAF to 0.25% VAF. In some embodiments, the baseline variant threshold is about 0.01% VAF, 0.015% VAF, 0.02% VAF, 0.025% VAF, 0.03% VAF, 0.035% VAF, 0.04% VAF, 0.045% VAF, 0.05% VAF, 0.06% VAF, 0.07% VAF, 0.075% VAF, 0.08% VAF, 0.09% VAF, 0.1% VAF, 0.15% VAF, 0.2% VAF, 0.25% VAF, 0.3% VAF, 0.35% VAF, 0.4% VAF, 0.45% VAF, 0.5% VAF, or greater. In some embodiments, the baseline variant threshold is different for variants located in a first region, *e.g.,* a region identified as a mutational hotspot and/or having high genomic complexity, than for variants located in a second region, *e.g.,* a region that is not identified as a mutational hotspot and/or having average genomic complexity. For example, in some embodiments, the baseline variant threshold is a value from 0.01% to 0.25% for variants located in the first region and is a value from 0.1% to 0.5% for variants located in the second region.

In some embodiments, the first region is a region of interest in the genome that may have been manually selected based on criteria (for example, selection may be based on a known likelihood that a region is associated with variants) and the second region is a region that did not meet the selection criteria. In some embodiments, the baseline variant threshold is a value from 0.01% to 0.5% for variants located in the first region and is a value from 1% to 5% for variants located in the second region. In some embodiments, the first region is a region of interest in the genome that may have been manually selected based on criteria (for example, selection may be based on a known likelihood that a region is associated with variants) and the second region is a region selected based on a second set of criteria.

In some embodiments, a baseline variant threshold is influenced by the sequencing depth of the reaction, *e.g.,* a locus-specific sequencing depth and/or an average sequencing depth (*e.g.,* across a targeted panel and/or complete reference sequence construct). In some embodiments, the baseline variant threshold is dependent upon the type of variant being detected. For example, in some embodiments, different baseline variant thresholds are set for SNPs/MNVs than for indels and/or genomic rearrangements. For instance, while an apparent SNP may be introduced by amplification and/or sequencing errors, it is much less likely that a genomic rearrangement is introduced this way and, thus, a lower baseline variant threshold may be appropriate for genomic rearrangements than for SNPs/NINVs.

In some embodiments, one or more additional criteria are required to be satisfied before a variant can be annotated as a somatic variant. For instance, in some embodiments, a threshold number of unique sequence reads encompassing the variant must be present to annotate the variant as somatic. In some embodiments, the threshold number of unique sequence reads is 2, 3, 4, 5, 7, 10, 12, 15, or greater. In some embodiments, the threshold number of unique sequence reads is only applied when certain conditions are met, *e.g.,* when the variant allele is located in a region of a certain genomic complexity. In some embodiments, the certain genomic complexity is a low genomic complexity. In some embodiments, the certain genomic complexity is an average genomic complexity. In some embodiments, the certain genomic complexity is a high genomic complexity.

In some embodiments, a threshold sequencing coverage, *e.g.,* a locus-specific and/or an average sequencing depth (*e.g.,* across a targeted panel and/or complete reference sequence construct) must be satisfied to annotate the variant as somatic. In some embodiments, the threshold sequencing coverage is 50X, 100X, 150X, 200X, 250X, 300X, 350X, 400X or greater. In some embodiments, the variant is located in a microsatellite instable (MSI) region. In some embodiments, the variant is not located in a microsatellite instable (MSI) region. In some embodiments, the variant has sufficient signal-to-noise ratio.

In some embodiments, bases contributing to the variant satisfy a threshold mapping quality to annotate the variant as somatic. In some embodiments, alignments contributing to the variant must satisfy a threshold alignment quality to annotate the variant as somatic. In some embodiments, a threshold value is determined for a variant detected in a somatic (cancer) sample by analyzing the threshold metric (for example, the baseline variant threshold is determined by analyzing VAF, or the threshold sequencing coverage is determined by analyzing coverage) associated with that variant in a group of germline (normal) samples that were each processed by the same sample processing and sequencing protocol as the somatic sample (process-matched). This may be used to ensure the variants are not caused by observed artifact generating processes.

In some embodiments, the threshold value is set above the median base fraction of the threshold metric value associated with the variant in more than a specified percentage of process-matched germline samples, e.g., 1, 2, 3, 4, 5, 6, 7, 8, 9, 10, or more standard deviations above the median base fraction of the threshold metric value associated with 25%, 30, 40, 50, 60, 70, 75, or more of the processed-matched germline samples. For example, in one embodiment, the threshold value is set to a value 5 standard deviations above the median base fraction of the threshold metric value associated with the variant in more than 50% of the process matched germline samples.

In some embodiments, variants around homopolymer and multimer regions known to generate artifacts may be specifically filtered to avoid such artifacts. For example, in some embodiments, strand specific filtering is performed in the direction of the read in order to minimize stranded artifacts. Similarly, in some embodiments, variants that do not exceed the stranded minimum deviation for their specific locus within a known artifact-generating region may be filtered to avoid artifacts.

Variants may be filtered using dynamic methods, such as through the application of Bayes' Theorem through a likelihood ratio test. The dynamic threshold may be based on, for example, factors such as sample specific error rate, the error rate from a healthy reference pool, and information from internal human solid tumors. Accordingly, in some embodiments, the dynamic filtering method employs a tri-nucleotide context-based Bayesian model. That is, in some embodiments, the threshold for filtering any particular putative variant is dynamically calibrated using a context-based Bayesian model that considers one or more of a sample-specific sequencing error rate, a process-matched control sequencing error rate, and/or a variant-specific frequency (*e.g.,* determined from similar cancers). In this fashion, a minimum number of alternative alleles required to positively identify a true variant is determined for individual alleles and/or loci.

In some embodiments, certain variants pre-identified on a whitelist may be rescued, i.e., not filtered out, when they fail to pass selective filters, *e.g.,* MSI/SN, a Bayesian filtering method, and/or a coverage, VAF or region-based filter. The rationale for whitelisting a variant is to apply less stringent filtering criteria to such a variant so that it can be reviewed and/or reported. The variants on the whitelist are usually common pathogenic variants with high clinical relevance. If a variant is on the whitelist and fails to pass certain filters, it will be rescued and not filtered out. As used herein, MSI/SN refers to a variant filter for filtering out potential artifactual variants based on the MSI (microsatellite instable) and SN (signal-to-noise ratio) values calculated by the variant caller VarDict. See, for example, VarDict documentation, available on the internet at github.com/AstraZeneca-NGS/VarDictJava.

In some embodiments, one or more locus and/or genomic region is blacklisted, preventing somatic variant annotation for variants identified at the locus or region. In some embodiments, the variant has a length of 120, 100, 80, 60, 40, 20, 10, 5 or less base pairs. In various embodiments, any combination of the additional criteria, as well as additional criteria not listed above, may be applied to the variant calling process. Again, in some embodiments, different criteria are applied to the annotation of different types of variants.

In some embodiments, liquid biopsy assays are used to detect variant alterations present at low circulating fractions in the patient's blood. In such circumstances, it may be warranted to lower the requirements for positively identifying a variant. That is, in some embodiments, low levels of support may be sufficient to call a variant, dependent upon the reason for using the liquid biopsy assay.

In some embodiments, SNV/INDEL detection is accomplished using VarDict (available on the Internet at github.com/AstraZeneca-NGS/VarDictJava). Both SNVs and INDELs are called and then sorted, deduplicated, normalized and annotated. The annotation uses SnpEff to add transcript information, 1000 genomes minor allele frequencies, COSMIC reference names and counts, ExAC allele frequencies, and Kaviar population allele frequencies. The annotated variants are then classified as germline, somatic, or uncertain using a Bayesian model based on prior expectations informed by databases of germline and cancer variants. In some embodiments, uncertain variants are treated as somatic for filtering and reporting purposes.

In some embodiments, genomic rearrangements (*e.g.,* inversions, translocations, and gene fusions) are detected following de-multiplexing by aligning tumor FASTQ files against a human reference genome using a local alignment algorithm, such as BWA. In some embodiments, DNA reads are sorted and duplicates may be marked with a software, for example, SAMBlaster. Discordant and split reads may be further identified and separated. These data may be read into a software, for example, LUMPY, for structural variant detection. In some embodiments, structural alterations are grouped by type, recurrence, and presence and stored within a database and displayed through a fusion viewer software tool. The fusion viewer software tool may reference a database, for example, Ensembl, to determine the gene and proximal exons surrounding the breakpoint for any possible transcript generated across the breakpoint. The fusion viewer tool may then place the breakpoint 5' or 3' to the subsequent exon in the direction of transcription. For inversions, this orientation may be reversed for the inverted gene. After positioning of the breakpoint, the translated amino acid sequences may be generated for both genes in the chimeric protein, and a plot may be generated containing the remaining functional domains for each protein, as returned from a database, for example, Uniprot.

For instance, in an example implementation, gene rearrangements are detected using the SpeedSeq analysis pipeline. Chiang et al., 2015, "SpeedSeq: ultra-fast personal genome analysis and interpretation," Nat Methods, (12), pg. 966. Briefly, FASTQ files are aligned to hg19 using BWA. Split reads mapped to multiple positions and read pairs mapped to discordant positions are identified and separated, then utilized to detect gene rearrangements by LUMPY. Layer et al., 2014, "LUMPY: a probabilistic framework for structural variant discovery," Genome Biol, (15), pg. 84. Fusions can then be filtered according to the number of supporting reads.

In some embodiments, putative fusion variants supported by fewer than a minimum number of unique sequence reads are filtered. In some embodiments, the minimum number of unique sequence reads is 2, 3, 4, 5, 6, 7, 8, 9, 10, 12, 15, or 20 unique sequence reads.

Allelic Fraction Determination

In some embodiments, the analysis of aligned sequence reads, *e.g.,* in SAM or BAM format, includes determination of variant allele fractions (133) for one or more of the variant alleles 132 identified as described above. In some embodiments, a variant allele fraction module 151 tallies the instances that each allele is represented by a unique sequence read encompassing the variant locus of interest, generating a count for each allele represented at that locus. In some embodiments, these tallies are used to determine the ratio of the variant allele, *e.g.,* an allele other than the most prevalent allele in the subject's population for a respective locus, to a reference allele. This variant allele fraction 133 can be used in several places in the feature extraction 206 workflow. For instance, in some embodiments, a variant allele fraction is used during annotations of identified variants, *e.g.,* when determining whether the allele originated from a germline cell or a somatic cell. In other instances, a variant allele fraction is used in a process for estimating a tumor fraction for a liquid biopsy sample or a tumor purity for a solid tumor fraction. For instance, variant allele fractions for a plurality of somatic alleles can be used to estimate the percentage of sequence reads originating from one copy of a cancerous chromosome. Assuming a 100% tumor purity and that each cancer cell caries one copy of the variant allele, the overall purity of the tumor can be estimated. This estimate, of course, can be further corrected based on other information extracted from the sequencing data, such as copy number alterations, tumor ploidy aberrations, tumor heterozygosity, *etc.*

Methylation Determination

In some embodiments, where nucleic acid sequencing library was processed by bisulfite treatment or enzymatic methyl-cytosine conversion, as described above, the analysis of aligned sequence reads, *e.g.,* in SAM or BAM format, includes determination of methylation states 132 for one or more loci in the genome of the patient. In some embodiments, methylation sequencing data is aligned to a reference sequence construct 158 in a different fashion than nonmethylation sequencing, because non-methylated cytosines are converted to uracils, and the resulting uracils are ultimately sequenced as thymines, whereas methylated cytosine are not converted and sequenced as cytosine. Different approaches, therefore, have to be used to align these modified sequences to a reference sequence construct, such as seeding alignments with shorter regions of identity or converting all cytosines to thymidines in the sequencing data and then aligning the data to reference sequence constructs for both the plus and minus strand of the sequence construct. For review of these approaches, see Zhou Q. et al., BMC Bioinformatics, 20(47):1-11 (2019). Algorithms for calling methylated bases are known in the art. For example, Bismark is able to distinguish between cytosines in CpG, CHG, and CHH contexts. Krueger F. and Andrews SR, Bioinformatics, 27(11):1571-71 (2011).

Copy Number Variation Analysis:

In some embodiments, the analysis of aligned sequence reads, *e.g.,* in SAM or BAM format, includes determination of the copy number 135 for one or more locus, using a copy number variation analysis module 153. In some embodiments, where both a liquid biopsy sample and a normal tissue sample of the patient are analyzed, de-duplicated BAM files and a VCF generated from the variant calling pipeline are used to compute read depth and variation in heterozygous germline SNVs between sequencing reads for each sample. By contrast, in some embodiments, where only a liquid biopsy sample is being analyzed, comparison between a tumor sample and a pool of process-matched normal controls is used. In some embodiments, copy number analysis includes application of a circular binary segmentation algorithm and selection of segments with highly differential $\log_2$ ratios between the cancer sample and its comparator (*e.g.,* a matched normal or normal pool). In some embodiments, approximate integer copy number is assessed from a combination of differential coverage in segmented regions and an estimate of stromal admixture (for example, tumor purity, or the portion of a sample that is cancerous vs. non-cancerous, such as a tumor fraction for a liquid biopsy sample) is generated by analysis of heterozygous germline SNVs.

For instance, in an example implementation, copy number variants (CNVs) are analyzed using the CNVkit package. Talevich et al., PLoS Comput Biol, 12:1004873 (2016). CNVkit is used for genomic region binning, coverage calculation, bias correction, normalization to a reference pool, segmentation and visualization. The $\log_2$ ratios between the tumor sample and a pool of process matched healthy samples from the CNVkit output are then annotated and filtered using statistical models whereby the amplification status (amplified or not-amplified) of each gene is predicted and non-focal amplifications are removed.

In some embodiments, copy number variations (CNVs) are analyzed using a combination of an open-source tool, such as CNVkit, and an annotation/filtering algorithm, *e.g.,* implemented via a python script. CNVkit is used initially to perform genomic region binning, coverage calculation, bias correction, normalization to a reference pool, segmentation and, optionally, visualization. The bin-level copy ratios and segment-level copy ratios, in addition to their corresponding confidence intervals, from the CNVkit output are then used in the annotation and filtering where the copy number state (amplified, neutral, deleted) of each segment and bin are determined and non-focal amplifications/deletions are filtered out based on a set of acceptance criteria. In some embodiments, one or more copy number variations selected from amplifications in the MET, EGFR, ERBB2, CD274, CCNE1, and MYC genes, and deletions in the BRCA1 and BRCA2 genes are analyzed. However, the methods described herein is not limited to only these reportable genes.

In some embodiments, CNV analysis is performed using a tumor BAM file, a target region BED file, a pool of process matched normal samples, and inputs for initial reference pool construction. Inputs for initial reference pool construction include one or more of normal BAM files, a human reference genome file, mappable regions of the genome, and a blacklist that contains recurrent problematic areas of the genome.

CNVkit utilizes both targeted captured sequencing reads and non-specifically captured off-target reads to infer copy number information. The targeted genomic regions specified in the probe target BED file are divided to target bins with an average size of, *e.g.,* 100 base pairs, which can be specified by the user. The genomic regions between the target regions, *e.g.,* excluding regions that cannot be mapped reliably, are automatically divided into off-target (also referred to as anti-target) bins with an average size of, *e.g.,* 150 kbp, which again can be specified by the user. Raw $\log_2$-transformed depths are then calculated from the alignments in the input BAM file and written to two tab-delimited .cnn files, one for each of the target and off-target bins.

A pooled reference is constructed from a panel of process matched normal samples. The raw $\log_2$ depths of target and off-target bins in each normal sample are computed as described above, and then each are median-centered and corrected for bias including GC content, genome sequence repetitiveness, target size, and/or spacing. The corrected target and off-target $\log_2$ depths are combined, and a weighted average and spread are calculated as Tukey's biweight location and midvariance in each bin. These values are written to a tab delimited reference .cnn file, which is used to normalize an input tumor sample as follows.

The raw $\log_2$ depths of an input sample are median-centered and bias-corrected as described in the reference construction. The corrected $\log_2$ depth of each bin is then subtracted from the corresponding $\log_2$ depth in the reference file, resulting in the $\log_2$ copy ratios (also referred to as copy ratios or $\log_2$ ratios) between the input tumor sample and the reference pool. These values are written to a tab-delimited .cnr file.

The copy ratios are then segmented, *e.g.,* via a circular binary segmentation (CBS) algorithm or another suitable segmentation algorithm, whereby adjacent bins are grouped to larger genomic regions (segments) of equal copy number. The segment's copy ratio is calculated as the weighted mean of all bins within the segment. The confidence interval of the segment mean is estimated by bootstrapping the bin-level copy ratios within the segment. The segments' genomic ranges, copy ratios and confidence intervals are written to a tab-delimited .cns file.

$\log_2$ transformed copy ratios, $\log_2$ copy ratios, $\log_2$-transformed depths, $\log_2$-transformed read depths, $\log_2$ depths, corrected $\log_2$ depths, $\log_2$ ratios, $\log_2$ read depths, and $\log_2$ depth correction values have been discussed herein by way of example. In each instance where such a term is used, it will be appreciated that log base 2 is presented by way of example only and that the present disclosure is not so limited. Indeed, logarithms to any base N may be used, (*e.g.*, where N is a positive number greater than 1 for instance), and thus the present disclosure fully supports $\log_N$ transformed copy ratios, $\log_N$ copy ratios, $\log_N$-transformed depths, $\log_N$-transformed read depths, $\log_N$ depths, corrected $\log_N$ depths, $\log_N$ ratios, $\log_N$ read depths, and $\log_N$ depth correction values as respective substitutes for $\log_2$ transformed copy ratios, $\log_2$ copy ratios, $\log_2$-transformed depths, $\log_2$-transformed read depths, $\log_2$ depths, corrected $\log_2$ depths, $\log_2$ ratios, $\log_2$ read depths, and $\log_2$ depth correction values.

Microsatellite Instability (MSI):

In some embodiments, analysis of aligned sequence reads, *e.g.,* in SAM or BAM format, includes analysis of the microsatellite instability status 137 of a cancer, using a microsatellite instability analysis module 154. In some embodiments, an MSI classification algorithm classifies a cancer into three categories: microsatellite instability-high (MSI-H), microsatellite stable (MSS), or microsatellite equivocal (MSE). Microsatellite instability is a clinically actionable genomic indication for cancer immunotherapy. In microsatellite instability-high (MSI-H) tumors, defects in DNA mismatch repair (MMR) can cause a hypermutated phenotype where alterations accumulate in the repetitive microsatellite regions of DNA. MSI detection is conventionally performed by subjecting tumor tissue ("solid biopsy") to clinical next-generation sequencing or specific assays, such as MMR IHC or MSI PCR.

For example, microsatellite instability status can be assessed by determining the number of repeating units present at a plurality of microsatellite loci, *e.g.,* 5, 10, 15, 20, 25, 30, 40, 50, 75, 100, 250, 500, 750, 1000, 2500, 5000, or more loci. In some embodiments, only reads encompassing a microsatellite locus that include a significant number of flanking nucleotides on both ends, *e.g.,* at least 5, 10, 15, or more nucleotides flanking each end, are used for the analysis in order to avoid using reads that do not completely cover the locus. In some embodiments, a minimal number of reads, *e.g.,* at least 5, 10, 20, 30, 40, 50, or more reads have to meet this criteria in order to use a particular microsatellite locus, in order to ensure the accuracy of the determination given the high incidence of polymerase slipping during replication of these repeated sequences.

In some embodiments, each locus is tested individually for instability, *e.g.,* as measured by a change or variance in the number of nucleotide base repeats, *e.g.,* in cancerderived nucleotide sequences relative to a normal sample or standard, for example, using the Kolmogorov-Smimov test. For example, if $p \leq 0.05$, the locus is considered unstable. The proportion of unstable microsatellite loci may be fed into a logistic regression classifier trained on samples from various cancer types, especially cancer types which have clinically determined MSI statuses, for example, colorectal and endometrial cohorts. For MSI testing where only a liquid biopsy sample is analyzed, the mean and variance for the number of repeats may be calculated for each microsatellite locus. A vector containing the mean and variance data may be put into a classifier *(e.g.,* a support vector machine classification algorithm) trained to provide a probability that the patient is MSI-H, which may be compared to a threshold value. In some embodiments, the threshold value for calling the patient as MSI-H is at least 60% probability, or at least 65% probability, 70% probability, 75% probability, 80% probability, or greater. In some embodiments, a baseline threshold may be established to call the patient as MSS. In some embodiments, the baseline threshold is no more than 40%, or no more than 35% probability, 30% probability, 25% probability, 20% probability, or less. In some embodiments, when the output of the classifier falls within the range between the MSI-H and MSS thresholds, the patient is identified as MSE.

Tumor Mutational Burden (TMB):

In some embodiments, the analysis of aligned sequence reads, *e.g.,* in SAM or BAM format, includes determination of a mutation burden for the cancer *(e.g.,* a tumor mutational burden 136), using a tumor mutational burden analysis module 155. Generally, a tumor mutational burden is a measure of the mutations in a cancer per unit of the patient's genome. For example, a tumor mutational burden may be expressed as a measure of central tendency *(e.g.,* an average) of the number of somatic variants per million base pairs in the genome. In some embodiments, a tumor mutational burden refers to only a set of possible mutations, *e.g.,* one or more of SNVs, MNVs, indels, or genomic rearrangements. In some embodiments, a tumor mutational burden refers to only a subset of one or more types of possible mutations, *e.g.,* non-synonymous mutations, meaning those mutations that alter the amino acid sequence of an encoded protein. In other embodiments, for example, a tumor mutational burden refers to the number of one or more types of mutations that occur in protein coding sequences, *e.g.,* regardless of whether they change the amino acid sequence of the encoded protein.

As an example, in some embodiments, a tumor mutational burden (TMB) is calculated by dividing the number of mutations *(e.g.,* all variants or non-synonymous variants) identified in the sequencing data *(e.g.,* as represented in a VCF file) by the size *(e.g.,* in megabases) of a capture probe panel used for targeted sequencing. In some embodiments, a variant is included in tumor mutation burden calculation only when certain criteria are met. For instance, in some embodiments, a threshold sequence coverage for the locus associated with the variant must be met before the variant is included in the calculation, *e.g.,* at least 25x, 50x, 75x, 100x, 250x, 500x, or greater. Similarly, in some embodiments, a minimum number of unique sequence reads encompassing the variant allele must be identified in the sequencing data, *e.g.,* at least 4, 5, 6, 7, 8, 9, 10, or more unique sequence reads. In some embodiments, a threshold variant allelic fraction threshold must be satisfied before the variant is included in the calculation, e.g., at least 0.01%, 0.1%, 0.25%, 0.5%, 0.75%, 1%, 1.5%, 2%, 2.5%, 3%, 4%, 5%, or greater. In some embodiments, an inclusion criteria may be different for different types of variants and/or different variants of the same type. For instance, a variant detected in a mutation hotspot within the genome may face less rigorous criteria than a variant detected in a more stable locus within the genome.

Other methods for calculating tumor mutation burden in liquid biopsy samples and/or solid tissue samples are known in the art. See, for example, Fenizia F. et al., Transl Lung Cancer Res., 7(6):668-77 (2018) and Georgiadis A. et al., Clin. Cancer Res., 25(23):7024-34 (2019).

Homologous Recombination Status (HRD):

In some embodiments, analysis of aligned sequence reads, *e.g.,* in SAM or BAM format, includes analysis of whether the cancer is homologous recombination deficient (HRD status 137-3), using a homologous recombination pathway analysis module 157.

Homologous recombination (HR) is a normal, highly conserved DNA repair process that enables the exchange of genetic information between identical or closely related DNA molecules. It is most widely used by cells to accurately repair harmful breaks (i.e. damage) that occur on both strands of DNA. DNA damage may occur from exogenous (external) sources like UV light, radiation, or chemical damage; or from endogenous (internal) sources like errors in DNA replication or other cellular processes that create DNA damage. Double strand breaks are a type of DNA damage. Using poly (ADP-ribose) polymerase (PARP) inhibitors in patients with HRD compromises two pathways of DNA repair, resulting in cell death (apoptosis). The efficacy of PARP inhibitors is improved not only in ovarian cancers displaying germline or somatic BRCA mutations, but also in cancers in which HRD is caused by other underlying etiologies.

In some embodiments, HRD status can be determined by inputting features correlated with HRD status into a classifier trained to distinguish between cancers with homologous recombination pathway deficiencies and cancers without homologous recombination pathway deficiencies. For example, in some embodiments, the features include one or more of (i) a heterozygosity status for a first plurality of DNA damage repair genes in the genome of the cancerous tissue of the subject, (ii) a measure of the loss of heterozygosity across the genome of the cancerous tissue of the subject, (iii) a measure of variant alleles detected in a second plurality of DNA damage repair genes in the genome of the cancerous tissue of the subject, and (iv) a measure of variant alleles detected in the second plurality of DNA damage repair genes in the genome of the non-cancerous tissue of the subject. In some embodiments, all four of the features described above are used as features in an HRD classifier. More details about HRD classifiers using these and other features are described in U.S. Patent Application Serial No. 16/789,363, filed February 12, 2020.

Quality Control

In some embodiments, a positive sensitivity control sample is processed and sequenced along with one or more clinical samples. In some embodiments, the control sample is included in at least one flow cell of a multi-flow cell reaction and is processed and sequenced each time a set of samples is sequenced or periodically throughout the course of a plurality of sets of samples. In some embodiments, the control includes a pool of controls. In some embodiments, a quality control analysis requires that read metrics of variants present in the control sample fall within acceptable criteria. In some embodiments, a quality control requires approval by a pathologist before the results are reported.

In some embodiments, the quality control system includes methods that pass samples for reporting if various criteria are met. Similarly, in some embodiments, the system includes methods that allow for more manual review if a sample does not meet the criteria established for automatic pass. In some embodiments, the criteria for pass of panel sequencing results include one or more of the following:

- A criterion for the on-target rate of the sequencing reaction, defined as a comparison *(e.g.,* a ratio) of (i) the number of sequenced nucleotides or reads falling within the targeted panel region of a genome and (ii) the number of sequenced nucleotides or reads falling outside of the targeted panel region of the genome. Generally, an on-target rate threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a minimum on-target rate threshold of at least 30%, at least 40%, at least 50%, at least 60%, at least 70%, or greater. In some embodiments, the on-target rate criteria is implemented as a range of acceptable on-target rates, e.g., requiring that the on-target rate for a reaction is from 30% to 70%, from 30% to 80%, from 40% to 70%, from 40% to 80%, and the like.

- A criterion for the number of total reads generated by the sequencing reaction, including both unique sequence reads and non-unique sequence reads. Generally, a total read number threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a minimum number of total reads threshold of at least 100 million, 110 million, 120 million, 130 million, 140 million, 150 million, 160 million, 170 million, 180 million, 190 million, 200 million, or more total sequence reads. In some embodiments, the criterion is implemented as a range of acceptable number of total reads, *e.g.*, requiring that the sequencing reaction generate from 50 million to 300 million total sequence reads, from 100 million to 300 million sequence reads, from 100 million to 200 million sequence reads, and the like.

- A criterion for the number of unique reads generated by the sequencing reaction. Generally, a unique read number threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a minimum number of total reads threshold of at least 3 million, 4 million, 5 million, 6 million, 7 million, 8 million, 9 million, or more unique sequence reads. In some embodiments, the criterion is implemented as a range of acceptable number of unique reads, *e.g.,* requiring that the sequencing reaction generate from 2 million to 10 million total sequence reads, from 3 million to 9 million sequence reads, from 3 million to 9 million sequence reads, and the like.

- A criterion for unique read depth across the panel, defined as a measure of central tendency (e.g., a mean or median) for a distribution of the number of unique reads in the sequencing reaction encompassing the genomic regions targeted by each probe. The average unique read depth is calculated for each targeted region defined in a target region BED file, using a formula of "# of reads mapped to the region" * "read length" / "length of the region" if the "length of the region" is longer than "read length", or otherwise using a formula of "# of reads fall in the region" * "read length". The median of unique read depth across the panel is then calculated as the median of those average unique read depths of all targeted regions. In some embodiments, the resolution as to how depth is calculated is increased or decreased, *e.g.,* in cases where it is necessary or desirable to calculate depth for each base, or for a single gene. Generally, a unique read depth threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a minimum unique read depth threshold of at least 1500, 1750, 2000, 2250, 2500, 2750, 3000, 3250, 3500, or higher unique read depth. In some embodiments, the criterion is implemented as a range of acceptable unique read depth, e.g., requiring that the sequencing reaction generate a unique read depth of from 1000 to 4000, from 1500 to 4000, from 1500 to 4000, and the like.

- A criterion for the unique read depth of a lowest percentile across the panel, defined as a measure of central tendency (e.g., a mean or median) for a distribution of the number of unique reads in the sequencing reaction encompassing the genomic regions targeted by each probe that fall within the lowest percentile of genomic regions by read depth (*e.g.,* the first, second, third, fourth, fifth, tenth, fifteenth, twentieth, twenty-fifth, or similar percentile). Generally, a unique read depth at a lowest percentile threshold will be selected based on the sequencing technology used, the size of the targeted panel, the lowest percentile selected, and the expected number of sequence reads generated by the combination of the technology and targeted panel used. For example, in some embodiments where next generation sequencing-by-synthesis technology is used, the criterion is implemented as a minimum unique read depth threshold at the fifth percentile of at least 500, 750, 1000, 1250, 1500, 1750, 2000, 2250, 2500, or higher unique read depth. In some embodiments, the criterion is implemented as a range of acceptable unique read depth at the fifth percentile, *e.g.,* requiring that the sequencing reaction generate a unique read depth at the fifth percentile of from 250 to 3000, from 500 to 3000, from 500 to 2500, and the like.

- A criterion for the deamination or OxoG Q-score of a sequencing reaction, defined as a Q-score for the occurrence of artifacts arising from template oxidation/deamination. Generally, a deamination or OxoG Q-score threshold will be selected based on the sequencing technology used. For example, in some embodiments where next generation sequencing-by-synthesis technology is used, the criterion is implemented as a minimum deamination or OxoG Q-score threshold of at least 10, 20, 30, 40, 5,0 6,0 70, 80, 90, or higher. In some embodiments, the criterion is implemented as a range of acceptable deamination or OxoG Q-scores, e.g., from 10 to 100, from 10 to 90, and the like.

- A criterion for the estimated contamination fraction is of a sequencing reaction, defined as an estimate of the fraction of template fragments in the sample being sequenced arising from contamination of the sample, commonly expressed as a decimal, e.g., where 1% contamination is expressed as 0.01. An example method for estimating contamination in a sequencing method is described in Jun G. et al., Am. J. Hum. Genet., 91:839-48 (2012). For example, in some embodiments, the criterion is implemented as a maximum contamination fraction threshold of no more than 0.001, 0.0015, 0.002, 0.0025, 0.003, 0.0035, 0.004. In some embodiments, the criterion is implemented as a range of acceptable contamination fractions, *e.g.,* from 0.0005 to 0.005, from 0.0005 to 0.004, from 0.001 to 0.004, and the like.

- A criterion for the fingerprint correlation score of a sequencing reaction, defined as a pearson correlation coefficient calculated between the variant allele fractions of a set of pre-defined single nucleotide polymorphisms (SNPs) in two samples. An example method for determining a fingerprint correlation score is described in Sejoon L. et al., Nucleic Acids Research, Volume 45, Issue 11, 20 June 2017, Page e103. For example, in some embodiments, the criterion is implemented as a minimum fingerprint correlation score threshold of at least 0.1, 0.2, 0.3, 0.4, 0.5, 0.6, 0.7, 0.8, 0.9, or higher. In some embodiments, the criterion is implemented as a range of acceptable fingerprint correlation scores, *e.g.,* from 0.1 to 0.9, from 0.2 to 0.9, from 0.3 to 0.9, and the like.

- A criterion for the raw coverage of a minimum percentage of the genomic regions targeted by a probe, defined as a minimum number of unique reads in the sequencing reaction encompassing each of a minimum percentage (*e.g.,* at least 80%, 85%, 90%, 95%, 98%, 99%, 99.5%, 99.9%, and the like) of the genomic regions targeted by the probe panel. In some embodiments, the term "unique read depth" is used to distinguish deduplicated reads from raw reads that may contain multiple reads sequenced from the same original DNA molecule via PCR. Generally, a raw coverage of a minimum percentage of the genomic regions targeted by a probe threshold will be selected based on the sequencing technology used, the size of the targeted panel, the minimum percentage selected, and the expected number of sequence reads generated by the combination of the technology and targeted panel used. For example, in some embodiments where next generation sequencing-by-synthesis technology is used, the criterion is implemented as a raw coverage of 95% of the genomic regions targeted by a probe threshold of at least 500, 750, 1000, 1250, 1500, 1750, 2000, 2250, 2500, or higher unique read depth. In some embodiments, the criterion is implemented as a range of acceptable unique read depth for 95% of the genomic regions targeted by a probe, e.g., requiring that the sequencing reaction generate a unique read depth for 95% of the targeted regions of from 250 to 3000, from 500 to 3000, from 500 to 2500, and the like.

- A criterion for the PCR duplication rate of a sequencing reaction, defined as the percentage of sequence reads that arise from the same template molecule as at least one other sequence read generated by the reaction. Generally, a PCR duplication rate threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a minimum PCR duplication rate threshold of at least 91%, 92% ,93%, 94%, 95%, 96%, 97%, 98%, 99%, or higher. In some embodiments, the criterion is implemented as a range of acceptable PCR duplication rates, *e.g.,* of from 90% to 100%, from 90% to 99%, from 91% to 99%, and the like.

Similarly, in some embodiments, the quality control system includes methods that fail samples for reporting if various criteria are met. In some embodiments, the system includes methods that allow for more manual review if a sample does meet the criteria established for automatic fail. In some embodiments, the criteria for failing panel sequencing results include one or more of the following:

- A criterion for the on-target rate of the sequencing reaction, defined as a comparison (*e.g.,* a ratio) of (i) the number of sequenced nucleotides or reads falling within the targeted panel region of a genome and (ii) the number of sequenced nucleotides or reads falling outside of the targeted panel region of the genome. Generally, an on-target rate threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a maximum on-target rate threshold of no more than 30%, 40%, 50%, 60%, 70%, or greater. That is, the criterion for failing the sample is satisfied when the on-target rate for the sequencing reaction is below the maximum on-target rate threshold. In some embodiments, the on-target rate criteria is implemented as not falling within a range of acceptable on-target rates, *e.g.,* falling outside of an on-target rate for a reaction of from 30% to 70%, from 30% to 80%, from 40% to 70%, from 40% to 80%, and the like.

- A criterion for the number of total reads generated by the sequencing reaction, including both unique sequence reads and non-unique sequence reads. Generally, a total read number threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a maximum number of total reads threshold of no more than 100 million, 110 million, 120 million, 130 million, 140 million, 150 million, 160 million, 170 million, 180 million, 190 million, 200 million, or more total sequence reads. That is, the criterion for failing the sample is satisfied when the number of total reads for the sequencing reaction is below the maximum total read threshold. In some embodiments, the criterion is implemented as not falling within a range of acceptable number of total reads, *e.g.,* falling outside of a range of from 50 million to 300 million total sequence reads, from 100 million to 300 million sequence reads, from 100 million to 200 million sequence reads, and the like.

- A criterion for the number of unique reads generated by the sequencing reaction. Generally, a unique read number threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a maximum number of total reads threshold of no more than 3 million, 4 million, 5 million, 6 million, 7 million, 8 million, 9 million, or more unique sequence reads. That is, the criterion for failing the sample is satisfied when the number of unique reads for the sequencing reaction is below the maximum total read threshold. In some embodiments, the criterion is implemented as not falling within a range of acceptable number of unique reads, e.g., falling outside of a range of from 2 million to 10 million total sequence reads, from 3 million to 9 million sequence reads, from 3 million to 9 million sequence reads, and the like.

- A criterion for unique read depth across the panel, defined as a measure of central tendency (*e.g.,* a mean or median) for a distribution of the number of unique reads in the sequencing reaction encompassing the genomic regions targeted by each probe. Generally, a unique read depth threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a maximum unique read depth threshold of no more than 1500, 1750, 2000, 2250, 2500, 2750, 3000, 3250, 3500, or higher unique read depth. That is, the criterion for failing the sample is satisfied when the unique read depth across the panel for the sequencing reaction is below the maximum total read threshold. In some embodiments, the criterion is implemented as falling outside of a range of acceptable unique read depth, *e.g.,* falling outside of a unique read depth range of from 1000 to 4000, from 1500 to 4000, from 1500 to 4000, and the like.

- A criterion for the unique read depth of a lowest percentile across the panel, defined as a measure of central tendency (*e.g.,* a mean or median) for a distribution of the number of unique reads in the sequencing reaction encompassing the genomic regions targeted by each probe that fall within the lowest percentile of genomic regions by read depth (*e.g.,* the first, second, third, fourth, fifth, tenth, fifteenth, twentieth, twenty-fifth, or similar percentile). Generally, a unique read depth at a lowest percentile threshold will be selected based on the sequencing technology used, the size of the targeted panel, the lowest percentile selected, and the expected number of sequence reads generated by the combination of the technology and targeted panel used. For example, in some embodiments where next generation sequencing-by-synthesis technology is used, the criterion is implemented as a maximum unique read depth threshold at the fifth percentile of no more than 500, 750, 1000, 1250, 1500, 1750, 2000, 2250, 2500, or higher unique read depth. That is, the criterion for failing the sample is satisfied when the unique read depth at a lowest percentile threshold for the sequencing reaction is below the maximum unique read depth at a lowest percentile threshold. In some embodiments, the criterion is implemented as falling outside of a range of acceptable unique read depth at the fifth percentile, e.g., falling outside of a unique read depth at the fifth percentile range of from 250 to 3000, from 500 to 3000, from 500 to 2500, and the like.

- A criterion for the deamination or OxoG Q-score of a sequencing reaction, defined as a Q-score for the occurrence of artifacts arising from template oxidation/deamination. Generally, a deamination or OxoG Q-score threshold will be selected based on the sequencing technology used. For example, in some embodiments where next generation sequencing-by-synthesis technology is used, the criterion is implemented as a maximum deamination or OxoG Q-score threshold of no more than 10, 20, 30, 40, 5,0 6,0 70, 80, 90, or higher. That is, the criterion for failing the sample is satisfied when the deamination or OxoG Q-score for the sequencing reaction is below the maximum deamination or OxoG Q-score threshold. In some embodiments, the criterion is implemented as falling outside of a range of acceptable deamination or OxoG Q-scores, *e.g.,* falling outside of a deamination or OxoG Q-score range of from 10 to 100, from 10 to 90, and the like.

- A criterion for the estimated contamination fraction is of a sequencing reaction, defined as an estimate of the fraction of template fragments in the sample being sequenced arising from contamination of the sample, commonly expressed as a decimal, e.g., where 1% contamination is expressed as 0.01. An example method for estimating contamination in a sequencing method is described in Jun G. et al., Am. J. Hum. Genet., 91:839-48 (2012). For example, in some embodiments, the criterion is implemented as a minimum contamination fraction threshold of at least 0.001, 0.0015, 0.002, 0.0025, 0.003, 0.0035, 0.004. That is, the criterion for failing the sample is satisfied when the contamination fraction for the sequencing reaction is above the minimum contamination fraction threshold. In some embodiments, the criterion is implemented as falling outside of a range of acceptable contamination fractions, e.g., falling outside of a contamination fraction range of from 0.0005 to 0.005, from 0.0005 to 0.004, from 0.001 to 0.004, and the like.

- A criterion for the fingerprint correlation score of a sequencing reaction, defined as a pearson correlation coefficient calculated between the variant allele fractions of a set of pre-defined single nucleotide polymorphisms (SNPs) in two samples. An example method for determining a fingerprint correlation score is described in Sejoon L. et al., Nucleic Acids Research, Volume 45, Issue 11, 20 June 2017, Page e103. For example, in some embodiments, the criterion is implemented as a maximum fingerprint correlation score threshold of no more than 0.1, 0.2, 0.3, 0.4, 0.5, 0.6, 0.7, 0.8, 0.9, or higher. That is, the criterion for failing the sample is satisfied when the fingerprint correlation score for the sequencing reaction is below the maximum fingerprint correlation score threshold. In some embodiments, the criterion is implemented as falling outside of a range of acceptable fingerprint correlation scores, e.g., falling outside of a fingerprint correlation range of from 0.1 to 0.9, from 0.2 to 0.9, from 0.3 to 0.9, and the like.

- A criterion for the raw coverage of a minimum percentage of the genomic regions targeted by a probe, defined as a minimum number of unique reads in the sequencing reaction encompassing each of a minimum percentage (*e.g.,* at least 80%, 85%, 90%, 95%, 98%, 99%, 99.5%, 99.9%, and the like) of the genomic regions targeted by the probe panel. Generally, a raw coverage of a minimum percentage of the genomic regions targeted by a probe threshold will be selected based on the sequencing technology used, the size of the targeted panel, the minimum percentage selected, and the expected number of sequence reads generated by the combination of the technology and targeted panel used. For example, in some embodiments where next generation sequencing-by-synthesis technology is used, the criterion is implemented as a raw coverage of 95% of the genomic regions targeted by a probe threshold of no more than 500, 750, 1000, 1250, 1500, 1750, 2000, 2250, 2500, or higher unique read depth. That is, the criterion for failing the sample is satisfied when the raw coverage of a minimum percentage of the genomic regions targeted by a probe for the sequencing reaction is below the maximum raw coverage of a minimum percentage of the genomic regions targeted by a probe threshold. In some embodiments, the criterion is implemented as falling outside of a range of acceptable unique read depth for 95% of the genomic regions targeted by a probe, *e.g.,* requiring that the sequencing reaction generate a unique read depth for 95% of the targeted regions falling outside of a range of from 250 to 3000, from 500 to 3000, from 500 to 2500, and the like.

- A criterion for the PCR duplication rate of a sequencing reaction, defined as the percentage of sequence reads that arise from the same template molecule as at least one other sequence read generated by the reaction. Generally, a PCR duplication rate threshold will be selected based on the sequencing technology used, the size of the targeted panel, and the expected number of sequence reads generated by the combination of the technology and targeted panel used. For example, in some embodiments where next generation sequencing-by-synthesis technology is used, the criterion is implemented as a maximum PCR duplication rate threshold of at least 91%, 92% ,93%, 94%, 95%, 96%, 97%, 98%, 99%, or higher. That is, the criterion for failing the sample is satisfied when the PCR duplication rate for the sequencing reaction is below the maximum PCR duplication rate threshold. In some embodiments, the criterion is implemented as falling outside of a range of acceptable PCR duplication rates, *e.g.,* of from 90% to 100%, from 90% to 99%, from 91% to 99%, and the like.

Thresholds for the auto-pass and auto-fail criteria may be established with reference to one another, but are not necessarily set at the same level. For instance, in some embodiments, samples with a metric that falls between auto-pass and auto-fail criteria may be routed for manual review by a qualified bioinformatics scientist. Samples that are failed either automatically or by manual review may be routed to medical and laboratory teams for final review and can be released for downstream processing at the discretion of the laboratory medical director or designee.

Systems and Methods for Improved Microsatellite Stability Classification

An overview of methods for providing clinical support for personalized cancer therapy is described above with reference to Figures 2-4 above. Below, systems and methods for improving microsatellite stability classification, *e.g.,* within the context of the methods and systems described above, are described with reference to Figures 4F, 4G, 19A-19B, 20A-20B, 21A-21C, 22A-22C, 23A-23B, 24A-24B, 25A-25C, and 29A-29C.

Many of the embodiments described below, in conjunction with Figures 4F, 4G, 19A-19B, 20A-20B, 21A-21C, 22A-22C, 23A-23B, 24A-24B, 25A-25C, and 29A-29C, relate to analyses performed using sequencing data for cfDNA obtained from a liquid biopsy sample of a cancer patient. Generally, these embodiments are independent and, thus, not reliant upon any particular DNA sequencing methods. However, in some embodiments, the methods described below include generating the sequencing data.

The disclosure provides a method 400 of determining a microsatellite instability (MSI) status of a cancer in a subject from a liquid biopsy sample of the subject, as illustrated in Figure 4F. The method includes obtaining a plurality of aligned nucleotide sequences (406) from a sequencing reaction of cell-free DNA isolated from a liquid biopsy sample obtained from the subject. As described above, in some embodiments, the methods described herein include one or more of obtaining the liquid biopsy sample from the subject, isolating cell-free DNA molecules from the liquid biopsy sample, obtaining sequencing data for the isolated cell-free DNA molecules (402), and aligning the sequencing data to a reference human genetic construct (404), e.g., a reference human genome, exome, or assay-specific panel of human genomic regions. In other embodiments, the methods described herein start by electronically obtaining the aligned sequencing data.

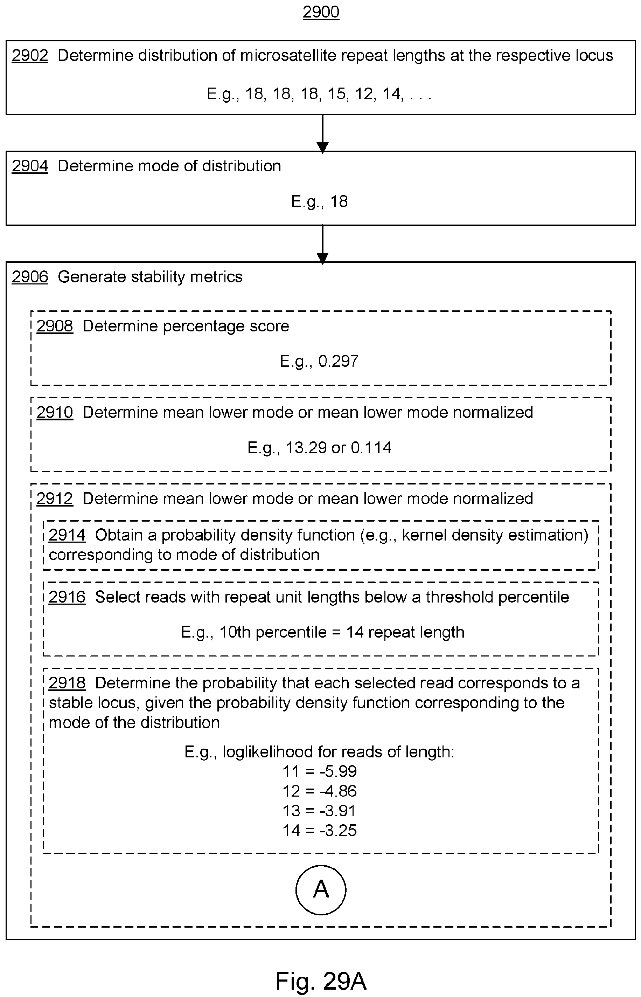

Method 400 then includes generating one or more repeat unit stability metrics for each of a plurality of microsatellite loci (408). In some embodiments, as described in more details herein, the one or more repeat unit metrics 410 include a percentage lower metric 412, a mean lower mode (or normalized mean lower mode) metric 414, and/or a likelihood metric 416. In some embodiments, e.g., as described in Example 2 and with reference to Figures 29A-29C, the method includes determining each of a percentage lower metric 412, a mean lower mode (or normalized mean lower mode) metric 414, and/or a likelihood metric 416, for each of the plurality of microsatellite loci. In some embodiments, the metric values are normalized (418) to generate normalized repeat unit metrics 420 for each microsatellite locus.

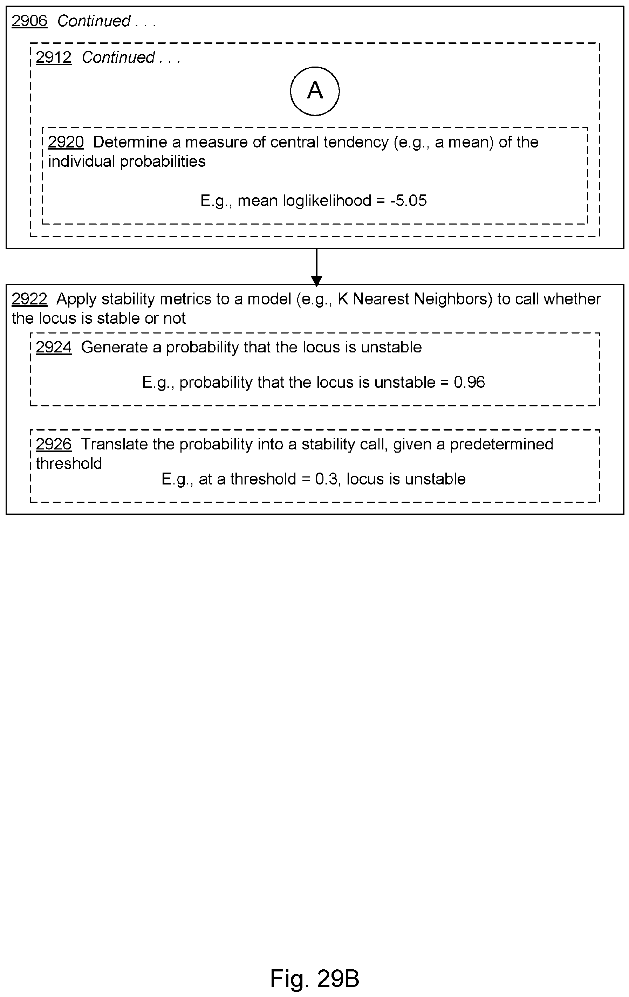

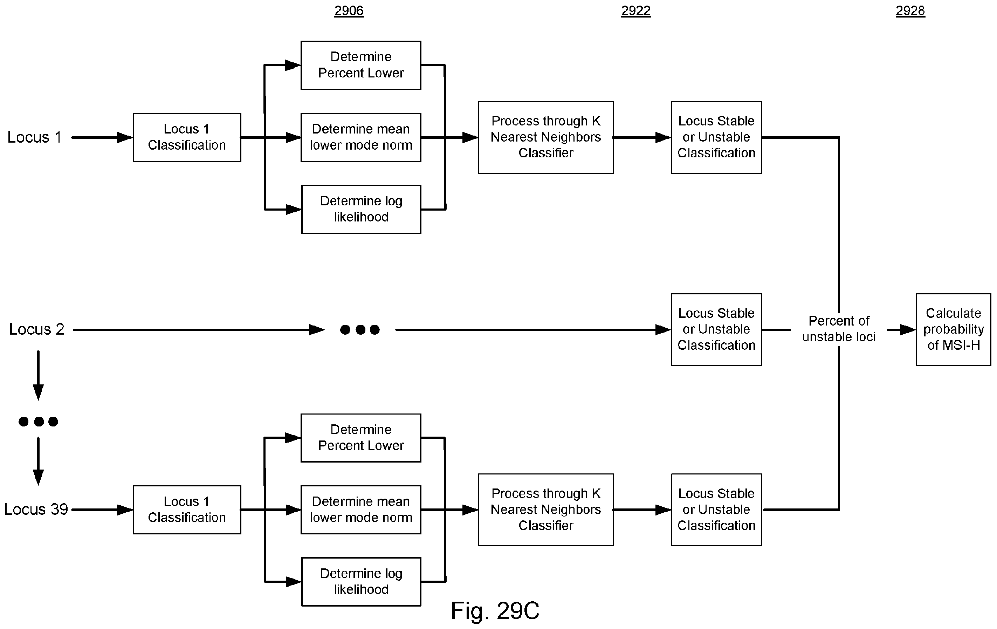

Method 400 then includes using the repeat unit metrics 410 or normalized repeat unit metrics 420 in order to classify the microsatellite stability of the cancer of the subject, e.g., as microsatellite stable (MSS) or microsatellite unstable (MSI-H). In some embodiments, e.g., as described in Example 2 and with reference to Figures 29A-29C, the repeat unit metrics 410 or normalized repeat unit metrics 420 are used to determine the stability status of individual microsatellite loci, and then classify the cancer of the subject based on these individual determinations at each loci. For instance, as illustrated in Figure 4G and Figure 29, and described below with reference to Example 2, a stability call is made for each of the 39 microsatellite loci listed in Table 2 (e.g., determinations made at steps 462 in Figure 4G and 2922 in Figure 29). These individual stability calls are then used in the aggregate to determine the MSI status of the cancer, for example by determining whether a threshold number of individual loci were determined to be unstable.

In some embodiments, the determination of whether individual loci are stable or unstable is made using a training set that includes sequencing data from biological samples with stable microsatellite loci, e.g., normal tissue samples and/or solid tumor samples of microsatellite stable cancers, and sequencing data from biological samples with unstable microsatellite loci, e.g., solid tumor samples of microsatellite unstable cancers. In some embodiments, a vector containing values for the repeat unit metrics 410 for a respective microsatellite loci is input into a K Nearest Neighbors algorithm populated (trained) with vectors containing values for the repeat unit metrics 410 determined from the training set. The process is repeated for each of the microsatellite loci, e.g., using a different KNN algorithm for each respective microsatellite locus trained against repeat unit metrics 410 generated for the respective microsatellite locus across the training data.

In some embodiments, method 400 includes matching (424) one or more therapies and/or one or more clinical trials to the subject based on the MSI status of the cancer. In some embodiments, method 400 includes generating a patient report 426 indicating the MSI status determined for the cancer and/or reporting one or more matched therapies and/or clinical trials based on the MSI status of the subject. Of course, the patient report 426 may contain additional classifications of the cancer, matched therapies, and/or matched clinical trials that are based on evaluation of other features of the liquid biopsy sequencing reaction, e.g., allelic states 132, variant allele fractions 133, methylation states, 134, genomic copy numbers 135, tumor mutational burden 136, tumor ploidy 137-2, HRD status 137-3, and the like.

Figure 4G illustrates a related method 450. Briefly, the method includes obtaining a plurality of aligned nucleotide sequences (e.g., BAM files 452) from a liquid biopsy sequencing assay. The method includes extracting (452) aligned nucleotide sequences for each of a plurality of microsatellite loci (e.g., including a subset or all of the loci listed in Table 2), and counting (454) the number of repeat units at each locus. As described with reference to method 400, a plurality of features (e.g., MSI stability metrics 412, 414, and/or 416) are generated (452) from the counts of the number of repeat units. The features generated for each locus are then applied (458) to a stability model specific for the particular locus, to generate a probability 460 that the particular locus is stable or unstable. Based on this probability, the individual locus is called (462) as either stable or unstable.

In some embodiments, the determination of whether individual loci are stable or unstable is made using a training set that includes sequencing data from biological samples with stable microsatellite loci, e.g., normal tissue samples and/or solid tumor samples of microsatellite stable cancers, and sequencing data from biological samples with unstable microsatellite loci, e.g., solid tumor samples of microsatellite unstable cancers. In some embodiments, a vector containing values for the repeat unit metrics 410 for a respective microsatellite loci is input into a K Nearest Neighbors algorithm populated (trained) with vectors containing values for the repeat unit metrics 410 determined from the training set. The process is repeated for each of the microsatellite loci, e.g., using a different KNN algorithm for each respective microsatellite locus trained against repeat unit metrics 410 generated for the respective microsatellite locus across the training data.

Then, the individual stability calls for each locus are used together to determine (462) the microsatellite stability status for the cancer of the subject. In some embodiments, the determination is made based on a threshold number of unstable microsatellite loci. For instance, in some embodiments, when at least 20% of the individual microsatellite loci are determined to be unstable, the cancer of the subject is classified as unstable. In other embodiments, when at least 25%, at least 30%, at least 35%, at least 40%, at least 45%, at least 50%, at least 55%, at least 60%, at least 65%, or at least 70% of the individual microsatellite loci are determined to be unstable, the cancer of the subject is classified as unstable.

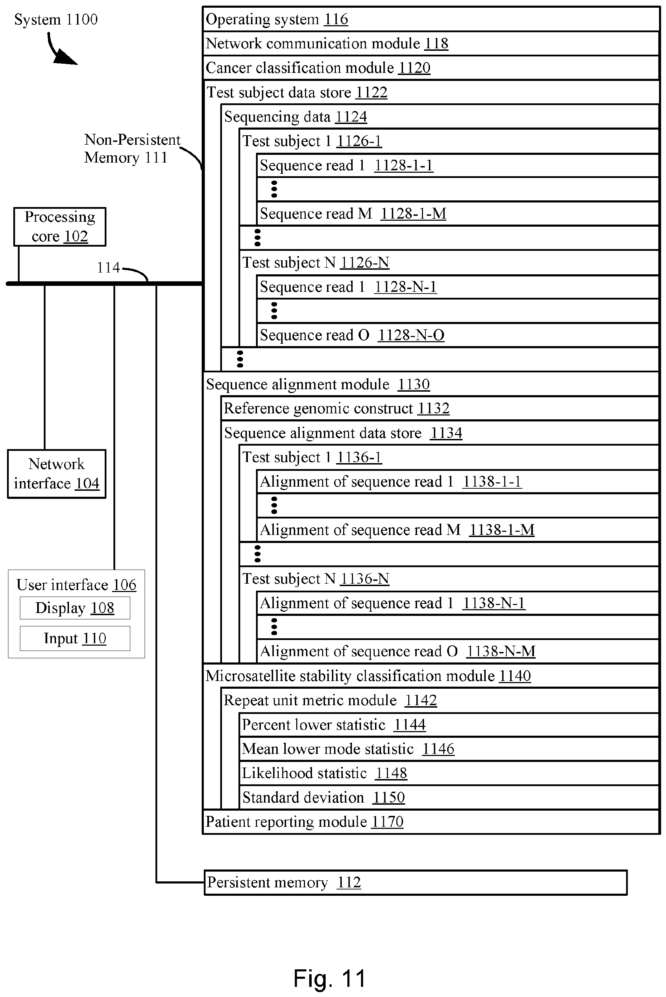

The disclosure provides a method 1900 of determining a microsatellite instability (MSI) status of a subject from a liquid biopsy sample of the subject by comparing metrics derived from the distribution of lengths of a plurality of microsatellite loci against metrics derived from the distribution of lengths of the same microsatellite loci from solid tissue samples (e.g., of tissues with stable microsatellite loci and/or unstable microsatellite loci) from a cohort of training subjects. Advantageously, it was found that training a classifier for a liquid biopsy MSI assay with training data from solid biopsy samples improved the performance of the liquid biopsy classifier. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 1902, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 1904-1908, in some embodiments, the obtaining includes, for each respective DNA fragment in the plurality of DNA fragments, obtaining a corresponding set of raw sequence reads for the respective DNA fragment (Block 1904). Each respective raw sequence read in the set of raw sequence reads is aligned to the reference genome for the test subject, thus obtaining a corresponding set of aligned raw sequence reads for the respective DNA fragment (Block 1906). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (Block 1908).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

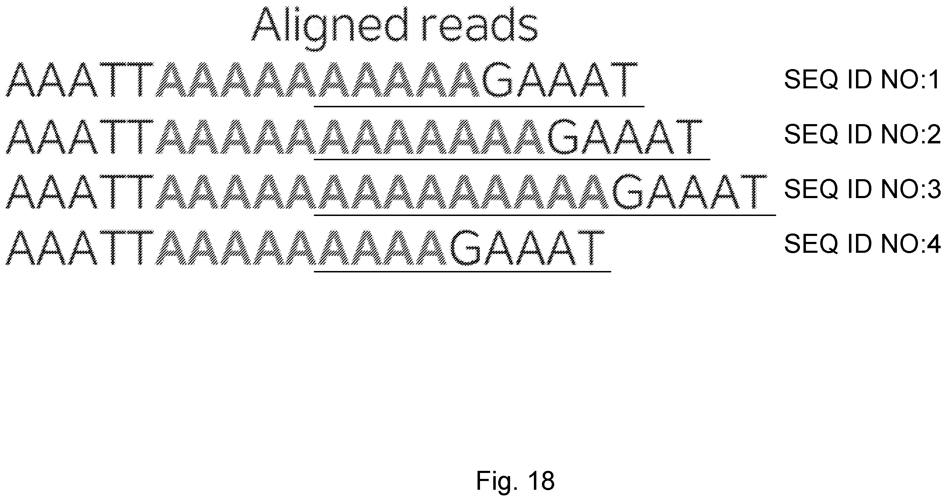

Referring to Blocks 1912-1920, method 1900 includes using the plurality of aligned nucleotide sequences to determine, for each respective microsatellite locus in a plurality of predetermined microsatellite loci a corresponding metric value for each of one or more metrics. Each respective microsatellite locus in the plurality of microsatellite loci has a nucleic acid sequence that includes a variable number of repeat units. Each respective metric in the one or more metrics is determined, at least in part, by a distribution of the number of repeat units in each aligned nucleotide sequence, in the plurality of aligned nucleotide sequences, encompassing the respective microsatellite locus. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

Referring to Block 1914, in some embodiments, the one or more metrics comprises a percentage score. The corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

Referring to Block 1916, in some embodiments, the one or more metrics comprises a lower mean score. The corresponding metric value for the lower mean score is determined at least in part by the mean number of repeat units in aligned nucleotide sequences, in the plurality of aligned nucleotide sequences, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

Referring to Block 1918, in some embodiments, the one or more metrics comprises a likelihood score. The corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirtyfifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

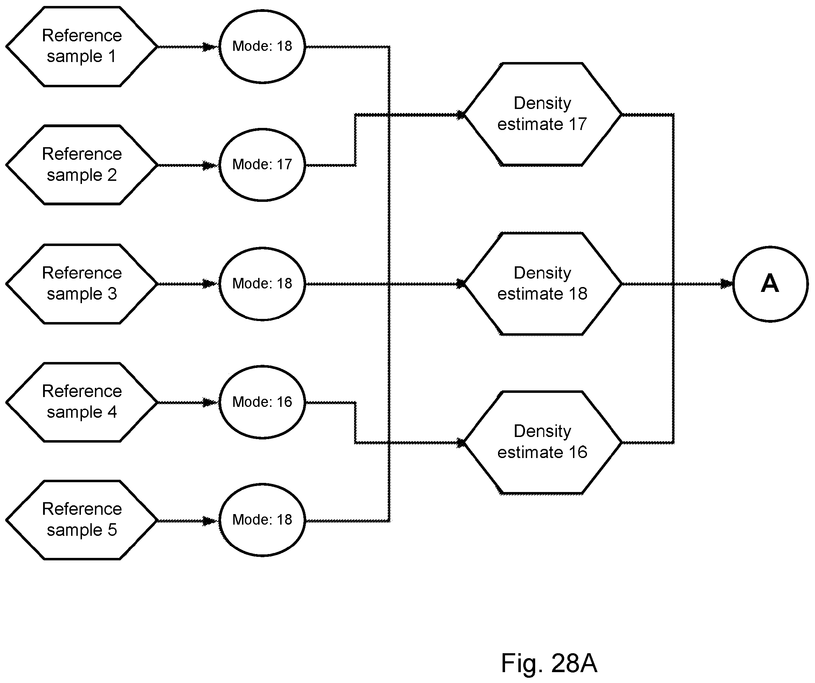

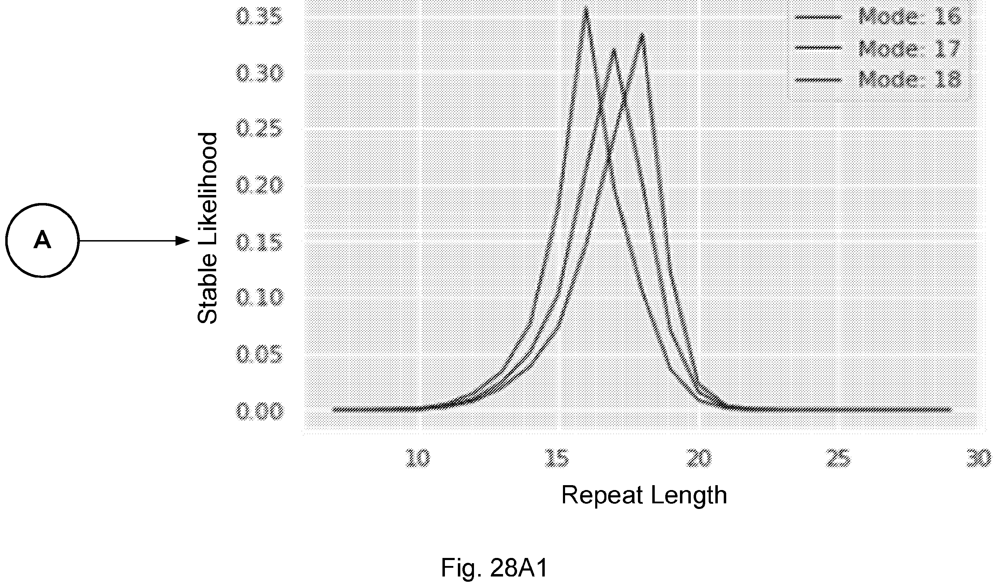

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

Referring to Block 1920, in some embodiments, the one or more corresponding metric values comprises a corresponding standard deviation of the number of repeat units in the aligned nucleotide sequences, in the plurality of aligned nucleotide sequences, encompassing the respective microsatellite locus. For instance, the set of nucleotide sequences shown in Figure 18 would have a standard deviation of 1.92.

In some embodiments, the one or more metrics includes at least two of the metrics described herein. For instance, in some embodiments, the one or more metrics include a percentage score and a lower mean score. In some embodiments, the one more or more metrics include a percentage score and a likelihood score. In some embodiments, the one of more metrics include a percentage score and a standard deviation. In some embodiments, the one or more metrics includes a lower mean score and a likelihood score. In some embodiments, the one or more metrics includes a lower mean score and a standard deviation. In some embodiments, the one or more metrics includes a likelihood score and a standard deviation.

In some embodiments, the one or more metrics includes at least three of the metrics described herein. For instance, in some embodiments, the one or more metrics include a percentage score, a lower mean score, and a likelihood score. In some embodiments, the one or more metrics include a percentage score, a lower mean score, and a standard deviation. In some embodiments, the one or more metrics include a percentage score, a likelihood score, and a standard deviation. In some embodiments, the one or more metrics include a lower mean score, a likelihood score, and a standard deviation. In some embodiments, the one or more metrics includes at least the percentage score, the lower mean score, the likelihood score, and standard deviation, as described herein.

Referring to Block 1922, in some embodiments the method further includes normalizing one or more of the metrics across the plurality of microsatellite loci. For example, in some embodiments, a power transform function is used to normalize the one or more metrics.

Referring to Block 1924, method 1900 includes comparing for each respective microsatellite locus in the plurality of predetermined microsatellite loci, the corresponding metric values for the one or more metrics to corresponding reference metric values for the one or more metrics representative of a stable construct at the respective microsatellite locus. The reference metric values are determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the comparing include inputting, for each respective microsatellite locus in the plurality of microsatellite loci, the corresponding metric value for each of the one or more metrics to a classifier trained to distinguish between stable and unstable microsatellite loci, where the classifier was trained using the reference metric values determined across the nucleotide sequences obtained from the samples from cells with stable microsatellites, e.g., biological samples from solid tumor samples from training subjects with stable microsatellite cancer and/or normal tissue samples. In some embodiments, the classifier is trained against both solid tumor samples from training subjects with stable microsatellite cancer and normal tissue samples. In some embodiments, the classifier is also trained against reference metric values determined across nucleotide sequences obtained from solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci.

The disclosure provides a method 2000 of determining a microsatellite instability (MSI) status of a subject from a liquid biopsy sample of the subject using two or more novel metrics of microsatellite stability, as described herein, derived from the distribution of lengths of a plurality of microsatellite loci. Advantageously, it was found that using multiple metrics for microsatellite stability improved the overall performance of the classifier. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2002, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 2004-2008, in some embodiments, the obtaining includes, for each respective DNA fragment in the plurality of DNA fragments, obtaining a corresponding set of raw sequence reads for the respective DNA fragment (Block 2004). Each respective raw sequence read in the set of raw sequence reads is aligned to the reference genome for the test subject, thus obtaining a corresponding set of aligned raw sequence reads for the respective DNA fragment (Block 2006). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (Block 2008).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Blocks 2012-2020, method 2000 includes using the plurality of aligned nucleotide sequences to determine, for each respective microsatellite locus in a plurality of predetermined microsatellite loci, where each respective microsatellite locus in the plurality of microsatellite loci has a nucleic acid sequence that includes a variable number of repeat units, a corresponding metric value for each of two or more independent metrics. Each respective metric in the two or more metrics is determined, at least in part, by a distribution of the number of repeat units in each aligned nucleotide sequence, in the plurality of nucleotide sequences, encompassing the respective microsatellite locus. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

Referring to Block 2014, in some embodiments, the two or more metrics comprises a percentage score. The corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

Referring to Block 2016, in some embodiments, the two or more metrics comprises a lower mean score. The corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

Referring to Block 2018, in some embodiments, the two or more metrics comprises a likelihood score. The corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirtyfifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

Referring to Block 2020, in some embodiments, the two or more corresponding metric values comprises a corresponding standard deviation of the number of repeat units in the aligned nucleotide sequences, in the plurality of aligned nucleotide sequences, encompassing the respective microsatellite locus. For instance, the set of nucleotide sequences shown in Figure 18 would have a standard deviation of 1.92.

In some embodiments, the two or more metrics include a percentage score and a lower mean score. In some embodiments, the two more or more metrics include a percentage score and a likelihood score. In some embodiments, the two of more metrics include a percentage score and a standard deviation. In some embodiments, the two or more metrics includes a lower mean score and a likelihood score. In some embodiments, the two or more metrics includes a lower mean score and a standard deviation. In some embodiments, the two or more metrics includes a likelihood score and a standard deviation.

In some embodiments, the two or more metrics includes at least three of the metrics described herein. For instance, in some embodiments, the two or more metrics include a percentage score, a lower mean score, and a likelihood score. In some embodiments, the two or more metrics include a percentage score, a lower mean score, and a standard deviation. In some embodiments, the two or more metrics include a percentage score, a likelihood score, and a standard deviation. In some embodiments, the two or more metrics include a lower mean score, a likelihood score, and a standard deviation. In some embodiments, the two or more metrics includes at least the percentage score, the lower mean score, the likelihood score, and standard deviation, as described herein.

Referring to Block 2022, in some embodiments the method further includes normalizing one or more of the metrics across the plurality of microsatellite loci. For example, in some embodiments, a power transform function is used to normalize the one or more metrics.

Referring to Block 2024, method 2000 includes classifying the MSI status of the test subject by inputting, for each respective microsatellite locus in the plurality of predetermined microsatellite loci, the corresponding metric values for the two or more metrics to a classifier that distinguishes between stable and unstable microsatellite loci, thereby determining the MSI status of the test subject.

In some embodiments, the classifier is trained against metric values determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci.

The disclosure provides a method 2100 of determining a microsatellite instability (MSI) status of a subject using a percent lower statistic, as described herein. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2102, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 2104-2108, in some embodiments, the obtaining includes, for each respective DNA fragment in the plurality of DNA fragments, obtaining a corresponding set of raw sequence reads for the respective DNA fragment (Block 2104). Each respective raw sequence read in the set of raw sequence reads is aligned to the reference genome for the test subject, thus obtaining a corresponding set of aligned raw sequence reads for the respective DNA fragment (Block 2106). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (Block 2108).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Block 2110, the method includes using the plurality of aligned nucleotide sequences to determine, for each respective microsatellite locus in a plurality of predetermined microsatellite loci, a corresponding metric value for a percentage score metric. Each respective microsatellite locus in the plurality of microsatellite loci has a nucleic acid sequence that includes a variable number of repeat units. The percentage score metric is determined, at least in part, by the percentage of sequence reads, in the set of sequence reads encompassing the respective microsatellite locus in the plurality of sequence reads, having a number of repeat units that is less than a predetermined number of repeat units (*e.g.,* a reference number of repeat units) representative of a stable construct at the respective microsatellite locus. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units. Assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

In some embodiments, referring to Block 2111, the method also includes determining one or more additional metrics of microsatellite stability for each of the microsatellite loci. For instance, in some embodiments, referring to Block 2112, the method further includes using the plurality of sequence reads to determine, for each respective microsatellite locus in the plurality of predetermined microsatellite loci, a corresponding metric value for a lower mean score metric. The lower mean score metric is determined, at least in part, by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. In some such embodiments, the one or more metrics used in the classifying a MSI status of the subject comprise the lower mean score for the respective microsatellite locus. For instance, assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

Similarly referring to Block 2114, in some embodiments, the method further includes using the first plurality of sequence reads to determine, for each respective microsatellite locus in the plurality of predetermined microsatellite loci, a corresponding metric value for a likelihood score metric. The likelihood score metric is determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units. In some such embodiments, the one or more metrics used in the classifying a MSI status of the subject comprise the likelihood score for the respective microsatellite locus.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirtyfifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

Similarly referring to Block 2116, in some embodiments, the method further includes using the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. In some such embodiments, the one or more metrics used in the classifying a MSI status of the subject comprise the corresponding standard deviation for the respective microsatellite locus. For instance, the set of nucleotide sequences shown in Figure 18 would have a standard deviation of 1.92.

In some embodiments, two or more metrics are used. For instance, in some embodiment, the two or more metrics include a percentage score and a lower mean score. In some embodiments, the two more or more metrics include a percentage score and a likelihood score. In some embodiments, the two of more metrics include a percentage score and a standard deviation. In some embodiments, the two or more metrics includes a lower mean score and a likelihood score. In some embodiments, the two or more metrics includes a lower mean score and a standard deviation. In some embodiments, the two or more metrics includes a likelihood score and a standard deviation.

In some embodiments, three or more metrics are used. For instance, in some embodiments, the three or more metrics include a percentage score, a lower mean score, and a likelihood score. In some embodiments, the three or more metrics include a percentage score, a lower mean score, and a standard deviation. In some embodiments, the three or more metrics include a percentage score, a likelihood score, and a standard deviation. In some embodiments, the three or more metrics include a lower mean score, a likelihood score, and a standard deviation. In some embodiments, the three or more metrics includes at least the percentage score, the lower mean score, the likelihood score, and standard deviation, as described herein.

Referring to Block 2118, in some embodiments the method further includes normalizing the one or more metrics across the plurality of microsatellite loci. For example, in some embodiments, a power transform function is used to normalize the one or more metrics.

Referring to Block 2120, the method further includes classifying the MSI status of the test subject by inputting, for each respective microsatellite locus in the plurality of predetermined microsatellite loci, a corresponding metric value for each of one or more metrics, including the corresponding percentage score, to a classifier that distinguishes between stable and unstable microsatellite loci, thus determining the MSI status of the test subject.

In some embodiments, the classifier is trained against metric values determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci.

The disclosure provides a method 2200 of determining a microsatellite instability (MSI) status of a subject using a lower mean score metric, as described herein. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2202, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 2204-2208, in some embodiments, the obtaining includes, for each respective DNA fragment in the plurality of DNA fragments, obtaining a corresponding set of raw sequence reads for the respective DNA fragment (Block 2204). Each respective raw sequence read in the set of raw sequence reads is aligned to the reference genome for the test subject, thus obtaining a corresponding set of aligned raw sequence reads for the respective DNA fragment (Block 2206). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (Block 2208).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Block 2210, the method includes using the plurality of aligned nucleotide sequences to determine, for each respective microsatellite locus in a plurality of predetermined microsatellite loci, a corresponding metric value for a lower mean score metric. Each respective microsatellite locus in the plurality of microsatellite loci has a nucleic acid sequence that includes a variable number of repeat units. The lower mean score metric is determined, at least in part, by the mean number of repeat units in the set of aligned nucleotide sequences encompassing the respective microsatellite locus, in the plurality of aligned nucleotide sequences, having a number of repeat units that is less than a predetermined number of repeat units (*e.g.,* a reference number of repeat units) representative of a stable construct at the respective microsatellite locus. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units. Assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

In some embodiments, referring to Block 2211, the method also includes determining one or more additional metrics of microsatellite stability for each of the microsatellite loci. For instance, in some embodiments, referring to Block 2212, the method further includes using the plurality of sequence reads to determine, for each respective microsatellite locus in the plurality of predetermined microsatellite loci, a corresponding metric value for a percentage score metric. The percentage score metric is determined, at least in part, by the percentage of aligned nucleotide sequences, in the plurality of aligned nucleotides, encompassing the respective microsatellite locus that have a number of repeat units that is less than a reference number of repeat units representative of a stable construct at the respective microsatellite locus. In some such embodiments, the one or more metrics used in the classifying a MSI status of the subject comprise the corresponding percentage score for the respective microsatellite locus. Assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

In some embodiments, referring to Block 2214, the method further includes using the first plurality of sequence reads to determine, for each respective microsatellite locus in the plurality of predetermined microsatellite loci, a corresponding metric value for a likelihood score metric. The likelihood score metric is determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units. In some such embodiments, the one or more metrics used in the classifying a MSI status of the subject comprise the corresponding likelihood score for the respective microsatellite locus.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirtyfifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

In some embodiments, referring to Block 2216, the method further includes using the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. In some such embodiments, the one or more metrics used in the classifying a MSI status of the subject comprise the corresponding standard deviation for the respective microsatellite locus. For instance, the set of nucleotide sequences shown in Figure 18 would have a standard deviation of 1.92.

In some embodiments, two or more metrics are used. For instance, in some embodiment, the two or more metrics include a percentage score and a lower mean score. In some embodiments, the two more or more metrics include a percentage score and a likelihood score. In some embodiments, the two of more metrics include a percentage score and a standard deviation. In some embodiments, the two or more metrics includes a lower mean score and a likelihood score. In some embodiments, the two or more metrics includes a lower mean score and a standard deviation. In some embodiments, the two or more metrics includes a likelihood score and a standard deviation.

In some embodiments, three or more metrics are used. For instance, in some embodiments, the three or more metrics include a percentage score, a lower mean score, and a likelihood score. In some embodiments, the three or more metrics include a percentage score, a lower mean score, and a standard deviation. In some embodiments, the three or more metrics include a percentage score, a likelihood score, and a standard deviation. In some embodiments, the three or more metrics include a lower mean score, a likelihood score, and a standard deviation. In some embodiments, the three or more metrics includes at least the percentage score, the lower mean score, the likelihood score, and standard deviation, as described herein.

Referring to Block 2118, in some embodiments, the method further includes normalizing the one or more metrics across the plurality of microsatellite loci. For example, in some embodiments, a power transform function is used to normalize the one or more metrics.

Referring to Block 2220, the method further includes classifying the MSI status of the test subject by inputting, for each respective microsatellite locus in the plurality of predetermined microsatellite loci, a corresponding metric value for each of one or more metrics, including the corresponding lower mean score, to a classifier that distinguishes between stable and unstable microsatellite loci, thus determining the MSI status of the test subject.

In some embodiments, the classifier is trained against metric values determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci.

The disclosure provides a method 2300 of determining a microsatellite instability (MSI) status of a subject using a likelihood score metric, as described herein. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2302, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 2304-2308, in some embodiments, the obtaining includes, for each respective DNA fragment in the plurality of DNA fragments, obtaining a corresponding set of raw sequence reads for the respective DNA fragment (2304). Each respective raw sequence read in the set of raw sequence reads is aligned to the reference genome for the test subject, thus obtaining a corresponding set of aligned raw sequence reads for the respective DNA fragment (2306). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (2308).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Block 2310, the method includes using the plurality of aligned nucleotide sequences to determine, for each respective microsatellite locus in a plurality of predetermined microsatellite loci, a corresponding metric value for a likelihood score metric. Each respective microsatellite locus in the plurality of microsatellite loci has a nucleic acid sequence that includes a variable number of repeat units. The likelihood score metric is determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirtyfifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

In some embodiments, referring to Block 2311, the method also includes determining one or more additional metrics of microsatellite stability for each of the microsatellite loci. For instance, in some embodiments, referring to Block 2312, the method further includes using the plurality of sequence reads to determine, for each respective microsatellite locus in the plurality of predetermined microsatellite loci, a corresponding metric value for a percentage score metric. The percentage score metric is determined, at least in part, by the percentage of aligned nucleotide sequences, in the plurality of aligned nucleotides, encompassing the respective microsatellite locus that have a number of repeat units that is less than a reference number of repeat units representative of a stable construct at the respective microsatellite locus. In some such embodiments, the one or more metrics used in the classifying a MSI status of the subject comprise the corresponding percentage score for the respective microsatellite locus. Assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

Referring to Block 2314, in some embodiments, the method further includes using the plurality of sequence reads to determine, for each respective microsatellite locus in the plurality of predetermined microsatellite loci, a corresponding metric value for a lower mean score metric. The lower mean score metric is determined, at least in part, by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. In some such embodiments, the one or more metrics used in the classifying a MSI status of the subject comprise the corresponding lower mean score for the respective microsatellite locus. Assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

In some embodiments, referring to Block 2316, the method further includes using the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. In some such embodiments, the one or more metrics used in the classifying a MSI status of the subject comprise the corresponding standard deviation for the respective microsatellite locus. For instance, the set of nucleotide sequences shown in Figure 18 would have a standard deviation of 1.92.

In some embodiments, two or more metrics are used. For instance, in some embodiment, the two or more metrics include a percentage score and a lower mean score. In some embodiments, the two more or more metrics include a percentage score and a likelihood score. In some embodiments, the two of more metrics include a percentage score and a standard deviation. In some embodiments, the two or more metrics includes a lower mean score and a likelihood score. In some embodiments, the two or more metrics includes a lower mean score and a standard deviation. In some embodiments, the two or more metrics includes a likelihood score and a standard deviation.

In some embodiments, three or more metrics are used. For instance, in some embodiments, the three or more metrics include a percentage score, a lower mean score, and a likelihood score. In some embodiments, the three or more metrics include a percentage score, a lower mean score, and a standard deviation. In some embodiments, the three or more metrics include a percentage score, a likelihood score, and a standard deviation. In some embodiments, the three or more metrics include a lower mean score, a likelihood score, and a standard deviation. In some embodiments, the three or more metrics includes at least the percentage score, the lower mean score, the likelihood score, and standard deviation, as described herein.

Referring to Block 2118, in some embodiments, the method further includes normalizing the one or more metrics across the plurality of microsatellite loci. For example, in some embodiments, a power transform function is used to normalize the one or more metrics.

Referring to Block 2220, the method further includes classifying the MSI status of the test subject by inputting, for each respective microsatellite locus in the plurality of predetermined microsatellite loci, a corresponding metric value for each of one or more metrics, including the corresponding lower mean score, to a classifier that distinguishes between stable and unstable microsatellite loci, thus determining the MSI status of the test subject.

In some embodiments, the classifier is trained against metric values determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci. The disclosure provides a method 2400 of determining a microsatellite instability (MSI) status of a subject using an internal measure of central tendency (e.g., a mode) of the number of repeat units at a microsatellite locus as a subject-specific reference value representative of a stable locus when determining one or more microsatellite stability metric for the subject from a liquid biopsy sample. Advantageously, a more accurate assessment of microsatellite stability can be made by considering what the individual's baseline stable construct is, rather than using the same predetermined reference value for all subjects. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2402, the method includes obtaining, for each respective DNA fragment in a plurality of DNA fragments from a liquid biopsy sample from the test subject, a corresponding nucleotide sequence that has been aligned to a reference genome for the species of the test subject, thereby obtaining a plurality of aligned nucleotide sequences.

For example, referring to Blocks 2404-2408, in some embodiments, the obtaining includes, for each respective DNA fragment in the plurality of DNA fragments, obtaining a corresponding set of raw sequence reads for the respective DNA fragment (2404). Each respective raw sequence read in the set of raw sequence reads is aligned to the reference genome for the test subject, thus obtaining a corresponding set of aligned raw sequence reads for the respective DNA fragment (2406). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (2408).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Block 2410, the method includes using the plurality of aligned nucleotide sequences to determine, for each respective microsatellite locus in a plurality of predetermined microsatellite loci, a corresponding mode of the number of repeat units at the respective microsatellite locus in aligned nucleotide sequences encompassing the respective microsatellite locus. Each respective microsatellite locus in the plurality of microsatellite loci has a nucleic acid sequence that includes a variable number of repeat units. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units.

Referring to Block 2412, the method further includes using the plurality of aligned nucleotide sequences to determine, for each respective microsatellite locus in the plurality of predetermined microsatellite loci, a corresponding metric value for each of one or more metrics. Each respective metric in the one or more metrics is determined, at least in part, by a distribution of the number of repeat units in each aligned nucleotide sequence, in the plurality of aligned nucleotide sequences, encompassing the respective microsatellite locus. At least one of the one or more metrics is a comparison of a distribution of the number of repeat units, in each aligned nucleotide sequence encompassing the respective microsatellite locus, to a corresponding measure of central tendency (e.g., a mode) of the number of repeat units at the respective microsatellite locus. For instance, the set of aligned nucleotide sequences shown in Figure 18 has a mode of 11. Thus, one or more metric for the particular microsatellite locus would be a comparison of the distribution of the number of repeated units in the sequence reads to this mode.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

In some embodiments, the one or more metrics comprises a percentage score. The corresponding metric value for the percentage score is the percentage of aligned nucleotide sequences, in the plurality of aligned nucleotides, encompassing the respective microsatellite locus that have a number of repeat units that is less than the reference number of repeat units representative of a stable construct at the respective microsatellite locus (e.g., the internal measure of central tendency, e.g., mode, for the set of nucleotide sequences encompassing the microsatellite locus derived from the liquid biopsy sample). For instance, assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, the one or more metrics comprises a lower mean score. The corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus (e.g., the internal measure of central tendency, e.g., mode, for the set of nucleotide sequences encompassing the microsatellite locus derived from the liquid biopsy sample). For instance, assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, the one or more metrics comprises a likelihood score. The corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirty-fifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

In some embodiments, the one or more corresponding metric values comprises a corresponding standard deviation of the number of repeat units in the aligned nucleotide sequences, in the plurality of aligned nucleotide sequences, encompassing the respective microsatellite locus. For instance, the set of nucleotide sequences shown in Figure 18 would have a standard deviation of 1.92.

In some embodiments, the one or more metrics includes at least two of the metrics described herein. For instance, in some embodiments, the one or more metrics include a percentage score and a lower mean score. In some embodiments, the one more or more metrics include a percentage score and a likelihood score. In some embodiments, the one of more metrics include a percentage score and a standard deviation. In some embodiments, the one or more metrics includes a lower mean score and a likelihood score. In some embodiments, the one or more metrics includes a lower mean score and a standard deviation. In some embodiments, the one or more metrics includes a likelihood score and a standard deviation.

In some embodiments, the one or more metrics includes at least three of the metrics described herein. For instance, in some embodiments, the one or more metrics include a percentage score, a lower mean score, and a likelihood score. In some embodiments, the one or more metrics include a percentage score, a lower mean score, and a standard deviation. In some embodiments, the one or more metrics include a percentage score, a likelihood score, and a standard deviation. In some embodiments, the one or more metrics include a lower mean score, a likelihood score, and a standard deviation. In some embodiments, the one or more metrics includes at least the percentage score, the lower mean score, the likelihood score, and standard deviation, as described herein.

Referring to Block 2414, in some embodiments, the method further includes normalizing the one or more metrics across the plurality of microsatellite loci. For example, in some embodiments, a power transform function is used to normalize the one or more metrics.

Referring to Block 2416, the method further includes classifying the MSI status of the test subject by inputting, for each respective microsatellite locus in the plurality of predetermined microsatellite loci, a corresponding metric value for each of one or more metrics to a classifier that distinguishes between stable and unstable microsatellite loci, thus determining the MSI status of the test subject.

In some embodiments, the classifier is trained against metric values determined across nucleotide sequences obtained from biological samples from a training cohort that includes samples from cells with stable microsatellites, thereby determining the MSI status of the test subject. For example, in some embodiments, the biological samples from the training cohort include solid tumor samples from training subjects with stable microsatellite cancer. In some embodiments, the biological samples from the training cohort include normal (e.g., non-cancerous) tissue samples. In some embodiments, the biological samples from the training cohort include both solid tumor samples from training subjects with stable microsatellite cancer and normal tissue samples.

In some embodiments, the biological samples from the training cohort also include solid tumor samples from training subjects with unstable microsatellite cancer. For example, in some embodiments, the biological samples from the training cohort include solid tumor samples from training subjects with stable microsatellite cancer (MSS) and solid tumor samples from training subjects with unstable microsatellite cancer (MSI). Similarly, in some embodiments, the biological samples from the training cohort include normal (e.g., non-cancerous) tissue samples and solid tumor samples from training subjects with unstable microsatellite cancer. In some embodiments, the biological samples from the training cohort include solid tumor samples from training subjects with stable microsatellite cancer, normal tissue samples, and solid tumor samples from training subjects with unstable microsatellite cancer (MSI).

In some embodiments, the classifier is a k-nearest neighbors algorithm. In other embodiments, the classifier is any one or more of the classifiers described herein above. In some embodiments, a separate classifier is used to determine the stability status for each microsatellite locus. For instance, in some embodiments, a different k-nearest neighbors algorithm is used to determine the individual status of each microsatellite locus, and the individual microsatellite statuses are used in aggregate to classify the microsatellite stability of the cancer in the subject.

For instance, in some embodiments, the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable. In some such embodiments, the threshold number of microsatellite loci is at least 30% of the total number of microsatellite loci in the plurality of microsatellite loci. In some such embodiments, the threshold number of microsatellite loci is at least 50% of the total number of microsatellite loci in the plurality of microsatellite loci. In other such embodiments, the threshold number of microsatellite loci is at least 25%, at least 30%, at least 35%, at least 40%, at least 45%, at least 50%, at least 55%, at least 60%, at least 65%, at least 70%, at least 75%, or at least 75% of the total number of microsatellite loci in the plurality of microsatellite loci.

The disclosure provides a method 2500 of determining a microsatellite instability (MSI) status of a subject from a liquid biopsy sample of the subject using a scoring vector that includes the highest N metric values for each of one or more microsatellite stability metrics, as described herein. In some embodiments, the method is performed at a computer system having one or more processors, and memory storing one or more programs for execution by the one or more processors, such as system 100 illustrated in Figure 1 or system 1100 as illustrated in Figure 11. In some embodiments, the subject is a human.

Referring to Block 2502, the method includes obtaining, for each respective DNA fragment in a plurality of DNA fragments from a liquid biopsy sample from the test subject, a corresponding nucleotide sequence that has been aligned to a reference genome for the species of the test subject, thus obtaining a plurality of aligned nucleotide sequences.

For example, referring to Blocks 2504-2508, in some embodiments, the obtaining includes, for each respective DNA fragment in the plurality of DNA fragments, obtaining a corresponding set of raw sequence reads for the respective DNA fragment (Block 2504). Each respective raw sequence read in the set of raw sequence reads is aligned to the reference genome for the test subject, thus obtaining a corresponding set of aligned raw sequence reads for the respective DNA fragment (Block 2506). The corresponding set of aligned raw sequence reads for the respective DNA fragment is collapsed into a corresponding nucleotide sequence for the respective DNA fragment that has been aligned to the human reference genome, thus obtaining a plurality of aligned nucleotide sequences (Block 2508).

In some embodiments, a single, unique aligned nucleotide sequence for each DNA fragment is used for the methods described herein. In some embodiments, all of the aligned nucleotide sequences obtained from each DNA fragment are used for the methods described herein. That is, in some embodiments, a plurality of aligned nucleotide sequences refers to the set of collapsed aligned nucleotide sequences and therefore includes only a single nucleotide sequence for each unique DNA fragment sequenced. In other embodiments, a plurality of aligned nucleotide sequences refers to the set of raw sequence reads that have been aligned to the reference construct (e.g., a reference human genome) and therefore generally includes multiple nucleotide sequences for each unique DNA fragment sequenced. While using the set of only unique aligned nucleotide sequences or using the set of all raw aligned nucleotide sequences may generate slightly different values for the microsatellite stability metrics described herein, the two methodologies will generally provide the same classification outcome, with similar specificity and sensitivity.

In some embodiments, as described herein above, the method includes sequencing nucleic acid fragments from the liquid biopsy sample. In other embodiments, as described herein above, the method begins by electronically receiving a dataset containing nucleotide sequences that have already been aligned to a reference genome.

In some embodiments, the plurality of aligned nucleotide sequences includes at least 250,000 aligned nucleotide sequences. In some embodiments, the plurality of aligned nucleotide sequences includes at least 10,000, at least 25,000, at least 50,000, at least 100,000, at least 200,000, at least 300,000, at least 400,000, at least 500,000, at least 750,000, or at least 1,000,000 aligned sequence reads. In some embodiments, the plurality of aligned nucleotide sequences includes an average of at least 1000 aligned nucleotide sequences per microsatellite locus. In some embodiments, the plurality of aligned nucleotide sequences includes an average of at least 2500, at least 5000, at least 10,000, at least 15,000, at least 20,000, or at least 25,000 aligned nucleotide sequences per microsatellite locus.

Referring to Blocks 2512-2520, method 2500 includes using the plurality of aligned nucleotide sequences to determine, for each respective microsatellite locus in a plurality of predetermined microsatellite loci a corresponding metric value for each of one or more metrics. Each respective microsatellite locus in the plurality of microsatellite loci has a nucleic acid sequence that includes a variable number of repeat units. Each respective metric in the one or more metrics is determined, at least in part, by a distribution of the number of repeat units in each aligned nucleotide sequence, in the plurality of aligned nucleotide sequences, encompassing the respective microsatellite locus. For instance, SEQ ID NOs: 1-4, as shown in Figure 18, represent a set of four aligned nucleotide sequences for a microsatellite locus having a repeating unit of a single adenine ('A') with a distribution of 9, 10, 12, and 14 repeated units.

In some embodiments, the plurality of predetermined microsatellite loci is at least 10 microsatellite loci. In some embodiments, the plurality of predetermined microsatellite loci is at least 20 microsatellite loci. In some embodiments, the plurality of predetermined microsatellite loci is at least 30 microsatellite loci. In other embodiments, the plurality of predetermined microsatellite loci is at least 5, at least 10, at least 15, at least 20, at least 25, at least 30, at least 35, at least 40, at least 45, at least 50, at least 75, or at least 100 microsatellite loci. In some embodiments, the plurality of predetermined microsatellite loci includes microsatellite loci from at least 10 of the genes listed in Table 2. In some embodiments, the plurality of predetermined microsatellite loci includes microsatellite loci from at least 20 of the genes listed in Table 2. In some embodiments, the plurality of predetermined microsatellite loci includes microsatellite loci from at least 30 of the genes listed in Table 2. In some embodiments, the plurality of predetermined microsatellite loci includes microsatellite loci from all of the genes listed in Table 2. In other embodiments, the plurality of predefined microsatellite loci includes microsatellite loci from at least 5, at least 10, at least 15, at least 20, at least 25, at least 30, or at least 35 of the genes listed in Table 2.

Referring to Block 2514, in some embodiments, the one or more metrics comprises a percentage score. The corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a percentage score of 0.5 (or 50%), because 2 of 4 of the nucleotides sequences have a fewer number of the repeated unit ("A") than the reference number.

In some embodiments, for each respective microsatellite locus in the plurality of predetermined microsatellite loci, the reference number of repeat units representative of a stable construct for the percentage score is a number of repeat units present in a reference genome for the species of the subject. In some embodiments, for each respective microsatellite locus in the plurality of predetermined microsatellite loci, the reference number of repeat units representative of a stable construct for the percentage score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the percentage score is the mode of the number of repeat units at the respective microsatellite locus in aligned nucleotide sequences, in the plurality of aligned nucleotide sequences, encompassing the respective microsatellite locus.

Referring to Block 2516, in some embodiments, the one or more metrics comprises a lower mean score. The corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. For instance, assuming a reference number of 11 repeat units, the set of nucleotide sequences shown in Figure 18 would have a lower mean score of 9.5 (the mean of 9 and 10), in some embodiments. In some embodiments, a measure of central tendency other than the mean (e.g., a mode) is used to generate the lower mean score.

In some embodiments, the mean number of repeat units is normalized by the reference number of repeat units representative of a stable construct at the respective microsatellite locus. In some embodiments, the normalization includes subtracting the lower mean number of repeat units (or other measure of central tendency of the number of repeat units in the subset of sequence reads) from the reference number (e.g., the mode of the full distribution of repeat units from the test data) and then dividing the difference by the lower mean. This normalizes for the value of the mode, which is variable across different loci. For instance, assuming a reference number of 11 repeat units, the set of nucleotide sequences shown in Figure 18 would have a normalized lower mean score of 0.136 (the mean of 9 and 10 (9.5) subtracted from the reference number (11), and then divided by the reference number (11) = 0.136), in some embodiments. Of course, other methods for normalizing and/or standardizing data are known in the art and could be used in conjunction with the methods described herein.

In some embodiments, for each respective microsatellite locus in the plurality of predetermined microsatellite loci, the reference number of repeat units representative of a stable construct for the lower mean score is a number of repeat units present in a reference genome for the species of the subject. In some embodiments, for each respective microsatellite locus in the plurality of predetermined microsatellite loci, the reference number of repeat units representative of a stable construct for the lower mean score is a measure of central tendency (e.g., an arithmetic mean, weighted mean, midrange, midhinge, trimean, geometric mean, geometric median, Winsorized mean, median, or mode of the distribution of values) of a plurality of reference values, wherein each respective reference value in the plurality of reference values is determined at least in part based on a distribution of the number of repeat units at the respective microsatellite locus in a respective reference sample in a plurality of reference standard. In some embodiments, for each respective microsatellite locus in the plurality of predetermined microsatellite loci, the reference number of repeat units representative of a stable construct for the lower mean score is the mode of the number of repeat units at the respective microsatellite locus in aligned nucleotide sequences, in the plurality of aligned nucleotide sequences, encompassing the respective microsatellite locus.

Referring to Block 2518, in some embodiments, the one or more metrics comprises a likelihood score. The corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units.

In some embodiments, the threshold percentile of the smallest number of repeat units falls between the first percentile and the seventy-fifth percentile of the smallest number of repeat units. In some embodiments, the threshold percentile of the smallest number of repeat units falls between the first percentile and the fiftieth percentile of the smallest number of repeat units. In some embodiments, the threshold percentile of the smallest number of repeat units falls between the fifth percentile and the fiftieth percentile of the smallest number of repeat units. In some embodiments, the threshold percentile of the smallest number of repeat units falls between the fifth percentile and the twenty-fifth percentile of the smallest number of repeat units. In some embodiments, the threshold percentile of the smallest number of repeat units is the tenth percentile. In other embodiment, the threshold percentile of the smallest number of repeat units is the first, second, third, fourth, fifth, tenth, fifteenth, twentieth, twenty-fifth, thirtieth, thirty-fifth, fortieth, forty-fifth, fiftieth, fifty-fifth, sixtieth, sixty-fifth, seventieth, seventy-fifth, eightieth, eighty-fifth percentile, or the like.

In some embodiments, the probability that a respective aligned nucleotide sequence is a member of a distribution encompassing a stable construct is determined using a density model of the distribution of the number of repeat units in a set of reference samples. For instance, in some embodiments, a density model is formed for the distribution of the number of repeat units at a particular microsatellite locus in one or more reference biological samples having stable microsatellite loci, e.g., one or more normal tissue samples and/or one or more solid tumor samples from microsatellite stable cancer (MSS). In some embodiments, the density estimate is a kernel density estimate (KDE). However, other methodologies for forming a density estimate are known in the field, and may be used in conjunction with the methods described herein.

In some embodiments, e.g., where the mode of a distribution of repeat units representative of a stable microsatellite locus varies within a population, the density model for the stable distribution is formed using reference samples having the same mode for the distribution of repeat units at the microsatellite locus as the mode of the distribution of repeat units at the microsatellite locus in the test sample. An example of forming mode-specific density estimates for a particular microsatellite locus is shown in Figure 28A. As illustrated, a plurality of reference samples having stable microsatellite loci are used, e.g., reference samples 1 through 5. For a particular microsatellite locus being evaluated, the mode (or other measure of central tendency) of the distribution of repeat unit lengths at the particular microsatellite locus is determined, e.g., 16, 17, or 18. Mode-specific density estimates are then formed from the pooled distributions of the repeat unit lengths from each reference sample having a particular mode. For instance, the repeat unit length distribution of reference sample 4 is used to create a density estimate for a mode of 16 repeat units; the repeat unit length distribution of reference sample 2 is used to create a density estimate for a mode of 17 repeat units; and the repeat unit length distributions of reference samples 1, 3, and 5 are used to create a density estimate for a mode of 18 repeat units. This process is repeated for each microsatellite locus, such that the density estimate for a first particular mode and microsatellite locus may be formed using a different subset of stable reference samples than the density estimate for a second particular mode and microsatellite locus.

In some embodiments, the statistical measure is a measure of central tendency (e.g., a mean) of the distribution of probabilities that each aligned nucleotide sequence in the subset of aligned nucleotide sequences encompassing the respective microsatellite locus is a member of the distribution of aligned nucleotide sequences encompassing a stable construct at the respective microsatellite locus. In some embodiments, the distribution of probabilities is a plurality of p-values, wherein each respective p-value in the plurality of p-values is a probability value that a respective aligned nucleotide sequence in the subset of aligned nucleotide sequences is a member of the distribution of aligned nucleotide sequences encompassing a stable construct at the respective microsatellite locus.

Referring to Block 2520, in some embodiments, the one or more corresponding metric values comprises a corresponding standard deviation of the number of repeat units in the aligned nucleotide sequences, in the plurality of aligned nucleotide sequences, encompassing the respective microsatellite locus. For instance, the set of nucleotide sequences shown in Figure 18 would have a standard deviation of 1.92.

In some embodiments, the one or more metrics includes at least two of the metrics described herein. For instance, in some embodiments, the one or more metrics include a percentage score and a lower mean score. In some embodiments, the one more or more metrics include a percentage score and a likelihood score. In some embodiments, the one of more metrics include a percentage score and a standard deviation. In some embodiments, the one or more metrics includes a lower mean score and a likelihood score. In some embodiments, the one or more metrics includes a lower mean score and a standard deviation. In some embodiments, the one or more metrics includes a likelihood score and a standard deviation.

In some embodiments, the one or more metrics includes at least three of the metrics described herein. For instance, in some embodiments, the one or more metrics include a percentage score, a lower mean score, and a likelihood score. In some embodiments, the one or more metrics include a percentage score, a lower mean score, and a standard deviation. In some embodiments, the one or more metrics include a percentage score, a likelihood score, and a standard deviation. In some embodiments, the one or more metrics include a lower mean score, a likelihood score, and a standard deviation. In some embodiments, the one or more metrics includes at least the percentage score, the lower mean score, the likelihood score, and standard deviation, as described herein.

Referring to Block 2522, in some embodiments the method further includes normalizing one or more of the metrics across the plurality of microsatellite loci. For example, in some embodiments, a power transform function is used to normalize the one or more metrics.

Referring to Block 2524, method 2500 includes generating one or more MSI scoring vectors for the test subject, each respective MSI scoring vector in the one or more MSI scoring vector comprising, for a respective metric in the one or more metrics, the highest N metric values. In some embodiments, the highest N metric values is no more than the highest 50% of metric values. For example, for an assay that determines a metric value for each of 39 microsatellite loci, the highest N metric values would be no more than the highest 19 metric values. In some embodiments, the highest $N$ metric values is no more than the highest 45% of metric values. In some embodiments, the highest $N$ metric values is no more than the highest 40% of metric values. In some embodiments, the highest $N$ metric values is no more than the highest 35% of metric values. In other embodiments, the highest $N$ metric values is no more than the highest 30% of metric values, no more than the highest 25% of metric values, no more than the highest 20% of metric values, no more than the highest 15% of metric values, or no more than the highest 10% of metric values.

Referring to Block 2526, method 2500 includes determining, for each respective MSI scoring vectors in the one or more MSI scoring vectors, a difference between the respective MSI scoring vector and a reference MSI vector, wherein the reference MSI vector comprises corresponding metric values derived from one or more biological samples from one or more reference subjects with stable microsatellite loci. In some embodiments, the difference is a geometric distance. In some embodiments, the geometric distance is a Wasserstein distance between two distributions.

Referring to Block 2528, method 2500 includes classifying the MSI status of the subject by inputting, for each respective MSI scoring vector in the one or more MSI scoring vectors, the difference between the respective MSI scoring vector and the reference MSI vector to a classifier that distinguishes between stable and unstable microsatellite loci, thereby determining the MSI status of the subject. In some embodiments, the classifier is a k-nearest neighbors algorithm. In some embodiments, the classifier is a logistic regression algorithm. In other embodiments, the classifier is any one or more of the classifiers described herein above.

In some embodiments, the methods described herein include generating a clinical report 139-3 *(e.g.,* a patient report), providing clinical support for personalized cancer therapy, and/or using the information curated from sequencing of a liquid biopsy sample, as described above. In some embodiments, the report is provided to a patient, physician, medical personnel, or researcher in a digital copy (for example, a JSON object, a pdf file, or an image on a website or portal), a hard copy (for example, printed on paper or another tangible medium). A report object, such as a JSON object, can be used for further processing and/or display. For example, information from the report object can be used to prepare a clinical laboratory report for return to an ordering physician. In some embodiments, the report is presented as text, as audio (for example, recorded or streaming), as images, or in another format and/or any combination thereof.

The report includes information related to the specific characteristics of the patient's cancer, e.g., detected genetic variants, epigenetic abnormalities, associated oncogenic pathogenic infections, and/or pathology abnormalities. In some embodiments, other characteristics of a patient's sample and/or clinical records are also included in the report. For example, in some embodiments, the clinical report includes information on clinical variants, e.g., one or more of copy number variants (*e.g.*, for actionable genes CCNE1, CD274(PD-L1), EGFR, ERBB2(HER2), MET, MYC, BRCA1, and/or BRCA2), fusions, translocations, and/or rearrangements (*e.g.*, in actionable genes ALK, ROS1, RET, NTRK1, FGFR2, FGFR3, NTRK2 and/or NTRK3), pathogenic single nucleotide polymorphisms, insertion-deletions (*e.g.,* somatic/tumor and/or germline/normal), therapy biomarkers, microsatellite instability status, and/or tumor mutational burden.

In some embodiments, the results of the liquid biopsy assays used to classify the microsatellite stability of a subject's cancer, are used to further classify a cancer status of the subject. For instance, in some embodiments, additional types of information derived from the same biological sample, a different biological sample for the individual, and/or a personal survey of the subject, are combined with the classification results to provide diagnosis, prognosis, or treatment recommendations for the subject. These additional types of information can include one or more of genomic information (*e.g.*, sequencing information such as germline or cancer variant allele identification, copy number variation, chromosomal aberration data, etc.), exome information (*e.g.,* gene expression data), epigenetic information (*e.g.,* methylation data, and histone modification data), proteomic information (*e.g.*, protein expression data), metabolome information (*e.g.,* data on the metabolism of the subject), and personal characteristics (*e.g.*, age, weight, smoking status, familial disease history, *etc.*). For instance, as shown in Figure 2A, different portions of the liquid biopsy sample, or different biological samples such as solid tumor biopsies, may be analyzed at different diagnostic environments, *e.g.,* a clinical environment 220, a sequencing lab 230, a pathology lab 240, or a molecular biology lab 250, and the information analyzed at a remove processing/storage center 260.

Methods for classifying the cancer status of an individual are known in the art. For instance, U.S. Provisional Application Serial No. 62/855,750, filed May 31, 2019, describes various methods for combining different types of data about a subject in order to classify the cancer status of the subject. In some embodiments, the methods for classifying the microsatellite stability of a cancer described herein are combined with any of the methods for classifying the cancer status of a subject, as described in USSN 62/855,750.

In some embodiments, the methods for classifying the microsatellite stability of a cancer described herein are integrated with a test to determine whether the subject has a type of cancer. In some embodiments, the test determines whether the subject has a type of cancer selected from one or more of breast cancer, lung cancer, prostate cancer, colorectal cancer, renal cancer, uterine cancer, pancreatic cancer, esophagus cancer, head/neck cancer, ovarian cancer, hepatobiliary cancer, cervical cancer, thyroid cancer, or bladder cancer. In some embodiments, the test determines a likelihood that the subject has a particular type of cancer, *e.g.,* a likelihood that the subject has breast cancer, lung cancer, prostate cancer, colorectal cancer, renal cancer, uterine cancer, pancreatic cancer, esophagus cancer, head/neck cancer, ovarian cancer, hepatobiliary cancer, cervical cancer, thyroid cancer, or bladder cancer.

In some embodiments, the methods for classifying the microsatellite stability of a cancer described herein are integrated with a test to classify a stage of a cancer in the subject, e.g., whether the subject's cancer is stage I, stage II, stage III, or stage IV cancer. In some embodiments, the test determines the stage of a breast cancer, lung cancer, prostate cancer, colorectal cancer, renal cancer, uterine cancer, pancreatic cancer, esophagus cancer, head/neck cancer, ovarian cancer, hepatobiliary cancer, cervical cancer, thyroid cancer, or bladder cancer.

In some embodiments, the methods for classifying the microsatellite stability of a cancer described herein are integrated with a test to classify a prognosis for a cancer in a subject, e.g., a survival rate without treatment, a survival rate with treatment, a disease-free survival rate, a cancer recursion rate, etc. In some embodiments, the prognosis is a 1-year, 2-year, 3-year, 4-year, 5-year, or 10-year prognosis, e.g., a ten year disease-free survival rate.

In some embodiments, the methods for classifying the microsatellite stability of a cancer described herein are integrated with a test to determine a recommended treatment for a cancer in a subject. In some embodiments, the recommended treatment is dependent upon whether or not the subject has an MSS or MSI-H cancer.

In some embodiments, the methods described herein further includes assigning therapy and/or administering therapy to the subject based on the classification of the microsatellite stability of the cancer, e.g., based on whether or not the subject's cancer is MSS or MSI-H. In some embodiments, when the subject's cancer is classified as MSI-H, the subject is assigned or administered an immunotherapy, e.g., checkpoint inhibitor immunotherapy, and when the subject's cancer is classified as MSI-H, the subject is not assigned or administered an immunotherapy.

Variant Characterization

In some embodiments, a predicted functional effect and/or clinical interpretation for one or more identified variants is curated by using information from variant databases. In some embodiments, a weighted-heuristic model is used to characterize each variant.

In some embodiments, identified clinical variants are labeled as "potentially actionable", "biologically relevant", "variants of unknown significance (VUSs)", or "benign". Potentially actionable alterations are protein-altering variants with an associated therapy based on evidence from the medical literature. Biologically relevant alterations are protein-altering variants that may have functional significance or have been observed in the medical literature but are not associated with a specific therapy. Variants of unknown significance (VUSs) are protein-altering variants exhibiting an unclear effect on function and/or without sufficient evidence to determine their pathogenicity. In some embodiments, benign variants are not reported. In some embodiments, variants are identified through aligning the patient's DNA sequence to the human genome reference sequence version hg19 (GRCh37). In some embodiments, actionable and biologically relevant somatic variants are provided in a clinical summary during report generation.

For instance, in some embodiments, variant classification and reporting is performed, where detected variants are investigated following criteria from known evolutionary models, functional data, clinical data, literature, and other research endeavors, including tumor organoid experiments. In some embodiments, variants are prioritized and classified based on known gene-disease relationships, hotspot regions within genes, internal and external somatic databases, primary literature, and other features of somatic drivers. Variants can be added to a patient (or sample, for example, organoid sample) report based on recommendations from the AMP/ASCO/CAP guidelines. Additional guidelines may be followed. Briefly, pathogenic variants with therapeutic, diagnostic, or prognostic significance may be prioritized in the report. Non-actionable pathogenic variants may be included as biologically relevant, followed by variants of uncertain significance. Translocations may be reported based on features of known gene fusions, relevant breakpoints, and biological relevance. Evidence may be curated from public and private databases or research and presented as 1) consensus guidelines 2) clinical research, or 3) case studies, with a link to the supporting literature. Germline alterations may be reported as secondary findings in a subset of genes for consenting patients. These may include genes recommended by the ACMG and additional genes associated with cancer predisposition or drug resistance.

In some embodiments, a clinical report 139-3 includes information about clinical trials for which the patient is eligible, therapies that are specific to the patient's cancer, and/or possible therapeutic adverse effects associated with the specific characteristics of the patient's cancer, *e.g.,* the patient's genetic variations, epigenetic abnormalities, associated oncogenic pathogenic infections, and/or pathology abnormalities, or other characteristics of the patient's sample and/or clinical records. For example, in some embodiments, the clinical report includes such patient information and analysis metrics, including cancer type and/or diagnosis, variant allele fraction, patient demographic and/or institution, matched therapies (e.g., FDA approved and/or investigational), matched clinical trials, variants of unknown significance (VUS), genes with low coverage, panel information, specimen information, details on reported variants, patient clinical history, status and/or availability of previous test results, and/or version of bioinformatics pipeline.

In some embodiments, the results included in the report, and/or any additional results (for example, from the bioinformatics pipeline), are used to query a database of clinical data, for example, to determine whether there is a trend showing that a particular therapy was effective or ineffective in treating (e.g., slowing or halting cancer progression), and/or adverse effects of such treatments in other patients having the same or similar characteristics.

In some embodiments, the results are used to design cell-based studies of the patient's biology, e.g., tumor organoid experiments. For example, an organoid may be genetically engineered to have the same characteristics as the specimen and may be observed after exposure to a therapy to determine whether the therapy can reduce the growth rate of the organoid, and thus may be likely to reduce the growth rate of cancer in the the patient associated with the specimen. Similarly, in some embodiments, the results are used to direct studies on tumor organoids derived directly from the patient. An example of such experimentation is described in U.S. Provisional Patent Application No. 62/944,292, filed December 5, 2019.

As illustrated in Figure 2A, in some embodiments, a clinical report is checked for final validation, review, and sign-off by a medical practitioner (*e.g.*, a pathologist). The clinical report is then sent for action (*e.g.,* for precision oncology applications).

Digital and Laboratory Health Care Platform:

The methods and systems described herein may be utilized in combination with, or as part of, a digital and laboratory health care platform that is generally targeted to medical care and research. It should be understood that many uses of the methods and systems described above, in combination with such a platform, are possible. One example of such a platform is described in U.S. Patent Application No. 16/657,804, filed October 18, 2019.

For example, an implementation of the methods and systems as described above may include microservices constituting a digital and laboratory health care platform supporting analysis of liquid biopsy samples to provide clinical support for personalized cancer therapy. Examples may include a single microservice for executing and delivering analysis of liquid biopsy samples to clinical support for personalized cancer therapy or may include a plurality of microservices each having a particular role. In one example, a first microservice may execute sequence analysis in order to deliver genomic features to a second microservice for curating clinical support for personalized cancer therapy based on the identified features. Similarly, the second microservice may execute therapeutic analysis of the curated clinical support to deliver recommended therapeutic modalities.

Where examples above are executed in one or more micro-services with or as part of a digital and laboratory health care platform, one or more of such micro-services may be part of an order management system that orchestrates the sequence of events as needed at the appropriate time and in the appropriate order necessary to instantiate examples above. A microservices-based order management system is disclosed, for example, in U.S. Prov. Patent Application No. 62/873,693, filed July 12, 2019.

For example, continuing with the above first and second microservices, an order management system may notify the first microservice that an order for curating clinical support for personalized cancer therapy has been received and is ready for processing. The first microservice may execute and notify the order management system once the delivery of genomic features for the patient is ready for the second microservice. Furthermore, the order management system may identify that execution parameters (prerequisites) for the second microservice are satisfied, including that the first microservice has completed, and notify the second microservice that it may continue processing the order to curate clinical support for personalized cancer therapy.

Where the digital and laboratory health care platform further includes a genetic analyzer system, the genetic analyzer system may include targeted panels and/or sequencing probes. An example of a targeted panel is disclosed, for example, in U.S. Prov. Patent Application No. 62/902,950, filed September 19, 2019. In one example, targeted panels may enable the delivery of next generation sequencing results for providing clinical support for personalized cancer therapy. An example of the design of next-generation sequencing probes is disclosed, for example, in U.S. Prov. Patent Application No. 62/924,073, filed October 21, 2019.

Where the digital and laboratory health care platform further includes a bioinformatics pipeline, the methods and systems described above may be utilized after completion or substantial completion of the systems and methods utilized in the bioinformatics pipeline. As one example, the bioinformatics pipeline may receive next-generation genetic sequencing results and return a set of binary files, such as one or more BAM files, reflecting nucleic acid (*e.g.,* cfDNA, DNA and/or RNA) read counts aligned to a reference genome. The methods and systems described above may be utilized, for example, to ingest the cfDNA, DNA and/or RNA read counts and produce genomic features as a result.

When the digital and laboratory health care platform further includes an RNA data normalizer, any RNA read counts may be normalized before processing as described above. An example of an RNA data normalizer is disclosed, for example, in U.S. Patent Application No. 16/581,706, filed September 24, 2019.

When the digital and laboratory health care platform further includes a genetic data deconvoluter, any system and method for deconvoluting may be utilized for analyzing genetic data associated with a specimen having two or more biological components to determine the contribution of each component to the genetic data and/or determine what genetic data would be associated with any component of the specimen if it were purified. An example of a genetic data deconvoluter is disclosed, for example, in U.S. Patent Application No. 16/732,229 and PCT/US19/69161, filed December 31, 2019, U.S. Prov. Patent Application No. 62/924,054, filed October 21, 2019, and U.S. Prov. Patent Application No. 62/944,995, filed December 6, 2019.

When the digital and laboratory health care platform further includes an automated RNA expression caller, RNA expression levels may be adjusted to be expressed as a value relative to a reference expression level, which is often done in order to prepare multiple RNA expression data sets for analysis to avoid artifacts caused when the data sets have differences because they have not been generated by using the same methods, equipment, and/or reagents. An example of an automated RNA expression caller is disclosed, for example, in U.S. Prov. Patent Application No. 62/943,712, filed December 4, 2019.

The digital and laboratory health care platform may further include one or more insight engines to deliver information, characteristics, or determinations related to a disease state that may be based on genetic and/or clinical data associated with a patient and/or specimen. Exemplary insight engines may include a tumor of unknown origin engine, a human leukocyte antigen (HLA) loss of homozygosity (LOH) engine, a tumor mutational burden engine, a PD-L1 status engine, a homologous recombination deficiency engine, a cellular pathway activation report engine, an immune infiltration engine, a microsatellite instability engine, a pathogen infection status engine, and so forth. An example tumor of unknown origin engine is disclosed, for example, in U.S. Prov. Patent Application No. 62/855,750, filed May 31, 2019. An example of an HLA LOH engine is disclosed, for example, in U.S. Prov. Patent Application No. 62/889,510, filed August 20, 2019. An example of a tumor mutational burden (TMB) engine is disclosed, for example, in U.S. Prov. Patent Application No. 62/804,458, filed February 12, 2019. An example of a PD-L1 status engine is disclosed, for example, in U.S. Prov. Patent Application No. 62/854,400, filed May 30, 2019. An additional example of a PD-L1 status engine is disclosed, for example, in U.S. Prov. Patent Application No. 62/824,039, filed March 26, 2019. An example of a homologous recombination deficiency engine is disclosed, for example, in U.S. Prov. Patent Application No. 62/804,730, filed February 12, 2019. An example of a cellular pathway activation report engine is disclosed, for example, in U.S. Prov. Patent Application No. 62/888,163, filed August 16, 2019. An example of an immune infiltration engine is disclosed, for example, in U.S. Patent Application No. 16/533,676, filed August 6, 2019. An additional example of an immune infiltration engine is disclosed, for example, in U.S. Patent Application No. 62/804,509, filed February 12, 2019. An example of an MSI engine is disclosed, for example, in U.S. Patent Application No. 16/653,868, filed October 15, 2019. An additional example of an MSI engine is disclosed, for example, in U.S. Prov. Patent Application No. 62/931,600, filed November 6, 2019.

When the digital and laboratory health care platform further includes a report generation engine, the methods and systems described above may be utilized to create a summary report of a patient's genetic profile and the results of one or more insight engines for presentation to a physician. For instance, the report may provide to the physician information about the extent to which the specimen that was sequenced contained tumor or normal tissue from a first organ, a second organ, a third organ, and so forth. For example, the report may provide a genetic profile for each of the tissue types, tumors, or organs in the specimen. The genetic profile may represent genetic sequences present in the tissue type, tumor, or organ and may include variants, expression levels, information about gene products, or other information that could be derived from genetic analysis of a tissue, tumor, or organ. The report may include therapies and/or clinical trials matched based on a portion or all of the genetic profile or insight engine findings and summaries. For example, the therapies may be matched according to the systems and methods disclosed in U.S. Prov. Patent Application No. 62/804,724, filed February 12, 2019. For example, the clinical trials may be matched according to the systems and methods disclosed in U.S. Prov. Patent Application No. 62/855,913, filed May 31, 2019.

The report may include a comparison of the results to a database of results from many specimens. An example of methods and systems for comparing results to a database of results are disclosed in U.S. Prov. Patent Application No. 62/786,739, filed December 31, 2018. The information may be used, sometimes in conjunction with similar information from additional specimens and/or clinical response information, to discover biomarkers or design a clinical trial.

When the digital and laboratory health care platform further includes application of one or more of the embodiments herein to organoids developed in connection with the platform, the methods and systems may be used to further evaluate genetic sequencing data derived from an organoid to provide information about the extent to which the organoid that was sequenced contained a first cell type, a second cell type, a third cell type, and so forth. For example, the report may provide a genetic profile for each of the cell types in the specimen. The genetic profile may represent genetic sequences present in a given cell type and may include variants, expression levels, information about gene products, or other information that could be derived from genetic analysis of a cell. The report may include therapies matched based on a portion or all of the deconvoluted information. These therapies may be tested on the organoid, derivatives of that organoid, and/or similar organoids to determine an organoid's sensitivity to those therapies. For example, organoids may be cultured and tested according to the systems and methods disclosed in U.S. Patent Application No. 16/693,117, filed November 22, 2019; U.S. Prov. Patent Application No. 62/924,621, filed October 22, 2019; and U.S. Prov. Patent Application No. 62/944,292, filed December 5, 2019.

When the digital and laboratory health care platform further includes application of one or more of the above in combination with or as part of a medical device or a laboratory developed test that is generally targeted to medical care and research, such laboratory developed test or medical device results may be enhanced and personalized through the use of artificial intelligence. An example of laboratory developed tests, especially those that may be enhanced by artificial intelligence, is disclosed, for example, in U.S. Provisional Patent Application No. 62/924,515, filed October 22, 2019.

It should be understood that the examples given above are illustrative and do not limit the uses of the systems and methods described herein in combination with a digital and laboratory health care platform.

The results of the bioinformatics pipeline may be provided for report generation 208. Report generation may comprise variant science analysis, including the interpretation of variants (including somatic and germline variants as applicable) for pathogenic and biological significance. The variant science analysis may also estimate microsatellite instability (MSI) or tumor mutational burden. Targeted treatments may be identified based on gene, variant, and cancer type, for further consideration and review by the ordering physician. In some aspects, clinical trials may be identified for which the patient may be eligible, based on mutations, cancer type, and/or clinical history. Subsequent validation may occur, after which the report may be finalized for sign-out and delivery. A first or second report may include additional data provided through a clinical dataflow 202, such as patient progress notes, pathology reports, imaging reports, and other relevant documents. Such clinical data is ingested, reviewed, and abstracted based on a predefined set of curation rules. The clinical data is then populated into the patient's clinical history timeline for report generation.

Further details on clinical report generation are disclosed in US Patent Application No. 16/789,363 (PCT/US20/180002), filed February 12, 2020.

**Specific Embodiments of the Disclosure - not part of the invention**

In some aspects, the systems and methods disclosed herein may be used to support clinical decisions for personalized treatment of cancer. For example, the methods described herein identify actionable genomic variants and/or genomic states with associated recommended cancer therapies. The recommended treatment may be dependent upon whether or not the subject has a particular actionable variant and/or genomic status. Recommended treatment modalities can be therapeutic drugs and/or assignment to one or more clinical trials. Generally, current treatment guidelines for various cancers are maintained by various organizations, including the National Cancer Institute and Merck & Co., in the Merck Manual.

In some embodiments, the methods described herein further includes assigning therapy and/or administering therapy to the subject based on the identification of an actionable genomic variant and/or genomic state, e.g., based on whether or not the subject's cancer will be responsive to a particular personalized cancer therapy regimen. For example, when the subject's cancer is classified as having a first actionable variant and/or genomic state, the subject is assigned or administered a first personalized cancer therapy that is associated with the first actionable variant and/or genomic state, and when the subject's cancer is classified as having a second actionable variant and/or genomic state, the subject is assigned or administered a second personalized cancer therapy that is associated with the second actionable variant. Assignment or administration of a therapy or a clinical trial to a subject is thus tailored for treatment of the actionable variants and/or genomic states of the cancer patient.

**Examples**

***Example 1 - The Cancer Genome Atlas (TCGA).***

The Cancer Genome Atlas (TCGA) is a publicly available dataset comprising more than two petabytes of genomic data for over 11,000 cancer patients, including clinical information about the cancer patients, metadata about the samples (e.g., the weight of a sample portion, *etc.*) collected from such patients, histopathology slide images from sample portions, and molecular information derived from the samples (e.g., mRNA/miRNA expression, protein expression, copy number, *etc.*). The TCGA dataset includes data on 33 different cancers: breast (breast ductal carcinoma, bread lobular carcinoma) central nervous system (glioblastoma multiforme, lower grade glioma), endocrine (adrenocortical carcinoma, papillary thyroid carcinoma, paraganglioma & pheochromocytoma), gastrointestinal (cholangiocarcinoma, colorectal adenocarcinoma, esophageal cancer, liver hepatocellular carcinoma, pancreatic ductal adenocarcinoma, and stomach cancer), gynecologic (cervical cancer, ovarian serous cystadenocarcinoma, uterine carcinosarcoma, and uterine corpus endometrial carcinoma), head and neck (head and neck squamous cell carcinoma, uveal melanoma), hematologic (acute myeloid leukemia, Thymoma), skin (cutaneous melanoma), soft tissue (sarcoma), thoracic (lung adenocarcinoma, lung squamous cell carcinoma, and mesothelioma), and urologic (chromophobe renal cell carcinoma, clear cell kidney carcinoma, papillary kidney carcinoma, prostate adenocarcinoma, testicular germ cell cancer, and urothelial bladder carcinoma).

***Example 2 - Classification of microsatellite instability in a liquid biopsy sample.***

In order to classify a human cancer for precision oncology, a hybrid-capture next generation sequencing assay was developed to detect somatic alterations and microsatellite instability in a liquid biopsy sample. The hybrid-capture next generation sequencing assay uses a panel of hybridization probes to enrich for cfDNA in the liquid biopsy sample from a plurality of loci associated with actionable somatic alterations, as well as microsatellite loci. Specifically, the panel of hybridization probes includes probes to the 39 highly informative microsatellite loci listed in Table 2. The bioinformatics pipeline for MSI detection used in with the assay is illustrated in Figures 29A-29C.

The ability of the hybrid-capture next generation sequencing assay to detect MSI on a new independent validation dataset was validated. Briefly, for each microsatellite locus, all sequencing reads that mapped to the corresponding microsatellite region were identified and the number of repeat units contained within the sequencing read was quantified. Figures 29A and 29B show the feature calcluation for a single locus and example MSI status results for a single patient, as described in the next several paragraphs.

Next, three distinct summary statistics were calculated to characterize the distribution of the number of repeat units for each locus. The first summary statistic was a percentage score, calculated as the percentage of sequence reads encompassing the respective microsatellite locus in which the number of repeat units is less than a reference number of repeat units representative of a stable reference construct at the respective microsatellite locus.

The second summary statistic was a lower mean normalized score, calculated as the mean number of repeat units in sequence reads encompassing the respective microsatellite locus in which the number of repeat units is less than a reference number of repeat units representative of a stable construct at the respective microsatellite locus, normalized by the number of repeat units most commonly occurring in the distribution (i.e., the mode), as show in Equation I:

$$\frac{mean_{lower} - mode}{mode}, \qquad \text{(Equation I)}$$

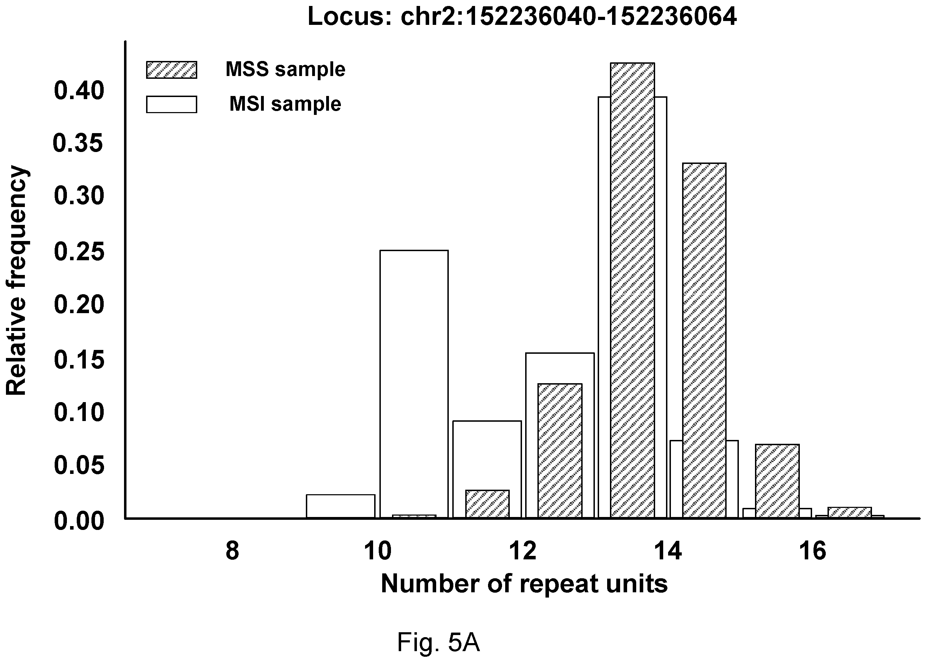

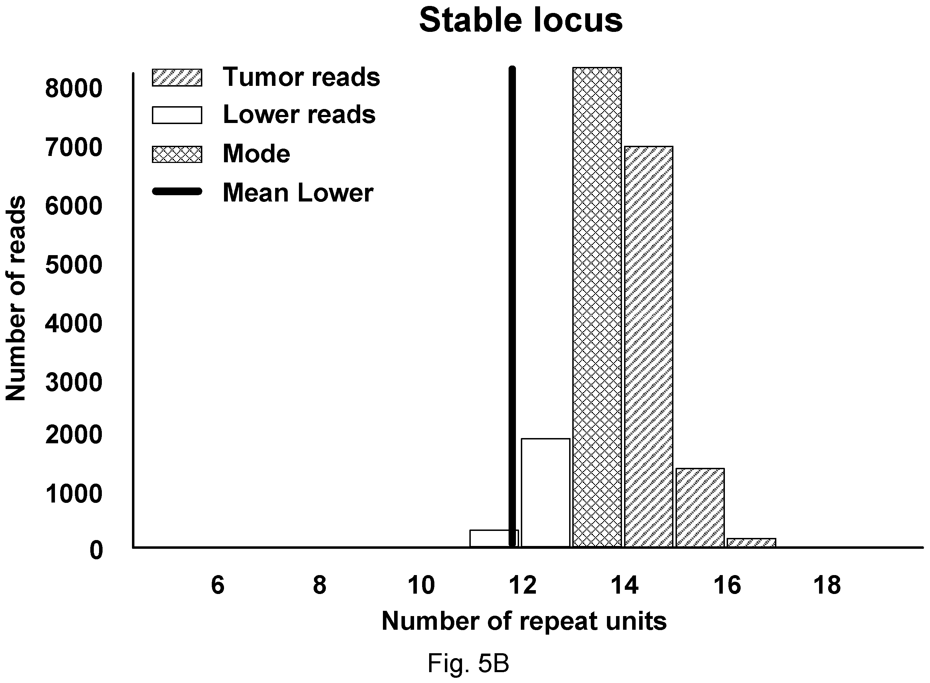

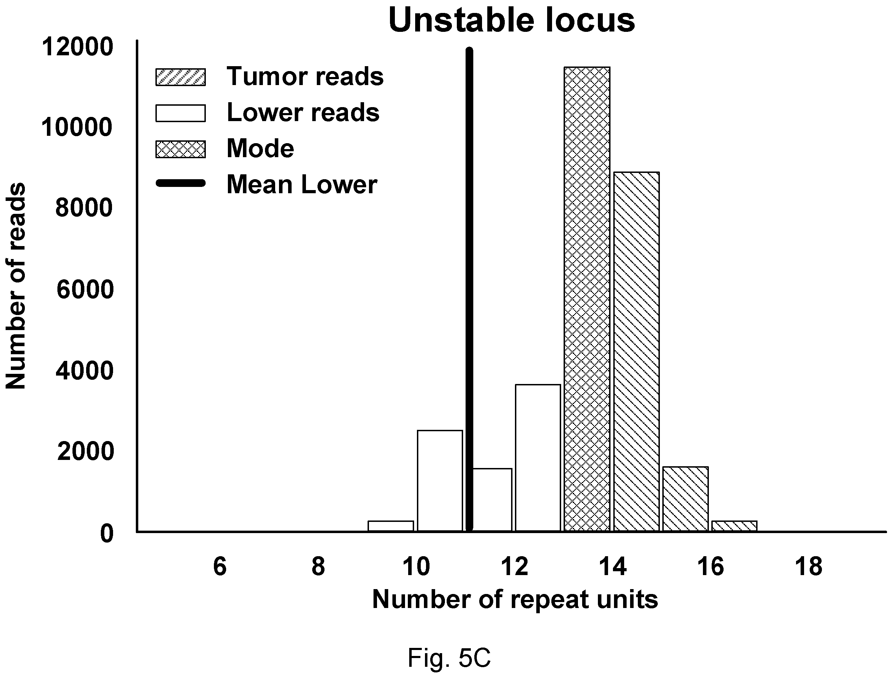

where mode is the most frequently occurring number of repeat units in the distribution of the given sample and mean_lower is the mean number of repeat units in the reads with fewer repeat units than the mode. For instance, calculation of a lower mean normalized score for two different distributions in shown in Figure 5 for an MSS sample (Figure 5B) and an MSI-H sample (Figure 5C).

The third summary statistic was a likelihood score, which is a statistical measure of a distribution of probabilities that each sequence read, in a subset of the set of sequence reads encompassing the respective microsatellite locus, is a member of a distribution of sequence reads encompassing a stable construct at the respective microsatellite locus, wherein the subset of sequence reads are the sequence reads encompassing the respective microsatellite locus falling within the tenth percentile of the smallest number of repeat units (i.e., the 10% of sequence reads with the fewest number of repeat units).

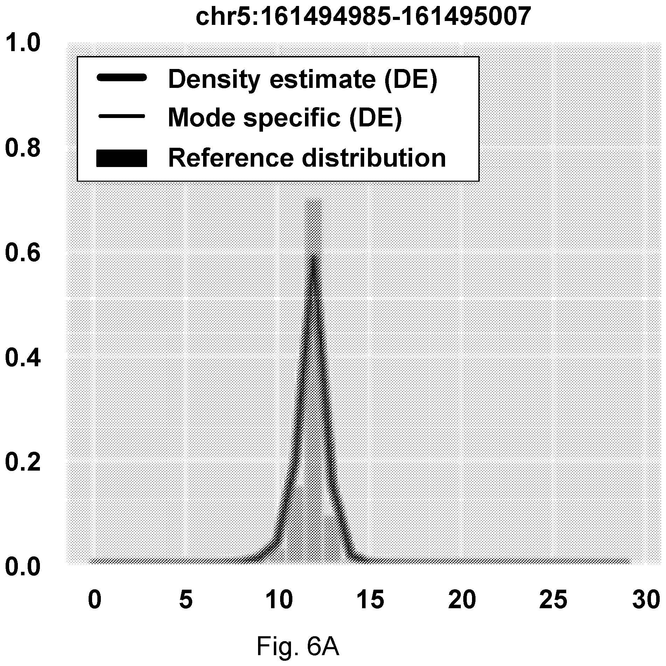

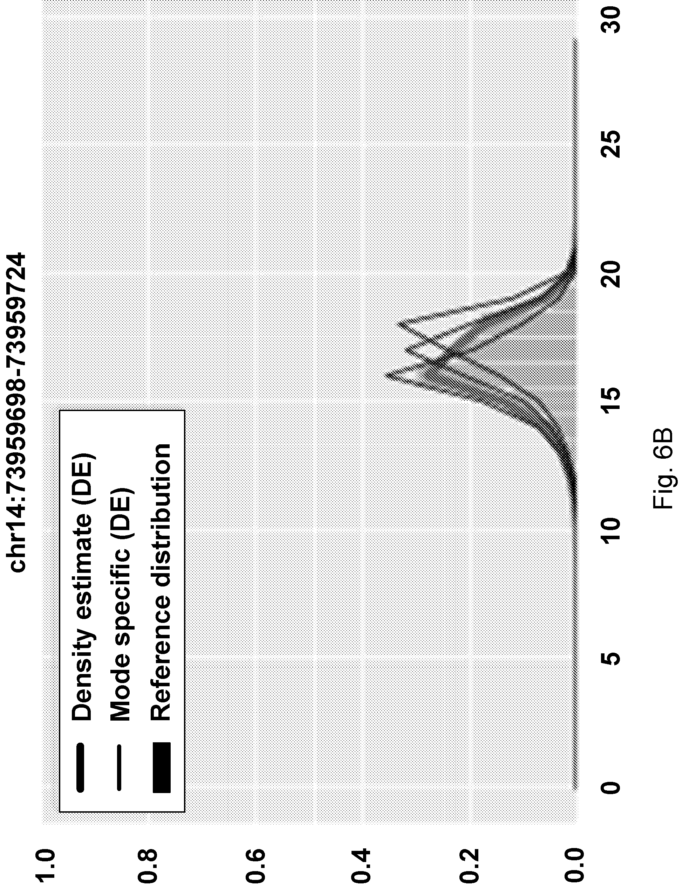

It is important to note that some loci have more than one stable number of repeat units in the population. For example, for a given locus A, patient 1 may have 14 repeat units in their germline DNA while patient 2 only has 12 repeat units. To account for this variability, a training set of 212 solid normal tissue samples and 133 solid tumor samples are used to generate, on a per locus basis, a sample-specific reference distribution and density estimate where only the reference samples that share the same mode of the number of repeat units as the sample being analyzed are included the reference distribution and density estimate. In the aforementioned example, the locus for patient 1 would be compared to a reference distribution and density estimate made up of reference samples having a mode of 14 repeat units at that locus, while the locus for patient 2 would be compared to a reference distribution and density estimate made up of reference samples having a mode of 12 repeat units at that locus. For some loci, there is a single, stable number of repeat units in the population of reference samples (e.g., all samples have 12 repeat units at that locus). Examples of density estimates and reference distributions for a locus with a single stable number of repeat units in a population is shown in Figure 6A, while examples of a global density estimate, mode-specific density estimates, and a reference distribution for a locus with multiple stable numbers of repeat units in the population is shown in Figure 6B.

Accordingly, for each locus in the set of 39 loci, the mode of the number of repeated units was determined. Sequencing data for reference distributions for each locus, which are each based on solid normal tissue samples and solid tumor samples from microsatellite stable cancers having a similar mode of the number of repeated units at the particular locus, were generated using a commercially available hybridization-based next generation sequencing methodology. Each reference distribution is used to model a density estimate of the number of repeat units in sequencing reads of a stable locus (using scikit-learn's KernelDensity function with a gaussian kernel and a bandwidth of 0.5). This density estimate gives an estimation of the probability of each sequencing read coming from a stable locus given the number of repeat units it contains.

After calculating the three summary statistics, a normalization step was applied. Each of the features in the resulting vector was normalized either with scikit-learn's RobustScaler or scikit-learn's PowerTransformer. RobustScaler normalized the vector by removing the median and scaling the data according to the interquartile range. PowerTransformer uses the Yeo-Johnson transform to make the data more "Gaussian-like."

Finally, using 54 labeled patient samples (17 MSI-H, 37 microsatellite stable) sequenced with the hybrid-capture next generation cfDNA sequencing assay, a k-Nearest Neighbor (k-NN) classifier was trained to classify each locus for the test samples. Briefly, 100 neighbors weighted by distance were used.

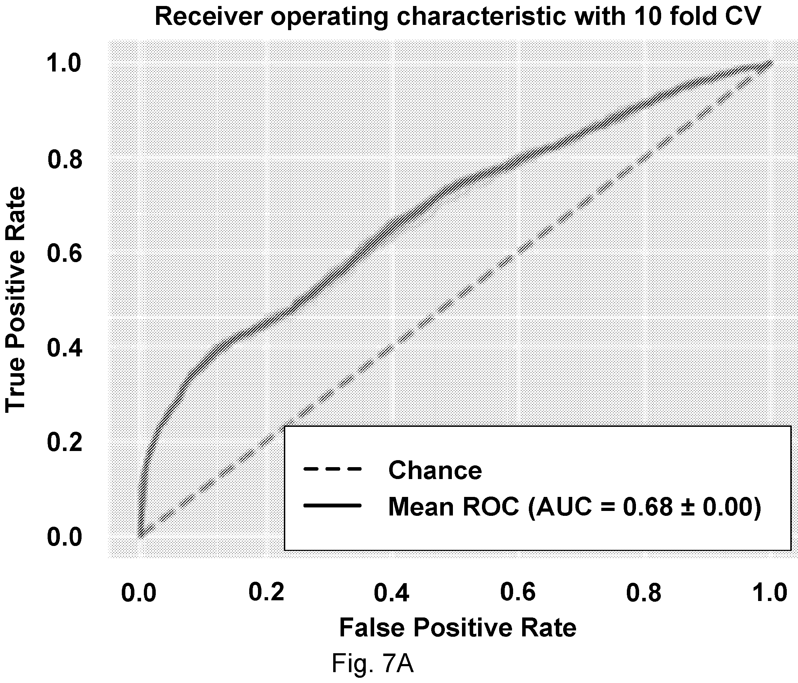

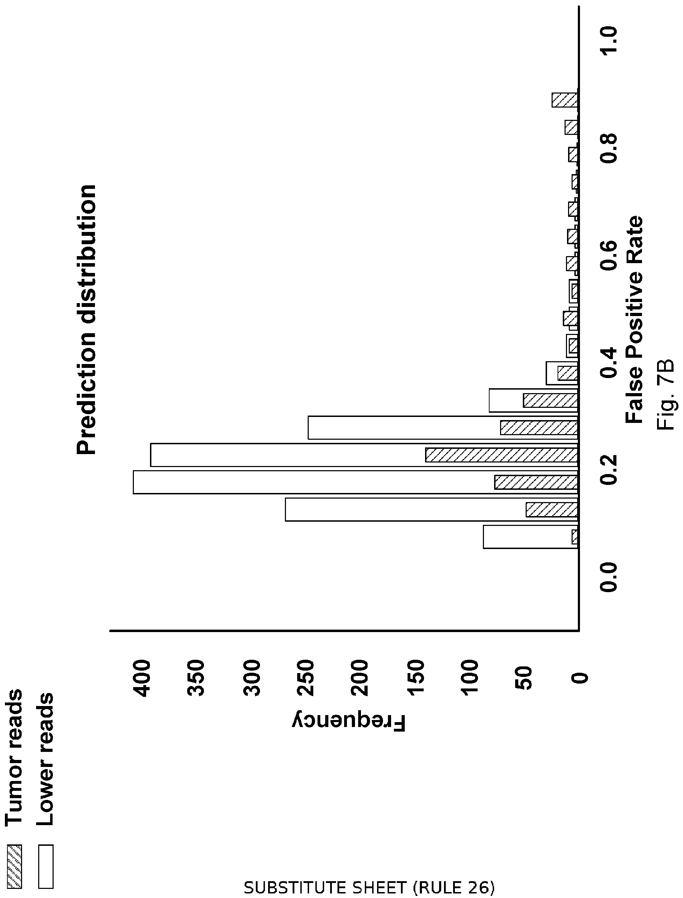

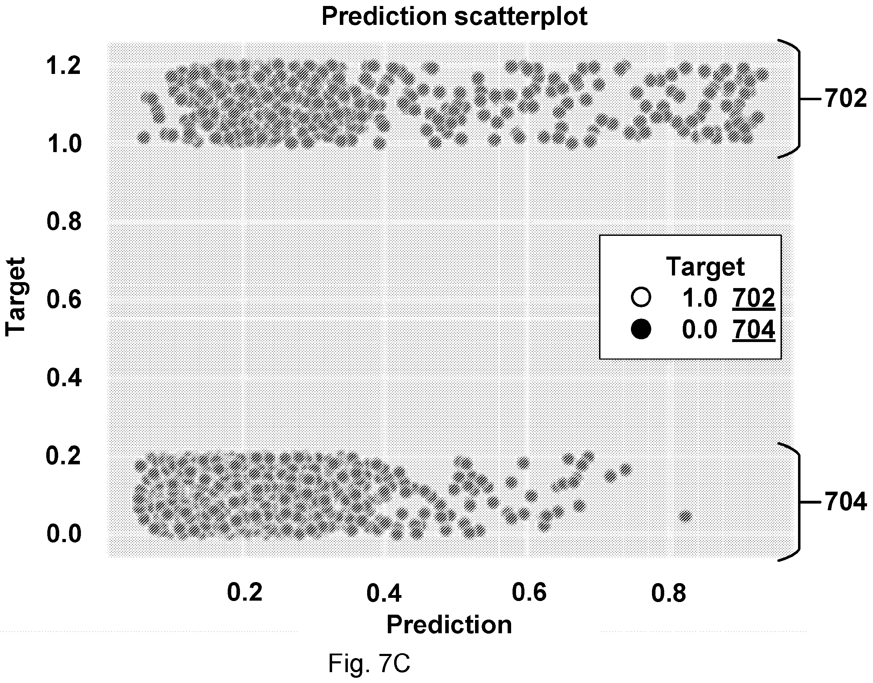

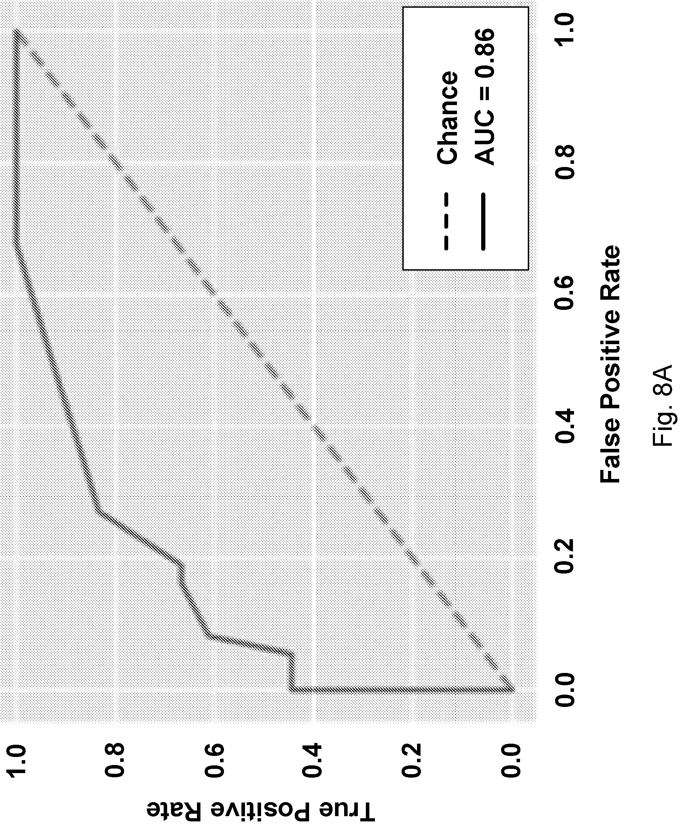

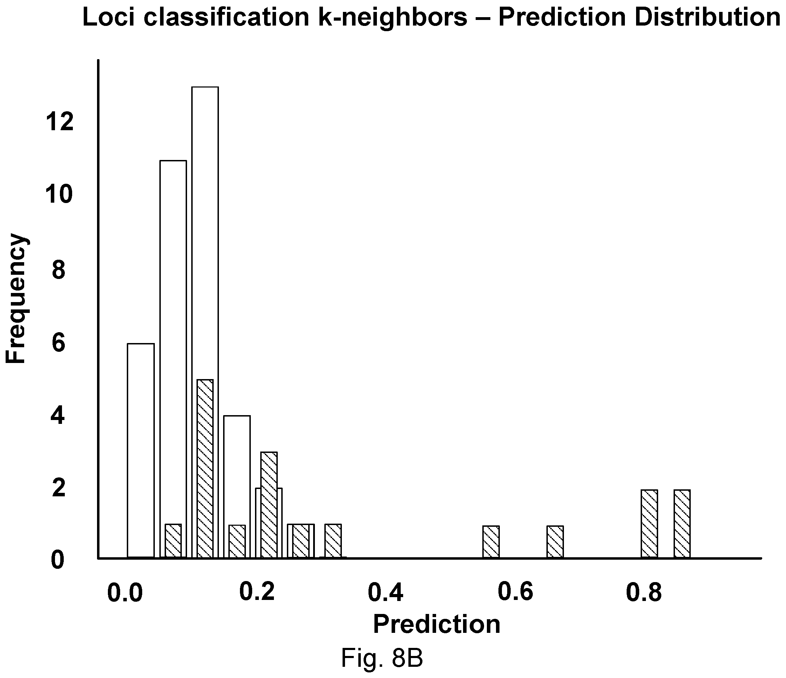

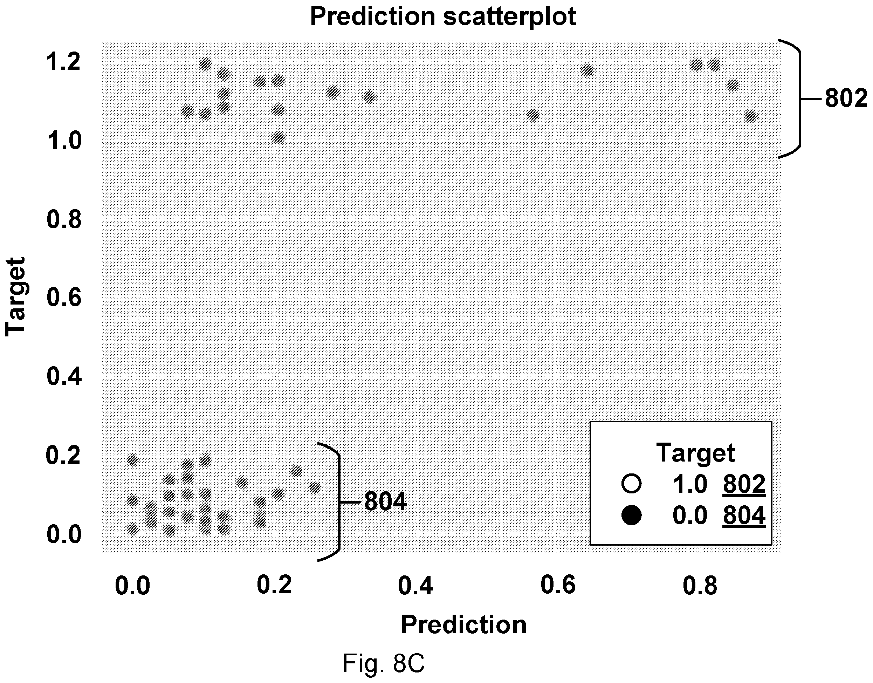

Performance of the locus classifier on the training dataset was then assessed. The model was evaluated using a 30-fold cross validation methodology. Figures 7A-7C show the predictions of the model for each locus. Figures 8A-8C show the percentage of unstable loci in each sample. A locus was labeled as unstable if the probability of it being unstable was superior to 30%.

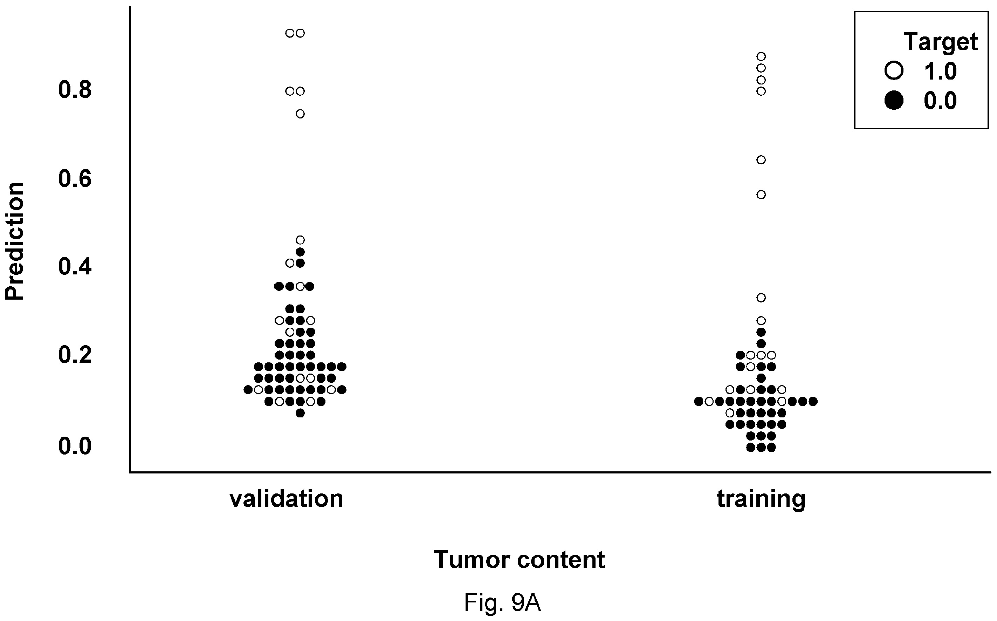

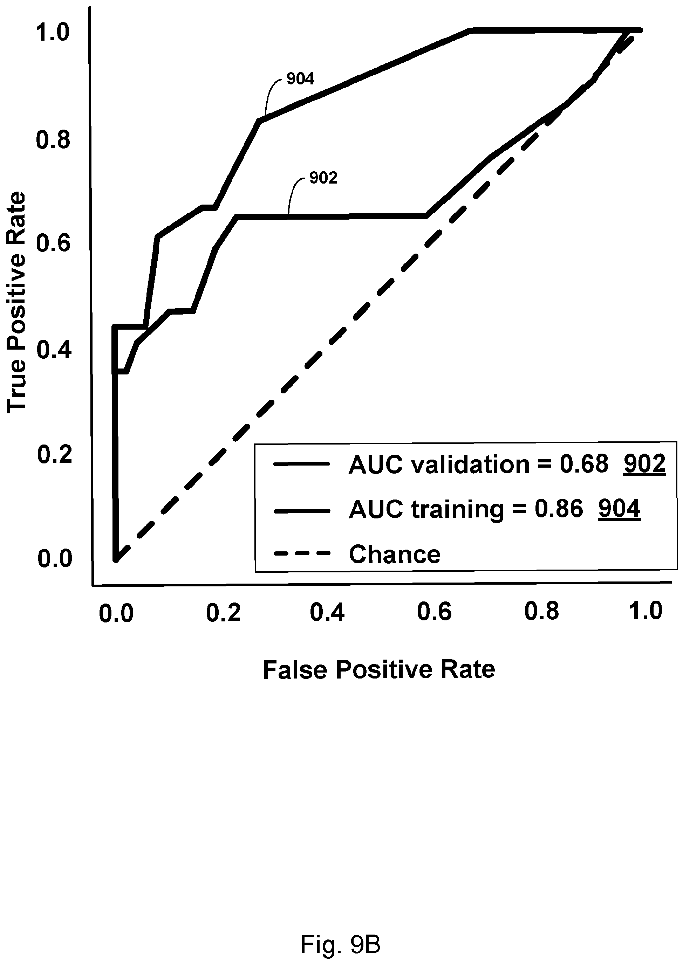

Finally, patient samples with more than 50% unstable loci were classified as MSI-H. Performance of the final classifier was then assessed, as shown in Figure 9A and 9B.

MSI-H status was detected in 6 patient samples. In 3 of these patients (2 colorectal, 1 skin cancer), abnormal MMR IHC confirmed the detected MSI-H status. In the other 3 patients (1 colorectal, 1 non-small cell lung cancer, and 1 endometrial cancer), MSI-H status was confirmed using a clinically validated solid tumor MSI assay. Furthermore, the reliability of the model was validated in 10 technical replicates from 2 MSI-H patients in a training dataset. The results were 100% concordant with all 10 replicates classified as MSI-H.

These results demonstrate the ability of the assay to detect MSI in cfDNA with high specificity, providing a transformative opportunity to report a clinically actionable insight alongside other somatic changes detected from cfDNA.

*Example 3 - Determining the effect of tumor content percentage on specificity when classifying microsatellite instability in several liquid biopsy samples.*

In general, cfDNA samples used for liquid biopsies have a low tumor content (approximately 1-10% of total circulating DNA coming from cancerous cells, and much of the remaining DNA fragments coming from non-cancerous cells). In some instances, the patient from which the liquid biopsy blood sample was taken has an unknown germline phenotype and the length of the patient's microsatellite repeats in the genetic material of non-cancerous cells is unknown. In this example, we explored the possibility of detecting MSI in cfDNA and developed a cfDNA MSI classifier to use as a detection assay with high specificity (greater than 95%).

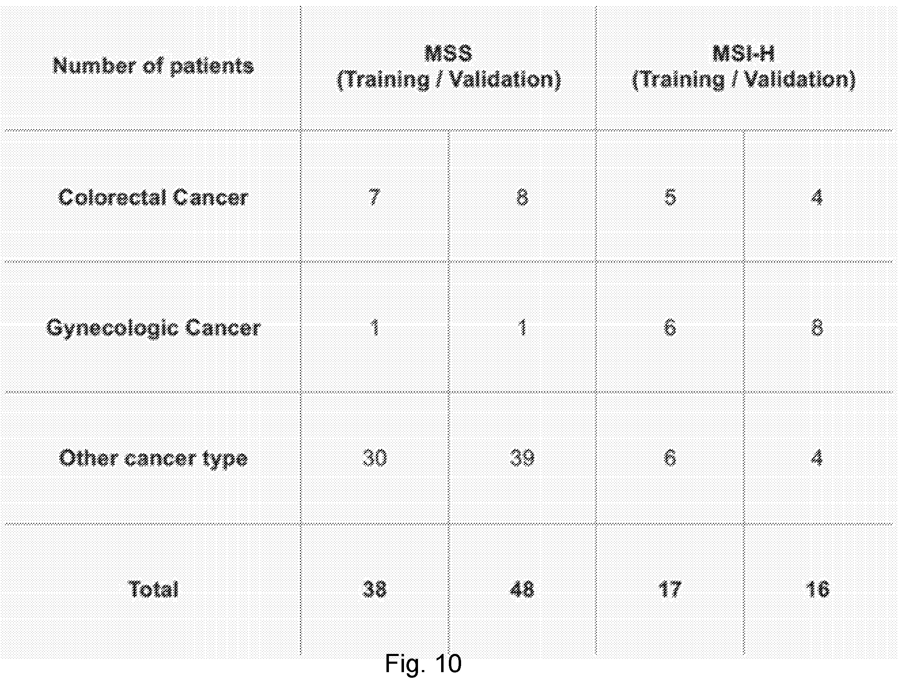

In this example, the cohort used to train and validate the cfDNA MSI model includes liquid biopsy blood samples from patients having colorectal cancer, gynecologic cancer, or another type of cancer. Some of these liquid biopsies were from patients designated as having MSS tumors and some were from patients designated as having MSI-H tumors. The sequencing data from some of these liquid biopsies were used as training data and the data from the other liquid biopsies were used as validation data. (See Figure 10)

A first step of preparing NGS data for training and validating the model, or generating a prediction with the model, included selecting the NGS reads mapping to a microsatellite locus, including any repeated sequence units in the read and a number of base pairs flanking the repeated sequence units. In this example, the number of nucleotide bases on each end of the repeated sequence units is five. (See Figure 18)

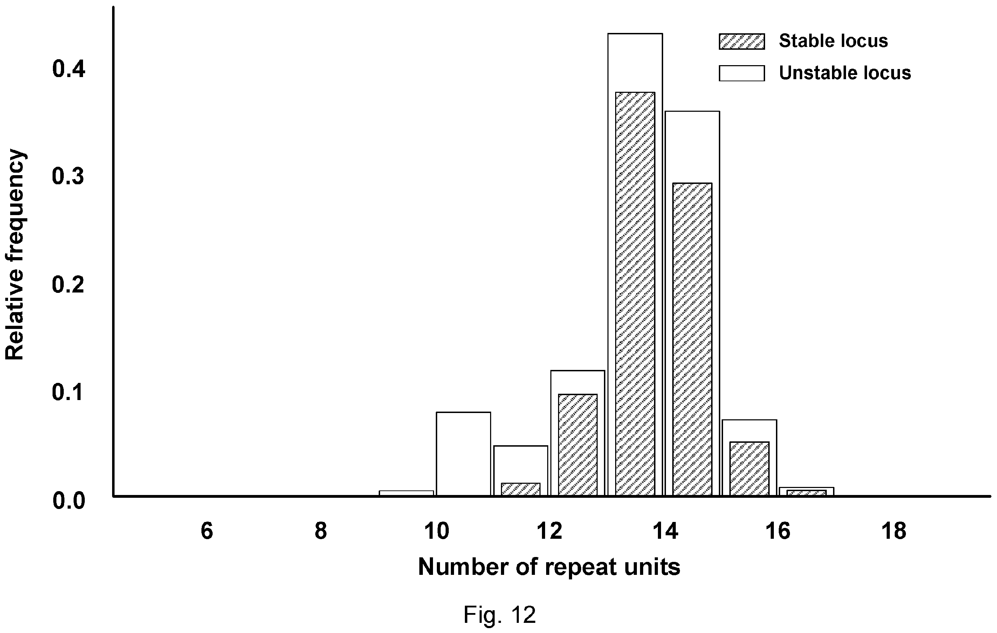

A second step of preparing NGS data for training and validating the model, or generating a prediction with the model, included counting the number of copies of each repeated sequence in each of the reads in a group of reads aligned to a given locus (for example, the group of reads selected in step one) and displaying the distribution of the number of reads containing a given number of copies (repeated units) as a bar chart with the x-axis showing the number of repeated units and the y-axis showing the frequency or percentage of reads having that number of repeated units. In this example, there is a bimodal distribution of the number of repeated units in unstable loci. The bimodal distribution may be due to short reads of DNA fragments originating from cancerous cells. (See Figure 12, red bars) In this example, the stable locus displays a unimodal distribution. (See Figure 12, blue bars)

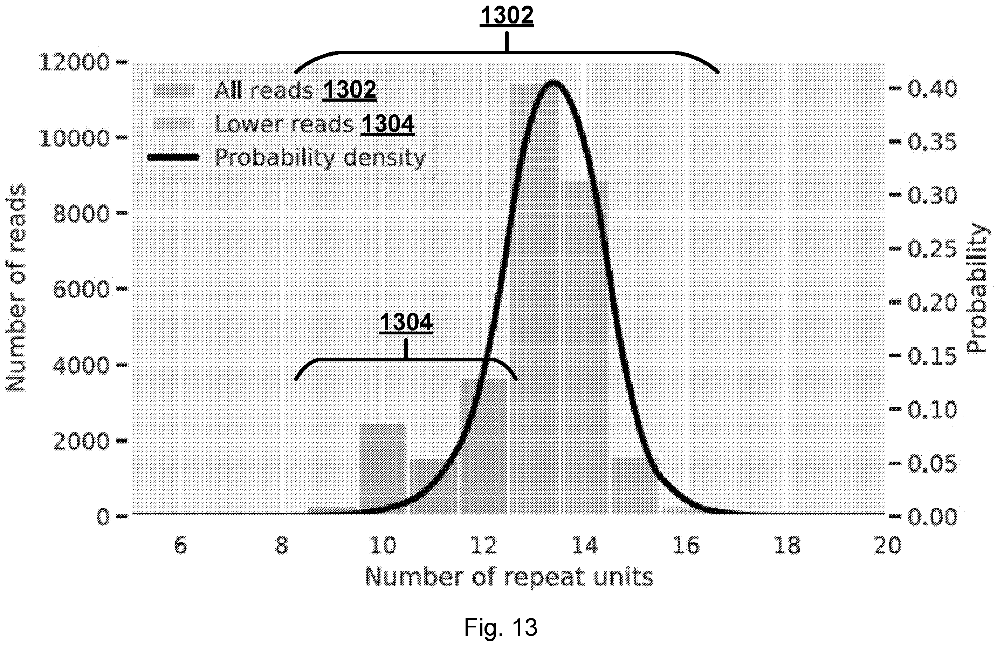

A third step of preparing NGS data for training and validating the model, or generating a prediction with the model, included generating a percent lower metric, a mean lower mode metric, and a likelihood metric as described above. In this example, the percent lower metric is the percentage of reads having less repeated units than the most frequently occurring number of repeated units (See Figure 13, green bars representing reads having less than 13 repeated units, where the x-axis shows the number of repeated units and the left y-axis shows the number of reads having a certain number of repeated units), the mean lower mode metric is the mean number of repeated units in the reads that have less repeated units than the most frequently occurring number of repeated units, and the likelihood metric is the mean log of the likelihood that each read originated from a stable locus based on the number of repeated units it contains (See Figure 13, black line representing the probability density, where the right y-axis shows the probability that a read having a certain number of repeated units originated from a stable locus and the number of repeated units is shown on the x-axis). In this example, the probability model was built from a reference of 133 MSS solid tumor samples and 212 normal, non-cancerous blood samples as described above.

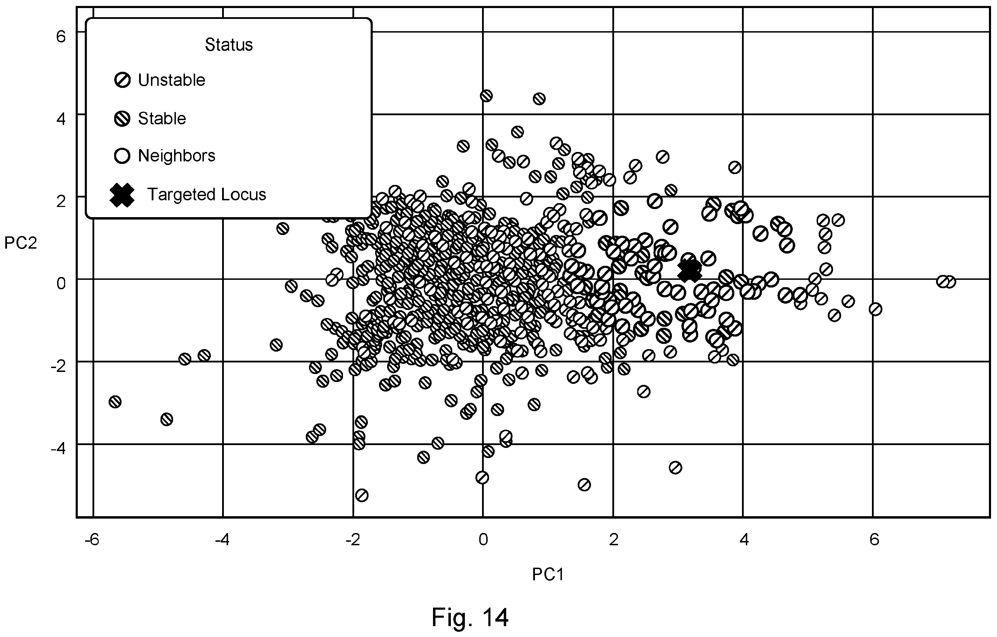

A fourth step of generating a prediction with the model included predicting the probability of a target locus being unstable, using the three metrics generated in the third step and a trained k Nearest Neighbors classifier where k=100. The classifier performed a majority vote among the target locus' 100 closest neighbors, where the designated stable or unstable status of each neighbor locus was used to calculate a percentage of the 100 that are unstable, and the status of each neighbor locus was weighted according to the distance between that neighbor locus and the target locus as described above. See Figure 14, which is a principal component analysis (PCA) of the three metrics associated with each data point in the training cohort data, where each data point represents a single locus. The targeted locus is shown as a black x, the 100 nearest neighbors are shown as purple circles, each representing a locus and the remaining loci are shown as either red circles if they have been designated unstable or blue circles if they have been designated stable. In this example, the three metrics were normalized as described above. (See block 318)

A fifth step of generating a prediction with the model included designating all loci having a probability greater than 30% as unstable and calculating for each liquid biopsy the percentage of all loci in the liquid biopsy designated unstable. For liquid biopsies having greater than 50% of loci designated unstable, the liquid biopsy and associated patient were classified as MSI-H.

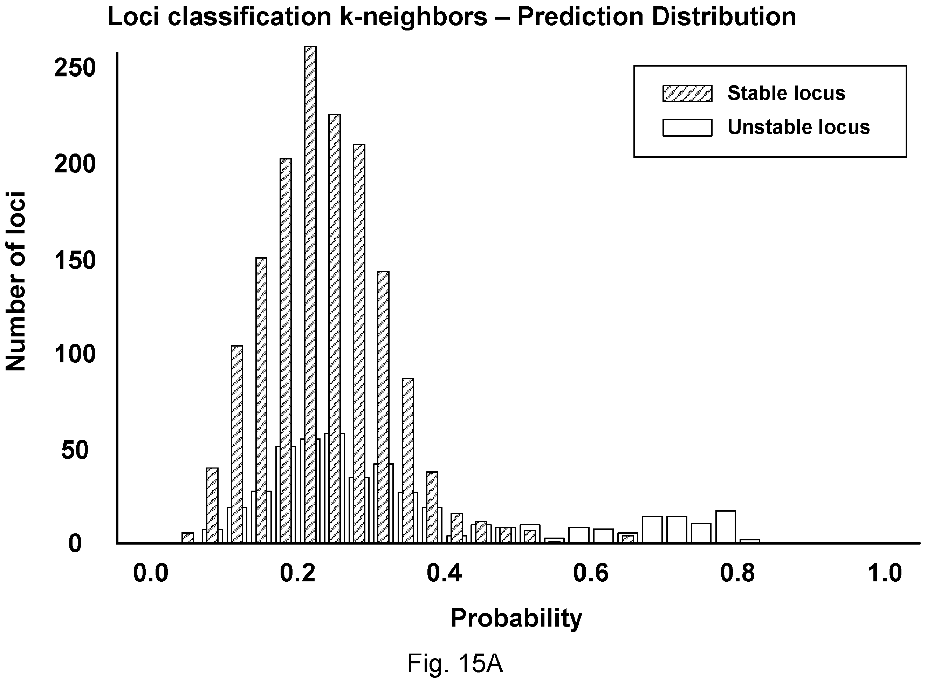

An optional sixth step of validating the model included charting the probability distribution of each locus of the validation cohort, showing that the model detects unstable loci with high specificity and relatively low sensitivity. (See Figure 15A) In this example, stability of a locus is determined using sequencing of the patient's solid tumor and germline cells (blood) and comparing the distribution of the number of repeat units in both.

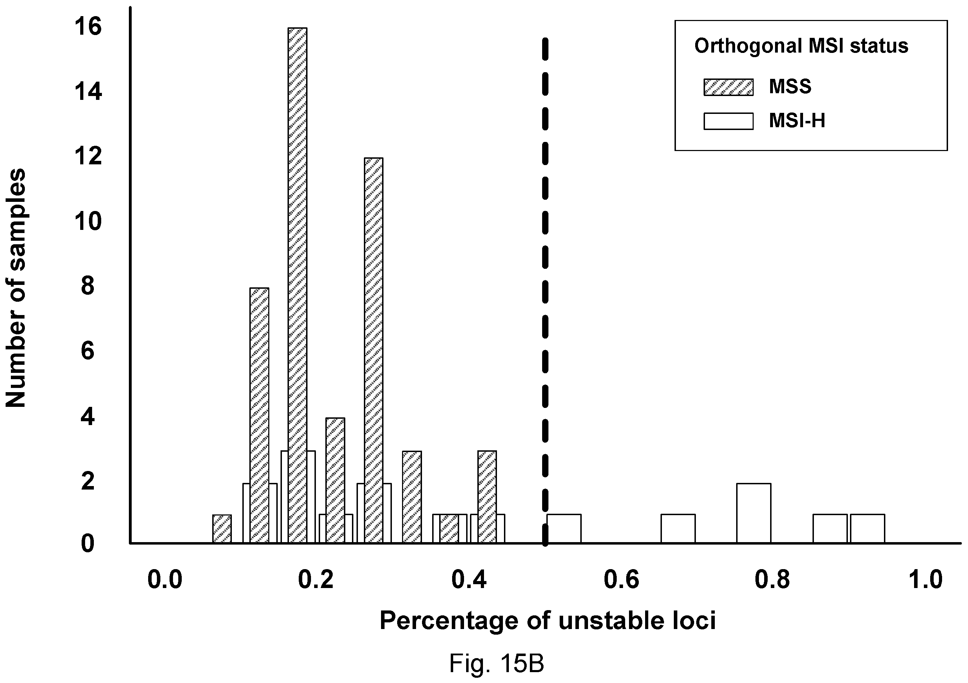

An optional seventh step of validating the model included charting the number of validation samples that had a given percentage of loci designated unstable by the model. (See Figure 15B) The model detected microsatellite instability in 37.5% (6/16) patients at 100% (6/6) positive predictive value. Orthogonal MSI status of the patient was determined by MMR IHC or Tempus' clinically validated solid tumor MSI test.

An optional eighth step of selecting a limit of detection for the model included comparing the percent of unstable loci detected by the model, the orthogonal MSI status, and levels of circulating tumor DNA (ctDNA) relative to total cell free DNA, also known as tumor content, for each liquid biopsy in the model training dataset. (See Figure 16A, colors in legend show the tumor content of each liquid biopsy, x-axis shows orthogonal MSI-H or MSS designation of the liquid biopsy and the y-axis notes the percent of unstable loci detected by the model in the liquid biopsy) In this relatively small cohort, 70% of the false negative samples have a tumor content lower than 6%. In this example, Tumor content was calculated using Low Pass Whole Genome Sequencing (LPWGS) and ichorCNA. (See Viktor A. Adalsteinsson et al. 2017 Nature Communications 8:1324)

An optional ninth step of selecting a limit of detection for the model included charting the effect of the limit of detection on sensitivity, specificity, and negative prediction value of the model, as well as the proportion of samples having tumor content above the limit of detection. (See Figure 16B, red line, green line, blue line, and dashed black line, respectively) In this example, setting a limit of detection at 6% allows the model to achieve a sensitivity of 66% and a negative prediction value of 94%.

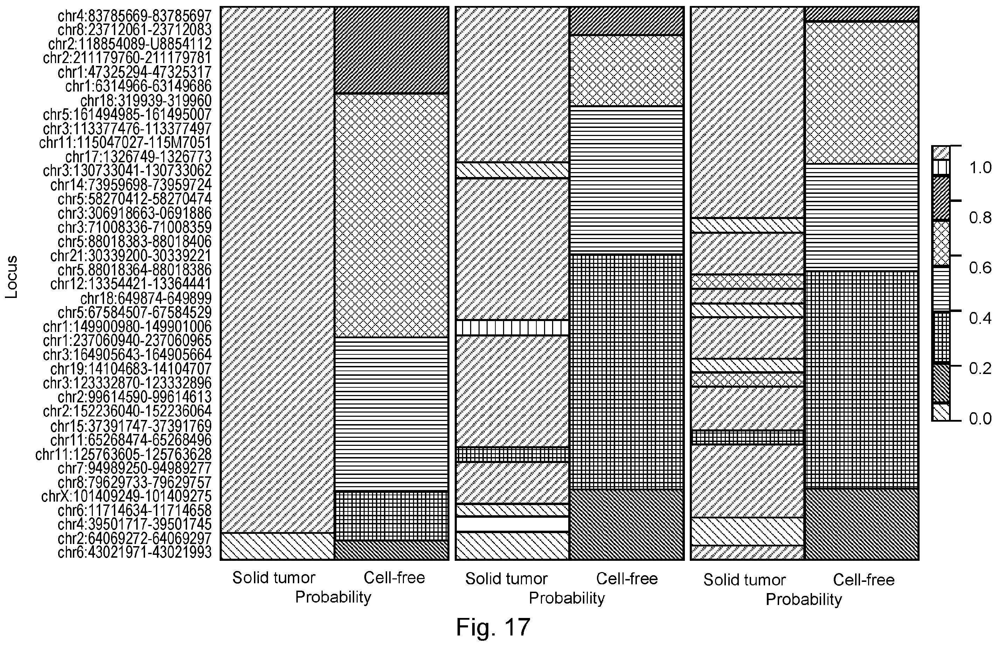

An optional tenth step of validating the model included comparing the results of the cell-free DNA MSI model and the solid tumor MSI test for several individual loci in three separate samples. See Figure 17, which is a heatmap showing the concordance between the solid tumor and cell-free assay predictions of locus instability. Each row is a locus, and each column of the heat map shows the results for a single sample for either the solid tumor assay or the cell-free assay. The legend displays the color associated with a given value of the probabilities of instability determined by an assay for a given locus, for a given sample.

These results demonstrated the ability of the assay to detect MSI in cfDNA with high specificity, providing a transformative opportunity to report a clinically actionable insight alongside other somatic changes detected from cfDNA.

In this example, the MSI status of cfDNA samples with a tumor content of 6% or greater may be classified with a specificity of 96% by the methods disclosed here. The training cohort in this example has 38 MSS samples and 17 MSI-H samples. (See Figure 10). In this example, the sensitivity of our assay was improved by setting a limit of detection of 6% tumor content and may be further improved by increasing the size of the training cohort.

### Example 4 - Determining microsatellite instability by comparing feature distribution.

As an alternative method for determining the microsatellite stability status of a cancer, a feature vector containing only the highest values for each of one or more microsatellite stability metrics is created for a liquid biopsy sample. By selecting only a subset of the feature values for each metric (e.g., those feature values having the highest values, which are the most likely to be unstable), only the loci that are most likely to be unstable in the subject are evaluated, increasing the sensitivity of the model.

Briefly, nucleotide sequences obtained from a liquid biopsy sample are sorted to identify those mapping to a microsatellite locus in a plurality of microsatellite loci, e.g., microsatellite loci in the 39 genes listed in Table 2. The number of copies of each repeated sequence in each of the reads aligned to a given locus (for example, the group of reads shown in Fig. 18) are counted, forming a distribution of the number of repeat units for each microsatellite locus. Microsatellite stability metrics, e.g., a percent lower metric, a mean lower mode metric, and a likelihood metric as described above, are then determined for each microsatellite locus. The $N$ values for each metric that are most indicative of an unstable locus are then selected and compared to one or more reference distribution, e.g., the $N$ values from each of a plurality of stable microsatellite samples.

For example, Table 3 shows an example of the standard deviation metric determined at each of five microsatellite loci in an MSI-H sample, an MSS sample, and two stable reference samples. The highest 2 values of the standard deviation metric are selected for each sample, as underlined in Table 3 (e.g., 0.5 and 1.5 for the MSI-H sample). A Wasserstein geometric distance between the MSI-H values and the reference values ([0.5, 1.5], [0.4, 0.4, 0.4, 0.5]) is determined and input into a classification model trained to predict the probability that the sample is from a subject with microsatellite unstable cancer.

**Table 3.** An example 39 genes containing informative microsatellite loci, for precision oncology.

| | std locus 1 | std locus 1 | std locus 1 | std locus 1 | std locus 1 | std distance |
|---|---|---|---|---|---|---|
| **MSI-H** | **0.5** | 0.3 | 0.4 | 0.2 | **1.5** | 0.58 |
| **MSS** | **0.4** | 0.2 | **0.3** | 0.1 | 0.3 | 0.08 |
| **Ref. 1** | 0.3 | **0.4** | 0.1 | **0.5** | 0.4 | |
| **Ref. 2** | 0.3 | 0.1 | 0.3 | **0.4** | **0.4** | |

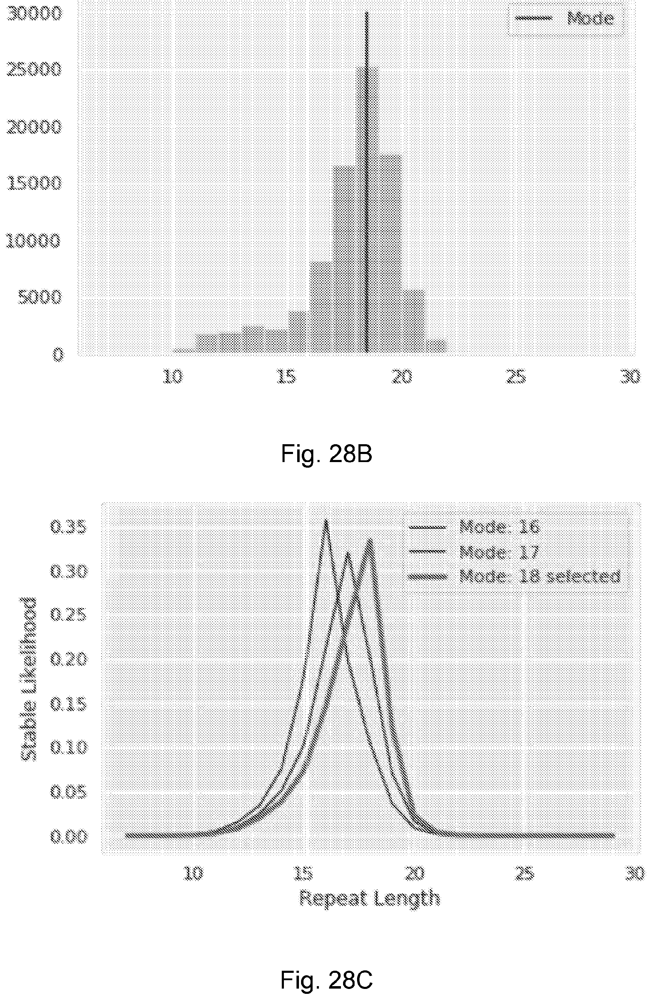

Figures 28 and 29 illustrate performance statistics for a linear regression model trained against the 15 highest metric values, from 39 microsatellite loci corresponding to the genes listed in Table 2, for each of a percent lower metric, a mean lower mode metric, and a likelihood metric. In this example, the probability model was built from a reference of 133 MSS solid tumor samples and 212 normal, non-cancerous blood samples as described above.

**Claims**

1.   A computer-implemented method of determining a microsatellite instability (MSI) status in a liquid biopsy sample obtained from a test subject, the method comprising:
at a computer system having one or more processors, and memory storing one or more programs for execution by the one or more processors:

(A) obtaining, for each respective DNA fragment in a plurality of DNA fragments from the liquid biopsy sample, a corresponding nucleotide sequence that has been aligned to a reference genome for the species of the test subject, thereby obtaining a plurality of aligned nucleotide sequences;
(B) using the plurality of aligned nucleotide sequences to determine, for each respective microsatellite locus in a plurality of predetermined microsatellite loci, wherein each respective microsatellite locus in the plurality of microsatellite loci has a nucleic acid sequence that includes a variable number of repeat units, a first corresponding metric value for a first metric, a second corresponding metric value for a second metric, and a third corresponding metric value for a third metric, wherein the first corresponding metric value, the second corresponding metric value and the third corresponding metric value is determined, at least in part, by a distribution of the number of repeat units in each aligned nucleotide sequence, in the plurality of nucleotide sequences, encompassing the respective microsatellite locus, wherein

the first metric is a percentage score and the corresponding first metric value for the percentage score is a percentage of aligned nucleotide sequences, in the plurality of aligned nucleotides, encompassing the respective microsatellite locus that have a number of repeat units that is less than a first corresponding reference number of repeat units representative of a corresponding stable construct at the respective microsatellite locus,
the second metric is a lower mean score and the corresponding metric value for the lower mean score is determined by the corresponding mean number of repeat units in aligned nucleotide sequences in the plurality of aligned nucleotides that both (i) encompass the respective microsatellite locus and (ii) have a number of repeat units that is less than a second corresponding reference number of repeat units representative of a corresponding stable construct at the respective microsatellite locus, and
the third metric is a likelihood score and the corresponding metric value for the likelihood score is a mean of a distribution of probabilities, wherein each respective probability in the distribution of probabilities is a probability that a corresponding aligned nucleotide sequence in a respective subset of the set of aligned nucleotide sequences encompassing the respective microsatellite locus is a member of a distribution of aligned nucleotide sequences encompassing a corresponding stable construct at the respective microsatellite locus, wherein the respective subset of aligned nucleotide sequences consists of the aligned nucleotide sequences that (i) encompass the respective microsatellite locus and (ii) have a number of repeat units within a threshold percentile of the smallest number of repeat units observed in the respective subset of the set of aligned nucleotide sequences; and

(C) classifying the MSI status of the test subject by inputting, for each respective microsatellite locus in the plurality of predetermined microsatellite loci, at least the first corresponding metric value, the second corresponding metric value and the third corresponding metric value into a classifier that distinguishes between stable and unstable microsatellite loci, thereby determining the MSI status of the test subject.

2. The method of claim 1, wherein, for each respective microsatellite locus in the plurality of predetermined microsatellite loci, the first corresponding reference number of repeat units representative of a corresponding stable construct is a corresponding number of repeat units present in a reference genome at the respective microsatellite locus for the species of the subject.

3. The method of claim 1, wherein, for each respective microsatellite locus in the plurality of predetermined microsatellite loci, the first corresponding reference number of repeat units representative of a corresponding stable construct is a mode of the number of repeat units at the respective microsatellite locus in aligned nucleotide sequences, in the plurality of aligned nucleotides, encompassing the respective microsatellite locus.

4. The method of any one of claims 1-3, wherein the corresponding mean number of repeat units is normalized by the second corresponding reference number of repeat units representative of the corresponding stable construct at the respective microsatellite locus.

5. The method of any one of claims 1-4, wherein, for each respective microsatellite locus in the plurality of predetermined microsatellite loci, the second corresponding reference number of repeat units representative of the corresponding stable construct is a corresponding number of repeat units present in a reference genome for the species of the subject at the respective microsatellite locus.

6. The method of any one of claims 1-4, wherein, for each respective microsatellite locus in the plurality of predetermined microsatellite loci, the second corresponding reference number of repeat units representative of the corresponding stable construct is a mode of the number of repeat units at the respective microsatellite locus in aligned nucleotide sequences, in the plurality of aligned nucleotides, encompassing the respective microsatellite locus.

7. The method of any one of claims 1-6, wherein the distribution of probabilities is a plurality of p-values, wherein each respective p-value in the plurality of p-values is a probability value that a respective aligned nucleotide sequence in the respective subset of aligned nucleotide sequences is a member of the distribution of aligned nucleotide sequences encompassing a the corresponding stable construct at the respective microsatellite locus.

8. The method of any one of claims 1-7, further comprising, prior to the classifying C), normalizing the first corresponding metric value, the second corresponding metric value and the third corresponding metric value across the first corresponding metric value, the second corresponding metric value and the third corresponding metric value of each respective microsatellite loci in the plurality of microsatellite loci.

9. The method of claim 8, the method further comprising using a power transform function for the normalizing of the first corresponding metric value, the second corresponding metric value and the third corresponding metric value.

10. The method of any one of claims 1-9, the method further comprising, prior to the (C) classifying, training the classifier against metric values determined across nucleotide sequences obtained from biological samples from a training cohort that includes (i) solid tumor samples from training subjects with high microsatellite instability (MSI-H) cancer and one or both of (ii)(a) solid tumor samples from training subjects with stable microsatellite (MSS) cancer and (ii)(b) normal tissue samples.

11. The method of any one of claims 1-10, wherein the classifier is a k-nearest neighbors algorithm.

12. The method of any one of claims 1-11, wherein the classifier assigns a stability status selected from unstable and stable to each respective microsatellite locus in the plurality of predetermined microsatellite loci, and the subject is classified as having a high level of microsatellite instability when at least a threshold number of microsatellite loci are assigned a status of unstable.

13. The method of claim 12, wherein the threshold number of microsatellite loci is at least 30% or at least 50% of the total number of microsatellite loci in the plurality of microsatellite loci.

14. The method of any one of claims 1-13, wherein the liquid biopsy sample comprises blood, whole blood, plasma, serum, urine, cerebrospinal fluid, fecal material, saliva, sweat, tears, pleural fluid, pericardial fluid, or peritoneal fluid of the subject.

**15.** The method of any one of claims 1-14, wherein the subject is human and the plurality of predetermined microsatellite loci comprises at least 25 microsatellite loci.

**16.** The method of any one of claims 1-15, wherein the subject is human and the plurality of predetermined microsatellite loci comprises at least 10 or at least all of the microsatellite loci listed in Table 2.


**Patentansprüche**

**1.** Ein computergestütztes Verfahren zur Bestimmung des Mikrosatelliteninstabilitäts-Status (MSI) in einer Flüssigbiopsieprobe, die von einem Probanden entnommen wurde, das Verfahren umfassend:
in einem Computersystem mit einem oder mehreren Prozessoren und einem Speicher, der ein oder mehrere Programme zur Ausführung durch den einen oder die mehreren Prozessoren speichert:

(A) Erhalten einer entsprechenden Nukleotidsequenz für jedes jeweilige DNA-Fragment in einer Vielzahl von DNA-Fragmenten aus der Flüssigbiopsieprobe, die an ein Referenzgenom für die Spezies des Probanden angeglichen wurde, wodurch eine Vielzahl von angeglichenen Nukleotidsequenzen erhalten wird.;
(B) Verwendung der Vielzahl von ausgerichteten Nukleotidsequenzen, um für jeden jeweiligen Mikrosatellitenlocus in einer Vielzahl von vorbestimmten Mikrosatellitenloci, wobei jeder jeweilige Mikrosatellitenlocus in der Vielzahl von Mikrosatellitenloci eine Nukleinsäuresequenz aufweist, die eine variable Anzahl von Wiederholungseinheiten umfasst, einen ersten entsprechenden Metrikwert für eine erste Metrik, einen zweiten entsprechenden Metrikwert für eine zweite Metrik und einen dritten entsprechenden Metrikwert für eine dritte Metrik, wobei der erste entsprechende Metrikwert, der zweite entsprechende Metrikwert und der dritte entsprechende Metrikwert zumindest teilweise durch eine Verteilung der Anzahl der Wiederholungseinheiten in jeder ausgerichteten Nukleotidsequenz in der Vielzahl von Nukleotidsequenzen bestimmt wird, die den jeweiligen Mikrosatellitenlocus umfassen, wobei

die erste Metrik eine prozentuale Bewertung ist und der entsprechende erste Metrikwert für die prozentuale Bewertung ein Prozentsatz der ausgerichteten Nukleotidsequenzen in der Vielzahl von ausgerichteten Nukleotiden ist, die den jeweiligen Mikrosatellitenlocus umfassen, der eine Anzahl von Wiederholungseinheiten aufweist, die kleiner ist als eine erste entsprechende Referenzanzahl von Wiederholungseinheiten, die für ein entsprechendes stabiles Konstrukt an dem jeweiligen Mikrosatellitenlocus repräsentativ ist, die zweite Metrik ein niedrigerer Mittelwert ist und der entsprechende Metrikwert für den niedrigeren Mittelwert durch die entsprechende mittlere Anzahl von Wiederholungseinheiten in den ausgerichteten Nukleotidsequenzen in der Vielzahl von ausgerichteten Nukleotiden bestimmt wird, die sowohl (i) den jeweiligen Mikrosatellitenlocus umfassen als auch (ii) eine Anzahl von Wiederholungseinheiten aufweisen, die kleiner ist als eine zweite entsprechende Referenzanzahl von Wiederholungseinheiten, die für ein entsprechendes stabiles Konstrukt am jeweiligen Mikrosatellitenlocus repräsentativ ist, und die dritte Metrik ein Wahrscheinlichkeitswert ist und der entsprechende Metrikwert für den Wahrscheinlichkeitswert ein Mittelwert einer Wahrscheinlichkeitsverteilung ist, wobei jede jeweilige Wahrscheinlichkeit in der Wahrscheinlichkeitsverteilung eine Wahrscheinlichkeit ist, dass eine entsprechende ausgerichtete Nukleotidsequenz in einer jeweiligen Teilmenge der Menge ausgerichteter Nukleotidsequenzen, die den jeweiligen Mikrosatellitenlocus umfasst, ein Element einer Verteilung ausgerichteter Nukleotidsequenzen ist, die ein entsprechendes stabiles Konstrukt am jeweiligen Mikrosatellitenlocus umfasst, wobei die jeweilige Teilmenge ausgerichteter Nukleotidsequenzen aus den ausgerichteten Nukleotidsequenzen besteht, die (i) den jeweiligen Mikrosatellitenlocus umfassen und (ii) eine Anzahl von Wiederholungseinheiten innerhalb eines Schwellenperzentils der kleinsten Anzahl von Wiederholungseinheiten aufweisen, die in der jeweiligen Teilmenge der Menge der ausgerichteten Nukleotidsequenzen beobachtet wurde; und

(C) Klassifizieren des MSI-Status des Probanden durch Eingeben von mindestens des ersten entsprechenden Metrikwerts, des zweiten entsprechenden Metrikwerts und des dritten entsprechenden Metrikwerts für jeden jeweiligen Mikrosatellitenlocus in der Vielzahl von vorbestimmten Mikrosatellitenloci in einen Klassifizierer, der zwischen stabilen und instabilen Mikrosatellitenloci unterscheidet, wodurch der MSI-Status des Probanden bestimmt wird.

**2.** Das Verfahren nach Anspruch 1, wobei für jeden jeweiligen Mikrosatellitenlocus in der Vielzahl von vorbestimmten Mikrosatellitenloci die erste entsprechende Referenznummer von Wiederholungseinheiten, die für ein entsprechendes stabiles Konstrukt repräsentativ sind, eine entsprechende Anzahl von Wiederholungseinheiten ist, die in einem Referenzgenom an dem jeweiligen Mikrosatellitenlocus für die Spezies des Probanden vorhanden sind.

3. Das Verfahren nach Anspruch 1, wobei für jeden jeweiligen Mikrosatellitenlocus in der Vielzahl von vorbestimmten Mikrosatellitenloci die erste entsprechende Referenznummer von Wiederholungseinheiten, die für ein entsprechendes stabiles Konstrukt repräsentativ sind, ein Modus der Anzahl von Wiederholungseinheiten an dem jeweiligen Mikrosatellitenlocus in ausgerichteten Nukleotidsequenzen in der Vielzahl von ausgerichteten Nukleotiden ist, die den jeweiligen Mikrosatellitenlocus umfassen.

4. Das Verfahren nach einem der Ansprüche 1 - 3, wobei der entsprechende Mittelwert der Anzahl von Wiederholungseinheiten durch die zweite entsprechende Referenzzahl von Wiederholungseinheiten normalisiert wird, die für das entsprechende stabile Konstrukt an dem jeweiligen Mikrosatellitenlocus repräsentativ ist.

5. Das Verfahren nach einem der Ansprüche 1 - 4, wobei für jeden jeweiligen Mikrosatellitenlocus in der Vielzahl von vorbestimmten Mikrosatellitenloci die zweite entsprechende Referenznummer von Wiederholungseinheiten, die für das entsprechende stabile Konstrukt repräsentativ sind, eine entsprechende Anzahl von Wiederholungseinheiten ist, die in einem Referenzgenom für die Spezies des Probanden an dem jeweiligen Mikrosatellitenlocus vorhanden sind.

6. Das Verfahren nach einem der Ansprüche 1 - 4, wobei für jeden jeweiligen Mikrosatellitenlocus in der Vielzahl von vorbestimmten Mikrosatellitenloci die zweite entsprechende Referenznummer von Wiederholungseinheiten, die für das entsprechende stabile Konstrukt repräsentativ sind, ein Modus der Anzahl von Wiederholungseinheiten an dem jeweiligen Mikrosatellitenlocus in ausgerichteten Nukleotidsequenzen in der Vielzahl von ausgerichteten Nukleotiden ist, die den jeweiligen Mikrosatellitenlocus umfassen.

7. Das Verfahren nach einem der Ansprüche 1 - 6, wobei die Wahrscheinlichkeitsverteilung eine Vielzahl von p-Werten ist, wobei jeder jeweilige p-Wert in der Vielzahl von p-Werten ein Wahrscheinlichkeitswert dafür ist, dass eine jeweilige ausgerichtete Nukleotidsequenz in der jeweiligen Teilmenge ausgerichteter Nukleotidsequenzen ein Element der Verteilung ausgerichteter Nukleotidsequenzen ist, die ein entsprechendes stabiles Konstrukt an dem jeweiligen Mikrosatellitenlocus umfasst.

8. Das Verfahren nach einem der Ansprüche 1 - 7, vor dem (C) Klassifizieren ferner umfassend das Normalisierendes ersten entsprechenden Metrikwerts, des zweiten entsprechenden Metrikwerts und des dritten entsprechenden Metrikwerts über den ersten entsprechenden Metrikwert, den zweiten entsprechenden Metrikwert und den dritten entsprechenden Metrikwert jedes jeweiligen Mikrosatellitenlocus in der Vielzahl von Mikrosatellitenloci normalisiert werden.

9. Das Verfahren nach Anspruch 8, wobei das Verfahren ferner die Verwendung einer Leistungstransformationsfunktion zum Normalisieren des ersten entsprechenden Metrikwerts, des zweiten entsprechenden Metrikwerts und des dritten entsprechenden Metrikwerts umfasst.

10. Das Verfahren nach einem der Ansprüche 1 - 9, wobei das Verfahren vor dem (C) Klassifizieren ferner das Trainieren des Klassifizierers anhand von Metrikwerten umfasst, die über Nukleotidsequenzen bestimmt wurden, die aus biologischen Proben einer Trainingskohorte gewonnen wurden, die (i) solide Tumorproben von Trainingsprobanden mit hochgradig mikrosatelliteninstabilem (MSI-H) Krebs und eine oder beide der folgenden Gruppen: (ii)(a) solide Tumorproben von Trainingsprobanden mit stabilem Mikrosatelliten (MSS) Krebs und (ii)(b) normale Gewebeproben beinhaltet.

11. Das Verfahren nach einem der Ansprüche 1 - 10, wobei der Klassifizierer ein k-nächste-Nachbarn-Algorithmus ist.

12. Das Verfahren nach einem der Ansprüche 1 - 11, wobei der Klassifizierer jedem der in der Vielzahl von vorbestimmten Mikrosatellitenloci einen Stabilitätsstatus zuweist, der von instabil bis stabil ausgewählt ist, und der Proband als hochgradig mikrosatelliteninstabil klassifiziert wird, wenn mindestens einer Schwellenanzahl von Mikrosatellitenloci ein instabiler Status zugewiesen wird.

13. Das Verfahren nach Anspruch 12, wobei die Schwellenanzahl an Mikrosatellitenloci mindestens 30 % oder mindestens 50 % der Gesamtzahl an Mikrosatellitenloci in der Vielzahl von Mikrosatellitenloci beträgt.

14. Das Verfahren nach einem der Ansprüche 1 - 13, wobei die Flüssigbiopsieprobe Blut, Vollblut, Plasma, Serum, Urin, Cerebrospinalflüssigkeit, Stuhlmaterial, Speichel, Schweiß, Tränen, Pleuraflüssigkeit, Perikardergussflüssigkeit oder Peritonealflüssigkeit des Probanden umfasst.

15. Das Verfahren nach einem der Ansprüche 1 - 14, wobei der Proband ein Mensch ist und die Vielzahl vorbestimmter Mikrosatellitenloci mindestens 25 Mikrosatellitenloci umfasst.

16. Das Verfahren nach einem der Ansprüche 1 - 15, wobei der Proband ein Mensch ist und die Vielzahl vorbestimmter Mikrosatellitenloci mindestens 10 oder mindestens alle in Tabelle 2 aufgeführten Mikrosatellitenloci umfasst.

**Revendications**

1. Un procédé mis en œuvre par ordinateur de détermination d'un état d'instabilité microsatellitaire (MSI) dans un échantillon de biopsie liquide obtenu à partir d'un sujet de test, le procédé comprenant :
   sur un système informatique présentant un ou plusieurs processeurs, et une mémoire stockant un ou plusieurs programmes à exécuter par le ou les processeurs :

   (A) l'obtention, pour chaque fragment d'ADN respectif parmi une pluralité de fragments d'ADN provenant de l'échantillon de biopsie liquide, d'une séquence nucléotidique correspondante qui a été alignée sur un génome de référence pour l'espèce du sujet de test, permettant ainsi d'obtenir une pluralité de séquences nucléotidiques alignées ;
   (B) l'utilisation de la pluralité de séquences nucléotidiques alignées pour déterminer, pour chaque locus microsatellitaire respectif dans une pluralité de loci microsatellitaires prédéterminés, dans lequel chaque locus microsatellitaire respectif dans la pluralité de loci microsatellitaires présente une séquence d'acide nucléique incluant un nombre variable d'unités répétées, une valeur de première métrique correspondante pour une première métrique, une valeur de deuxième métrique correspondante pour une deuxième métrique, et une valeur de troisième métrique correspondante pour une troisième métrique, dans lequel la valeur de première métrique correspondante, la valeur de deuxième métrique correspondante et la valeur de troisième métrique correspondante sont déterminées, au moins en partie, par une distribution du nombre d'unités répétées dans chaque séquence nucléotidique alignée, dans la pluralité de séquences nucléotidiques, englobant le locus microsatellitaire respectif, dans lequel

   la première métrique est un score en pourcentage et la valeur de première métrique correspondante pour le score en pourcentage est un pourcentage de séquences nucléotidiques alignées, dans la pluralité de nucléotides alignés, englobant le locus microsatellitaire respectif qui présente un nombre d'unités répétées qui est inférieur à un premier nombre de référence correspondant d'unités répétées représentatif d'une construction stable correspondante au niveau du locus microsatellitaire respectif,
   la deuxième métrique est un score moyen inférieur et la valeur de métrique correspondante pour le score moyen inférieur est déterminée par le nombre moyen correspondant d'unités répétées dans des séquences nucléotidiques alignées au sein de la pluralité de nucléotides alignés qui à la fois (i) englobent le locus microsatellitaire respectif et (ii) présentent un nombre d'unités répétées inférieur à un second nombre de référence correspondant d'unités répétées représentatif d'une construction stable correspondante au niveau du locus microsatellitaire respectif, et
   la troisième métrique est un score de vraisemblance et la valeur de métrique correspondante pour le score de vraisemblance est une moyenne d'une distribution de probabilités, dans lequel chaque probabilité respective dans la distribution de probabilités est une probabilité qu'une séquence nucléotidique alignée correspondante dans un sous-ensemble respectif de l'ensemble de séquences nucléotidiques alignées englobant le locus microsatellitaire respectif soit membre d'une distribution de séquences nucléotidiques alignées englobant une construction stable correspondante au niveau du locus microsatellitaire respectif, dans lequel le sous-ensemble respectif de séquences nucléotidiques alignées se compose des séquences nucléotidiques alignées qui (i) englobent le locus microsatellitaire respectif et (ii) présentent un nombre d'unités répétées dans un percentile seuil du plus petit nombre d'unités répétées observées dans le sous-ensemble respectif de l'ensemble de séquences nucléotidiques alignées ; et

   (C) la classification de l'état MSI du sujet de test en entrant, pour chaque locus microsatellitaire respectif dans la pluralité de loci microsatellitaires prédéterminés, au moins la valeur de première métrique correspondante, la valeur de deuxième métrique correspondante et la valeur de troisième métrique correspondante dans un classificateur qui permet la distinction entre les loci microsatellitaires stables et instables, déterminant ainsi l'état MSI du sujet de test.

**2.** Le procédé selon la revendication 1, dans lequel, pour chaque locus microsatellitaire respectif dans la pluralité de loci microsatellitaires prédéterminés, le premier nombre de référence correspondant d'unités répétées représentatif d'une construction stable correspondante est un nombre correspondant d'unités répétées présentes dans un génome de référence au niveau du locus microsatellitaire respectif pour l'espèce du sujet.

**3.** Le procédé selon la revendication 1, dans lequel, pour chaque locus microsatellitaire respectif dans la pluralité de loci microsatellitaires prédéterminés, le premier nombre de référence correspondant d'unités répétées représentatif d'une construction stable correspondante est un mode du nombre d'unités répétées au niveau du locus microsatellitaire respectif dans des séquences nucléotidiques alignées, dans la pluralité de nucléotides alignés, englobant le locus microsatellitaire respectif.

**4.** Le procédé selon l'une quelconque des revendications 1 à 3, dans lequel le nombre moyen correspondant d'unités répétées est normalisé par le second nombre de référence correspondant d'unités répétées représentatif de la construction stable correspondante au niveau du locus microsatellitaire respectif.

**5.** Le procédé selon l'une quelconque des revendications 1 à 4, dans lequel, pour chaque locus microsatellitaire respectif dans la pluralité de loci microsatellitaires prédéterminés, le second nombre de référence correspondant d'unités répétées représentatif de la construction stable correspondante est un nombre correspondant d'unités répétées présentes dans un génome de référence pour l'espèce du sujet au niveau du locus microsatellitaire respectif.

**6.** Le procédé selon l'une quelconque des revendications 1 à 4, dans lequel, pour chaque locus microsatellitaire respectif dans la pluralité de loci microsatellitaires prédéterminés, le second nombre de référence correspondant d'unités répétées représentatif de la construction stable correspondante est un mode du nombre d'unités répétées au niveau du locus microsatellitaire respectif dans des séquences nucléotidiques alignées, dans la pluralité de nucléotides alignés, englobant le locus microsatellitaire respectif.

**7.** Le procédé selon l'une quelconque des revendications 1 à 6, dans lequel la distribution de probabilités est une pluralité de valeurs p, dans lequel chaque valeur p respective dans la pluralité de valeurs p est une valeur de probabilité qu'une séquence nucléotidique alignée respective dans le sous-ensemble respectif de séquences nucléotidiques alignées soit un membre de la distribution de séquences nucléotidiques alignées englobant la construction stable correspondante au niveau du locus microsatellitaire respectif.

**8.** Le procédé selon l'une quelconque des revendications 1 à 7, comprenant en outre, avant (C) la classification, la normalisation de la valeur de première métrique correspondante, de la valeur de deuxième métrique correspondante et de la valeur de troisième métrique correspondante par rapport à la valeur de première métrique correspondante, à la valeur de deuxième métrique correspondante et à la valeur de troisième métrique correspondante de chaque locus microsatellitaire respectif dans la pluralité de loci microsatellitaires.

**9.** Le procédé selon la revendication 8, le procédé comprenant en outre l'utilisation d'une fonction de transformée de puissance pour la normalisation de la valeur de première métrique correspondante, de la valeur de deuxième métrique correspondante et de la valeur de troisième métrique correspondante.

**10.** Le procédé selon l'une quelconque des revendications 1 à 9, le procédé comprenant en outre, avant (C) la classification, l'entraînement du classificateur avec des valeurs métriques déterminées par rapport à des séquences nucléotidiques obtenues à partir d'échantillons biologiques d'une cohorte d'entraînement qui inclut (i) des échantillons de tumeurs solides provenant de sujets d'entraînement atteints d'un cancer à instabilité microsatellitaire élevée (MSI-H) et un ou les deux parmi (ii)(a) des échantillons de tumeurs solides provenant de sujets d'entraînement atteints d'un cancer à microsatellites stables (MSS) et (ii)(b) des échantillons de tissus normaux.

**11.** Le procédé selon l'une quelconque des revendications 1 à 10, dans lequel le classificateur est un algorithme de voisins les plus proches k.

**12.** Le procédé selon l'une quelconque des revendications 1 à 11, dans lequel le classificateur attribue un état de stabilité choisi parmi instable et stable à chaque locus microsatellitaire respectif dans la pluralité de loci microsatellitaires prédéterminés, et le sujet est classé comme présentant un niveau élevé d'instabilité microsatellitaire lorsque au moins un nombre seuil de loci microsatellitaires est associé à un état d'instabilité.

13. Le procédé selon la revendication 12, dans lequel le nombre seuil de loci microsatellitaires est d'au moins 30 % ou d'au moins 50 % du nombre total de loci microsatellitaires dans la pluralité de loci microsatellitaires.

14. Le procédé selon l'une quelconque des revendications 1 à 13, dans lequel l'échantillon de biopsie liquide comprend du sang, du sang total, du plasma, du sérum, de l'urine, du liquide céphalorachidien, des matières fécales, de la salive, de la sueur, des larmes, du liquide pleural, du liquide péricardique ou du liquide péritonéal du sujet.

15. Le procédé selon l'une quelconque des revendications 1 à 14, dans lequel le sujet est humain et la pluralité de loci microsatellitaires prédéterminés comprend au moins 25 loci microsatellitaires.

16. Le procédé selon l'une quelconque des revendications 1 à 15, dans lequel le sujet est humain et la pluralité de loci microsatellitaires prédéterminés comprend au moins 10 ou au moins tous les loci microsatellitaires énumérés dans le Tableau 2.

| System 100 | | |
|---|---|---|
| | Operating System  116 | |
| | Network communication module  118 | |
| | Test patient data store  120 | |
| Non-persistent memory 111 | Bioinformatics module  140 | |
| | | Sequence data processing module  141 |
| | | Pre-processing algorithms  142 |
| | | Alignment algorithm  143 |
| | | Demultiplexing algorithm  144 |
| | | Feature extraction module 145 |
| | | Variant identification module  146 |
| | | SNV/MNV calling algorithm 147 |
| | | Indel calling algorithm 148 |
| | | Genomic rearrangement calling algorithm 149 |
| | | Variant allele fraction module 151 |
| | | Methylation analysis module 152 |
| | | Copy Number Variation (CNV) analysis module 153 |
| | | Microsatellite Instability (MSI) analysis module  154 |
| | | Tumor Mutational Burden (TMB) analysis module  155 |
| | | Tumor ploidy analysis module  156 |
| | | Homologous recombination pathway analysis module  157 |
| | | Reference sequence constructs  158 |
| | Feature analysis module  160 | |
| | | Genomic alteration interpretation algorithms  161 |
| | | Pathogenic variant analysis algorithms  162 |
| | | Genomic variant analysis algorithms  163 |
| | | Compound feature analysis algorithms  164 |
| | | Clinical data analysis algorithms  165 |
| | | Therapeutic curation algorithms  166 |
| | | Recommendation validation module  167 |
| | Reporting module  170 | |

Network 105

114

Processing core 102

Persistent memory 112

User interface 106
Display 108
Keyboard 110

Network interface 104

**Fig. 1A**

Non-persistent memory 111

| Test patient data store 120 |
|---|

| Patient 1 121-1 |
|---|

| Sequencing data 122-1 |
|---|

| Sequencing run 1 122-1-1 |
|---|

| Sequence read 1 123-1-1-1 |
|---|
| ⋮ |
| Sequence read K 123-1-1-K |
| Aligned sequences (e.g., BAM file) 124-1-1 |

⋮

| Sequencing run L 122-1-L |
|---|

| Feature data 125-1 |
|---|

| Personal characteristics 126-1 |
|---|
| Medical history 127-1 |
| Clinical features 128-1 |

| Pathology data 128-1-1 |
|---|
| Imaging data 128-2-1 |
| Tissue culture / organoid data 128-3-1 |

| Genomic features 131-1 |
|---|

| Allelic states 132-1 |
|---|
| Variant allele fractions 133-1 |
| Methylation states 134-1 |
| Genomic copy numbers 135-1 |
| Tumor mutational burden 136-1 |
| Microsatellite instability status 137-1-1 |
| Tumor ploidy 137-1-2 |
| HRD status 137-1-3 |

| Other -omics data 138-1 |
|---|

| Clinical assessment 139-1 |
|---|

| Actionable variants and characteristics 139-1-1 |
|---|
| Matched therapies 139-1-2 |
| Clinical reports 139-1-3 |

⋮

| Patient M 121-M |
|---|

Fig. 1B

Genomic features  131

Microsatellite instability status  137-1-1

Liquid biopsy repeat lengths  137-1-1-1-cf

MSI locus 1  137-1-1-1-cf-1

Repeat units for sequence read 1  137-1-1-1-cf-1-1

⋮

Repeat units for sequence read O  137-1-1-1-cf-1-O

⋮

MSI locus P  137-1-1-1-cf-P

Non-persistent memory
111

Fig. 1C

Feature extraction module  145

Microsatellite Instability (MSI) analysis module  154

Repeat unit metric module  154-a

Percent lower statistic algorithm  154-a-1

Mean lower mode statistic algorithm  154-a-2

Likelihood statistic algorithm  154-a-3

Standard deviation algorithm  154-a-4

Repeat unit length references  154-b

MSI locus 1 repeat unit length reference  154-b-1

⋮

MSI locus P repeat unit length reference  154-b-P

Non-persistent memory
111

Fig. 1D

EP 4 008 005 B1

**Sample Collection 201**    **200**    **Clinical Data Analysis 202**

Patient Intake → Physical Specimens Received → Receive Clinical Data which can include patient progress notes, pathology reports, imaging reports, and any other documents

**Wet Lab Processing 204**

Accessioning → Pathology Review → Extraction → Library Prep

Capture + Hybridize → Pooling → Sequencing

Ingest clinical data into platform

**Feature Extraction 206**

**Variant Analysis 208**

Variant Science Analysis

Interpret somatic and germline variants for pathogenic and biological significance

MSI & TMB: Microsatellite instability and tumor mutational burden are important determinants of a patient's eligibility for immunotherapies

Review Clinical Data and abstract relevant clinical information based on predefined set of curation rules

Therapy Curation

Targeted treatments are matched based on gene, variant and cancer types

Clinical Trials

Selects clinical trials based on mutations, cancer type, and clinical history

Validation by Report Team

Final validation checks are also completed by the clinical trials team, including patient and sample information

As available and when applicable, Clinical Data populates the patient's clinical history timeline

Pathologist Final Review and Sign Off

Report Delivered

Fig. 2A

Fig. 2B

**204  Wet Lab Processing**

**302  Accessioning**

Tissue biopsy                    Liquid biopsy

Clinical Review
316

Pathology review

Medical imaging review

Tissue culture review

**304  Extraction**

DNA            RNA                    cfDNA

**306  Library Prep**

DNA library       RNA library             cfDNA library

**308  Pooling**

Pool of DNA     Pool of RNA          Library of cfDNA

**310  Capture + Hybridize**

Captured pool DNA     Captured pool RNA     Captured pool cfDNA

**312  Sequencing**

Sequenced DNA     Sequenced RNA     Sequenced cfDNA

BCL file  314

**206  Feature Extraction**

Fig. 3

Fig. 4A

Fig. 4B

```
order.tar.gz
└── order/
    ├── order_N_1.fastq.gz
    ├── order_N_2.fastq.gz
    ├── order_T_1.fastq.gz
    └── order_T_2.fastq.gz
```

Sequence identifier
Sequence base calls
Base call quality scores
(phred)

Fig. 4C

Raw FASTQ files → FASTQ Quality Control (trimming, filtering, correcting for artifacts, etc.) → Processesed FASTQ files

Fig. 4D

Fig. 4E

400

**402** Obtain cfDNA Sequencing Data

**404** Align to reference human construct

Aligned reads
**406**

**408** Generate repeat unit metrics for microsatellite loci

Repeat unit metrics **410**

Percentage lower **412**

Mean lower mode **414**

Likelihood **416**

**418** Normalize repeat unit metrics

Normalized repeat
unit metrics 4**20**

**422** Classify MSI status using repeat
unit metrics

**424** Match therapies and/or clinical
trials based on MSI status

Patient report indicating
MSI status **426**

Fig. 4F

**450**

Aligned BAMs **452**

*Locus 1*

**452-1** Extract reads

**454-1** Count number of repeat units

**452-1** Generate features

**458-1** Apply model

Probability **460-1**

**462-1** Call locus stability

• • •

*Locus P*

**452-P** Extract reads

**454-P** Count number of repeat units

**452-P** Generate features

**458-P** Apply model

Probability **460-P**

**462-P** Call locus stability

**462** Determine MSI Status

Fig. 4G

EP 4 008 005 B1

**Locus: chr2:152236040-152236064**

Fig. 5A

# Stable locus

Fig. 5B

Fig. 5C

Fig. 6A

chr14:73959698-73959724

Legend:
— Density estimate (DE)
— Mode specific (DE)
■ Reference distribution

Fig. 6B

Receiver operating characteristic with 10 fold CV

Fig. 7A

Prediction distribution

Tumor reads
Lower reads

Fig. 7B

False Positive Rate

Frequency

Fig. 7C

Fig. 8A

Loci classification k-neighbors – Prediction Distribution

Fig. 8B

Fig. 8C

Fig. 9A

Fig. 9B

| Number of patients | MSS (Training / Validation) | | MSI-H (Training / Validation) | |
|---|---|---|---|---|
| Colorectal Cancer | 7 | 8 | 5 | 4 |
| Gynecologic Cancer | 1 | 1 | 6 | 8 |
| Other cancer type | 30 | 39 | 6 | 4 |
| Total | 38 | 48 | 17 | 16 |

Fig. 10

System 1100

Non-Persistent Memory 111

Processing core 102

114

Network interface 104

User interface 106

Display 108

Input 110

Operating system 116

Network communication module 118

Cancer classification module 1120

Test subject data store 1122

Sequencing data 1124

Test subject 1 1126-1

Sequence read 1 1128-1-1

⋮

Sequence read M 1128-1-M

⋮

Test subject N 1126-N

Sequence read 1 1128-N-1

⋮

Sequence read O 1128-N-O

⋮

Sequence alignment module 1130

Reference genomic construct 1132

Sequence alignment data store 1134

Test subject 1 1136-1

Alignment of sequence read 1 1138-1-1

⋮

Alignment of sequence read M 1138-1-M

⋮

Test subject N 1136-N

Alignment of sequence read 1 1138-N-1

⋮

Alignment of sequence read O 1138-N-M

Microsatellite stability classification module 1140

Repeat unit metric module 1142

Percent lower statistic 1144

Mean lower mode statistic 1146

Likelihood statistic 1148

Standard deviation 1150

Patient reporting module 1170

Persistent memory 112

Fig. 11

Fig. 12

Fig. 13

Fig. 14

# Loci classification k-neighbors – Prediction Distribution

Fig. 15A

Fig. 15B

EP 4 008 005 B1

Fig. 16A

Fig. 16B

Fig. 17

Aligned reads

AAATTAAAAAAAAAAAGAAAT     SEQ ID NO:1

AAATTAAAAAAAAAAAAAAGAAAT     SEQ ID NO:2

AAATTAAAAAAAAAAAAAAAAAAAGAAAT     SEQ ID NO:3

AAATTAAAAAAAAAAGAAAT     SEQ ID NO:4

Fig. 18

1900

1902 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

1904 Obtain, for each respective DNA fragment in a plurality of DNA fragments from a liquid biopsy sample from a test subject, a set of raw sequence reads for the respective DNA fragment

1906 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

1908 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

1910 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome

1912 Use the plurality of aligned nucleotide sequences to determine, for each respective microsatellite locus in a plurality of predetermined microsatellite loci, wherein each respective microsatellite locus in the plurality of microsatellite loci has a nucleic acid sequence that includes a variable number of repeat units, a corresponding metric value for each of one or more metrics, wherein each respective metric in the one or more metrics is determined, at least in part, by a distribution of the number of repeat units in each aligned nucleotide sequence, in the plurality of aligned nucleotide sequences, encompassing the respective microsatellite locus

(A)

Fig. 19A

1912 *continued . . .*

(A)

1914 The one or more metrics comprises a percentage score, and the corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus

1916 The one or more metrics comprises a lower mean score, and the corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus

1918 The one or more metrics comprises a likelihood score, and the corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units

1920 The one or more corresponding metric values comprises a corresponding standard deviation of the number of repeat units in the aligned nucleotide sequences, in the plurality of aligned nucleotide sequences, encompassing the respective microsatellite locus

1922 Normalize the one or more metrics across the plurality of microsatellite loci

1924 Compare, for each respective microsatellite locus in the plurality of predetermined microsatellite loci, the corresponding metric values for the one or more metrics to corresponding reference metric values for the one or more metrics representative of a stable construct at the respective microsatellite locus, the reference metric values determined across nucleotide sequences obtained from biological samples from a training cohort that includes (i) solid tumor samples from training subjects with stable microsatellite cancer or (ii) normal tissue samples, thereby determining the MSI status of the test subject

Fig. 19B

2000

2002 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

2004 Obtain, for each respective DNA fragment in a plurality of DNA fragments from a liquid biopsy sample from a test subject, a set of raw sequence reads for the respective DNA fragment

2006 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

2008 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

2010 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome

2012 Use the plurality of aligned nucleotide sequences to determine, for each respective microsatellite locus in a plurality of predetermined microsatellite loci, wherein each respective microsatellite locus in the plurality of microsatellite loci has a nucleic acid sequence that includes a variable number of repeat units, a corresponding metric value for each of two or more independent metrics, wherein each respective metric in the two or more metrics is determined, at least in part, by a distribution of the number of repeat units in each aligned nucleotide sequence, in the plurality of nucleotide sequences, encompassing the respective microsatellite locus

(A)

Fig. 20A

2012 *continued . . .*

(A)

2014 The two or more metrics comprises a percentage score, and the corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus

2016 The two or more metrics comprises a lower mean score, and the corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus

2018 The two or more metrics comprises a likelihood score, and the corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units

2020 The two or more corresponding metric values comprises a corresponding standard deviation of the number of repeat units in the aligned nucleotide sequences, in the plurality of aligned nucleotide sequences, encompassing the respective microsatellite locus

2022 Normalize the two or more metrics across the plurality of microsatellite loci

2024 Classify the MSI status of the test subject by inputting, for each respective microsatellite locus in the plurality of predetermined microsatellite loci, the corresponding metric values for the two or more metrics to a classifier that distinguishes between stable and unstable microsatellite loci, thereby determining the MSI status of the test subject

Fig. 20B

2100

2102 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

2104 Obtain, for each respective DNA fragment in a plurality of DNA fragments from a liquid biopsy sample from a test subject, a set of raw sequence reads for the respective DNA fragment

2106 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

2108 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

2110 Use the plurality of aligned nucleotide sequences to determine, for each respective microsatellite locus in a plurality of predetermined microsatellite loci, where each respective microsatellite locus in the plurality of microsatellite loci has a nucleic acid sequence that includes a variable number of repeat units, a corresponding metric value for a percentage score metric which is determined, at least in part, by the percentage of sequence reads, in the set of sequence reads encompassing the respective microsatellite locus in the plurality of sequence reads, having a number of repeat units that is less than a predetermined number of repeat units representative of a stable construct at the respective microsatellite locus

(B)

Fig. 21A

B

2111

2112 Use the plurality of sequence reads to determine, for each respective microsatellite locus in the plurality of predetermined microsatellite loci, a corresponding metric value for a lower mean score metric which is determined, at least in part, by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. The one or more metrics used in the classifying comprise the lower mean score for the respective microsatellite locus

2114 Use the first plurality of sequence reads to determine, for each respective microsatellite locus in the plurality of predetermined microsatellite loci, a corresponding metric value for a likelihood score metric determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units. The one or more metrics used in the classifying comprise the likelihood score for the respective microsatellite locus

2116 Use the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. The one or more metrics used in the classifying comprise the corresponding standard deviation for the respective microsatellite locus

C

Fig. 21B

( C )

2118 Normalize the one or more metrics across the plurality of microsatellite loci

2120 Classify the MSI status of the test subject by inputting, for each respective microsatellite locus in the plurality of predetermined microsatellite loci, a corresponding metric value for each of one or more metrics, including the corresponding percentage score, to a classifier that distinguishes between stable and unstable microsatellite loci, thereby determining the MSI status of the test subject

Fig. 21C

2200

2202 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

2204 Obtain, for each respective DNA fragment in a plurality of DNA fragments from a liquid biopsy sample from a test subject, a set of raw sequence reads for the respective DNA fragment

2206 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

2208 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

2210 Use the plurality of aligned nucleotide sequences to determine, for each respective microsatellite locus in a plurality of predetermined microsatellite loci, where each respective microsatellite locus in the plurality of microsatellite loci has a nucleic acid sequence that includes a variable number of repeat units, a corresponding metric value for a lower mean score metric that is determined, at least in part, by the mean number of repeat units in the set of aligned nucleotide sequences encompassing the respective microsatellite locus, in the plurality of aligned nucleotide sequences, having a number of repeat units that is less than a predetermined number of repeat units representative of a stable construct at the respective microsatellite locus

B

Fig. 22A

B

2212 Use the plurality of sequence reads to determine, for each respective microsatellite locus in the plurality of predetermined microsatellite loci, a corresponding metric value for a percentage score metric which is determined, at least in part, by the percentage of aligned nucleotide sequences, in the plurality of aligned nucleotides, encompassing the respective microsatellite locus that have a number of repeat units that is less than a reference number of repeat units representative of a stable construct at the respective microsatellite locus. The one or more metrics used in the classifying comprise the corresponding percentage score for the respective microsatellite locus

2214 Use the first plurality of sequence reads to determine, for each respective microsatellite locus in the plurality of predetermined microsatellite loci, a corresponding metric value for a likelihood score metric determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units. The one or more metrics used in the classifying comprise the likelihood score for the respective microsatellite locus

2216 Use the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. The one or more metrics used in the classifying comprise the corresponding standard deviation for the respective microsatellite locus

C

Fig. 22B

C

2218 Normalize the one or more metrics across the plurality of microsatellite loci

2220 Classify the MSI status of the test subject by inputting, for each respective microsatellite locus in the plurality of predetermined microsatellite loci, a corresponding metric value for each of one or more metrics, including the corresponding lower mean score, to a classifier that distinguishes between stable and unstable microsatellite loci, thereby determining the MSI status of the test subject

Fig. 22C

2300

2302 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

2304 Obtain, for each respective DNA fragment in a plurality of DNA fragments from a liquid biopsy sample from a test subject, a set of raw sequence reads for the respective DNA fragment

2306 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

2308 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

2310 Use the plurality of aligned nucleotide sequences to determine, for each respective microsatellite locus in a plurality of predetermined microsatellite loci, where each respective microsatellite locus in the plurality of microsatellite loci has a nucleic acid sequence that includes a variable number of repeat units, a corresponding metric value for a likelihood score metric determined, at least in part, by a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus. The subset of aligned nucleotide sequences consists of the aligned nucleotide sequences encompassing the respective microsatellite locus that have a number of repeat units that falls within a threshold percentile of the smallest number of repeat units

B

Fig. 23A

B

**2312** Use the plurality of sequence reads to determine, for each respective microsatellite locus in the plurality of predetermined microsatellite loci, a corresponding metric value for a percentage score metric which is determined, at least in part, by the percentage of aligned nucleotide sequences, in the plurality of aligned nucleotides, encompassing the respective microsatellite locus that have a number of repeat units that is less than a reference number of repeat units representative of a stable construct at the respective microsatellite locus. The one or more metrics used in the classifying comprise the corresponding percentage score for the respective microsatellite locus

**2314** Use the plurality of sequence reads to determine, for each respective microsatellite locus in the plurality of predetermined microsatellite loci, a corresponding metric value for a lower mean score metric which is determined, at least in part, by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus. The one or more metrics used in the classifying comprise the corresponding lower mean score for the respective microsatellite locus

**2316** Use the first plurality of sequence reads to determine, for each respective microsatellite locus in a plurality of predetermined microsatellite loci, a corresponding metric value for the standard deviation of the number of repeat units in the aligned nucleotide sequences, in the plurality of aligned nucleotide sequences, encompassing the respective microsatellite locus. The one or more metrics used in the classifying comprise the corresponding standard deviation for the respective microsatellite locus

**2318** Normalize the one or more metrics across the plurality of microsatellite loci

**2320** Classify the MSI status of the test subject by inputting, for each respective microsatellite locus in the plurality of predetermined microsatellite loci, a corresponding metric value for each of one or more metrics, including the likelihood score, to a classifier that distinguishes between stable and unstable microsatellite loci, thereby determining the MSI status of the test subject

Fig. 23B

2400

2402 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

2404 Obtain, for each respective DNA fragment in a plurality of DNA fragments from a liquid biopsy sample from a test subject, a set of raw sequence reads for the respective DNA fragment

2406 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

2408 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

2410 Use the plurality of aligned nucleotide sequences to determine, for each respective microsatellite locus in a plurality of predetermined microsatellite loci, where each respective microsatellite locus in the plurality of microsatellite loci has a nucleic acid sequence that includes a variable number of repeat units, a corresponding mode of the number of repeat units at the respective microsatellite locus in aligned nucleotide sequences encompassing the respective microsatellite locus

B

Fig. 24A

B

2412 Use the plurality of aligned nucleotide sequences to determine, for each respective microsatellite locus in the plurality of predetermined microsatellite loci, a corresponding metric value for each of one or more metrics, where each respective metric in the one or more metrics is determined, at least in part, by a distribution of the number of repeat units in each aligned nucleotide sequence, in the plurality of aligned nucleotide sequences, encompassing the respective microsatellite locus

2414 Normalize the one or more metrics across the plurality of microsatellite loci

2416 Classify the MSI status of the test subject by inputting, for each respective microsatellite locus in the plurality of predetermined microsatellite loci, a corresponding metric value for each of one or more metrics to a classifier that distinguishes between stable and unstable microsatellite loci, thereby determining the MSI status of the test subject. At least one of the one or more metrics is a comparison of a distribution of the number of repeat units, in each aligned nucleotide sequence encompassing the respective microsatellite locus, to the corresponding mode of the number of repeat units at the respective microsatellite locus

Fig. 24B

2500

2502 Obtain, for each respective DNA fragment in a plurality of DNA fragments from a liquid biopsy sample from a test subject, a corresponding nucleotide sequence that has been aligned to a reference genome for the species of the test subject, thereby obtaining a plurality of aligned nucleotide sequences

> 2504 Obtain, for each respective DNA fragment in a plurality of DNA fragments from a liquid biopsy sample from a test subject, a set of raw sequence reads for the respective DNA fragment

> 2506 Align, for each respective DNA fragment in the plurality of DNA fragments, each respective raw sequence read in the corresponding set of raw sequence reads to a reference genome for the test subject, thereby obtaining a corresponding set of aligned raw sequence reads for the respective DNA fragment

> 2508 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome, thereby obtaining a plurality of aligned nucleotide sequences

> 2510 Collapse, for each respective DNA fragment in the plurality of DNA fragments, the corresponding set of aligned raw sequence reads for the respective DNA fragment into a corresponding nucleotide sequence for the respective DNA fragment that has been aligned to the human reference genome

2512 Use the plurality of aligned nucleotide sequences to determine, for each respective microsatellite locus in a plurality of predetermined microsatellite loci, wherein each respective microsatellite locus in the plurality of microsatellite loci has a nucleic acid sequence that includes a variable number of repeat units, a corresponding metric value for each of one or more metrics, wherein each respective metric in the one or more metrics is determined, at least in part, by a distribution of the number of repeat units in each aligned nucleotide sequence, in the plurality of aligned nucleotide sequences, encompassing the respective microsatellite locus

$$\left(\text{A}\right)$$

Fig. 25A

<u>2512</u> *continued . . .*

( A )

---
<u>2514</u> The one or more metrics comprises a percentage score, and the corresponding metric value for the percentage score is the percentage of aligned nucleotide sequences, in the plurality of aligned nucleotides, encompassing the respective microsatellite locus that have a number of repeat units that is less than a reference number of repeat units representative of a stable construct at the respective microsatellite locus

---
<u>2516</u> The one or more metrics comprises a lower mean score, and the corresponding metric value for the lower mean score is determined at least in part by the mean number of repeat units in aligned nucleotide sequences, in the plurality of aligned nucleotides, encompassing the respective microsatellite locus that have a number of repeat units that is less than a reference number of repeat units representative of a stable construct at the respective microsatellite locus

---
<u>2518</u> The one or more metrics comprises a likelihood score, and the corresponding metric value for the likelihood score is a statistical measure of a distribution of probabilities that each respective aligned nucleotide sequence in a subset of the set of aligned nucleotide sequences encompassing the respective microsatellite locus is a member of a distribution of aligned nucleotide sequences encompassing a stable construct at the respective microsatellite locus, wherein the subset of aligned nucleotide sequences consists of the aligned nucleotide sequences encompassing the respective microsatellite locus that have a number of repeat units that falls within a threshold percentile of the smallest number of repeat units

---
<u>2520</u> The one or more corresponding metric values comprises a corresponding standard deviation of the number of repeat units in the aligned nucleotide sequences, in the plurality of aligned nucleotide sequences, encompassing the respective microsatellite locus

---
<u>2522</u> Normalize the one or more metrics across the plurality of microsatellite loci

---
<u>2524</u> Generate one or more MSI scoring vectors for the test subject, each respective MSI scoring vector in the one or more MSI scoring vector comprising, for a respective metric in the one or more metrics, the highest N metric values

( B )

Fig. 25B

B

2526 Determine, for each respective MSI scoring vectors in the one or more MSI scoring vectors, a difference between the respective MSI scoring vector and a reference MSI vector, wherein the reference MSI vector comprises corresponding metric values derived from one or more biological samples from one or more reference subjects with stable microsatellite loci

2528 Classify the MSI status of the subject by inputting, for each respective MSI scoring vector in the one or more MSI scoring vectors, the difference between the respective MSI scoring vector and the reference MSI vector to a classifier that distinguishes between stable and unstable microsatellite loci, thereby determining the MSI status of the subject

Fig. 25C

Receiver operating characteristic with 10 fold CV

Fig. 26A

Feature Distribution (logistic regression) Prediction Distribution

Fig. 26B

Fig. 26C

EP 4 008 005 B1

Fig. 27A

Fig. 27B

Fig. 28A

Fig. 28A1

Fig. 28B

Fig. 28C

2900

2902 Determine distribution of microsatellite repeat lengths at the respective locus

E.g., 18, 18, 18, 15, 12, 14, . . .

2904 Determine mode of distribution

E.g., 18

2906 Generate stability metrics

2908 Determine percentage score

E.g., 0.297

2910 Determine mean lower mode or mean lower mode normalized

E.g., 13.29 or 0.114

2912 Determine mean lower mode or mean lower mode normalized

2914 Obtain a probability density function (e.g., kernel density estimation) corresponding to mode of distribution

2916 Select reads with repeat unit lengths below a threshold percentile

E.g., 10th percentile = 14 repeat length

2918 Determine the probability that each selected read corresponds to a stable locus, given the probability density function corresponding to the mode of the distribution

E.g., loglikelihood for reads of length:
11 = -5.99
12 = -4.86
13 = -3.91
14 = -3.25

(A)

Fig. 29A

**2906** *Continued . . .*

> **2912** *Continued . . .*
>
> (A)
>
> > **2920** Determine a measure of central tendency (e.g., a mean) of the individual probabilities
> >
> > E.g., mean loglikelihood = -5.05

**2922** Apply stability metrics to a model (e.g., K Nearest Neighbors) to call whether the locus is stable or not

> **2924** Generate a probability that the locus is unstable
>
> E.g., probability that the locus is unstable = 0.96

> **2926** Translate the probability into a stability call, given a predetermined threshold
>
> E.g., at a threshold = 0.3, locus is unstable

Fig. 29B

Fig. 29C

Fig. 30A

Fig. 30B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 80212620 A
- US 62978067 A
- US 83018620 A
- US 78936320 A
- US 63007874 A
- US 1969149 W
- US 9138205 B
- US 62924621 A
- US 69311719 A
- US 62855750 A
- US 62855750
- US 62944292 A
- US 65780419 A
- US 62873693 B
- US 62902950 A
- US 62924073 A
- US 58170619 A
- US 732229 A
- US 1969161 W
- US 62924054 A
- US 62944995 A
- US 62943712 A
- US 62889510 A
- US 62804458 A
- US 62854400 A
- US 62824039 A
- US 62804730 A
- US 62888163 A
- US 53367619 A
- US 62804509 A
- US 65386819 A
- US 62931600 A
- US 62804724 A
- US 62855913 A
- US 62786739 A
- US 62924515 A
- US 789363
- US 20180002 W

### Non-patent literature cited in the description

- **RADOVICH M. et al.** *Oncotarget*, 2016, vol. 7 (35), 56491-500
- **TSIMBERIDOU AM et al.** *ASCO 2018*, 2018
- **WHEELER JJ et al.** *Cancer Res.*, 2016, vol. 76, 3690-701
- **SCHWAEDERLE M. et al.** *J Clin Oncol.*, 2015, vol. 33 (32), 3817-25
- **SCHWAEDERLE M. et al.** *, JAMA Oncol.*, 2016, vol. 2 (11), 1452-59
- **WHELER JJ et al.** *Cancer Res.*, 2016, vol. 76 (13), 3690-701
- **COYNE GO et al.** *Curr. Probl. Cancer*, 2017, vol. 41 (3), 182-93
- **MARKMAN M.** *Oncology*, vol. 31 (3), 158, 168
- **OLIVEIRA AF et al.** *Front Oncol.*, 2019, vol. 9, 396
- **LOZAC'HMEUR et al.** *Journal for Immunotherapy of Cancer*, 2019, vol. 7 (1)
- **ARTYOMENKO et al.** *Annales of Oncology*, 2018, vol. 29 (8)
- **FERNANDES GS et al.** *Clinics*, vol. 72 (10), 588-94
- **ROSS JS et al.** *JAMA Oncol.*, 2015, vol. 1 (1), 40-49
- **ROSS JS et al.** *, Arch. Pathol. Lab Med.*, 2015, vol. 139, 642-49
- **HIRSHFIELD KM et al.** *, Oncologist*, 2016, vol. 21 (11), 1315-25
- **GROISBERG R. et al.** *, Oncotarget*, 2017, vol. 8, 39254-67
- **ILIE; HOFMAN.** *Transl. Lung Cancer Res.*, 2016, vol. 5 (4), 420-23
- **ILIE; HOFMAN.** *Transl Lung Cancer Res.*, 2016, vol. 5 (4), 420-23
- **ATKINS et al.** *Molecular Diagnosis and Therapy*, 2019, vol. 23 (4), 521-535
- **CHAN et al.** *Ann. Clin. Biochem.*, 2003, vol. 40, 122-30
- **STROUN et al.** *Oncology*, 1989, vol. 46 (5), 318-22
- **GOESSL et al.** *Cancer Res.*, 2000, vol. 60 (21), 5941-45
- **FRENEL et al.** *, Clin. Cancer Res.*, 2015, vol. 21 (20), 4586-96
- **WADAPURKAR; VYAS.** *Informatics in Medicine Unlocked*, 2018, vol. 11, 75-82
- **BAICHOO; OUZOUNIS.** *BioSystems*, 2017, vol. 156-157, 72-85
- **LI; DURBIN.** *Bioinformatics*, 2009, vol. 25 (14), 1754-60
- **AGRESTI.** An Introduction to Categorical Data Analysis. John Wiley & Son, 1996, 103-144

- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. Fundamentals of Artificial Neural Networks. Massachusetts Institute of Technology, 1995
- **DUDA et al.** Pattern Classification. John Wiley & Sons, Inc., 2001
- **HASTIE et al.** The Elements of Statistical Learning. Springer-Verlag, 2001
- **DRAGHICI**. Data Analysis Tools for DNA Microarrays,. Chapman & Hall/CRC, 2003
- **MOUNT**. Bioinformatics: sequence and genome analysis. Cold Spring Harbor Laboratory Press, 2001
- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK**. Statistical Learning Theory,. Wiley, 1998
- **DUDA**. Pattern Classification,. John Wiley & Sons, Inc, 2001, 259, 262-265
- **HASTIE**. The Elements of Statistical Learning. Springer, 2001
- **FUREY et al.** *Bioinformatics*, 2000, vol. 16, 906-914
- **NG et al.** On discriminative vs. generative classifiers: A comparison of logistic regression and naive Bayes. *Advances in Neural Information Processing Systems*, 2002, vol. 14
- **DUDA**. Pattern Classification. John Wiley & Sons, Inc, 2001, 395-396
- **DUDA**. Pattern Classification. John Wiley & Sons, Inc., 2001, 396-408, 411-412
- Random Forests--Random Features. **BREIMAN**. Technical Report 567. Statistics Department, 1999
- **DUDA** ; **HART**. Pattern Classification and Scene Analysis. John Wiley & Sons, Inc., 1973, 211-256
- **DUDA et al.** Pattern Classification. John Wiley & Sons, Inc.
- **KAUFMAN** ; **ROUSSEEUW**. Finding Groups in Data: An Introduction to Cluster Analysis,. Wiley, 1990
- **EVERITT**. Cluster analysis. Wiley, 1993
- **BACKER**. Computer-Assisted Reasoning in Cluster Analysis. Prentice Hall, 1995
- **DUDA**. Pattern Classification. John Wiley & Sons, Inc, 2001
- **BERA, K. et al.** *Nat. Rev. Clin. Oncol.*, 2019, vol. 16, 703-15
- **CHOMCZYNSKI** ; **SACCHI**. *Nat Protoc*, 2006, vol. 1 (2), 581-85
- **POECKH, T. et al.** *Anal Biochem.*, 2008, vol. 373 (2), 253-62
- **KIVIOJA et al.** *Nat. Methods*, 2011, vol. 9 (1), 72-74
- **ISLAM et al.** *Nat. Methods*, 2014, vol. 11 (2), 163-66
- **JIANG, H. et al.** *BMC Bioinformatics*, 2014, vol. 15 (182), 1-12
- **SMITH** ; **WATERMAN**. *J Mol. Biol.*, 1981, vol. 147 (1), 195-97
- **HUANG** ; **MILLER**. *Adv. Appl. Math*, 1991, vol. 12, 337-57
- **MA B. et al.** *Bioinformatics*, 2002, vol. 18 (3), 440-45
- **HATEM et al.** Benchmarking short sequence mapping tools. *BMC Bioinformatics*, 2013, vol. 14, 184
- **FLICEK** ; **BIRNEY**. Sense from sequence reads: methods for alignment and assembly. *Nat Methods*, 2009, vol. 6 (11), S6-S12
- **LI** ; **HOMER**. A survey of sequence alignment algorithms for next-generation sequencing. *Brief Bioinformatics*, 2010, vol. 11, 473-483
- **SCHWARTZ et al.** *PLoS ONE*, 2011, vol. 6 (1), e16685
- **BENJAMINI** ; **SPEED**. *Nucleic Acids Research*, 2012, vol. 40 (10), e72
- **CAMERON, D.L. et al.** *Nat. Commun.*, 2019, vol. 10 (3240), 1-11
- **E. GARRISON**. *Vcflib: A C++ library for parsing and manipulating VCF files, GitHub, available on the Internet*, 2012
- **CHIANG et al.** SpeedSeq: ultra-fast personal genome analysis and interpretation. *Nat Methods*, 2015, 966
- **LAYER et al.** LUMPY: a probabilistic framework for structural variant discovery. *Genome Biol*, 2014, 84
- **ZHOU Q et al.** *BMC Bioinformatics*, 2019, vol. 20 (47), 1-11
- **KRUEGER F.** ; **ANDREWS SR**. *Bioinformatics*, 2011, vol. 27 (11), 1571-71
- **TALEVICH et al.** *PLoS Comput Biol*, 2016, vol. 12, 1004873
- **FENIZIA F. et al.** *Transl Lung Cancer Res.*, 2018, vol. 7 (6), 668-77
- **GEORGIADIS A. et al.** *, Clin. Cancer Res.*, 2019, vol. 25 (23), 7024-34
- **JUN G. et al.** *Am. J. Hum. Genet.*, 2012, vol. 91, 839-48
- **SEJOON L. et al.** *Nucleic Acids Research*, 20 June 2017, vol. 45 (11), e103
- Merck Manual. National Cancer Institute and Merck & Co.

- **VIKTOR A. ADALSTEINSSON et al.** *Nature Communications*, 2017, vol. 8, 1324